(19) Europäisches Patentamt
European Patent Office
Office européen des brevets

(11) EP 4 400 596 A1

(12) EUROPEAN PATENT APPLICATION
published in accordance with Art. 153(4) EPC

(43) Date of publication:
**17.07.2024 Bulletin 2024/29**

(21) Application number: **22864372.2**

(22) Date of filing: **24.08.2022**

(51) International Patent Classification (IPC):
*C12P 19/12* (2006.01) *A61K 31/7008* (2006.01)
*A61K 31/7048* (2006.01) *A61K 31/7056* (2006.01)
*A61P 3/10* (2006.01) *A61P 43/00* (2006.01)
*C07H 5/06* (2006.01) *C07H 15/12* (2006.01)
*C07H 15/26* (2006.01) *C12N 1/20* (2006.01)
*C12P 19/44* (2006.01)

(52) Cooperative Patent Classification (CPC):
**A61K 31/7008; A61K 31/7048; A61K 31/7056; A61P 3/10; A61P 43/00; C07H 5/06; C07H 15/12; C07H 15/26; C12N 1/20; C12P 19/12; C12P 19/44**

(86) International application number:
**PCT/JP2022/031876**

(87) International publication number:
**WO 2023/032788 (09.03.2023 Gazette 2023/10)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **06.09.2021 JP 2021144962**

(71) Applicant: **Microbial Chemistry Research Foundation**
**Tokyo 141-0021 (JP)**

(72) Inventors:
- **WADA, Shun-ichi**
  **Tokyo 141-0021 (JP)**
- **ARIMURA, Honami**
  **Tokyo 141-0021 (JP)**
- **IGARASHI, Masayuki**
  **Tokyo 141-0021 (JP)**
- **NAGAYOSHI, Miho**
  **Tokyo 141-0021 (JP)**
- **OHBA, Shun-ichi**
  **Tokyo 141-0021 (JP)**
- **SAWA, Ryuichi**
  **Tokyo 141-0021 (JP)**
- **KUBOTA, Yumiko**
  **Tokyo 141-0021 (JP)**

(74) Representative: **Maiwald GmbH**
**Elisenhof**
**Elisenstraße 3**
**80335 München (DE)**

Remarks:
The complete document including Reference Table(s) and the Sequence Listing(s) can be downloaded from the EPO website

# (54) NOVEL COMPOUND, USE THEREOF, METHOD FOR PRODUCING SAME, COMPOUND-CONTAINING COMPOSITION, METHOD FOR PRODUCING 4-TREHALOSAMINE, AND MICROORGANISM

(57) Provided are a protective agent, a surfactant composition, a composition for blood glucose level control, an autophagy inducer, a mycobacterium stain, or a protein extractant characterized by containing compounds represented by general formula (1).

FIG. 15C

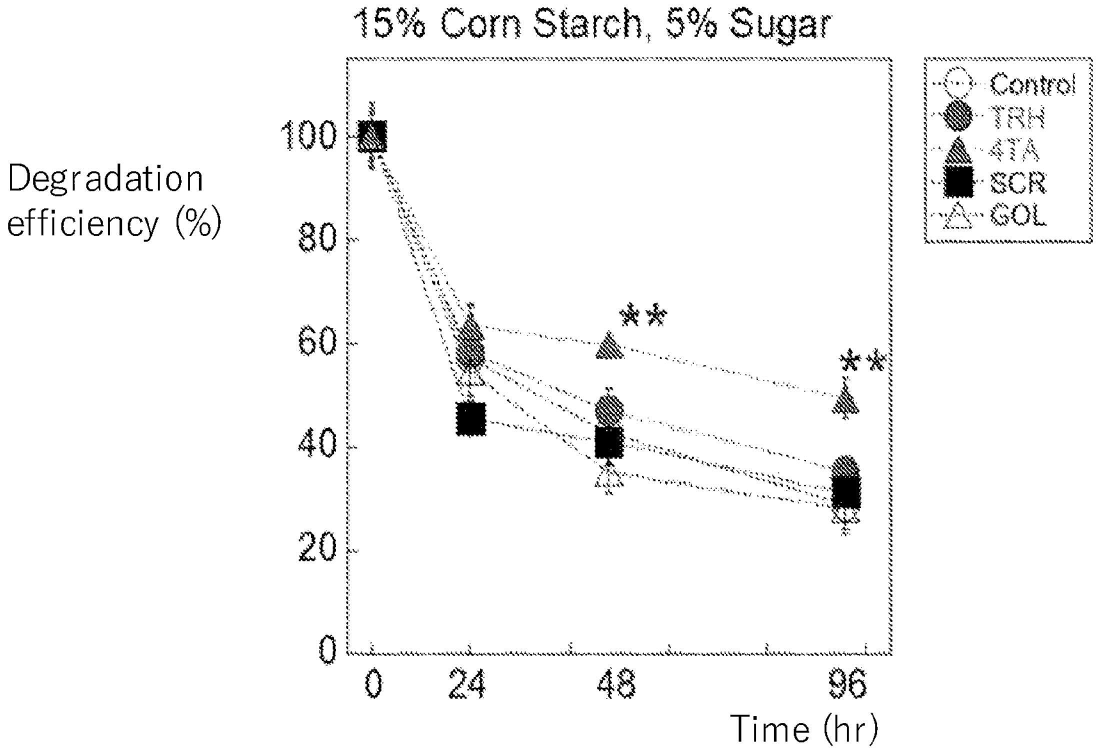
15% Corn Starch, 5% Sugar
Degradation efficiency (%)
100
80
60
40
20
0
0
24
48
96
Time (hr)
Control
TRH
4TA
SCR
GOL
**
**

## Description

### Technical Field

The present invention relates to novel compounds, or pharmaceutically acceptable salts thereof, or solvates thereof, a use thereof, and a method for producing the compounds, and a compound-containing composition, a method for producing 4-trehalosamine, and a microorganism.

### Background Art

Trehalose is inexpensive and exhibits a moisture retaining effect and a protective effect, thereby being used in a variety of fields including food. Further, its therapeutic effects on various diseases have been reported, whereby development as pharmaceuticals has been considered.

However, trehalose is quickly degraded into glucose by trehalase in bodies, and high-dose administration is therefore required to achieve the above-mentioned advantageous effects. This high-dose administration of trehalose has had a problem in that the glucose causes elevation of blood glucose levels. Further, there have also been a problem in that symbiotic bacterial flora is affected in living bodies and a problem in that it causes product spoilage because it is assimilated by microorganisms.

Therefore, it has been strongly required to provide a substitute for trehalose that exhibits similar advantageous effects to those of trehalose and is not degraded in living bodies. However, there have been a problem in that analogs of trehalose are not available on the market and a problem in that it is difficult to synthesize derivatives modified at specific sites because trehalose contains a plurality of hydroxyl groups having the same chemical properties in one molecule.

On the other hand, 4-trehalosamine, which is a known analog of trehalose, is known to have antibacterial activity, but its other uses are unknown (see Non-Patent Literature 1).

### Citation List

### Non-Patent Literature

Non-Patent Literature 1: THE JOURNAL OF ANTIBIOTICS, Vol. XXVII No. 2, 1974, p. 145

### Summary of Invention

### Technical Problem

The present invention is intended to solve the various conventional problems mentioned above and to achieve the following objects.

That is, an object of the present invention is to provide: protective agents which exhibit various excellent effects such as a starch aging inhibiting effect, an effect for preventing or inhibiting decrease in activity of proteins during storage in frozen or freeze-dried state, an effect for preventing or inhibiting damage or death of microorganisms during storage in frozen or freeze-dried state, and a pH buffer effect; a surfactant which exhibits an excellent surface activity effect; a composition for blood glucose level control which exhibits an effect for preventing elevation of blood glucose levels even when administered to individuals; an autophagy inducer which exhibits an excellent autophagy inducing effect; a mycobacterium stain which can easily and efficiently stain mycobacteria; and a protein extractant which exhibits an excellent protein extraction effect.

Further, another object of the present invention is to provide novel compounds, or pharmaceutically acceptable salts thereof, or solvates thereof and a compound-containing composition containing these compounds, which exhibit various excellent effects such as at least any one effect of: a starch aging inhibiting effect, an effect for preventing or inhibiting decrease in activity of proteins during storage in frozen or freeze-dried state, an effect for preventing or inhibiting damage or death of microorganisms during storage in frozen or freeze-dried state, a pH buffer effect, an excellent surface activity effect, an effect for preventing elevation of blood glucose levels even when administered to individuals, an autophagy inducing effect, a mycobacterium staining effect with which mycobacteria can be easily and efficiently stained, and a protein extraction effect.

Furthermore, still another object of the present invention is to provide a method for producing compound by which the above compounds can be inexpensively, easily, and efficiently produced.

Furthermore, yet another object of the present invention is to provide a method for producing 4-trehalosamine by which 4-trehalosamine can be inexpensively, easily, and efficiently produced.

Furthermore, yet another object of the present invention is to provide a microorganism which is capable of producing 4-trehalosamine.

**Solution to Problem**

Means for solving the above-mentioned problems are as the following. Namely,

<1> A protective agent, a surfactant composition, a composition for blood glucose level control, an autophagy inducer, a mycobacterium stain, or a protein extractant which contains compounds, or pharmaceutically acceptable salts thereof, or solvates thereof represented by general formula (1) below:

[Chemical formula 1]

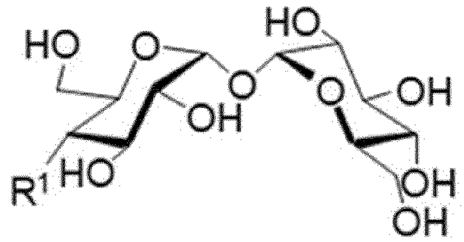

General formula (1)

where, in the general formula (1) above, $R^1$ is $-NH_2$, a substituent represented by structural formula (A) below, a substituent represented by structural formula (B) below, or $-NH(CH_2)_mCH_3$, and the m denotes an integer from 7 to 14,

[Chemical formula 2]

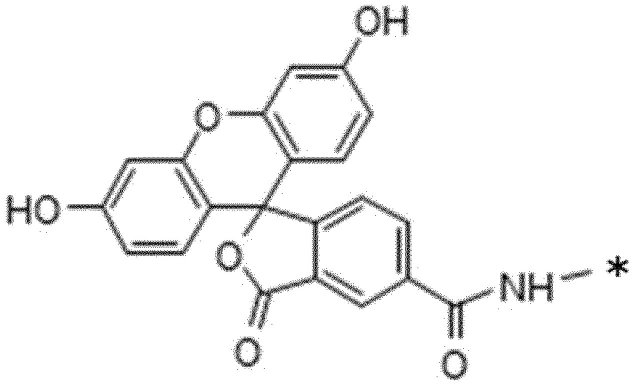

Structural formula (A)

[Chemical formula 3]

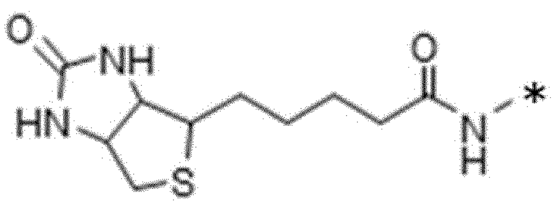

Structural formula (B)

where, in the structural formula (A) above or the structural formula (B) below, "*" denotes a dangling bond.

<2> Compounds, or pharmaceutically acceptable salts thereof, or solvates thereof represented by general formula (2) below:

[Chemical formula 4]

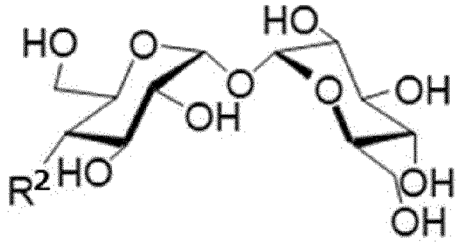

General formula (2)

where, in the general formula (2) above, $R^2$ is a substituent represented by structural formula (A) below, a substituent represented by structural formula (B) below, or $-NH(CH_2)_mCH_3$, and the m denotes an integer from 7 to 14,

[Chemical formula 5]

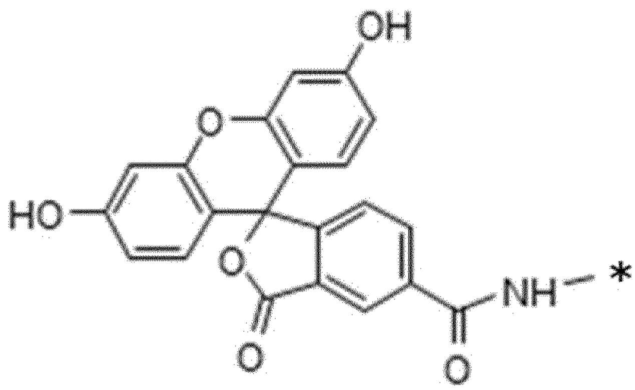

Structural formula (A)

[Chemical formula 6]

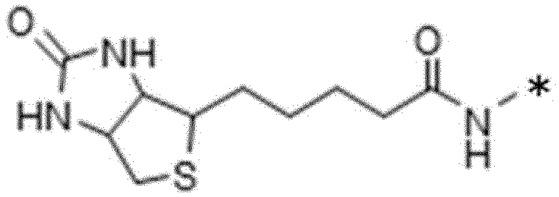

Structural formula (B)

where, in the structural formula (A) above or the structural formula (B) below, "*" denotes a dangling bond.

<3> A compound-containing composition containing the compounds, or pharmaceutically acceptable salts thereof, or solvates thereof described the above <2>.

<4> A method for producing 4-trehalosamine including a culture process for culturing Streptomyces sp. MK186-mF5 (accession number: NITE BP-03495).

<5> The method for producing 4-trehalosamine described in the above <4> in which a culture medium contains at least any one of 3% by mass to 9% by mass of soluble starch, 0.001% by mass to 0.02% by mass of zinc chloride, and 0.1% by mass to 1% by mass of potassium chloride in the culture process.

<6> A method for producing compound represented by general formula (3) below, which includes a process for reacting an amino group at position 4 of 4-trehalosamine with $CH_3(CH_2)_pCOH$ (where the p denotes an integer from 6 to 13), 2-azido-1,3-dimethylimidazolinium hexafluorophosphate, 5-carboxyfluorescein N-succinimidyl ester, or D-biotin N-succinimidyl, by using 4-trehalosamine as a starting material:

[Chemical formula 7]

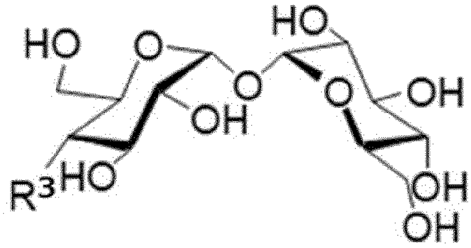

General formula (3)

where, in the general formula (3) above, $R^3$ is $-N(CH_2)_mCH_3$, $-N_3$, a substituent represented by structural formula (A) below, or a substituent represented by structural formula (B) below, and the m denotes an integer from 7 to 14,

[Chemical formula 8]

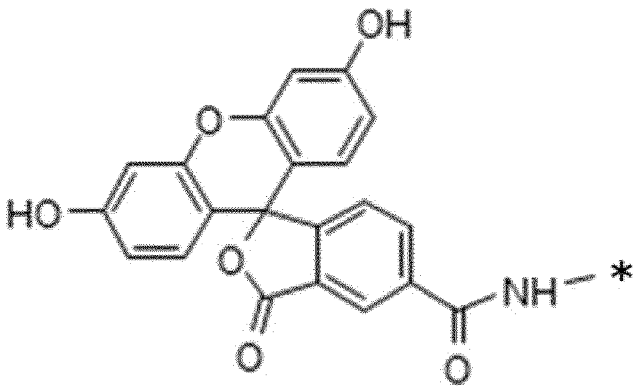

Structural formula (A)

[Chemical formula 9]

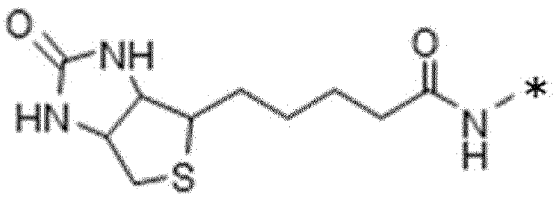

Structural formula (B)

where, in the structural formula (A) above or the structural formula (B) below, "*" denotes a dangling bond.
<7> A microorganism which is Streptomyces sp. MK186-mF5 (accession number: NITE BP-03495), and is capable of producing 4-trehalosamine.

## Advantageous Effects of Invention

According to the present invention, it is possible to solve the various conventional problems mentioned above, achieve the above-mentioned objects, and provide: protective agents which exhibit various excellent effects such as a starch aging inhibiting effect, an effect for preventing or inhibiting decrease in activity of proteins during storage in frozen or freeze-dried state, an effect for preventing or inhibiting damage or death of microorganisms during storage in frozen or freeze-dried state, and a pH buffer effect; a surfactant which exhibits an excellent surface activity effect; a composition for blood glucose level control which exhibits an effect for preventing elevation of blood glucose levels even when administered to individuals; an autophagy inducer which exhibits an excellent autophagy inducing effect; a mycobacterium stain which can easily and efficiently stain mycobacteria; and a protein extractant which exhibits an excellent protein extraction effect.

Further, according to the present invention, it is possible to solve the various conventional problems mentioned above, achieve the above-mentioned objects, and provide novel compounds, or pharmaceutically acceptable salts thereof, or solvates thereof and a compound-containing composition containing these compounds, which exhibit various excellent effects such as at least any one effect of: a starch aging inhibiting effect, an effect for preventing or inhibiting decrease in activity of proteins during storage in frozen or freeze-dried state, an effect for preventing or inhibiting damage or death of microorganisms during storage in frozen or freeze-dried state, a pH buffer effect, an excellent surface activity effect, an effect for preventing elevation of blood glucose levels even when administered to individuals, an autophagy inducing effect, a mycobacterium staining effect with which mycobacteria can be easily and efficiently stained, and a protein extraction effect.

Furthermore, according to the present invention, it is possible to solve the various conventional problems mentioned above, achieve the above-mentioned objects, and provide a method for producing compound by which the above compounds can be inexpensively, easily, and efficiently produced.

Furthermore, according to the present invention, it is possible to solve the various conventional problems mentioned above, achieve the above-mentioned objects, and provide a method for producing 4-trehalosamine by which 4-trehalosamine can be inexpensively, easily, and efficiently produced.

Furthermore, according to the present invention, it is possible to solve the various conventional problems mentioned above, achieve the above-mentioned objects, and provide a microorganism which is capable of producing 4-trehalosamine.

## Brief Description of Drawings

[Fig. 1A] Fig. 1A is a chart of an absorption spectrum measured by infrared spectroscopy for the compound (4-trehalosamine) represented by structural formula (1), where the vertical axis: transmittance (%T), and the horizontal axis: wave number ($cm^{-1}$).

[Fig. 1B] Fig. 1B is a chart of a proton nuclear magnetic resonance spectrum measured at 600 MHz in heavy water at 25°C for the compound (4-trehalosamine) represented by structural formula (1), where the horizontal axis: in ppm.

[Fig. 1C] Fig. 1C is a chart of a carbon 13 nuclear magnetic resonance spectrum measured at 150 MHz in heavy water at 25°C for the compound (4-trehalosamine) represented by structural formula (1), where the horizontal axis: in ppm.

[Fig. 2A] Fig. 2A is a chart of an absorption spectrum measured by infrared spectroscopy for the compound (IMCTA-C8) represented by structural formula (2), where the vertical axis: transmittance (%T), and the horizontal axis: wave number ($cm^{-1}$).

[Fig. 2B] Fig. 2B is a chart of a proton nuclear magnetic resonance spectrum measured at 600 MHz in deuterated methanol at 25°C for the compound (IMCTA-C8) represented by structural formula (2), where the horizontal axis: in ppm.

[Fig. 2C] Fig. 2C is a chart of a carbon 13 nuclear magnetic resonance spectrum measured at 150 MHz in deuterated methanol at 25°C for the compound (IMCTA-C8) represented by structural formula (2), where the horizontal axis: in ppm.

[Fig. 3A] Fig. 3A is a chart of an absorption spectrum measured by infrared spectroscopy for the compound (IMCTA-C9) represented by structural formula (3), where the vertical axis: transmittance (%T), and the horizontal axis: wave number ($cm^{-1}$).

[Fig. 3B] Fig. 3B is a chart of a proton nuclear magnetic resonance spectrum measured at 600 MHz in deuterated methanol at 25°C for the compound (IMCTA-C9) represented by structural formula (3), where the horizontal axis: in ppm.

[Fig. 3C] Fig. 3C is a chart of a carbon 13 nuclear magnetic resonance spectrum measured at 150 MHz in deuterated

methanol at 25°C for the compound (IMCTA-C9) represented by structural formula (3), where the horizontal axis: in ppm.

[Fig. 4A] Fig. 4A is a chart of an absorption spectrum measured by infrared spectroscopy for the compound (IMCTA-C10) represented by structural formula (4), where the vertical axis: transmittance (%T), and the horizontal axis: wave number ($cm^{-1}$).

[Fig. 4B] Fig. 4B is a chart of a proton nuclear magnetic resonance spectrum measured at 600 MHz in deuterated methanol at 25°C for the compound (IMCTA-C10) represented by structural formula (4), where the horizontal axis: in ppm.

[Fig. 4C] Fig. 4C is a chart of a carbon 13 nuclear magnetic resonance spectrum measured at 150 MHz in deuterated methanol at 25°C for the compound (IMCTA-C10) represented by structural formula (4), where the horizontal axis: in ppm.

[Fig. 5A] Fig. 5A is a chart of an absorption spectrum measured by infrared spectroscopy for the compound (IMCTA-C11) represented by structural formula (5), where the vertical axis: transmittance (%T), and the horizontal axis: wave number ($cm^{-1}$).

[Fig. 5B] Fig. 5B is a chart of a proton nuclear magnetic resonance spectrum measured at 600 MHz in deuterated methanol at 25°C for the compound (IMCTA-C11) represented by structural formula (5), where the horizontal axis: in ppm.

[Fig. 5C] Fig. 5C is a chart of a carbon 13 nuclear magnetic resonance spectrum measured at 150 MHz in deuterated methanol at 25°C for the compound (IMCTA-C11) represented by structural formula (5), where the horizontal axis: in ppm.

[Fig. 6A] Fig. 6A is a chart of an absorption spectrum measured by infrared spectroscopy for the compound (IMCTA-C12) represented by structural formula (6), where the vertical axis: transmittance (%T), and the horizontal axis: wave number ($cm^{-1}$).

[Fig. 6B] Fig. 6B is a chart of a proton nuclear magnetic resonance spectrum measured at 600 MHz in deuterated methanol at 25°C for the compound (IMCTA-C12) represented by structural formula (6), where the horizontal axis: in ppm.

[Fig. 6C] Fig. 6C is a chart of a carbon 13 nuclear magnetic resonance spectrum measured at 150 MHz in deuterated methanol at 25°C for the compound (IMCTA-C12) represented by structural formula (6), where the horizontal axis: in ppm.

[Fig. 7A] Fig. 7A is a chart of an absorption spectrum measured by infrared spectroscopy for the compound (IMCTA-C13) represented by structural formula (7), where the vertical axis: transmittance (%T), and the horizontal axis: wave number ($cm^{-1}$).

[Fig. 7B] Fig. 7B is a chart of a proton nuclear magnetic resonance spectrum measured at 600 MHz in deuterated methanol at 25°C for the compound (IMCTA-C13) represented by structural formula (7), where the horizontal axis: in ppm.

[Fig. 7C] Fig. 7C is a chart of a carbon 13 nuclear magnetic resonance spectrum measured at 150 MHz in deuterated methanol at 25°C for the compound (IMCTA-C13) represented by structural formula (7), where the horizontal axis: in ppm.

[Fig. 8A] Fig. 8A is a chart of an absorption spectrum measured by infrared spectroscopy for the compound (IMCTA-C14) represented by structural formula (8), where the vertical axis: transmittance (%T), and the horizontal axis: wave number ($cm^{-1}$).

[Fig. 8B] Fig. 8B is a chart of a proton nuclear magnetic resonance spectrum measured at 600 MHz in deuterated methanol at 25°C for the compound (IMCTA-C14) represented by structural formula (8), where the horizontal axis: in ppm.

[Fig. 8C] Fig. 8C is a chart of a carbon 13 nuclear magnetic resonance spectrum measured at 150 MHz in deuterated methanol at 25°C for the compound (IMCTA-C14) represented by structural formula (8), where the horizontal axis: in ppm.

[Fig. 9A] Fig. 9A is a chart of an absorption spectrum measured by infrared spectroscopy for the compound (IMCTA-C15) represented by structural formula (9), where the vertical axis: transmittance (%T), and the horizontal axis: wave number ($cm^{-1}$).

[Fig. 9B] Fig. 9B is a chart of a proton nuclear magnetic resonance spectrum measured at 600 MHz in deuterated DMSO at 25°C for the compound (IMCTA-C15) represented by structural formula (9), where the horizontal axis: in ppm.

[Fig. 9C] Fig. 9C is a chart of a carbon 13 nuclear magnetic resonance spectrum measured at 150 MHz in deuterated DMSO at 25°C for the compound (IMCTA-C15) represented by structural formula (9), where the horizontal axis: in ppm.

[Fig. 10A] Fig. 10A is a chart of an absorption spectrum measured by infrared spectroscopy for the compound (IMCTA-fluorescein) represented by structural formula (10), where the vertical axis: transmittance (%T), and the

horizontal axis: wave number ($cm^{-1}$).

[Fig. 10B] Fig. 10B is a chart of a proton nuclear magnetic resonance spectrum measured at 600 MHz in deuterated methanol at 25°C for the compound (IMCTA-fluorescein) represented by structural formula (10), where the horizontal axis: in ppm.

[Fig. 10C] Fig. 10C is a chart of a carbon 13 nuclear magnetic resonance spectrum measured at 150 MHz in deuterated methanol at 25°C for the compound (IMCTA-fluorescein) represented by structural formula (10), where the horizontal axis: in ppm.

[Fig. 11A] Fig. 11A is a chart of an absorption spectrum measured by infrared spectroscopy for the compound (IMCTA-biotin) represented by structural formula (11), where the vertical axis: transmittance (%T), and the horizontal axis: wave number ($cm^{-1}$).

[Fig. 11B] Fig. 11B is a chart of a proton nuclear magnetic resonance spectrum measured at 600 MHz in deuterated methanol at 25°C for the compound (IMCTA-biotin) represented by structural formula (11), where the horizontal axis: in ppm.

[Fig. 11C] Fig. 11C is a chart of a carbon 13 nuclear magnetic resonance spectrum measured at 150 MHz in deuterated methanol at 25°C for the compound (IMCTA-biotin) represented by structural formula (11), where the horizontal axis: in ppm.

[Fig. 12A] Fig. 12A is a chart of an absorption spectrum measured by infrared spectroscopy for the compound (IMCTA-azide) represented by structural formula (12), where the vertical axis: transmittance (%T), and the horizontal axis: wave number ($cm^{-1}$).

[Fig. 12B] Fig. 12B is a chart of a proton nuclear magnetic resonance spectrum measured at 600 MHz in deuterated methanol at 25°C for the compound (IMCTA-azide) represented by structural formula (12), where the horizontal axis: in ppm.

[Fig. 12C] Fig. 12C is a chart of a carbon 13 nuclear magnetic resonance spectrum measured at 150 MHz in deuterated methanol at 25°C for the compound (IMCTA-azide) represented by structural formula (12), where the horizontal axis: in ppm.

[Fig. 13A] Fig. 13A shows the results (phosphomolybdic acid staining patterns) of detecting 4-trehalosamine in culture fluid by TLC analysis in test example 1, where the horizontal axis: production example number.

[Fig. 13B] Fig. 13B shows the results of measuring the content of 4-trehalosamine in culture fluid by LC-MS analysis in test example 1, where the horizontal axis: production example number, and the vertical axis: 4-trehalosamine production (mg/mL).

[Fig. 14A] Fig. 14A shows the results of protein extraction efficiency with IMCTA-Cn in test example 4, where the vertical axis: extracted protein (mg/mL).

[Fig. 14B] Fig. 14B shows the result of SDS-PAGE after extraction of protein with IMCTA-Cn at a final concentration of 0.05% in test example 4, where the vertical axis: molecular weight (kDa).

[Fig. 14C] Fig. 14C shows the result of SDS-PAGE after extraction of protein with IMCTA-Cn at a final concentration of 0.5% in test example 4, where the vertical axis: molecular weight (kDa).

[Fig. 14D] Fig. 14D shows the result of western blotting of membrane protein extracted with IMCTA-Cn in test example 4, where "0.05%" or "0.5%" in the figure indicates the final concentration of a test sample.

[Fig. 14E] Fig. 14E shows the result of CIP activity of protein extracted with IMCTA-Cn in test example 4 (n = 3, mean ± S.D.), where the vertical axis: CIP activity (A420).

[Fig. 15A] Fig. 15A shows the evaluation results of the aging level of starch (corn starch) when the final concentration of test samples is 10% in enzyme assay 1 of test example 5 (n = 5, mean ± S.D.), where white circles indicate the results for control, gray circles for trehalose (TRH), gray triangles for 4-trehalosamine (4TA), black squares for sucrose (SCR), and white triangles for glycerol (GOL), and where the horizontal axis: time, and the vertical axis: degradation efficiency (%).

[Fig. 15B] Fig. 15B shows the evaluation results of the aging level of starch (corn starch) when the final concentration of test samples is 2% in enzyme assay 2 of test example 5 (n = 5, mean ± S.D.), where white circles indicate the results for control, gray circles for trehalose (TRH), gray triangles for 4-trehalosamine (4TA), black squares for sucrose (SCR), and white triangles for glycerol (GOL), and where the horizontal axis: time, and the vertical axis: degradation efficiency (%).

[Fig. 15C] Fig. 15C shows the evaluation results of the aging level of starch (corn starch) when the final concentration of test samples is 5% in enzyme assay 2 of test example 5 (n = 5, mean ± S.D.), where white circles indicate the results for control, gray circles for trehalose (TRH), gray triangles for 4-trehalosamine (4TA), black squares for sucrose (SCR), and white triangles for glycerol (GOL), and where the horizontal axis: time, and the vertical axis: degradation efficiency (%).

[Fig. 15D] Fig. 15D shows the evaluation results of the aging level of starch (corn starch) when the final concentration of test samples is 15% in enzyme assay 2 of test example 5 (n = 5, mean ± S.D.), where white circles indicate the results for control, gray circles for trehalose (TRH), gray triangles for 4-trehalosamine (4TA), black squares for

sucrose (SCR), and white triangles for glycerol (GOL), and where the horizontal axis: time, and the vertical axis: degradation efficiency (%).

[Fig. 15E] Fig. 15E shows the evaluation results of the aging level of starch (potato starch) when the final concentration of test samples is 10% in enzyme assay 3 of test example 5 (n = 5, mean ± S.D.), where white circles indicate the results for control, black circles for trehalose (TRH), gray triangles for 4-trehalosamine (4TA), black squares for sucrose (SCR), and white triangles for glycerol (GOL), and where the horizontal axis: time, and the vertical axis: degradation efficiency (%).

[Fig. 15F] Fig. 15F shows the evaluation results of the aging level of starch (potato starch) when the final concentration of test samples is 15% in enzyme assay 3 of test example 5 (n = 5, mean ± S.D.), where white circles indicate the results for control, black circles for trehalose (TRH), gray triangles for 4-trehalosamine (4TA), black squares for sucrose (SCR), and white triangles for glycerol (GOL), and where the horizontal axis: time, and the vertical axis: degradation efficiency (%).

[Fig. 15G] Fig. 15G shows the evaluation results of the aging level of starch (rice flour) when the final concentration of test samples is 10% in enzyme assay 4 of test example 5 (n = 5, mean ± S.D.), where white circles indicate the results for control, black circles for trehalose (TRH), gray triangles for 4-trehalosamine (4TA), black squares for sucrose (SCR), and white triangles for glycerol (GOL), and where the horizontal axis: time, and the vertical axis: degradation efficiency (%).

[Fig. 15H] Fig. 15H shows the evaluation results of the aging level of starch (rice flour) when the final concentration of test samples is 15% in enzyme assay 4 of test example 5 (n = 5, mean ± S.D.), where white circles indicate the results for control, black circles for trehalose (TRH), gray triangles for 4-trehalosamine (4TA), black squares for sucrose (SCR), and white triangles for glycerol (GOL), and where the horizontal axis: time, and the vertical axis: degradation efficiency (%).

[Fig. 15I] Fig. 15I shows the evaluation results of the aging level of starch (wheat flour) when the final concentration of test samples is 10% in enzyme assay 5 of test example 5 (n = 5, mean ± S.D.), where white circles indicate the results for control, black circles for trehalose (TRH), gray triangles for 4-trehalosamine (4TA), black squares for sucrose (SCR), and white triangles for glycerol (GOL), and where the horizontal axis: time, and the vertical axis: degradation efficiency (%).

[Fig. 15J] Fig. 15J shows the evaluation results of the aging level of starch (wheat flour) when the final concentration of test samples is 15% in enzyme assay 5 of test example 5 (n = 5, mean ± S.D.), where white circles indicate the results for control, black circles for trehalose (TRH), gray triangles for 4-trehalosamine (4TA), black squares for sucrose (SCR), and white triangles for glycerol (GOL), and where the horizontal axis: time, and the vertical axis: degradation efficiency (%).

[Fig. 15K] Fig. 15K shows the results of dynamic viscoelasticity measurement in test example 5 (n = 5, mean ± S.D.), where white circles indicate the results for control, gray circles for trehalose (TRH), gray triangles for 4-trehalosamine (4TA), black squares for sucrose (SCR), and white triangles for glycerol (GOL), and where the horizontal axis: time, and the vertical axis: Tanδ.

[Fig. 16A] Fig. 16A shows evaluation results of CIP-protective activity in test example 6-1 (n = 3, mean ± S.D.), where the horizontal axis: test sample number, and the vertical axis: CIP activity ($A_{420}$).

[Fig. 16B] Fig. 16B shows evaluation results of ADH-protective activity in test example 6-2 (n = 3, mean ± S.D.), where the horizontal axis: test sample number, and the vertical axis: ADH activity ($A_{340}$ test sample /$A_{340}$ control).

[Fig. 17A] Fig. 17A shows evaluation results of baker's yeast protective activity in test example 7-1.

[Fig. 17B] Fig. 17B shows evaluation results of Escherichia coli protective activity in test example 7-2.

[Fig. 17C] Fig. 17C shows evaluation results of Bacillus subtilis protective activity in test example 7-3.

[Fig. 17D] Fig. 17D shows evaluation results of mycobacterium-protective activity in test example 7-4.

[Fig. 18] Fig. 18 shows evaluation results of pH buffer effect in test example 8, where gray circles indicate the results for 4-trehalosamine (4TA), black circles for trehalose (TRH), white triangle (Δ) for tris, white squares for MES, and white inverted triangles (V) for HEPES, and where the horizontal axis: the amount of addition of sodium hydride or hydrogen chloride (μL), and the vertical axis: pH.

[Fig. 19A] Fig. 19A shows evaluation results of degradation by trehalase in test example 9-1, where black circles indicate the results for trehalose (TRH) and black squares for 4-trehalosamine (4TA), and where the horizontal axis: substrate concentration (mM), and the vertical axis: free glucose concentration (mM).

[Fig. 19B] Fig. 19B is a zoomed-in view of the graph of Fig. 19A for 4-trehalosamine (4TA), where black squares indicate the results for 4-trehalosamine (4TA), and where the horizontal axis: substrate concentration (mM), and the vertical axis: free glucose concentration (mM).

[Fig. 19C] Fig. 19C shows the results of western blotting to determine trehalase expression in cultured cells in test example 9-2.

[Fig. 19D] Fig. 19D shows the results of determining the rate of degradation by trehalase in test example 9-2, where white circles indicate the results for reaction solution (TRH C) of trehalose and cell extract liquid of human pancreatic

cancer cells KP4, black circles for reaction solution (TRH T) of trehalose and cell extract liquid of 10 strains of KP4-TREH, white triangles for reaction solution (4TA C) of 4-trehalosamine and cell extract liquid of human pancreatic cancer cells KP4, and black triangles for reaction solution (4TA T) of 4-trehalosamine and 10 strains of KP4-TREH, and where the horizontal axis: time, and the vertical axis: persistence rate (%).

[Fig. 19E] Fig. 19E shows cumulative excretion of 4-trehalosamine in urine over time in test example 9-3, where white circles indicate the results for trehalose (TRH) and black circles for 4-trehalosamine (4TA), and where the horizontal axis: time, and the vertical axis: cumulative urinary excretion (mg/mouse).

[Fig. 19F] Fig. 19F shows cumulative excretion of 4-trehalosamine in feces over time in test example 9-3 where white circles indicate the results for trehalose (TRH) and black circles for 4-trehalosamine (4TA), and where the horizontal axis: time, and the vertical axis: cumulative fecal excretion (mg/mouse).

[Fig. 19G] Fig. 19G shows blood level of 4-trehalosamine in individuals to which 4-trehalosamine was administered in test example 9-3, where the horizontal axis: time, and the vertical axis: 4-trehalosamine (μg/mL) (n = 5).

[Fig. 19H] Fig. 19H shows blood level of trehalose in individuals to which trehalose was administered in test example 9-3, where the horizontal axis: time, and the vertical axis: trehalose (μg/mL) (n = 5).

[Fig. 19I] Fig. 19I shows the blood glucose level of mice (n = 5) to which 4-trehalosamine was administered in test example 9-3, where the horizontal axis: time, and the vertical axis: blood glucose (mg/dL).

[Fig. 19J] Fig. 19J shows the blood glucose level of mice (n = 5) to which trehalose was administered in test example 9-3, where the horizontal axis: time, and the vertical axis: blood glucose (mg/dL).

[Fig. 20A] Fig. 20A shows the results of determining expression and phosphorylation pattern of autophagy-related protein when human ovarian cancer cells OVK18 were treated with 4-trehalosamine in test example 10-1 by western blotting.

[Fig. 20B] Fig. 20B shows the results of determining expression and phosphorylation pattern of autophagy-related protein when human ovarian cancer cells OVK18 were treated with trehalose analog (IMCTA-Cn) in test example 10-1 by western blotting.

[Fig. 20C] Fig. 20C shows the results of determining expression and phosphorylation pattern of autophagy-related protein when human ovarian cancer cells OVK18 were treated with trehalose analog (IMCTA-Cn) in test example 10-1 by western blotting.

[Fig. 20D] Fig. 20D shows the results of determining expression and phosphorylation pattern of autophagy-related protein when human malignant melanoma cells Mewo were treated with 4-trehalosamine in test example 10-1 by western blotting.

[Fig. 20E] Fig. 20E shows the results of determining expression and phosphorylation pattern of autophagy-related protein when human malignant melanoma cells Mewo were treated with trehalose analog (IMCTA-Cn) in test example 10-1 by western blotting.

[Fig. 20F] Fig. 20F shows the results of determining expression and phosphorylation pattern of autophagy-related protein when human malignant melanoma cells Mewo were treated with trehalose analog (IMCTA-Cn) in test example 10-1 by western blotting.

[Fig. 21A] Fig. 21A shows the results of determining expression of autophagy-related proteins LC3-I and LC3-II when human ovarian cancer cells OVK18 were treated with 4-trehalosamine or trehalose analog (IMCTA-C14) in the presence of autophagy inhibitor (BM or CQ) in test example 10-2 by western blotting, and the results of calculating the value of the amount of expression (expression amount ratio) of LC3-II for groups with different compounds added when the amount of expression of LC3-II in control is 1 through image analysis of said results of determination (n = 10, mean ± S.D.).

[Fig. 21B] Fig. 21B shows the results of determining expression of autophagy-related proteins LC3-I and LC3-II when human malignant melanoma cells Mewo were treated with 4-trehalosamine or trehalose analog (IMCTA-C14) in the presence of autophagy inhibitor (BM or CQ) in test example 10-2 by western blotting, and the results of calculating the value of the amount of expression (expression amount ratio) of LC3-II for groups with different compounds added when the amount of expression of LC3-II in control is 1 through image analysis of said results of determination (n = 10, mean ± S.D.).

[Fig. 22A] Fig. 22A shows the results of determining expression of aggregated protein Q74 when neuroblastoma cells SH-SY5Y were treated with 4-trehalosamine or trehalose analog (IMCTA-C14) in test example 10-3 by western blotting, and the results of calculating the value of the amount of expression (expression amount ratio) of Q74 for groups with different compounds added when the amount of expression of Q74 in control is 1 through image analysis of said results of determination (n = 5, mean ± S.D.).

[Fig. 22B] Fig. 22B shows the results of determining expression of aggregated protein SynA53T when neuroblastoma cells SH-SY5Y were treated with 4-trehalosamine or trehalose analog (IMCTA-C14) in test example 10-3 by western blotting, and the results of calculating the value of the amount of expression (expression amount ratio) of SynA53T for groups with different compounds added when the amount of expression of SynA53T in control is 1 through image analysis of said results of determination (n = 5, mean ± S.D.).

[Fig. 22C] Fig. 22C shows the results of determining expression of aggregated protein Q74 when human neuroblastoma cells NH-12 were treated with 4-trehalosamine or trehalose analog (IMCTA-C14) in test example 10-3 by western blotting, and the results of calculating the value of the amount of expression (expression amount ratio) of Q74 for groups with different compounds added when the amount of expression of Q74 in control is 1 through image analysis of said results of determination (n = 5, mean ± S.D.).

[Fig. 22D] Fig. 22D shows the results of determining expression of aggregated protein SynA53T when human neuroblastoma cells NH-12 were treated with 4-trehalosamine or trehalose analog (IMCTA-C14) in test example 10-3 by western blotting, and the results of calculating the value of the amount of expression (expression amount ratio) of SynA53T for groups with different compounds added when the amount of expression of SynA53T in control is 1 through image analysis of said results of determination (n = 5, mean ± S.D.).

[Fig. 23A] Fig. 23A shows the results of determining expression and nuclear localization of autophagy-related transcription factor TFEB when human ovarian cancer cells OVK18 were treated with 4-trehalosamine or trehalose analog (IMCTA-C14) in test example 10-4 by western blotting.

[Fig. 23B] Fig. 23B shows the results of determining expression and nuclear localization of autophagy-related transcription factor TFEB when human malignant melanoma cells Mewo were treated with 4-trehalosamine or trehalose analog (IMCTA-C14) in test example 10-4 by western blotting.

[Fig. 23C] Fig. 23C shows the results of determining expression and nuclear localization of autophagy-related transcription factor TFEB when human ovarian cancer cells OVK18 were treated with 4-trehalosamine or trehalose analog (IMCTA-C14) in test example 10-4 by immunostaining.

[Fig. 23D] Fig. 23D shows the results of determining expression and nuclear localization of autophagy-related transcription factor TFEB when human malignant melanoma cells Mewo were treated with 4-trehalosamine or trehalose analog (IMCTA-C14) in test example 10-4 by immunostaining.

[Fig. 24A] Fig. 24A shows the results of staining Mycobacterium smegmatis with trehalose analog (IMCTA-fluorescein) in test example 11-1.

[Fig. 24B] Fig. 24B shows the results of staining Mycobacterium smegmatis with trehalose analog (IMCTA-biotin) in test example 11-1.

[Fig. 24C] Fig. 24C shows the results of staining Mycobacterium smegmatis with trehalose analog (IMCTA-azide) in test example 11-1.

[Fig. 25A] Fig. 25A shows the intensity of fluorescence per bacterial cell volume when Mycobacterium smegmatis was stained with trehalose analog (IMCTA-fluorescein) in test example 11-1 (n = 3, mean ± S.D.).

[Fig. 25B] Fig. 25B shows the intensity of fluorescence per bacterial cell volume when Mycobacterium smegmatis was stained with trehalose analog (IMCTA-biotin) in test example 11-1 (n = 3, mean ± S.D.).

[Fig. 25C] Fig. 25C shows the intensity of fluorescence per bacterial cell volume when Mycobacterium smegmatis was stained with trehalose analog (IMCTA-azide) in test example 11-1 (n = 3, mean ± S.D.).

[Fig. 26A] Fig. 26A shows the result of TLC analysis (phosphomolybdic acid staining patterns) of lipid components of Mycobacterium smegmatis bacterial cells stained with trehalose analog (IMCTA-fluorescein) in test example 11-2.

[Fig. 26B] Fig. 26B shows the result of TLC analysis (fluorescence detection patterns) of lipid components of Mycobacterium smegmatis bacterial cells stained with trehalose analog (IMCTA-fluorescein) in test example 11-2.

[Fig. 27A] Fig. 27A shows the results of determining the persistence rate of 4-trehalosamine in culture fluid in which 12 kinds of microorganisms were cultured in the presence of 4-trehalosamine in test example 12-1, where the vertical axis: persistence rate (%).

[Fig. 27B] Fig. 27B shows the results of determining proliferation of microorganisms when 12 kinds of microorganisms were cultured in the presence of 4-trehalosamine in test example 12-1, where the vertical axis: $A_{620}$.

[Fig. 28A] Fig. 28A shows a binding model of trehalose to human trehalase in test example 13.

[Fig. 28B] Fig. 28B shows a binding model of 4-trehalosamine to human trehalase in test example 13.

## Description of Embodiments

(Novel compounds, or pharmaceutically acceptable salts thereof, or solvates thereof)

Compounds, or pharmaceutically acceptable salts thereof, or solvates thereof according to the present invention are compounds, or pharmaceutically acceptable salts thereof, or solvates thereof, which are represented by general formula (2) below, and are novel compounds, or pharmaceutically acceptable salts thereof, or solvates thereof, which were found by the present inventors.

[Chemical formula 10]

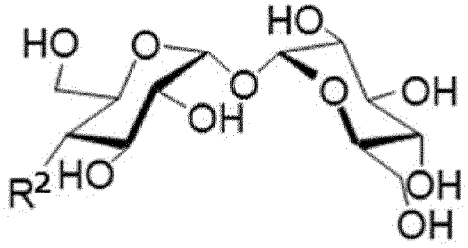

General formula (2)

Here, in the above general formula (2), $R^2$ denotes a substituent represented by structural formula (A) below, a substituent represented by structural formula (B) below, or $-NH(CH_2)_mCH_3$, and the m denotes an integer from 7 to 14.

[Chemical formula 11]

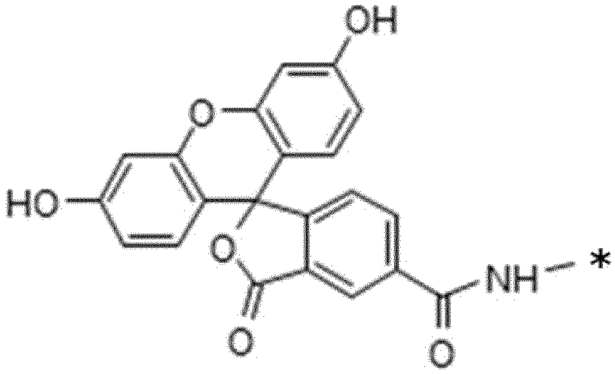

Structural formula (A)

[Chemical formula 12]

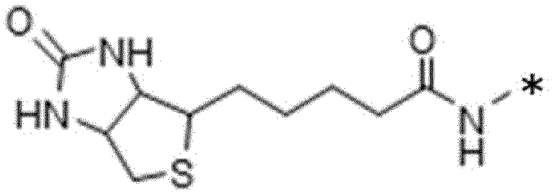

Structural formula (B)

In the above structural formula (A) or the structural formula (B) below, "*" denotes a dangling bond.

<IMCTA-fluorescein>

The general formula (2) in which $R^2$ is the substituent represented by the above structural formula (A) corresponds to compounds, or pharmaceutically acceptable salts thereof, or solvates thereof represented by structural formula (10) below. The compound represented by structural formula (10) below may be referred to as "IMCTA-fluorescein" hereinafter. The physico-chemical property of the IMCTA-fluorescein is as shown in synthesis example 9 which will be described later.

[Chemical formula 13]

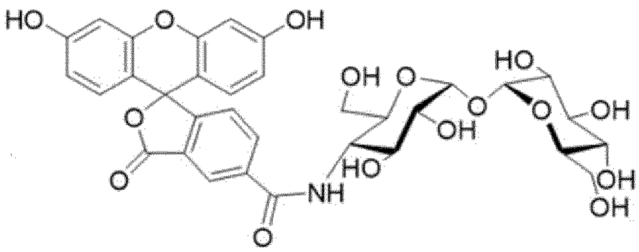

Structural formula (10)
(IMCTA-fluorescein)

<IMCTA-biotin>

The general formula (2) in which $R^2$ is the substituent represented by the above structural formula (B) corresponds to compounds, or pharmaceutically acceptable salts thereof, or solvates thereof represented by structural formula (11) below. The compound represented by structural formula (11) below may be referred to as "IMCTA-biotin" hereinafter. The physico-chemical property of the IMCTA-biotin is as shown in synthesis example 10 which will be described later.

[Chemical formula 14]

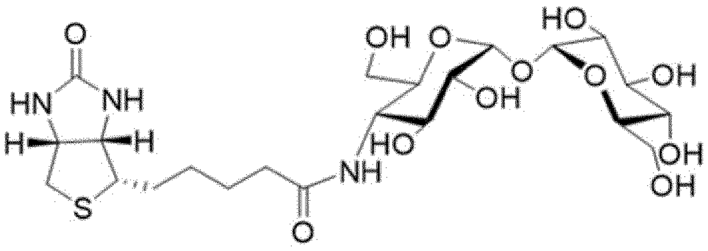

Structural formula (11)
(IMCTA-biotin)

- Use -

The compounds, or pharmaceutically acceptable salts thereof, or solvates thereof represented by the above structural formula (10) or (11) exhibit a mycobacterium staining effect with which mycobacterium can be easily and efficiently stained as shown in test examples described later, and therefore, can be suitably used, for example, as active components of a later-described mycobacterium stain according to the present invention.

<IMCTA-Cn>

The general formula (2) in which $R^2$ is the substituent represented by $-NH(CH_2)_mCH_3$ corresponds to compounds, or pharmaceutically acceptable salts thereof, or solvates thereof represented by any one of structural formulas (2) to (9) below. Compounds, or pharmaceutically acceptable salts thereof, or solvates thereof where the m is integers from 7 to 14 may be collectively referred to as "IMCTA-Cn" hereinafter. Here, the "n" in "IMCTA-Cn" is an integer and corresponds to the integer of "m+1" in the general formula (2) in which the substituent $R^2$ is $-NH(CH_2)_mCH_3$.

The case where the m is 7 (that is, a substituent where $R^2$ is represented by $-NH(CH_2)_7CH_3$) corresponds to

compounds, or pharmaceutically acceptable salts thereof, or solvates thereof represented by structural formula (2) below. Hereinafter, it may be referred to as "IMCTA-C8". The physico-chemical property of the IMCTA-C8 is as shown in synthesis example 1 which will be described later.

[Chemical formula 15]

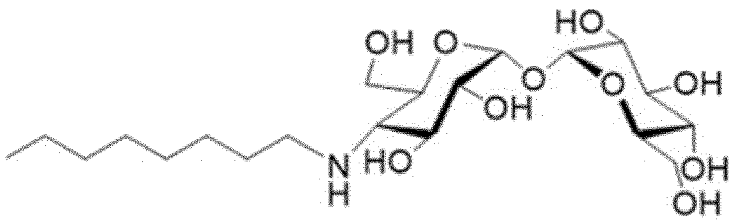

Structural formula (2)
(IMCTA-C8)

The case where the m is 8 (that is, a substituent where $R^2$ is represented by - $NH(CH_2)_8CH_3$) corresponds to compounds, or pharmaceutically acceptable salts thereof, or solvates thereof represented by structural formula (3) below. Hereinafter, it may be referred to as "IMCTA-C9". The physico-chemical property of the IMCTA-C9 is as shown in synthesis example 2 which will be described later.

[Chemical formula 16]

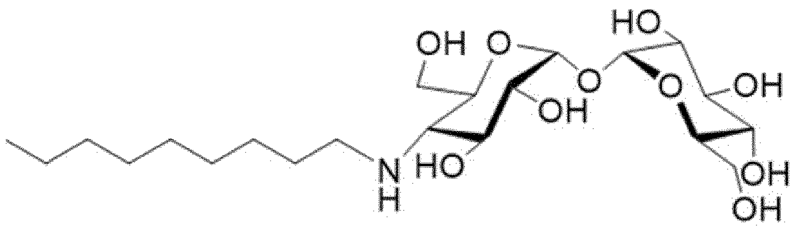

Structural formula (3)
(IMCTA-C9)

The case where the m is 9 (that is, a substituent where $R^2$ is represented by - $NH(CH_2)_9CH_3$) corresponds to compounds, or pharmaceutically acceptable salts thereof, or solvates thereof represented by structural formula (4) below. Hereinafter, it may be referred to as "IMCTA-C10". The physico-chemical property of the IMCTA-C10 is as shown in synthesis example 3 which will be described later.

[Chemical formula 17]

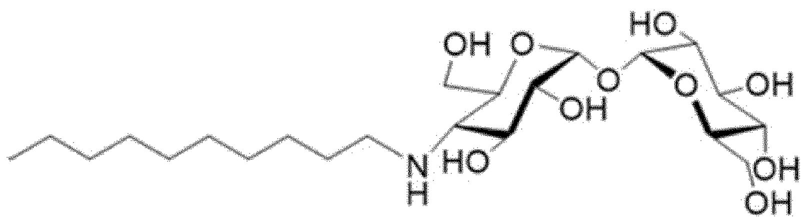

Structural formula (4)
(IMCTA-C10)

The case where the m is 10 (that is, a substituent where $R^2$ is represented by - $NH(CH_2)_{10}CH_3$) corresponds to compounds, or pharmaceutically acceptable salts thereof, or solvates thereof represented by structural formula (5) below. Hereinafter, it may be referred to as "IMCTA-C11". The physico-chemical property of the IMCTA-C11 is as shown in synthesis example 4 which will be described later.

[Chemical formula 18]

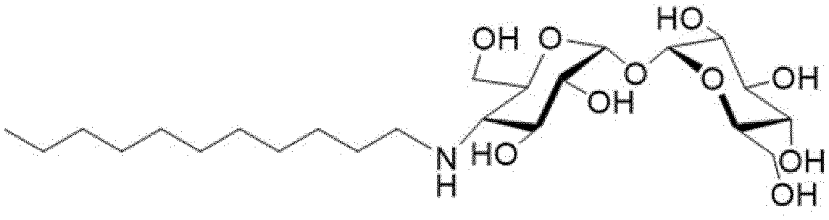

Structural formula (5)
(IMCTA-C11)

The case where the m is 11 (that is, a substituent where $R^2$ is represented by - $NH(CH_2)_{11}CH_3$) corresponds to compounds, or pharmaceutically acceptable salts thereof, or solvates thereof represented by structural formula (6) below. Hereinafter, it may be referred to as "IMCTA-C12". The physico-chemical property of the IMCTA-C12 is as shown in synthesis example 5 which will be described later.

[Chemical formula 19]

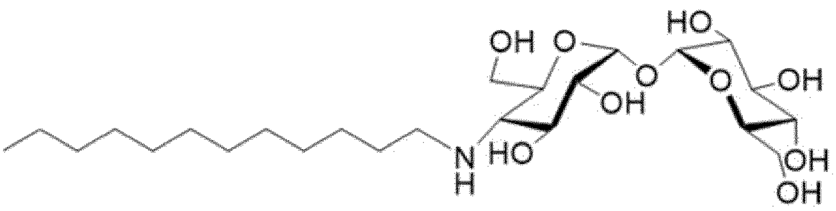

Structural formula (6)
(IMCTA-C12)

The case where the m is 12 (that is, a substituent where $R^2$ is represented by - $NH(CH_2)_{12}CH_3$) corresponds to compounds, or pharmaceutically acceptable salts thereof, or solvates thereof represented by structural formula (7) below. Hereinafter, it may be referred to as "IMCTA-C13". The physico-chemical property of the IMCTA-C13 is as shown in synthesis example 6 which will be described later.

[Chemical formula 20]

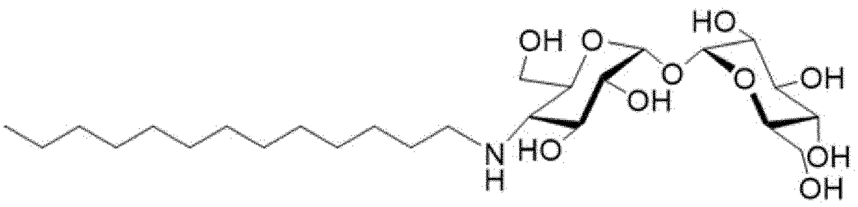

Structural formula (7)
(IMCTA-C13)

The case where the m is 13 (that is, a substituent where $R^2$ is represented by - $NH(CH_2)_{13}CH_3$) corresponds to compounds, or pharmaceutically acceptable salts thereof, or solvates thereof represented by structural formula (8) below. Hereinafter, it may be referred to as "IMCTA-C14". The physico-chemical property of the IMCTA-C14 is as shown in synthesis example 7 which will be described later.

[Chemical formula 21]

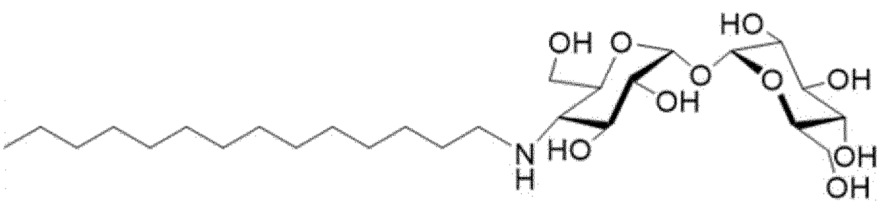

Structural formula (8)
(IMCTA-C14)

The case where the m is 14 (that is, a substituent where $R^2$ is represented by - $NH(CH_2)_{14}CH_3$) corresponds to compounds, or pharmaceutically acceptable salts thereof, or solvates thereof represented by structural formula (9) below. Hereinafter, it may be referred to as "IMCTA-C15". The physico-chemical property of the IMCTA-C15 is as shown in synthesis example 8 which will be described later.

[Chemical formula 22]

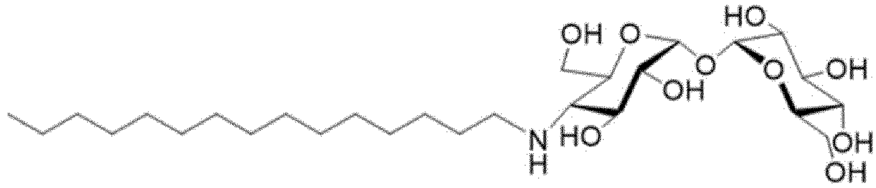

Structural formula (9) (IMCTA-C15)

- Use -

The compounds, or pharmaceutically acceptable salts thereof, or solvates thereof represented by any one of the above structural formulas (2) to (9) exhibit an excellent surface activity effect and an excellent autophagy inducing effect as shown in the test examples described later, and therefore, can be suitably used, for example, as active components of a surfactant composition according to the present invention, a protein extractant according to the present invention, and an autophagy inducer, which will be described later.

Whether the above-mentioned compounds have the structures of the compounds, or pharmaceutically acceptable salts thereof, or solvates thereof represented by the above general formula (2) can be confirmed by various analysis methods selected as appropriate. Examples of the analysis method include mass spectrometry, ultraviolet spectroscopy, infrared spectroscopy, proton nuclear magnetic resonance spectroscopy, carbon-13 nuclear magnetic resonance spectroscopy, and elemental analysis method. The above analysis methods may be used alone or in combination of two or more. Here, although there may be some errors in measured values obtained by the respective analysis methods mentioned above, those skilled in the art can easily identify that compounds have the structures of the compounds, or pharmaceutically acceptable salts thereof, or solvates thereof represented by the above general formula (2).

The above-mentioned salts are not particularly limited as long as the salts are pharmaceutically acceptable salts, and can be appropriately selected depending on a purpose. Examples of the salts include hydrohalogenic acid salts such as hydrofluoride, hydrochloride, hydrobromide, and hydroiodide; inorganic acid salts such as sulfate, nitrate, phosphate, perchlorate, and carbonate; carboxylate salts such as acetate, trichloroacetate, trifluoroacetate, hydroxyacetate, lactate, citrate, tartrate, oxalate, benzoate, mandelate, butyrate, maleate, propionate, formate, and malate; amino acid salts such as arginate, aspartate, and glutamate; and sulfonates such as methanesulfonate and paratoluenesulfonate.

The above-mentioned solvates are not particularly limited but can be appropriately selected depending on a purpose. Examples of the solvates include hydrate and ethanol hydrate.

The compounds, or pharmaceutically acceptable salts thereof, or solvates thereof represented by the above general formula (2) can be appropriately synthesized and obtained by chemical synthesis. A method for chemically synthesizing the compounds, or pharmaceutically acceptable salts thereof, or solvates thereof represented by the above general formula (2) is not particularly limited but can be appropriately selected from known synthesis methods depending on a purpose. However, the compounds, or pharmaceutically acceptable salts thereof, or solvates thereof can be suitably obtained by a later-described method for producing compound according to the present invention.

(Method for producing compound)

The method for producing compound according to the present invention includes a process for reacting an amino group at position 4 of 4-trehalosamine with $CH_3(CH_2)_pCOH$ (where the p denotes an integer from 6 to 13), 2-azido-1,3-dimethylimidazolinium hexafluorophosphate, 5-carboxyfluorescein N-succinimidyl ester, or D-biotin N-succinimidyl, using 4-trehalosamine as a starting material (hereinafter, this process may be referred to as the "reaction process"), and further includes other processes as necessary.

[Chemical formula 23]

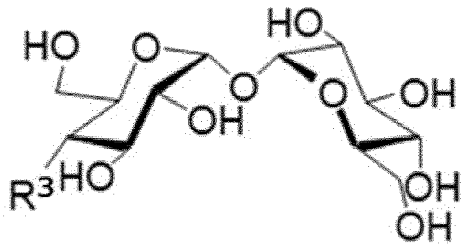

General formula (3)

Here, in the above general formula (3), $R^3$ denotes $-N(CH_2)_mCH_3$, $-N_3$, a substituent represented by structural formula (A) below, or a substituent represented by structural formula (B) below, and the m denotes an integer from 7 to 14.

[Chemical formula 24]

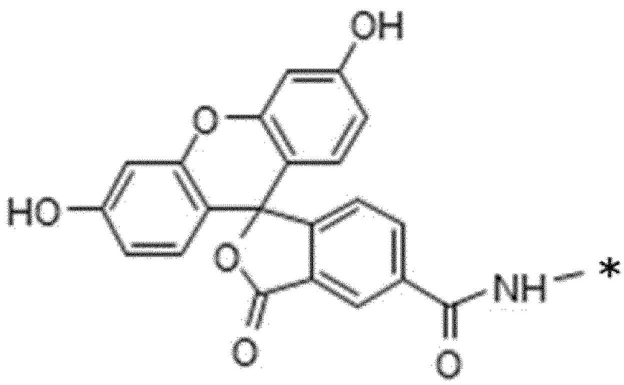

Structural formula (A)

[Chemical formula 25]

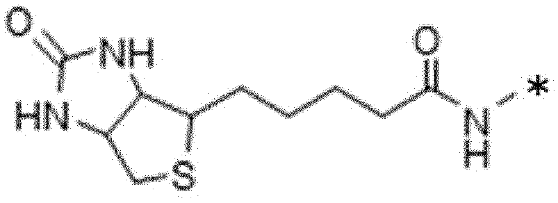

Structural formula (B)

In the above structural formula (A) or the structural formula (B) below, "*" denotes a dangling bond.

4-trehalosamine as a starting material to be used may be a commercially available product, or obtained from a microorganism producing 4-trehalosamine, or synthesized as appropriate by chemical synthesis. However, the one

obtained by a later-described method for producing 4-trehalosamine of the present invention is preferred on the point that a large quantity of 4-trehalosamine can be obtained simply, inexpensively, and efficiently.

<Reaction of amino group at position 4 of 4-trehalosamine with $CH_3(CH_2)_pCOH$ (where the p denotes integer from 6 to 13)>

The method for reacting the amino group at position 4 of 4-trehalosamine with $CH_3(CH_2)_pCO$ is not particularly limited but can be appropriately selected from known methods. Examples of the method include a method in which aqueous solution of 4-trehalosamine is mixed with $CH_3(CH_2)_pCOH$, which is dissolved in a solvent, and left to stand, and then aqueous solution of sodium cyanoborohydride is added to the solution and the solution is further left to stand.

There are no particular restrictions on the reaction conditions such as reaction temperature and reaction time in the reaction of the amino group at position 4 of 4-trehalosamine with $CH_3(CH_2)_pCOH$, a compound to be used and its use amount, solvent, and so forth, and these can be appropriately selected depending on a purpose.

Examples of the reaction temperature, reaction time, and so forth in leaving the aqueous solution of 4-trehalosamine to stand after mixing it with $CH_3(CH_2)_pCOH$ include 20°C to 45°C for 1 hour to 72 hours.

Further, examples of the reaction temperature, reaction time, and so forth in leaving the one to stand after adding the aqueous solution of sodium cyanoborohydride include 20°C to 45°C for 1 hour to 72 hours.

The above-mentioned solvent is not particularly limited as long as the solvent is able to react the amino group at position 4 of 4-trehalosamine with $CH_3(CH_2)_pCOH$, and can be appropriately selected depending on a purpose. Examples of the solvent include lower alcohols such as 1-propanol, 2-propanol, methanol, and ethanol, and dimethyl sulfoxide (DMSO). These may be used alone or in combination of two or more.

The above-mentioned reaction can produce compounds of the general formula (3) in which $R^3$ is $-N(CH_2)_mCH_3$ and the m denotes an integer from 7 to 14 (IMCTA-Cn).

The compounds of the general formula (3) in which $R^3$ is $-N(CH_2)_mCH_3$ and the m is an integer from 7 to 14 are similar to the case where $R^2$ is $-NH(CH_2)_mCH_3$ and the m denotes an integer from 7 to 14 in the general formula (2) in the above-mentioned section "(Novel compounds, or pharmaceutically acceptable salts thereof, or solvates thereof)", and their physico-chemical property is also similar.

<Reaction of amino group at position 4 of 4-trehalosamine with 2-azido-1,3-dimethylimidazolinium hexafluorophosphate>

The method for reacting the amino group at position 4 of 4-trehalosamine with 2-azido-1,3-dimethylimidazolinium hexafluorophosphate is not particularly limited but can be appropriately selected from known methods. Examples of this method include a method in which aqueous solution of 4-trehalosamine is mixed with a DMSO solution of 2-azido-1,3-dimethylimidazolinium hexafluorophosphate and a DMSO solution of N,N-dimethyl-4-aminopyridine (DMAP) and the mixed solution is left to stand.

There are no particular restrictions on the reaction conditions such as reaction temperature and reaction time in the reaction of the amino group at position 4 of 4-trehalosamine with 2-azido-1,3-dimethylimidazolinium hexafluorophosphate, a compound to be used and its use amount, solvent, and so forth, and these can be appropriately selected depending on a purpose.

The reaction temperature, reaction time, and so forth in leaving the aqueous solution of 4-trehalosamine to stand after mixing it with the DMSO solution of 2-azido-1,3-dimethylimidazolinium hexafluorophosphate and the DMSO solution of DMAP are not particularly limited but can be appropriately selected depending on a purpose, and examples of these include 20°C to 25°C for 2 hours to 72 hours.

<<IMCTA-azide>>

The above-mentioned reaction can produce the compounds of the general formula (3) in which $R^3$ is a substituent represented by $-N_3$.

The compounds of the general formula (3) in which $R^3$ is the substituent represented by $-N_3$ are compounds represented by structural formula (12) below. Hereinafter, it may be referred to as "IMCTA-azide". The physico-chemical property of the IMCTA-azide is as shown in synthesis example 11 which will be described later.

[Chemical formula 26]

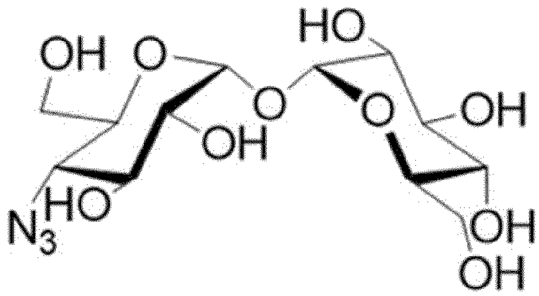

Structural formula (12) (IMCTA-azide)

<Reaction of amino group at position 4 of 4-trehalosamine with 5-carboxyfluorescein N-succinimidyl ester>

The method for reacting the amino group at position 4 of 4-trehalosamine with 5-carboxyfluorescein N-succinimidyl ester is not particularly limited but can be appropriately selected from known methods. Examples of this method include a method in which aqueous solution of 4-trehalosamine is mixed with 5-carboxyfluorescein N-succinimidyl ester, which is dissolved in a solvent, and the mixed solution is left to stand.

There are no particular restrictions on the reaction conditions such as reaction temperature and reaction time in the reaction of the amino group at position 4 of 4-trehalosamine with 5-carboxyfluorescein N-succinimidyl ester, a compound to be used and its use amount, solvent, and so forth, and these can be appropriately selected depending on a purpose.

The reaction temperature, reaction time, and so forth in leaving the aqueous solution of 4-trehalosamine to stand after mixing it with a DMSO solution of 5-carboxyfluorescein N-succinimidyl ester are not particularly limited but can be appropriately selected depending on a purpose, and examples of these include 20°C to 45°C for 24 hours to 72 hours.

The above-mentioned solvent is not particularly limited as long as the solvent is able to react the amino group at position 4 of 4-trehalosamine with 5-carboxyfluorescein N-succinimidyl ester, and can be appropriately selected depending on a purpose. Examples of the solvent include methanol, DMSO, and dimethylformamide. These may be used alone or in combination of two or more.

The above-mentioned reaction can produce compounds of the general formula (3) in which $R^3$ is a substituent represented by the above structural formula (A).

The compounds of the general formula (3) in which $R^3$ is the substituent represented by the above structural formula (A) are similar to the case where $R^2$ is a substituent represented by the above structural formula (A) in the general formula (2) in the above-mentioned section "(Novel compounds, or pharmaceutically acceptable salts thereof, or solvates thereof)", and their physico-chemical property is also similar.

<Reaction of amino group at position 4 of 4-trehalosamine with D-biotin N-succinimidyl>

The method for reacting the amino group at position 4 of 4-trehalosamine with D-biotin N-succinimidyl is not particularly limited but can be appropriately selected from known methods. Examples of this method include a method in which aqueous solution of 4-trehalosamine is mixed with D-biotin N-succinimidyl, which is dissolved in a solvent, and the mixed solution is left to stand.

There are no particular restrictions on the reaction conditions such as reaction temperature and reaction time in the reaction of the amino group at position 4 of 4-trehalosamine with D-biotin N-succinimidyl, a compound to be used and its use amount, solvent, and so forth, and these can be appropriately selected depending on a purpose.

Examples of the reaction temperature, reaction time, and so forth in leaving the aqueous solution of 4-trehalosamine to stand after mixing it with the DMSO solution of D-biotin N-succinimidyl include 20°C to 45°C for 24 hours to 72 hours.

The above-mentioned solvent is not particularly limited as long as the solvent is able to react the amino group at position 4 of 4-trehalosamine with D-biotin N-succinimidyl, and can be appropriately selected depending on a purpose. Examples of the solvent include DMSO and dimethylformamide. These may be used alone or in combination of two or more.

The above-mentioned reaction can produce compounds of the general formula (3) in which $R^3$ is a substituent represented by the above structural formula (B).

The compounds of the general formula (3) in which $R^3$ is the substituent represented by the above structural formula (B) are similar to the case where $R^2$ is a substituent represented by the above structural formula (B) in the

general formula (2) in the above-mentioned section "(Novel compounds, or pharmaceutically acceptable salts thereof, or solvates thereof)", and their physico-chemical property is also similar.

<Other processes>

Other processes mentioned above are not particularly limited but can be appropriately selected depending on a purpose. Examples of the processes include a purification process.

- Purification process -

The purification process is a process for purifying and isolating the compound obtained in the reaction process.
The purifying and isolating method is not particularly limited but can be appropriately selected depending on a purpose. Examples of the purifying and isolating method include liquid separation method, distillation method, sublimation method, precipitation method, crystallization method, silica gel column chromatography method using normal or reversed phase silica gel as a packing material, column chromatography method using ion exchange resin, column chromatography method using cellulose and the like, preparative thin-layer chromatography method, and high-performance liquid chromatography method. These may be used alone or in combination of two or more. Here, the compounds obtained in the production process described above can also be used in subsequent processes as appropriate without being subjected to isolation and purification.
Whether the obtained compounds have the structures represented by the above general formula (3) can be confirmed by various analysis methods selected as appropriate. The analysis method is not particularly limited but can be appropriately selected depending on a purpose. Examples of the analysis method include mass spectrometry, ultraviolet spectroscopy, infrared spectroscopy, proton nuclear magnetic resonance spectroscopy, carbon-13 nuclear magnetic resonance spectroscopy, and elemental analysis method. The above analysis methods may be used alone or in combination of two or more. Here, although there may be some errors in measured values obtained by the respective analysis methods mentioned above, those skilled in the art can easily identify that compounds have the structures represented by the above general formula (3).

(Compound-containing composition)

Compound-containing compositions according to the present invention contain at least the compounds, or pharmaceutically acceptable salts thereof, or solvates thereof represented by the above general formula (2) of the present invention, and further contain other components as necessary.
The compounds, or pharmaceutically acceptable salts thereof, or solvates thereof represented by the above general formula (2) may be used alone or in combination of two or more kinds.

<Compounds, or pharmaceutically acceptable salts thereof, or solvates thereof represented by general formula (2)>

Compounds, or pharmaceutically acceptable salts thereof, or solvates thereof represented by the above general formula (2) are the compounds, or pharmaceutically acceptable salts thereof, or solvates thereof represented by the general formula (2) according to the present invention described above.
There are no particular restrictions on a total content of the compounds, or pharmaceutically acceptable salts thereof, or solvates thereof represented by the above general formula (2) in the above-mentioned compound-containing composition and the total content can be appropriately selected depending on a purpose. The compound-containing composition may be composed only of the compounds, or pharmaceutically acceptable salts thereof, or solvates thereof represented by the above general formula (2).

<Other components>

Other components are not particularly limited as long as the components do not impair the effects of the present invention, and can be appropriately selected depending on a purpose. Examples of other components include components described in "- Other components -" under each of agents listed in the later-described section "(Protective agent, surfactant composition, composition for blood glucose level control, autophagy inducer, mycobacterium stain, or protein extractant)".
The content of other components in the compound-containing composition is not particularly limited but can be appropriately selected depending on a purpose within a range in which the effects of the compounds, or pharmaceutically acceptable salts thereof, or solvates thereof represented by the above general formula (2) are not impaired.

- Use -

The compound-containing composition containing the compounds, or pharmaceutically acceptable salts thereof, or solvates thereof represented by any one of the structural formulas (2) to (9) exhibits excellent surface activity effect and excellent protein extraction effect and therefore, can be suitably used, for example, as active components of a surfactant composition, a protein extractant, and the like.
The compound-containing composition containing the compounds, or pharmaceutically acceptable salts thereof, or solvates thereof represented by the above structural formula (10) or (11) exhibits an effect for easily and efficiently staining or labeling mycobacterium and therefore, can be suitably used, for example, as active components of a mycobacterium stain.
The compound-containing composition may be used alone or in combination with a medicine containing another component as an active component. Further, the compound-containing composition may be used in a blended form in a composition containing another component as an active component.
The composition containing another component as an active component is not particularly limited but can be appropriately selected depending on a purpose. Examples of this composition include food and beverages, pharmaceutical products, cosmetics, and reagents.

(Method for producing 4-trehalosamine)

The method for producing 4-trehalosamine according to the present invention includes a culture process and further includes other processes as necessary. Employing the method for producing 4-trehalosamine is advantageous in that 4-trehalosamine can be produced easily, simply, and efficiently. The 4-trehalosamine is the compound represented by structural formula (1) below.

[Chemical formula 27]

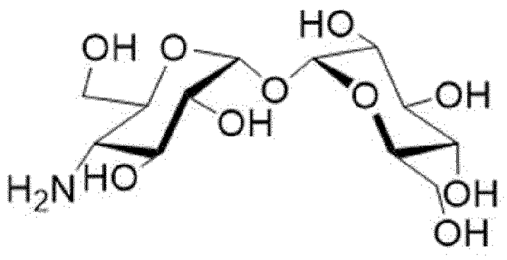

Structural formula (1)
(4-trehalosamine)

<Culture process>

The culture process is a process for culturing Streptomyces sp. MK186-mF5 (accession number: NITE BP-03495).
In the culture process, the production capacity of 4-trehalosamine can also be further increased by applying ultraviolet light, X-rays, radiation, chemicals, and other mutational treatments to Streptomyces sp. MK186-mF5. In addition, the production capacity of 4-trehalosamine can also be increased by genetic engineering methods.
Examples of a method for analyzing whether the above-mentioned microorganism is capable of producing 4-trehalosamine include a method in which 4-trehalosamine is detected from a culture product of this microorganism, preferably from a culture supernatant after liquid culture or solid culture medium after solid culture by various analysis methods.

«Culture medium»

The culture medium used in the culture process is not particularly limited as long as the culture medium can culture Streptomyces sp. MK186-mFS, and a culture medium which can be used is the one containing known culture medium components conventionally used for culturing bacteria belonging to the genus Streptomyces, which may be a liquid culture medium or a solid (agar) culture medium.
A nutrient source to be added to the culture medium is not particularly limited but can be appropriately selected depending on a purpose. Examples of the nutrient source include nitrogen sources such as commercially available soybean flour, wheat germ, rolled barley, peptone, cottonseed meal, yeast extract (for example, brewer's yeast extract,

baker's yeast extract), meat extract, corn steep liquor, ammonium sulfate, sodium nitrate, and urea; carbohydrates such as soluble starch, tomato paste, glycerin, glucose, galactose, dextrin, and bacto soytone; and carbon sources such as fat.

Further, inorganic salts such as common salt, calcium carbonate, dipotassium hydrogen phosphate, potassium dihydrogen phosphate, potassium chloride, and sodium chloride can be added to the culture medium, and furthermore, trace amounts of metal salts such as zinc chloride and magnesium sulfate can be added to the culture medium as necessary.

All known culture materials can be used as long as these materials are utilized by Streptomyces sp. MK186-mF5 and are useful for the production of 4-trehalosamine.

Among these, the culture medium used in the culture process preferably contains carbon sources such as soluble starch and glucose; nitrogen sources such as brewer's yeast and baker's yeast; inorganic salts such as calcium carbonate, dipotassium hydrogen phosphate, potassium dihydrogen phosphate, potassium chloride, and sodium chloride; or metal salts such as zinc chloride and magnesium sulfate; more preferably contains at least any one of soluble starch, potassium chloride, sodium chloride, calcium carbonate, and zinc chloride on the point that high volume of 4-trehalosamine can be produced; and particularly preferably contains soluble starch, potassium chloride, sodium chloride, and zinc chloride without containing magnesium sulfate.

The concentration of the soluble starch in the culture medium is not particularly limited but can be appropriately selected depending on a purpose. The concentration of the soluble starch is preferably 1% by mass to 10% by mass, more preferably 3% by mass to 9% by mass, and particularly preferably 5% by mass to 6% by mass, on the point that high volume of 4-trehalosamine can be produced.

The concentration of the zinc chloride in the culture medium is not particularly limited but can be appropriately selected depending on a purpose. The concentration of the zinc chloride is preferably 10% by mass or less, more preferably 0.001% by mass to 0.02% by mass, and particularly preferably 0.001% by mass to 0.015% by mass.

The concentration of the potassium chloride in the culture medium is not particularly limited but can be appropriately selected depending on a purpose. The concentration of the potassium chloride is preferably 0.1% by mass to 1% by mass, more preferably 0.1% by mass to 0.8% by mass, and particularly preferably 0.4% by mass to 0.7% by mass.

The concentration of the brewer's yeast in the culture medium is not particularly limited but can be appropriately selected depending on a purpose. The lower limit of the concentration of the brewer's yeast is preferably 2% by mass or greater, and more preferably 3% by mass or greater. The upper limit of the concentration of the brewer's yeast in the culture medium is not particularly limited in terms of production of 4-trehalosamine and can be appropriately selected depending on a purpose. However, the concentration of the brewer's yeast is preferably 4% by mass or less from the viewpoint of cost. The lower limit and the upper limit of the concentration of the brewer's yeast in the culture medium can be combined as appropriate, and are preferably 2% by mass to 4% by mass, more preferably 2% by mass to 3% by mass, and particularly preferably 3% by mass.

The pre-culture fluid for the production of 4-trehalosamine is not particularly limited but can be appropriately selected depending on a purpose. Examples of this pre-culture fluid include a growth of Streptomyces sp. MK186-mF5 cultured on a culture medium such as liquid culture medium, plate culture medium, slant culture medium, and semi-slant culture medium.

The culture method is not particularly limited but can be appropriately selected depending on a purpose. Examples of the culture method include shaking culture, static culture, and tank culture.

The culture temperature is not particularly limited as long as the temperature is within a range where 4-trehalosamine can be produced without substantially inhibiting the growth of Streptomyces sp. MK186-mF5, and the culture temperature can be appropriately selected depending on a purpose. However, the temperature is preferably 25°C to 35°C.

The pH of the culture is not particularly limited as long as the pH is within a range where 4-trehalosamine can be produced without substantially inhibiting the growth of Streptomyces sp. MK186-mF5, and the pH can be appropriately selected depending on a purpose.

The culture period is not particularly limited but can be appropriately selected in accordance with the accumulation of 4-trehalosamine.

The method for performing the culture process is not particularly limited but can be appropriately selected depending on a purpose. The culture may be performed at a laboratory level such as in a flask or may be performed using large-scale culture equipment.

Examples of the large-scale culture equipment include MPF-U3 (manufactured by Marubishi Bioengineering Co., Ltd.).

<Other processes>

Other processes mentioned above are not particularly limited but can be appropriately selected depending on a purpose. Examples of other processes include a collection process.

- Collection process -

The collection process is a process for collecting 4-trehalosamine from the culture product which is obtained in the above-mentioned culture process and contains 4-trehalosamine.
4-trehalosamine has the physico-chemical property described above and therefore, 4-trehalosamine can be purified and isolated from the culture product in accordance with its property.
The culture product is not particularly limited as long as the culture product is obtained in the culture process and contains 4-trehalosamine, and can be selected as appropriate depending on a purpose. Examples of the culture product include bacterial cell, culture supernatant after liquid culture, solid culture medium after solid culture, and mixtures of these.
When the above-mentioned bacterial cell is used as the culture product, 4-trehalosamine may be extracted from the bacterial cell by, for example, an extraction method using an appropriate organic solvent or by an elution method based on bacterial cell crushing, and then may be subjected to separation and/or purification.
The collection method is not particularly limited, and any method used to collect metabolites produced by microorganisms can be selected as appropriate. Examples of the collection method include solvent extraction method, method that utilizes differences in adsorption affinity for various adsorbents, and chromatography method. Separated and/or purified 4-trehalosamine can be collected by using these methods alone or in appropriate combination, and in some cases, by repeated use.
The solvent used in the solvent extraction method is not particularly limited but can be appropriately selected depending on a purpose. Examples of the solvent include ethanol, methanol, acetone, butanol, and acetonitrile.
The above-mentioned adsorbent is not particularly limited but can be appropriately selected from known adsorbents depending on a purpose. Examples of the adsorbent include polystyrene-based adsorption resin.
The above-mentioned chromatography method is not particularly limited but can be appropriately selected depending on a purpose. Examples of the chromatography method include thin-layer chromatography (TLC) method, and preparative high-performance liquid chromatography (preparative HPLC) method using normal-phase or reversed-phase columns.
The carrier used for the chromatography method is not particularly limited but can be appropriately selected depending on a purpose. Examples of the carrier include ion exchange resin, gel filtration, silica gel, alumina, and activated carbon.
Specific examples of commercially available carrier used in the chromatography method include Amberlite (registered trademark) CG50 (manufactured by Sigma-Aldrich), DOWEX 50W×4 column 50-100 (manufactured by MUROMACHI CHEMICALS INC.), Amberlite FPC3500 (manufactured by Organo Corporation), DIAION™ WA30 (manufactured by Mitsubishi Chemical Corporation), Sephadex (registered trademark) LH-20 (manufactured by GE Healthcare Japan Corporation), activated carbon (manufactured by FUJIFILM Wako Pure Chemical Corporation), Hydrosphere C18 (manufactured by YMC Co., Ltd.), Hydrophilic Interaction Chromatography column (manufactured by Waters Corporation), YMC-GEL ODS-A 6 nm S-150 μm (manufactured by YMC Co., Ltd.), YMC-Pack Polyamine II (manufactured by YMC Co., Ltd.), and ACQUITY UPLC Ethylene Bridged Hybrid (BEH) Amide 1.7 μm, 2.1 × 100 mm (manufactured by Waters Corporation).
The method for eluting 4-trehalosamine from the above-mentioned adsorbent and the carrier used in the chromatography method is not particularly limited but can be appropriately selected depending on the types, properties, and the like of the adsorbent and the carrier. For example, in the case of polystyrene-based adsorption resin, examples of the method include an elution method using hydrated alcohol, hydrated acetone, or the like as an elution solvent.
4-trehalosamine can be produced as described above.
4-trehalosamine is not a carbon source or a nutrient source to microorganisms and does not allow microorganisms to grow. Thus, 4-trehalosamine has the advantage of not causing spoilage.
The above-mentioned microorganisms are not particularly limited but can be appropriately selected depending on a purpose. Examples of the microorganisms include microorganisms belonging to the genus Escherichia (for example, Escherichia coli), microorganisms belonging to the genus Serratia (for example, Serratia marcescens), microorganisms belonging to the genus Enterococcus (for example, Enterococcus faecalis), microorganisms belonging to the genus Aspergillus (for example, Aspergillus niger), microorganisms belonging to the genus Salmonella (for example, Salmonella enteritidis), microorganisms belonging to the genus Mycobacterium (for example, Mycobacterium smegmatis), microorganisms belonging to the genus Bacillus (for example, Bacillus subtilis), microorganisms belonging to the genus Pseudomonas (for example, Pseudomonas aeruginosa), microorganisms belonging to the genus Micrococcus (for example, Micrococcus luteus), microorganisms belonging to the genus Bacteroides (for example, Bacteroides fragilis), microorganisms belonging to the genus Saccharomyces (for example, Saccharomyces cerevisiae), and microorganisms belonging to the genus Candida (for example, Candida albicans).
The above-mentioned microorganisms may be any one of commercially available products, microorganisms owned by the applicant, and newly separated bacterial strains of microorganisms belonging to the above-mentioned

genus.

Examples of Escherichia coli include Escherichia coli K-12 (owned by the Microbial Chemistry Research Foundation) and Escherichia coli K-12 (National BioResource Project (NBPR)).

Examples of Serratia marcescens include Serratia marcescens B-0524 (owned by the Microbial Chemistry Research Foundation) and Serratia marcescens JCM 1239T (RIKEN BRC).

Examples of Enterococcus faecalis include Enterococcus faecalis JCM5803 (RIKEN BRC).

Examples of Aspergillus niger include Aspergillus niger F 16 (owned by the Microbial Chemistry Research Foundation) and Aspergillus niger NBRC 13245T (NITE NBRC).

Examples of Salmonella enteritidis include Salmonella enteritidis 1891 (owned by the Microbial Chemistry Research Foundation) and Salmonella enteritidis JCM 1652 (RIKEN BRC).

Examples of Mycobacterium smegmatis include Mycobacterium smegmatis ATCC-607 (ATCC).

Examples of Bacillus subtilis include Bacillus subtilis 168.

Examples of Pseudomonas aeruginosa include Pseudomonas aeruginosa A3 (owned by the Microbial Chemistry Research Foundation) and Pseudomonas aeruginosa JCM 5962 T (RIKEN BRC).

Examples of Micrococcus luteus include Micrococcus luteus IFO3333 (Institute for Fermentation, Osaka (IFO)).

Examples of Bacteroides fragilis include Bacteroides fragilis JCM11019 (RIKEN BRC).

Examples of Saccharomyces cerevisiae include Saccharomyces cerevisiae F-7 (owned by the Microbial Chemistry Research Foundation) and Saccharomyces cerevisiae JCM 7255 T (RIKEN BRC).

Examples of Candida albicans include Candida albicans 3147 (IFO).

(Protective agent, surfactant composition, composition for blood glucose level control, autophagy inducer, mycobacterium stain, or protein extractant)

The protective agent, surfactant composition, composition for blood glucose level control, autophagy inducer, mycobacterium stain, or protein extractant according to the present invention contains compounds, or pharmaceutically acceptable salts thereof, or solvates thereof represented by general formula (1) below, and further contains other components as necessary.

[Chemical formula 28]

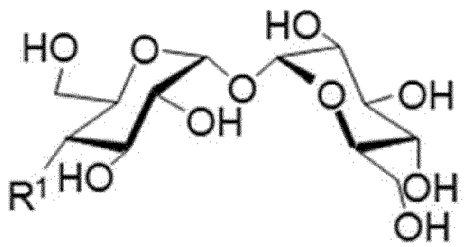

General formula (1)

Here, in the general formula (1), $R^1$ denotes $-NH_2$, a substituent represented by structural formula (A) below, a substituent represented by structural formula (B) below, or $-NH(CH_2)_mCH_3$, and the m denotes an integer from 7 to 14,

[Chemical formula 29]

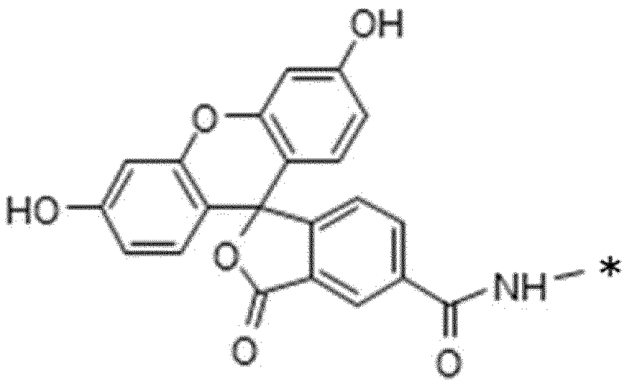

Structural formula (A)

[Chemical formula 30]

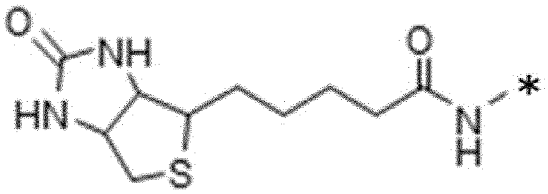

Structural formula (B)

In the above structural formula (A) or the structural formula (B) below, "*" denotes a dangling bond.

<Protective agent>

The present inventors have conducted extensive studies and have newly found that the compounds represented by the above general formula (1) exhibit excellent protective effect for starches, proteins, microorganisms, and so forth based on their moisture retaining effect and near-neutral pH buffer effect, and the compounds can be used as protective agents for these. Specifically, the protective agents are superior in various effects such as starch aging inhibiting effect, effect of preventing or inhibiting decrease in activity of proteins when stored in a frozen or freeze-dried state, and effect of preventing or inhibiting damage or death of microorganisms when stored in a frozen or freeze-dried state, based on the moisture retaining effect and near-neutral pH buffer effect of the compound represented by the above general formula (1), and the protective agents can be used as a starch aging inhibitor, protein protective agent, microorganism protective agent, pH buffer agent, and the like.

«Starch aging inhibitor»

Starch-containing foods have had a problem in which an elastic modulus of starch changes over time and takes time to stabilize in the producing process thereof, which causes variation in starch-containing foods and makes it difficult to maintain consistent quality. In addition, deterioration of texture (loss of softness, moistness, and so forth) due to aging of starch has been a problem.

To solve the above-mentioned problems, a method has been proposed in which swelling of starch is inhibited and gelatinization and associated food adhesion and texture deterioration are prevented by blending a natural polysaccharide or a decomposition product thereof having at least one of glucose, mannose, and galactose as a constituent sugar (see Japanese Unexamined Patent Application Publication No. H5-276882). However, the aging inhibiting effect of this method has not been fully satisfactory.

The present inventors have conducted extensive studies and newly found that the compound represented by the above general formula (1) exhibits an excellent starch aging inhibiting effect.

- Compounds represented by general formula (1) -

The compounds which are contained in the above-mentioned starch aging inhibitor and represented by the above general formula (1) are the compounds, or pharmaceutically acceptable salts thereof, or solvates thereof where $R^1$ is $-NH_2$.

The compounds of the general formula (1) in which $R^1$ is $-NH_2$ are the compounds represented by the above structural formula (1) in the above-mentioned section "(Method for producing 4-trehalosamine)", and their physico-chemical property is also as described in the above-mentioned section "(Method for producing 4-trehalosamine)".

There are no particular restrictions on a total content of the compounds (4-trehalosamine), or pharmaceutically acceptable salts thereof, or solvates thereof represented by the above general formula (1) in the above-mentioned starch aging inhibitor and the total content can be appropriately selected depending on a purpose. The starch aging inhibitor may be the compounds themselves represented by the above general formula (1).

- Other components -

Other components in the above-mentioned starch aging inhibitor are not particularly limited as long as the components do not impair the effects of the present invention, and can be appropriately selected depending on a purpose. Examples of other components include: amino acids such as sodium L-aspartate; nucleic acids such as disodium 5'-inosinate; seasonings represented by organic acids such as monopotassium citrate, and inorganic salts such as potassium chloride; storage life improving agents such as mustard extract, wasabi extract, and kojic acid; preservatives such as milt protein extract, polylysine, and sorbic acid; enzymes such as $\alpha$, $\beta$ amylase, $\alpha$, $\beta$ glucosidase, and papain; pH adjusters such as citric acid, fumaric acid, and succinic acid; emulsifiers such as sucrose fatty acid ester, glycerin fatty acid ester, organic acid monoglyceride, and lecithin; polysaccharide thickeners such as fragrance, dyes, watersoluble soy polysaccharide, carrageenan, xanthan gum, gellan gum, native gellan gum, sodium alginate, agar, konjac, pectin, Tara gum, karaya gum, tragacanth gum, ghatti gum, rhamsan gum, welan gum, curdlan, pullulan, and psyllium seed gum; expansion agents; proteins such as whey protein and soybean protein; sugars such as sucrose, fructose, reduced starch saccharides, erythritol, and xylitol; sweeteners such as sucralose, thaumatin, acesulfame potassium, and aspartame; vitamins such as vitamin A, vitamin C, vitamin E, and vitamin K; and minerals such as iron and calcium. Further, examples include addition agents described in "- Formulation form -" in "<Composition for blood glucose level control>", which will be described later. These may be used alone or in combination of two or more.

The content of above-mentioned other components in the starch aging inhibitor is not particularly limited as long as the content does not impair the effects of the present invention, and can be appropriately selected depending on a purpose.

- Use -

Since the above-mentioned starch aging inhibitor contains the compounds (4-trehalosamine), or pharmaceutically acceptable salts thereof, or solvates thereof represented by the above general formula (1), the starch aging inhibitor exhibits an excellent starch aging inhibiting effect when being contained in starch-containing foods. Accordingly, the starch aging inhibitor can be suitably used as a quality improving agent for starch contained in starch-containing foods. Thus, the present invention also relates to a starch quality improving agent and a production method thereof, which are characterized by containing the above-mentioned starch aging inhibitor, and also relates to food and beverage and a production method thereof, which are characterized by containing the above-mentioned starch aging inhibitor.

The above-mentioned starch-containing foods are not particularly limited as long as they are foods containing starch. Examples of the starch-containing foods include: breads such as sliced bread and sweet bread; noodles such as soba, udon, harusame, gyoza skin, shumai skin, Chinese noodles, and instant noodles; cooking sauces of white sauce gratin and so forth; cakes such as sponge cake, butter cake, and fruit cake; donuts; skins of gyoza, shumai, spring rolls, wonton, and so forth; steamed bread, Chinese buns, steamed buns, and so forth; and a wide variety of foods such as croquettes, okonomiyaki, takoyaki, taiyaki, imagawa-yaki, daifuku, dango, uirou, harusame, mitarashi dango sauce, custard cream, and flower paste.

<<Protein protective agent>>

Proteins such as enzymes and antibodies are useful as, for example, pharmaceuticals, diagnostic drugs, and various reagents, but they have a property of losing their activity due to physico-chemical denaturation, and special measures are required for their long-term storage. Aqueous solutions of enzymes and antibodies are generally frozen and stored at low temperatures, or freeze-dried and stored in a dry (solid) state. However, some enzymes and antibodies lose their activity during freezing or freeze-drying thereof. Therefore, protective agents have been developed and used to prevent decrease in activity during storage. Examples of the protective agents includes: (1) cryoprotectants produced by microorganisms of the genus Erwinia or the genus Xanthomonas (see Japanese Unexamined Patent Application Publication No. 2001-139599); silkworm-derived sericin (see Japanese Unexamined Patent Application Publication No. 2002-101869); (3) plant-derived protein dehydrin and its partial peptides (see Protein Science, 2011, January; 20(1): 42-50); (4) freezing tolerance proteins derived from gramineous plants Wcs19 and Wcor410 (see the specification of

U.S. Patent No. 5,731,419); (5) bovine serum albumin (BSA); and (6) human serum albumin (HSA).

When using enzymes, antibody proteins, and the like as biopharmaceuticals, it is necessary to take into account the risk that cryoprotective agents cause immune reactions within human bodies and cause side effects such as inflammation and anaphylaxis. The above (1) to (5) are proteins derived from organisms other than humans and therefore, there has been a problem in which the (1) to (5) are not suitable as additives for human pharmaceuticals. HSA in the above (6) already has other medicinal properties as blood derivatives, thereby being limited in use as pharmaceutical addition agents. Further, there has been a problem in which plasma derivatives HSA have the risk of medical accidents such as virus contamination. Furthermore, recombinant HSA is disadvantageously expensive. Therefore, it has been desired to provide a protein protective agent that is highly versatile and suitable for use in human medicine.

In light of this, the present inventors have conducted extensive studies and newly found that the compounds represented by the above general formula (1) are highly safe and have excellent protein-protective activity.

- Compounds represented by general formula (1) -

The compounds which are contained in the above-mentioned protein protective agent and represented by the above general formula (1) are the compounds, or pharmaceutically acceptable salts thereof, or solvates thereof where $R^1$ is $-NH_2$.

The compounds of the general formula (1) in which $R^1$ is $-NH_2$ are the compounds represented by the above structural formula (1) in the above-mentioned section "(Method for producing 4-trehalosamine)", and their physicochemical property is also as described in the above-mentioned section "(Method for producing 4-trehalosamine)".

There are no particular restrictions on a total content of the compounds (4-trehalosamine), or pharmaceutically acceptable salts thereof, or solvates thereof represented by the above general formula (1) in the above-mentioned protein protective agent and the total content can be appropriately selected depending on a purpose. The protein protective agent may be the compounds themselves represented by the above general formula (1).

The protein protective agent can prevent or inhibit decrease in activity of protein (including loss of activity) upon freezing or freeze-drying of the protein. Therefore, when the protein protective agent is applied at the time of freezing or freeze-drying of protein, the effect of freezing or freeze-drying is reduced. As a result, the activity of the protein after storage in the frozen or freeze-dried state is higher than when the protein protective agent is not applied.

The conditions for performing the freezing are not particularly limited as long as the conditions do not impair the effects of the present invention. The freezing can be performed by a conventional method after mixing protein to which the protein protective agent is applied (protection target) with the protein protective agent. Freezing can be performed at, for example, -20°C to -160°C or lower (using liquid nitrogen or the like), and freeze-drying can be performed at a shelf temperature of 35°C or lower and under a vacuum of about $1.0 \times 10^{-1}$ torr.

- Other components -

Other components in the above-mentioned protein protective agent are not particularly limited as long as the components do not impair the effects of the present invention, and can be appropriately selected depending on a purpose. Examples of other components include addition agents described in "- Formulation form -" in "<Composition for blood glucose level control>", which will be described later.

The content of above-mentioned other components in the protein protective agent is not particularly limited as long as the content does not impair the effects of the present invention, and can be appropriately selected depending on a purpose.

-Use-

The amount of the protein protective agent added to the protein is not particularly limited as long as the effects of the present invention are achieved, and the addition amount of the protein protective agent can be appropriately selected depending on a purpose.

The protein protective agent contains the compounds (4-trehalosamine), or pharmaceutically acceptable salts thereof, or solvates thereof represented by the above general formula (1). Therefore, the protein protective agent can easily protect proteins (prevent or inhibit decrease in activity of proteins (including loss of activity)) by being frozen or freeze-dried together with various proteins, and can be suitably used for reagents for protecting proteins as well as used in a protein protection method and protein storage method in clinical and research settings. Thus, the present invention also relates to a method for protecting protein characterized by containing the above-mentioned protein protective agent, a method for storing protein characterized by containing the above-mentioned protein protective agent, and a protein-containing composition characterized by containing an arbitrary protein and the above-mentioned protein protective agent.

There are no particular restrictions on proteins to which the above-mentioned protein protective agent is applied (protection targets), and proteins can be appropriately selected depending on a purpose. Examples of the proteins include: enzymes such as digestive enzymes (for example, amylase, lipase, cellulase, and so forth), proteases (for example, pepsin, trypsin, chymotrypsin, papain, bromelain, blood coagulation factor Xa, and so forth), glycolytic enzymes (for example, galactosidase, lactase, saccharase, and so forth), oxidoreductases (for example, lactate dehydrogenase, alcohol dehydrogenase (ADH), and so forth), dephosphorylating enzymes (for example, alkaline phosphatase (CIP), and so forth), nucleases (for example, DNase I, RNase H, and so forth), and restriction enzymes (EcoRI, Not I, and so forth); and antibodies such as polyclonal antibodies and monoclonal antibodies or fragments thereof.

The protein protective agent can be also suitably used for protection for not only single proteins but also various structures made of proteins (complex proteins such as glycoproteins, lipoproteins, nuclear proteins, and phosphoproteins; cells; tissues; and so forth) during freezing or freeze-drying.

<<Microorganism protective agent>>

Microorganisms used in researches and microorganisms added to food and beverages and the like have the property of being damaged or killed due to physico-chemical denaturation, and special measures are required for their long-term storage. Similar to the above-mentioned proteins, culture fluids containing microorganisms are generally frozen and stored at low temperatures, or freeze-dried and stored in a dry (solid) state. However, some microorganisms are damaged or killed during freezing or freeze-drying thereof. Therefore, protective agents have been developed and used to prevent decrease in activity during storage. Examples of the protective agents includes: skimmed milk, monosodium glutamate, gelatin, and sucrose (see, for example, Japanese Examined Patent Application Publication No. S53-8792), phenylalanine, histidine, citric acid, succinic acid, tartaric acid, and alkaline carbonate (see, for example, Japanese Unexamined Patent Application Publication No. S61-265085), lactose, trehalose, skimmed milk powder, sorbitol, and sodium ascorbate (see, for example, Ana S. Carvalho et al. Relevant factors for the preparation of freeze-dried lactic acid bacteria. International Dairy Journal. 14(10): 835-847, 2004.).

However, even with these protective agents, it has not been fully satisfactory to inhibit damage or death of microorganisms during freezing and freeze-drying thereof, and it has been difficult to prepare highly concentrated and stable frozen microorganisms or freeze-dried microorganisms. Especially, in a state in which a microorganism dispersion liquid and a protective substance, which is to be assimilated by microorganisms, are mixed together in microorganism preparation, a freezing operation has to be performed quickly at the lowest possible temperature, otherwise acid produced by the active metabolism of the microorganisms lowers the pH of a bacterial cell dispersion liquid which is to be frozen, resulting in a problem of a significant decrease in the viability. Furthermore, the pH of the microorganism dispersion liquid at the time of freezing significantly affects the damage or death of microorganisms during thawing or freeze-drying. Therefore, it has been necessary to pay sufficient attention to the pH of microorganism dispersion liquids before freezing and, in some cases, necessary to neutralize them with alkali or the like. Thus, it has been desired to provide a microorganism protective agent that is highly versatile and capable of preventing damage or death of microorganisms during freezing or freeze-drying of the microorganisms.

In light of this, the present inventors have conducted extensive studies and newly found that the compounds represented by the above general formula (1) are highly versatile, are not assimilated by microorganisms, and have excellent microorganism-protective activity.

- Compounds represented by general formula (1) -

The compounds which are contained in the above-mentioned microorganism protective agent and represented by the above general formula (1) are the compounds, or pharmaceutically acceptable salts thereof, or solvates thereof where $R^1$ is $-NH_2$.

The compounds of the general formula (1) in which $R^1$ is $-NH_2$ are the compounds represented by the above structural formula (1) in the above-mentioned section "(Method for producing 4-trehalosamine)", and their physicochemical property is also as described in the above-mentioned section "(Method for producing 4-trehalosamine)".

There are no particular restrictions on a total content of the compounds (4-trehalosamine), or pharmaceutically acceptable salts thereof, or solvates thereof represented by the above general formula (1) in the above-mentioned microorganism protective agent and the total content can be appropriately selected depending on a purpose. The microorganism protective agent may be the compounds themselves represented by the above general formula (1).

The microorganism protective agent can prevent or inhibit damage or death of microorganisms upon freezing or freeze-drying of the microorganisms. Therefore, when the microorganism protective agent is applied at the time of freezing or freeze-drying of microorganisms, the effect of freezing or freeze-drying is reduced. As a result, the viability of the microorganisms after storage in the frozen or freeze-dried state is higher than when the microorganism protective agent is not applied.

The conditions for performing the freezing can be the same as the conditions for performing the freezing described in the above section "<<Protein protective agent>>", except that "protein" is changed to "microorganism".

- Other components -

Other components in the above-mentioned microorganism protective agent are not particularly limited as long as the components do not impair the effects of the present invention, and can be appropriately selected depending on a purpose. Examples of other components include addition agents described in "- Formulation form -" in "<Composition for blood glucose level control>", which will be described later.
These may be used alone or in combination of two or more.
The content of above-mentioned other components in the microorganism protective agent is not particularly limited as long as the content does not impair the effects of the present invention, and can be appropriately selected depending on a purpose.

-Use-

The microorganism protective agent contains the compounds (4-trehalosamine), or pharmaceutically acceptable salts thereof, or solvates thereof represented by the above general formula (1). Therefore, by being frozen or freeze-dried together with various microorganisms, the microorganism protective agent can easily protect microorganisms (prevent or inhibit damage or death of microorganisms) and can be suitably used for reagents for protecting microorganisms as well as used in methods for protecting and storing microorganism in clinical and research settings. Thus, the present invention also relates to a method for protecting microorganism characterized by containing the above-mentioned microorganism protective agent, a method for storing microorganism characterized by containing the above-mentioned microorganism protective agent, and a microorganism-containing composition characterized by containing an arbitrary microorganism and the above-mentioned microorganism protective agent.
There are no particular restrictions on microorganisms to which the above-mentioned microorganism protective agent is applied (protection targets), and microorganisms can be appropriately selected depending on a purpose. Examples of the microorganisms include: microorganisms belonging to the genus Escherichia (for example, Escherichia coli), microorganisms belonging to the genus Saccharomyces (for example, Saccharomyces cerevisiae), microorganisms belonging to the genus Propionibacterium (for example, Propionibacterium freudenreichii), microorganisms belonging to the genus Bacillus (for example, Bacillus subtilis), microorganisms belonging to the genus Penicillium (for example, Penicillium roqueforti), microorganisms belonging to the genus Bifidobacterium (for example, Bifidobacterium bifidum, Bifidobacterium breve, Bifidobacterium longum subsp. infantis, Bifidobacterium longum, and Bifidobacterium adolescentis), microorganisms belonging to the genus Lactobacillus (for example, Lactobacillus casei), microorganisms belonging to the genus Streptomyces (for example, Streptomyces griseus and Streptomyces kanamyceticus), and microorganisms belonging to the genus Mycobacterium (for example, Mycobacterium smegmatis).
Microorganisms to which the above-mentioned microorganism protective agent is applied (protection targets) may be any one of commercially available products, microorganisms owned by the applicant, and newly separated bacterial strains of microorganisms belonging to the above-mentioned genus.
Examples of Saccharomyces cerevisiae include Saccharomyces cerevisiae F-7 (owned by the Microbial Chemistry Research Foundation) and Saccharomyces cerevisiae JCM 7255 T.
Examples of Escherichia coli include Escherichia coli K-12 (owned by the Microbial Chemistry Research Foundation) and Escherichia coli K-12 (National BioResource Project (NBPR)).

<<pH buffer agent>>

The present inventors have conducted extensive studies and newly found that the compounds represented by general formula (1) exhibit an excellent pH buffer effect.

- Compounds represented by general formula (1) -

The compounds which are contained in the above-mentioned pH buffer agent and represented by the above general formula (1) are the compounds, or pharmaceutically acceptable salts thereof, or solvates thereof where $R^1$ is $-NH_2$.
The compounds of the general formula (1) in which $R^1$ is $-NH_2$ are the compounds represented by the above structural formula (1) in the above-mentioned section "(Method for producing 4-trehalosamine)", and their physicochemical property is also as described in the above-mentioned section "(Method for producing 4-trehalosamine)".
There are no particular restrictions on a total content of the compounds (4-trehalosamine), or pharmaceutically acceptable salts thereof, or solvates thereof represented by the above general formula (1) in the above-mentioned pH

buffer agent and the total content can be appropriately selected depending on a purpose. The pH buffer agent may be the compounds themselves represented by the above general formula (1).

- Other components -

Other components in the above-mentioned pH buffer agent are not particularly limited as long as the components do not impair the effects of the present invention, and can be appropriately selected depending on a purpose. Examples of other components include addition agents described in "- Formulation form -" in "<Composition for blood glucose level control>", which will be described later.

The content of above-mentioned other components in the pH buffer agent is not particularly limited as long as the content does not impair the effects of the present invention, and can be appropriately selected depending on a purpose.

-Use-

The pH buffer agent contains the compounds, or pharmaceutically acceptable salts thereof, or solvates thereof represented by the above general formula (1) and accordingly exhibits an excellent pH buffer effect. Therefore, it can be used as a pH buffer agent for a variety of products in various fields such as household, medical, industrial, and agricultural fields. Specific examples thereof include detergents, emulsifiers, dispersants, penetrants, deinking agents for recycling used paper, agricultural spreading agents, cosmetics, cell culture, microorganism culture, plant cultivation, and therapeutic agents for acidosis. Furthermore, the pH buffer agent can be suitably used as a research reagent.

The pH buffer agent is capable of easily and efficiently inhibiting pH fluctuations. Accordingly, the present invention also relates to a pH adjuster characterized by containing the above-mentioned pH buffer agent and a pH buffer method characterized by using the above-mentioned pH buffer agent.

<Surfactant composition and protein extractant>

The present inventors have conducted extensive studies and newly found that the compounds which are represented by general formula (1) and are novel compounds exhibit an excellent surface activity effect and excellent protein extraction effect.

- Compounds represented by general formula (1) -

The compounds which are contained in the above-mentioned surfactant composition and protein extractant and represented by the above general formula (1) are the compounds (IMCTA-Cn), or pharmaceutically acceptable salts thereof, or solvates thereof where $R^1$ is $-NH(CH_2)_mCH_3$ and the m denotes an integer from 7 to 14. These may be used alone or in combination of two or more kinds.

The compounds of the general formula (1) in which $R^1$ is $-N(CH_2)_mCH_3$ and the m denotes integers from 7 to 14 are the compounds represented by any one of the above structural formulas (2) to (9) (IMCTA-Cn), which are the compounds where $R^2$ is $-NH(CH_2)_mCH_3$ in the general formula (2) in the above-described section "(Novel compounds, or pharmaceutically acceptable salts thereof, or solvates thereof)", and their physico-chemical property is also as described in the above section "(Novel compounds, or pharmaceutically acceptable salts thereof, or solvates thereof)".

There are no particular restrictions on a total content of the compounds (IMCTA-Cn), or pharmaceutically acceptable salts thereof, or solvates thereof represented by the above general formula (1) in the above-mentioned surfactant composition and protein extractant and the total content can be appropriately selected depending on a purpose. The surfactant composition and the protein extractant may be the compounds themselves represented by the above general formula (1).

- Other components -

Other components in the above-mentioned surfactant composition and protein extractant are not particularly limited as long as the components do not impair the effects of the present invention, and can be appropriately selected depending on a purpose. Examples of other components include: known surfactants such as anionic surfactants, cationic surfactants, and amphoteric surfactants; and various addition agents such as oily components, polymer compounds, ultraviolet absorbers, bactericidal agents, excipients, bulking agents, binders, wetting agents, disintegrants, lubricants, dispersants, buffer agents, preserving agents, solubilizing agents, flavoring agents, soothing agents, stabilizers, hydrotrope agents, opalizers, thickeners, zeolites, phosphates, sulfates, sulfites, silicones, enzymes, anti-recontamination agents, bleaching agents, fluorescent agents, fragrances, dyes, and solvents. These may be used alone or in combination of two or more.

The above-mentioned anionic surfactants are not particularly limited but can be appropriately selected depending on a purpose. Examples of the anionic surfactants include carboxylic acid type surfactants, sulfate ester type surfactants, sulfonic acid type surfactants, and phosphate ester type surfactants.

The above-mentioned cationic surfactants are not particularly limited but can be appropriately selected depending on a purpose. Examples of the cationic surfactants include higher amine salts, higher alkyl (or alkenyl) quaternary ammonium salts, and higher alkyl (or alkenyl) pyridinium quaternary ammonium salts.

The above-mentioned amphoteric surfactants are not particularly limited but can be appropriately selected depending on a purpose. Examples of the amphoteric surfactants include alkyl betaine type surfactants, alkylamide betaine type surfactants, imidazoline type surfactants, alkyl amino sulfonic acid type surfactants, alkyl aminocarboxylic acid type surfactants, alkyl amide carboxylic acid type surfactants, amide amino acid type surfactants, and phosphoric acid type surfactants.

The content of above-mentioned other components in the surfactant composition is not particularly limited as long as the content does not impair the effects of the present invention, and can be appropriately selected depending on a purpose.

-Use-

The surfactant composition contains the compounds, or pharmaceutically acceptable salts thereof, or solvates thereof represented by the above general formula (1) and accordingly exhibits an excellent surface activity effect. Therefore, it can be used as various surfactants in household, industrial, and agricultural fields. Specific examples thereof include detergents, emulsifiers, dispersants, oil phase component adjusters, penetrants, deinking agents for recycling used paper, agricultural spreading agents, and cosmetics. Furthermore, the surfactant composition can be suitably used as a research reagent.

<Composition for blood glucose level control>

Trehalose is used as food additives such as a sweetener. However, it has had a problem of causing an elevation in blood glucose levels because it is quickly degraded into glucose by trehalase in bodies. Therefore, it has been desired to provide a composition for blood glucose level control that can also be used as a sweetener without degrading in bodies and elevating blood glucose levels.

In light of this, the present inventors have conducted extensive studies and newly found that the compounds represented by the above general formula (1) are highly safe, do not degrade in bodies, and exhibit excellent blood glucose level control effect.

In the present specification, "blood glucose level control" refers to, for example, inhibition of elevation in blood glucose levels in individuals and maintenance of normal blood glucose levels before elevation of blood glucose levels.

- Compounds represented by general formula (1) -

The compounds which are contained in the above-mentioned composition for blood glucose level control and represented by the above general formula (1) are the compounds, or pharmaceutically acceptable salts thereof, or solvates thereof where $R^1$ is $-NH_2$.

The compounds of the general formula (1) in which $R^1$ is $-NH_2$ are the compounds represented by the above structural formula (1) in the above-mentioned section "(Method for producing 4-trehalosamine)", and their physicochemical property is also as described in the above-mentioned section "(Method for producing 4-trehalosamine)".

There are no particular restrictions on a total content of the compounds (4-trehalosamine), or pharmaceutically acceptable salts thereof, or solvates thereof represented by the above general formula (1) in the above-mentioned composition for blood glucose level control and the total content can be appropriately selected depending on a purpose. The composition for blood glucose level control may be the compounds themselves represented by the above general formula (1).

- Other components -

Other components in the above-mentioned composition for blood glucose level control are not particularly limited as long as the components do not impair the effects of the present invention, and can be appropriately selected depending on a purpose. Examples of other components include addition agents described in the section "-Formulation form -", which will be described later.

The content of above-mentioned other components in the composition for blood glucose level control is not particularly limited as long as the content does not impair the effects of the present invention, and can be appropriately

selected depending on a purpose.

-Use-

The composition for blood glucose level control contains the compounds, or pharmaceutically acceptable salts thereof, or solvates thereof represented by the above general formula (1). Accordingly, the composition for blood glucose level control does not elevate blood glucose levels even when administered to individuals, and can be suitably used for prevention or treatment of diseases caused by elevated blood glucose levels (for example, diabetes, myocardial infarction, liver cirrhosis, arteriosclerosis, and so forth). Thus, the present invention also relates to a pharmaceutical composition characterized by containing the above-mentioned composition for blood glucose level control and a method for preventing or treating diseases caused by elevated blood glucose levels which is characterized by administering the above-mentioned composition for blood glucose level control to individuals.

- Formulation form -

The formulation form of the above-mentioned composition for blood glucose level control is not particularly limited but can be appropriately selected depending on a purpose. Examples of the formulation form include: injection agents mainly used for intravenous injection, intramuscular injection, and so forth; oral preparations such as capsules, tablets, granules, powders, pills, fine granules, syrups, and lozenges; external preparations for parenteral administration such as ointments, eye drops, ear drops, nasal drops, eye ointments, mucocutaneous absorbents, external skin preparations, inhalants, and suppositories; other dry powders; and nebulized aerosol formulations.

The above-mentioned formulation can be produced by conventional methods using addition agents such as excipients, bulking agents, binders, wetting agents, disintegrants, surfactants, lubricants, dispersants, buffer agents, preserving agents, solubilizing agents, preservatives, flavoring agents, soothing agents, and stabilizers.

Specific examples of the addition agent include: dissolving agents or solubilizing agents that can constitute aqueous or dissolve-in-use dosage forms for injection agents, eye drops, ear drops, and nasal drops (distilled water for injection, saline, ethanol, glycerin, propylene glycol, corn oil, sesame oil, and so forth); pH adjusters (inorganic acid addition salts such as trisodium orthophosphate and sodium hydrogen carbonate; organic acid salts such as sodium citrate; organic basic salts such as L-lysine and L-arginine; and so forth); isotonic agents (sodium chloride, glucose, glycerin, and so forth); buffer agents (sodium chloride, benzalkonium chloride, sodium citrate, and so forth); surfactants (sorbitan monooleate, polysorbate 80, and so forth); dispersants (D-mannitol and so forth), stabilizers (antioxidants such as ascorbic acid, sodium sulfite, sodium pyrosulfite; chelating agents such as citric acid, tartaric acid, and so forth).

Examples of the eye ointments, mucocutaneous absorbents, and external skin preparations include formulation components suitable for ointments, creams, and patches (white petrolatum, macrogol, glycerin, liquid paraffin, cotton cloth, and so forth).

Examples of the liquid inhalants include pH adjusters (sodium citrate, sodium hydroxide, and so forth), isotonic agents (sodium chloride, benzalkonium chloride, sodium citrate, and so forth), and buffer agents (sodium chloride, benzalkonium chloride, sodium citrate, and so forth).

Examples of powder inhalants include lactose as a carrier.

Examples of the orally administered agents and suppositories include: excipients (lactose, D-mannitol, corn starch, crystalline cellulose, and so forth); disintegrants (carboxymethyl cellulose, carboxymethylcellulose calcium, and so forth); binders (hydroxypropyl cellulose, hydroxypropyl methylcellulose, polyvinylpyrrolidone, and so forth), lubricants (magnesium stearate, talc, and so forth); coating agents (shellac, hydroxypropyl methylcellulose, white sugar, titanium oxide, and so forth); plasticizers (glycerin, polyethylene glycol, and so forth); and substrates (cocoa butter, polyethylene glycol, hard fat, and so forth).

- Administration -

There are no particular restrictions on the administration route, administration amount, administration timing, and administration target of the composition for blood glucose level control, and these can be appropriately selected depending on a purpose.

The administration route is not particularly limited, but oral administration or parenteral administration can be employed depending on types of pathogens and diseases, natures of administration target individuals, and so forth. Examples of the parenteral one include intravenous injection, intramuscular injection, subcutaneous administration, rectal administration, transdermal administration, topical ocular administration, and transpulmonary administration.

The administration amount is not particularly limited, but can be appropriately selected in consideration of various factors such as usage, type of pathogen, and administration target individual's age, sex, weight, constitution, disease severity, and presence/absence of administration of a medicine or drug containing other ingredients as active

ingredients. For example, oral administration to humans can be performed in the range of 100 mg/kg to 2,000 mg/kg per adult per day, and intravenous administration can be performed in the range of 100 mg/kg to 2,000 mg/kg.

The administration timing is not particularly limited, but can be appropriately selected depending on a purpose.

Animal species which are to be the above-mentioned administration targets are not particularly limited but can be appropriately selected depending on a purpose. Examples of the animal species include humans, monkeys, pigs, cattle, sheep, goats, dogs, cats, mice, rats, and birds, among which humans are most suitable.

<Autophagy inducer>

Autophagy is a sequence of events in which isolation membranes generated from rough endoplasmic reticulum and mitochondrial contact sites in cells surround a substrate, form a double membrane structure called an autophagosome, and fuse with lysosomes (containing hydrolases inside) to be a single membrane structure of autolysosome, and the internal substrate is degraded. Autophagy has been found to play a fundamental role in various physiological and pathological functions of cells. In the course of the research of autophagy, a number of diseases thought to be caused by abnormalities in autophagy have been reported. One of the substrates of autophagy is known to contain $\alpha$-synuclein (Syn), which is known to cause neurodegeneration in Parkinson's disease and so forth, and huntingtin (Htt), which is known to cause Huntington's disease and so forth.

Trehalose is known to have autophagy-inducing activity. However, as mentioned above, it is quickly degraded into glucose by trehalase in bodies and has accordingly had a limit in the amount added as a pharmaceutical product, resulting in a problem of difficulty of exerting sufficient autophagy-inducing activity. Therefore, it has been desired to provide an autophagy inducer that does not degrade in bodies and has excellent autophagy-inducing activity.

In light of this, the present inventors have conducted extensive studies and newly found that the compounds represented by the above general formula (1) are highly safe, do not degrade in bodies, and have excellent autophagy-inducing activity.

The induction of autophagy can be confirmed by conventional methods such as western blotting, immunostaining, and PCR method, using known autophagy markers.

- Compounds represented by general formula (1) -

The compounds which are contained in the above-mentioned autophagy inducer and represented by the above general formula (1) are the compounds, or pharmaceutically acceptable salts thereof, or solvates thereof where $R^1$ is $-NH_2$ or $-NH(CH_2)_mCH_3$ and the m denotes an integer from 7 to 14. These may be used alone or in combination of two or more kinds.

The compounds of the general formula (1) in which $R^1$ is $-NH_2$ are the compounds represented by the above structural formula (1) in the above-mentioned section "(Method for producing 4-trehalosamine)", and their physico-chemical property is also as described in the above-mentioned section "(Method for producing 4-trehalosamine)".

The compounds of the general formula (1) in which $R^1$ is $-N(CH_2)_mCH_3$ and the m denotes integers from 7 to 14 are the compounds represented by any one of the above structural formulas (2) to (9) (IMCTA-Cn), which are the compounds where $R^2$ is $-NH(CH_2)_mCH_3$ in the general formula (2) in the above-described section "(Novel compounds, or pharmaceutically acceptable salts thereof, or solvates thereof)", and their physico-chemical property is also as described in the above section "(Novel compounds, or pharmaceutically acceptable salts thereof, or solvates thereof)".

There are no particular restrictions on a total content of the compounds (4-trehalosamine or IMCTA-Cn), or pharmaceutically acceptable salts thereof, or solvates thereof represented by the above general formula (1) in the above-mentioned autophagy inducer and the total content can be appropriately selected depending on a purpose. The autophagy inducer may be the compounds themselves represented by the above general formula (1).

- Other components -

Other components in the above-mentioned autophagy inducer are not particularly limited as long as the components do not impair the effects of the present invention, and can be appropriately selected depending on a purpose. Examples of other components include addition agents described in "- Formulation form -" in "<Composition for blood glucose level control>" described above.

The content of above-mentioned other components in the autophagy inducer is not particularly limited as long as the content does not impair the effects of the present invention, and can be appropriately selected depending on a purpose.

-Use-

Since the above-mentioned autophagy inducer contains the compounds (4-trehalosamine or IMCTA-Cn), or pharmaceutically acceptable salts thereof, or solvates thereof represented by the above general formula (1), the autophagy inducer is highly safe and does not degrade in bodies, thereby being able to be easily administered over a relatively long period of time. Therefore, the autophagy inducer can effectively prevent or treat various diseases or symptoms caused by autophagy abnormalities. Examples of the diseases or symptoms include neurodegenerative diseases (Alzheimer's disease, Huntington's disease, Parkinson's disease, and so forth), pulmonary diseases, muscle atrophy diseases, myopathies, cardiomyopathies, brain swelling, fatigue, lack of sleep, and cold constitution. Accordingly, the present invention also relates to a composition for preventing or treating diseases caused by autophagy abnormalities, which is characterized by containing the above-mentioned autophagy inducer, and a method for preventing or treating diseases caused by autophagy abnormalities, which is characterized by using the above-mentioned autophagy inducer.

<Mycobacterium stain>

Mycobacteria are Gram-positive bacilli that have anti-acid properties, and their testing is extremely important clinically because there are many pathogens causing severe lesions.
The mycobacterium stain is capable of staining mycobacteria. The mycobacteria commonly have a mycolic acid layer containing trehalose monomycolate (TMM) and trehalose dimycolate (TDM) as cell wall components. The mycobacteria protect themselves by the external side of arabinogalactan on the external side of the cell wall (peptidoglycan), that is, by their outermost peripheral portion. The mycolic acid layer is bound to arabinogalactan inside and mycolic acids are bound to each other with trehalose. The mycobacterium stain is incorporated into the mycolic acid layer of mycobacteria in place of trehalose, thereby being able to suitably staining the mycobacteria.
In this specification, "staining" means visualizing mycobacteria by some method, and is also synonymous with "labeling". Accordingly, the mycobacterium stain is also a mycobacterium labeling agent.
Mycobacterium to be stained with the above-mentioned mycobacterium stain is not particularly limited and can be appropriately selected depending on a purpose. Examples of the mycobacterium include Mycobacterium smegmatis, Mycobacterium avium, Mycobacterium intracellulare, Mycobacterium kansasii, Mycobacterium xenopi, Mycobacterium scrofulaceum, Mycobacterium gordonae, Mycobacterium szulgai, Mycobacterium fortuitum, Mycobacterium chelonae, Mycobacterium haemophilum, Mycobacterium marinum, Mycobacterium shinjukuense, Mycobacterium tuberculosis, Mycobacterium leprae, and Mycobacterium abscessus.

- Compounds represented by general formula (1) -

The compounds which are contained in the above-mentioned mycobacterium stain and represented by the above general formula (1) are the compounds, or pharmaceutically acceptable salts thereof, or solvates thereof where $R^1$ is a substituent represented by the above structural formula (A) or a substituent represented by the above structural formula (B). These may be used alone or in combination of two or more kinds.
Among compounds represented by the above general formula (1), the compounds where $R^1$ is the substituent represented by the above structural formula (A) are compounds represented by the above structural formula (10) (IMCTA-fluorescein), which are the compounds where $R^2$ is the substituent represented by the above structural formula (A) in the general formula (2) in the above-described section "(Novel compounds, or pharmaceutically acceptable salts thereof, or solvates thereof)", and their physico-chemical property is as described in the above-described section "(Novel compounds, or pharmaceutically acceptable salts thereof, or solvates thereof)".
Among compounds represented by the above general formula (1), the compounds where $R^1$ is the substituent represented by the above structural formula (B) are compounds represented by the above structural formula (11) (IMCTA-biotin), which are the compounds where $R^2$ is the substituent represented by the above structural formula (B) in the general formula (2) in the above-described section "(Novel compounds, or pharmaceutically acceptable salts thereof, or solvates thereof)", and their physico-chemical property is as described in the above-described section "(Novel compounds, or pharmaceutically acceptable salts thereof, or solvates thereof)".
There are no particular restrictions on a total content of the compounds (IMCTA-fluorescein or IMCTA-biotin), or pharmaceutically acceptable salts thereof, or solvates thereof represented by the above general formula (1) in the above-mentioned mycobacterium stain and the total content can be appropriately selected depending on a purpose. The mycobacterium stain may be the compounds themselves represented by the above general formula (1).

- Other components -

Other components in the above-mentioned mycobacterium stain are not particularly limited as long as the

components do not impair the effects of the present invention, and can be appropriately selected depending on a purpose. Examples of other components include addition agents described in "- Formulation form -" in "<Composition for blood glucose level control>" described above.

Further, compounds represented by the above structural formula (12) (IMCTA-azide), which are the compounds where $R^3$ is $-N^3$ in the general formula (3) in the above-described section "(Novel compounds, or pharmaceutically acceptable salts thereof, or solvates thereof)", are also capable of staining mycobacteria and therefore, the compounds may be contained as other components of the mycobacterium stain.

The content of above-mentioned other components in the mycobacterium stain is not particularly limited as long as the content does not impair the effects of the present invention, and can be appropriately selected depending on a purpose.

-Use-

The mycobacterium stain contains the compounds (IMCTA-fluorescein or IMCTA-biotin), or pharmaceutically acceptable salts thereof, or solvates thereof represented by the above general formula (1). Therefore, the mycobacterium stain can easily and efficiently stain mycobacteria when being cultured together with the mycobacteria. Accordingly, it can be suitably used for reagents for detection of mycobacteria as well as used in a mycobacterium detection method in clinical and research settings. Thus, the present invention also relates to a mycobacterium detection reagent characterized by containing the above-mentioned mycobacterium stain and a mycobacterium detection method characterized by using the above-mentioned mycobacterium stain.

(Microorganism)

A microorganism according to the present invention is Streptomyces sp. MK186-mF5 (accession number: NITE BP-03495), and is capable of producing 4-trehalosamine.

The Streptomyces sp. MK186-mF5 (strain MK186-mF5) is a bacterium belonging to the genus Streptomyces that was isolated from soil in Japan at Institute of Microbial Chemistry in the Microbial Chemistry Research Foundation.

The partial base sequence (1,446 bp) of the 16S rRNA gene of the strain MK186-mF5 was determined and compared to data of known bacterial strains registered in DNA databases. As a result, the base sequence of the strain MK186-mF5 was a base sequence represented by sequence number: 1, showing high homology with the 16S rRNA gene of Streptomyces cirratus. That is, the homology value with the base sequence of Streptomyces cirratus NRRL B-3250 (accession number: AY999794) was 99.72% (1,439/1,443).

From the above results, the strain MK186-mF5 is considered to belong to the genus Streptomyces. Therefore, Streptomyces sp. MK186-mF5 is determined.

International deposition of the strain MK186-mF5 was applied to Patent Microorganisms Depositary, National Institute of Technology and Evaluation (Room 122, 2-5-8 Kazusa Kamatari, Kisarazu, Chiba, Japan), and was accepted as MK186-mF5 on July 9, 2021.

As is the case with other bacteria, the strain MK186-mF5 tends to change in properties, but the microorganism of the present invention also includes, for example, any mutated strain derived from the strain MK186-mF5 (for example, spontaneous mutant strain and artificial mutant strain that can be obtained by mutation treatment with ultraviolet light, X-rays, radiation, chemicals, and so forth), trait zygote, or gene recombinant which is capable of producing 4-trehalosamine.

EXAMPLES

The present invention will be specifically explained below with reference to production examples, synthesis examples, and test examples, but the present invention is not limited to these production examples, synthesis examples, and test examples. Moreover, in the following production examples, synthesis examples, and test examples, "%" indicates "% by mass" unless otherwise specified.

(Production example 1: 4-trehalosamine production 1)

<Preparation of Ko culture medium>

"Ko culture medium" at pH 7.2 was prepared by adding the following components to sterile water up to the following final concentrations (%).

[Composition]

| | |
|---|---|
| • Soluble starch (manufactured by Koso Chemical Co., Ltd.) | 3% |
| • Ebios (brewer's yeast, manufactured by Asahi Group Foods, Ltd.) | 3% |
| • Dipotassium hydrogen phosphate ($K_2HPO_4$) | 0.3% |
| • Potassium dihydrogen phosphate ($KH_2PO_4$) | 0.1% |
| • Magnesium sulfate heptahydrate ($MgSO_4 \cdot 7H_2O$) | 0.05% |
| • Sodium chloride (NaCl) | 0.2% |
| • Calcium carbonate ($CaCO_3$) | 0.1% |

<Culture process>

30 mL of prepared Ko culture medium was put in a flask (capacity: 100 mL), Streptomyces sp. MK186-mF5 (accession number: NITE BP-03495) was inoculated into the culture medium, and cultured with shaking at 27°C and 220 rpm for 12 days.

(Production example 2: 4-trehalosamine production 2)

<Preparation of modified Ko culture medium 1>

"Modified Ko culture medium 1" at pH 7.2 was prepared by adding the following components to sterile water up to the following final concentrations (%). The "modified Ko culture medium 1" has composition obtained by changing the concentration of soluble starch in the "Ko culture medium" in the production example 1 to twice.

[Composition]

| | |
|---|---|
| • Soluble starch (manufactured by Koso Chemical Co., Ltd.) | 6% |
| • Ebios (brewer's yeast, manufactured by Asahi Group Foods, Ltd.) | 3% |
| • Dipotassium hydrogen phosphate ($K_2HPO_4$) | 0.3% |
| • Potassium dihydrogen phosphate ($KH_2PO_4$) | 0.1% |
| • Magnesium sulfate heptahydrate ($MgSO_4 \cdot 7H_2O$) | 0.05% |
| • Sodium chloride (NaCl) | 0.2% |
| • Calcium carbonate ($CaCO_3$) | 0.1% |

<Culture process>

Culturing was performed in the same manner as that in the production example 1 except that the "Ko culture medium" was changed to the "modified Ko culture medium 1" in the <Culture process> of the production example 1.

(Production example 3: 4-trehalosamine production 3)

<Preparation of modified Ko culture medium 2>

"Modified Ko culture medium 2" at pH 7.2 was prepared by adding the following components to sterile water up to the following final concentrations (%). The "modified Ko culture medium 2" has composition obtained by changing the soluble starch in the "Ko culture medium" in the production example 1 to glucose.

[Composition]

- Glucose (manufactured by FUJIFILM Wako Pure Chemical Corporation) 3%
- Ebios (brewer's yeast, manufactured by Asahi Group Foods, Ltd.) 3%
- Dipotassium hydrogen phosphate ($K_2HPO_4$) 0.3%
- Potassium dihydrogen phosphate ($KH_2PO_4$) 0.1%
- Magnesium sulfate heptahydrate ($MgSO_4 \cdot 7H_2O$) 0.05%
- Sodium chloride (NaCl) 0.2%
- Calcium carbonate ($CaCO_3$) 0.1%

<Culture process>

Culturing was performed in the same manner as that in the production example 1 except that the "Ko culture medium" was changed to the "modified Ko culture medium 2" in the <Culture process> of the production example 1.

(Production example 4: 4-trehalosamine production 4)

<Preparation of modified Ko culture medium 3>

"Modified Ko culture medium 3" at pH 7.2 was prepared by adding the following components to sterile water up to the following final concentrations (%). The "modified Ko culture medium 3" has composition obtained by changing ebios (brewer's yeast) in the "Ko culture medium" in the production example 1 to baker's yeast.

[Composition]

- Soluble starch (manufactured by Koso Chemical Co., Ltd.) 3%
- Dry yeast (baker's yeast, manufactured by Oriental Yeast Co., Ltd.) 3%
- Dipotassium hydrogen phosphate ($K_2HPO_4$) 0.3%
- Potassium dihydrogen phosphate ($KH_2PO_4$) 0.1%
- Magnesium sulfate heptahydrate ($MgSO_4 \cdot 7H_2O$) 0.05%
- Sodium chloride (NaCl) 0.2%
- Calcium carbonate ($CaCO_3$) 0.1%

<Culture process>

Culturing was performed in the same manner as that in the production example 1 except that the "Ko culture medium" was changed to the "modified Ko culture medium 3" in the <Culture process> of the production example 1.

(Production example 5: 4-trehalosamine production 5)

<Preparation of modified Ko culture medium 4>

"Modified Ko culture medium 4" at pH 7.2 was prepared by adding the following components to sterile water up to the following final concentrations (%). The "modified Ko culture medium 4" has composition obtained by further adding zinc chloride to the "Ko culture medium" in the production example 1.

[Composition]

- Soluble starch (manufactured by Koso Chemical Co., Ltd.) 3%
- Ebios (brewer's yeast, manufactured by Asahi Group Foods, Ltd.) 3%
- Dipotassium hydrogen phosphate ($K_2HPO_4$) 0.3%

(continued)

| | |
|---|---|
| • Potassium dihydrogen phosphate ($KH_2PO_4$) | 0.1% |
| • Magnesium sulfate heptahydrate ($MgSO_4 \cdot 7H_2O$) | 0.05% |
| • Sodium chloride (NaCl) | 0.2% |
| • Calcium carbonate ($CaCO_3$) | 0.1% |
| • Zinc chloride ($ZnCl_2$) | 0.011% |

<Culture process>

Culturing was performed in the same manner as that in the production example 1 except that the "Ko culture medium" was changed to the "modified Ko culture medium 4" in the <Culture process> of the production example 1.

(Production example 6: 4-trehalosamine production 6)

<Preparation of modified Ko culture medium 5>

"Modified Ko culture medium 5" at pH 7.2 was prepared by adding the following components to sterile water up to the following final concentrations (%). The "modified Ko culture medium 5" has composition obtained by removing magnesium sulfate heptahydrate from the "Ko culture medium" in the production example 1.

[Composition]

| | |
|---|---|
| • Soluble starch (manufactured by Koso Chemical Co., Ltd.) | 3% |
| • Ebios (brewer's yeast, manufactured by Asahi Group Foods, Ltd.) | 3% |
| • Dipotassium hydrogen phosphate ($K_2HPO_4$) | 0.3% |
| • Potassium dihydrogen phosphate ($KH_2PO_4$) | 0.1% |
| • Sodium chloride (NaCl) | 0.2% |
| • Calcium carbonate ($CaCO_3$) | 0.1% |

<Culture process>

Culturing was performed in the same manner as that in the production example 1 except that the "Ko culture medium" was changed to the "modified Ko culture medium 5" in the <Culture process> of the production example 1.

(Production example 7: 4-trehalosamine production 7)

<Preparation of modified Ko culture medium 6>

"Modified Ko culture medium 6" at pH 7.2 was prepared by adding the following components to sterile water up to the following final concentrations (%). The "modified Ko culture medium 6" has composition obtained by changing the concentration of calcium carbonate in the "Ko culture medium" in the production example 1 to 1/10 times.

[Composition]

| | |
|---|---|
| • Soluble starch (manufactured by Koso Chemical Co., Ltd.) | 3% |
| • Ebios (brewer's yeast, manufactured by Asahi Group Foods, Ltd.) | 3% |
| • Dipotassium hydrogen phosphate ($K_2HPO_4$) | 0.3% |
| • Potassium dihydrogen phosphate ($KH_2PO_4$) | 0.1% |
| • Sodium chloride (NaCl) | 0.2% |

(continued)

| | |
|---|---|
| • Calcium carbonate ($CaCO_3$) | 0.01% |

<Culture process>

Culturing was performed in the same manner as that in the production example 1 except that the "Ko culture medium" was changed to the "modified Ko culture medium 6" in the <Culture process> of the production example 1.

(Production example 8: 4-trehalosamine production 8)

<Preparation of modified Ko culture medium 7>

"Modified Ko culture medium 7" at pH 7.2 was prepared by adding the following components to sterile water up to the following final concentrations (%). The "modified Ko culture medium 7" has composition obtained by changing the dipotassium hydrogen phosphate and potassium dihydrogen phosphate in the "Ko culture medium" in the production example 1 to potassium chloride.

[Composition]

| | |
|---|---|
| • Soluble starch (manufactured by Koso Chemical Co., Ltd.) | 3% |
| • Ebios (brewer's yeast, manufactured by Asahi Group Foods, Ltd.) | 3% |
| • Potassium chloride (KCl) | 0.4% |
| • Sodium chloride (NaCl) | 0.2% |
| • Calcium carbonate ($CaCO_3$) | 0.1% |

<Culture process>

Culturing was performed in the same manner as that in the production example 1 except that the "Ko culture medium" was changed to the "modified Ko culture medium 7" in the <Culture process> of the production example 1.

(Production example 9: 4-trehalosamine production 9)

<Preparation of modified Ko culture medium 8>

"Modified Ko culture medium 8" at pH 7.2 was prepared by adding the following components to sterile water up to the following final concentrations (%).

[Composition]

| | |
|---|---|
| • Soluble starch (manufactured by Koso Chemical Co., Ltd.) | 6% |
| • Ebios (brewer's yeast, manufactured by Asahi Group Foods, Ltd.) | 3% |
| • Potassium chloride (KCl) | 0.4% |
| • Sodium chloride (NaCl) | 0.2% |
| • Calcium carbonate ($CaCO_3$) | 0.01% |
| • Zinc chloride ($ZnCl_2$) | 0.11% |

<Culture process>

Culturing was performed in the same manner as that in the production example 1 except that the "Ko culture medium" was changed to the "modified Ko culture medium 8" in the <Culture process> of the production example 1.

<Purification process>

Most of the 4-trehalosamine obtained by culturing of Streptomyces sp. MK186-mF5 is released into the culture fluid after culturing. Therefore, 8 L of the culture fluid obtained in the above <Culture process> was filtered using filter paper to remove bacterial cells, and filtrate was collected. The pH of the obtained filtrate was adjusted from 8.2 to 2.0 using 1 M aqueous solution of hydrochloric acid, and then the filtrate was applied to 200 mL carbon column (manufactured by FUJIFILM Wako Pure Chemical Corporation), and a passed-through fraction was collected. The carbon column was washed once with 1 L of water, and a wash fraction was also collected. The pass-through fraction and the wash fraction were mixed and applied to 250 mL column (DOWEX™ 50W × 4, 50-100 mesh, strongly acidic cation exchange resin, manufactured by FUJIFILM Wako Pure Chemical Corporation). The column was washed once with 1.25 L of water, and 4-trehalosamine was eluted with 0.1 M ammonium hydroxide ($NH_4OH$). After concentrating the eluate by an evaporator, the concentrated eluate was applied to 1 L column (weakly acidic cation exchange resin FPC3500, manufactured by Organo Corporation). The column was washed once with 5 L of water and eluted with 0.1 M ammonium hydroxide ($NH_4OH$). After concentrating the eluate by an evaporator, the concentrated eluate was centrifuged, and supernatant was vacuum-filtered and freeze-dried to obtain a purified product of 4-trehalosamine.

(Production example 10: 4-trehalosamine production 10)

<Preparation of modified Ko culture medium 9>

"Modified Ko culture medium 9" at pH 7.2 was prepared by adding the following components to sterile water up to the following final concentrations (%).

[Composition]

| | |
|---|---|
| • Soluble starch (manufactured by Koso Chemical Co., Ltd.) | 6% |
| • Ebios (brewer's yeast, manufactured by Asahi Group Foods, Ltd.) | 3% |
| • Potassium chloride (KCl) | 0.7% |
| • Sodium chloride (NaCl) | 0.2% |
| • Zinc chloride ($ZnCl_2$) | 0.0073% |

<Culture process>

Culturing was performed in the same manner as that in the production example 1 except that the "Ko culture medium" was changed to the "modified Ko culture medium 9" in the <Culture process> of the production example 1.

(Production example 11: 4-trehalosamine production 11)

<Culture process>

130 L of culture medium obtained by further adding an antifoaming agent (0.05% KS-496A, Shin-Etsu Chemical Co., Ltd.) to the "modified Ko culture medium 8" prepared in the production example 9 was put in a large culture device (Model MPF-U3 200L, manufactured by Marubishi Bioengineering Co., Ltd.), Streptomyces sp. MK186-mF5 was inoculated into the culture medium, and the culture medium was cultured for 10 days at 27°C with air supply at 100 NL/min and agitation at 370 rpm to obtain the culture product (first culture).

Subsequently, 140 L of culture medium obtained by further adding the antifoaming agent (0.05% KS-496A, Shin-Etsu Chemical Co., Ltd.) to the "modified Ko culture medium 8" prepared in the production example 9 was put in the above-mentioned large culture device, Streptomyces sp. MK186-mF5 was inoculated into the culture medium, and the culture medium was cultured for 10 days at 27°C with air supply at 100 NL/min and agitation at 370 rpm (first culture).

<Purification process>

From the culture fluid obtained in the first culture and second culture, 520 g or more of purified product of 4-trehalosamine was obtained in the same manner as described in the purification process in the production example 9.

- The physico-chemical properties of the compound (4-trehalosamine) represented by structural formula (1) -

The physico-chemical properties of the 4-trehalosamine obtained in production example 9 were as follows. From these, it was determined that the compound produced by culturing Streptomyces sp. MK186-mF5 was 4-trehalosamine having the structure represented by structural formula (1) below. The compounds obtained in production examples 1 to 8, 10, and 11 also had similar physico-chemical properties.

(1) Appearance: white powder
(2) Molecular formula: $C_{12}H_{24}NO_{10}$
In normal state, it is a monohydrate and is represented as $C_{12}H_{24}NO_{10}\cdot H_2O$ in elementary analysis.
(3) High resolution mass spectrometry (HRMS: ESI positive ion mode):

Experimental value: m/z 342.1397 $(M+H)^+$
Calculated value: m/z 342.1400 (as $C_{12}H_{24}NO_{10}$)

(4) Elementary analysis:

Experimental values: C40.27 ± 0.06%, H6.91 ± 0.02%, N3.81 ± 0.01% (n = 3, mean ± S.D.)
Calculated values ($C_{12}H_{24}NO_{10}\cdot$H2O): C40.11% H7.01% N3.90%

(5) Specific rotation: $[\alpha]_D^{25}$ = +147.5° (c = 1.065, methanol)
(6) Infrared absorption spectrum:

The measurements by infrared spectroscopy were as shown in Fig. 1A and below.
$v_{max}$ (KBr) $cm^{-1}$: 3,403, 2,929, 1,607, 1,455, 1,347, 1,149, 1,040, 987, 803, 607

(7) Proton nuclear magnetic resonance spectrum:

The results of measurement at 600 MHz in heavy water at 25°C were as shown in Fig. 1B and below.
$^1H$ NMR (600 MHz, $D_2O$): 5.21 (d, J = 3.7 Hz, 1H, H-1), 5.18 (d, J = 3.7 Hz, 1H, H-1'), 3.84 (t, J = 9.8 Hz, 1H, H-3'), 3.84 (dd, J = 12.0, 2.1 Hz, 1H, H-6), 3.84 (m, 1H, H-6'), 3.82 (m, 1H, H-5'), 3.78 (m, 1H, H-5), 3.75 (dd, J = 12.0, 5.2 Hz, 1H, H-6'), 3.74 (dd, J = 12.0, 5.0 Hz, 1H, H-6), 3.72 (t, J = 9.8 Hz, 1H, H-3), 3.643 (dd, J = 9.8, 3, 7 Hz, 1H, H-2), 3.636 (dd, J = 9.8, 3, 7 Hz, 1H, H-2'), 3.44 (t, J = 9.8 Hz, 1H, H-4'), 2.73 (t, J = 9.8 Hz, 1H, H-4).

(8) Carbon 13 nuclear magnetic resonance spectrum:
The results of measurement at 150 MHz in heavy water at 25°C were as shown in Fig. 1C and below.

$^{13}C$ NMR (150 MHz, $D_2O$): 96.2 (C-1), 96.0 (C-1'), 75.6 (C-5), 75.3 (C-3'), 75.2 (C-3), 74.9 (C-5'), 74.1 (C-2), 73.8 (C-2'), 72.5 (C-4'), 63.6 (C-6), 63.3 (C-6'), 55.3 (C-4).

[Chemical formula 31]

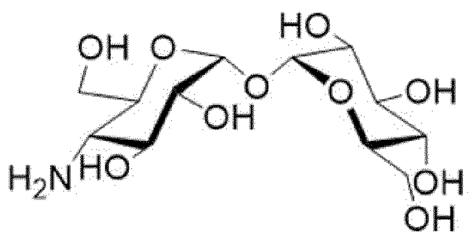

Structural formula (1)
(4-trehalosamine)

- Measurement of purity of 4-trehalosamine -

About 4 mg to 5 mg of the 4-trehalosamine obtained in the <Purification process> of production example 9 or the 4 -trehalosamine obtained in the <Purification process> of production example 11 and about 1 mg of certified reference material DSS-$d_6$ for quantitative NMR (the internal standard) were each accurately weighed out and dissolved in about 0.75 mL of heavy water, thus producing test solutions. Under the following conditions, $^1H$ in the test solutions was measured (n = 3).

[Quantitative NMR analysis conditions]

- Equipment: Varian NMR System 500 [Varian, Inc.] (hydrogen nucleus 500 MHz-NMR)
- Observation offset and observation width: $\delta$2 ppm $\pm$ 20 ppm
- Acquisition time: 4.0 seconds
- Pulse angle: 90°
- Wait time: 60 seconds
- Accumulation time: 16 times
- Measurement temperature: 50°C
- Dummy scan: twice
- Spinning: off
- $^{13}C$ decoupling: on

From the formula below, a quantitative value of 4-trehalosamine was calculated.

[Formula 1]

$$\text{The quantitative value of analyte (\%)} = \frac{\text{Integrated intensity ratio (analyte)}}{\text{Integrated intensity ratio (internal standard)}} \times \frac{\text{Number of protons (internal standard)}}{\text{Numbers of protons (analyte)}} \times \frac{\text{Molecular weight (analyte)}}{\text{Molecular weight (internal standard)}} \times \frac{\text{Amount of sampling (internal standard)}}{\text{Amount of sampling (analyte)}} \times \text{Purity (internal standard)}$$

(In the formula, "analyte" indicates 4-trehalosamine; the "integrated intensity ratio (analyte)" indicates the integrated intensity ratio of the signals originating from the analyte; the "integrated intensity ratio (internal standard)" indicates the integrated intensity ratio of the signals originating from the internal standard material; the "number of protons (internal standard)" is 9; the "number of protons (analyte)" is 1; the molecular weight (analyte) is 341.311; the molecular weight (internal standard) is 224.360; the amount of sampling indicates a value (n = 3) weighed to the order of 0.1 $\mu$g by means of a ultramicro balance (MSE2.7s-000-DM, manufactured by Sartorius AG); and the purity (internal standard) indicates 92.4% of the certified value.)

As a result of the purity measurement by quantitative NMR, the purity of the 4-trehalosamine of production example 9 was 92.7 $\pm$ 0.3%; assuming that all the molecules were monohydrates, the purity of the 4-trehalosamine monohydrates was estimated to be around 97.6%. Similar results were observed for the purity of the 4-trehalosamine of production example 11.

(Synthesis example 1: synthesis of IMCTA-C8)

<Preparation of aqueous solution of 4-trehalosamine>

400 mg of the purified product of 4-trehalosamine from the <Purification process> of production example 10 was dissolved in 2 mL of water to prepare aqueous solution of 4-trehalosamine (1.11 mmol).

<Preparation of octanal solution>

200 mg of octanal was dissolved in 4 mL of 1-propanol (1-PrOH) to prepare octanal solution (1.56 mmol).

<Preparation of aqueous solution of sodium cyanoborohydride>

4 g of sodium cyanoborohydride was dissolved in 40 mL of water to prepare aqueous solution of sodium cyanoborohydride ($NaCNBH_3$, 63.7 mmol).

<Synthesis of IMCTA-C8>

The 4 mL octanal solution was added to the 2 mL aqueous solution of 4-trehalosamine and the resulting solution was left to stand for 2 hours at room temperature (15 ± 2°C). Next, the 40 mL aqueous solution of sodium cyanoborohydride was added thereto and 4 mL of 1-propanol was added for defoaming. They were left to react at room temperature for 12 hours, synthesizing trehalose analog. The synthesized trehalose analog may be referred to as "IMCTA-C8".

Solution containing the synthesized IMCTA-C8 was passed through a 100-mL ODS column (YMC GEL ODS-A, pore size 6 nm and particle size 150 μm, manufactured by YMC Co., Ltd.) to allow IMCTA-C8 to bind to the column, after which the column was washed with 100 mL of water three times to remove watersoluble impurities. After that, the IMCTA-C8 was eluted with 300 mL of methanol. After drying and removing the solvent, HPLC purification was repeated several times with a gradient from water 100% to methanol 100% using a reversed-phase column (Hydrosphere C18, length 250 mm, diameter 20 mm, particle size 5 μm, and pore size 12 nm, manufactured by YMC Co., Ltd.) with water-methanol as solvent. The IMCTA-C8 produced was 129.6 mg and the yield was 25.7%.

[Chemical formula 32]

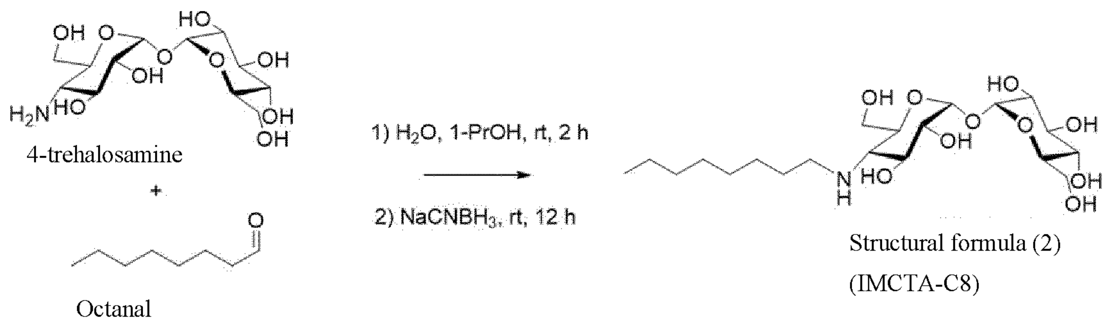


4-trehalosamine

+

Octanal

Structural formula (2)
(IMCTA-C8)

-The physico-chemical properties of the compound (IMCTA-C8) represented by structural formula (2) -

The physico-chemical properties of the IMCTA-C8 obtained were as follows. From these, it was determined that the compound produced in synthesis example 1 was the compound (IMCTA-C8) having the structure represented by the structural formula (2).

(1) Appearance: white powder
(2) Molecular formula: $C_{20}H_{40}NO_{10}$
(3) High resolution mass spectrometry (HRMS: ESI positive ion mode):

Experimental value: m/z 454.2651 $(M+H)^+$
Calculated value: m/z 454.2652 (as $C_{20}H_{40}NO_{10}$)

(4) Specific rotation: $[\alpha]_D^{23}$ = + 153.3° (c = 1.015, methanol)
(5) Infrared absorption spectrum:

The measurements by infrared spectroscopy were as shown in Fig. 2A and below.
$\nu_{max}$ (KBr) $cm^{-1}$: 3,389, 2,925, 2,854, 1,632, 1,462, 1,369, 1,148, 1,044, 992, 803, 606

(6) Proton nuclear magnetic resonance spectrum:

The results of measurement at 600 MHz in deuterated methanol at 25°C were as shown in Fig. 2B and below.
$^1H$ NMR (600 MHz, $CD_3OD$): 5.11 (d, J = 3.8 Hz, 1H), 5.09 (d, J = 3.8 Hz, 1H), 3.74 to 3.85 (m, 6H), 3.65 to 3.69 (m, 2H), 3.48 (dd, J = 9.4, 3.8 Hz, 1H), 3.46 (dd, J = 9.8, 3, 7 Hz, 1H), 3.31 (m, 1H), 2.71 (m, 2H), 2.49 (t, J = 10.0 Hz, 1H), 1.47 (m, 2H), 1.25 to 1.38 (m, 10H), 0.90 (t, J = 6.9 Hz, 3H).

(7) Carbon 13 nuclear magnetic resonance spectrum:
The results of measurement at 150 MHz in deuterated methanol at 25°C were as shown in Fig. 2C and below.

$^{13}C$ NMR (150 MHz, $CD_3OD$): 95.0 × 2, 74.6, 74.0, 73.8, 73.2, 72.8, 72.7, 72.0, 63.7, 62.6, 62.1, 49.4, 33.0,

31.5, 30.7, 30.4, 28.4, 23.7, 14.4.

(Synthesis example 2: synthesis of IMCTA-C9)

Trehalose analog was synthesized in a similar manner to synthesis example 1 except that <Preparation of octanal solution> in synthesis example 1 was changed to <Preparation of nonanal solution> described below and <Synthesis of IMCTA-C8> was changed to <Synthesis of IMCTA-C9> described below. The resulting trehalose analog may be referred to as "IMCTA-C9".

<Preparation of nonanal solution>

200 mg of nonanal was dissolved in 4 mL of 1-propanol to prepare nonanal solution (1.41 mmol).

<Synthesis of IMCTA-C9>

IMCTA-C9 (purified product) was obtained in a similar manner to <Synthesis of IMCTA-C8> in synthesis example 1 except that octanal solution was changed to nonanal solution in <Synthesis of IMCTA-C8> in synthesis example 1. The IMCTA-C9 produced was 138 mg and the yield was 26.6%.

[Chemical formula 33]

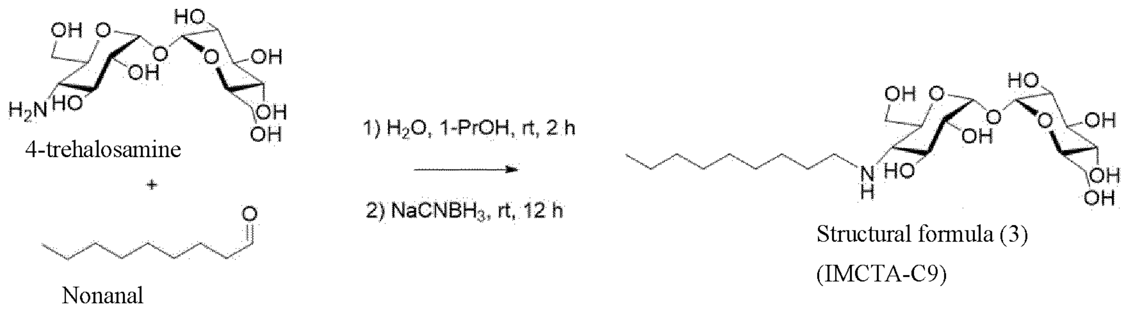


- The physico-chemical properties of the compound (IMCTA-C9) represented by structural formula (3) -

The physico-chemical properties of the IMCTA-C9 obtained were as follows. From these, it was determined that the compound produced in synthesis example 2 was the compound (IMCTA-C9) having the structure represented by the structural formula (3).

(1) Appearance: white powder
(2) Molecular formula: $C_{21}H_{42}NO_{10}$
(3) High resolution mass spectrometry (HRMS: ESI positive ion mode):

Experimental value: m/z 468.2803 $(M+H)^+$
Calculated value: m/z 468.2809 (as $C_{21}H_{42}NO_{10}$)

(4) Specific rotation: $[\alpha]_D^{23}$ = +155.5° (c = 1.025, methanol)
(5) Infrared absorption spectrum:

The measurements by infrared spectroscopy were as shown in Fig. 3A and below.
$\nu_{max}$ (KBr) $cm^{-1}$: 3,370, 2,925, 2,854, 1,643, 1,466, 1,371, 1,148, 1,077, 1,048, 992, 942, 802, 606

(6) Proton nuclear magnetic resonance spectrum:

The results of measurement at 600 MHz in deuterated methanol at 25°C were as shown in Fig. 3B and below.
$^1H$ NMR (600 MHz, $CD_3OD$): 5.11 (d, J = 3.7 Hz, 1H), 5.09 (d, J = 3.7 Hz, 1H), 3.74 to 3.85 (m, 6H), 3.64 to 3.69 (m, 2H), 3.48 (dd, J = 9.5, 3.7 Hz, 1H), 3.46 (dd, J = 9.8, 3, 7 Hz, 1H), 3.31 (m, 1H), 2.71 (m, 2H), 2.49 (t, J = 10.0 Hz, 1H), 1.47 (m, 2H), 1.24 to 1.38 (m, 12H), 0.90 (t, J = 7.1 Hz, 3H).

(7) Carbon 13 nuclear magnetic resonance spectrum:
The results of measurement at 150 MHz in deuterated methanol at 25°C were as shown in Fig. 3C and below.

$^{13}$C NMR (150 MHz, $CD_3OD$): 95.0, 94.9, 74.6, 74.0, 73.8, 73.2, 72.8, 72.7, 72.0, 63.7, 62.6, 62.1, 49.4, 33.1, 31.5, 30.7 × 2, 30.4, 28.4, 23.7, 14.4.

(Synthesis example 3: synthesis of IMCTA-C10)

Trehalose analog was synthesized in a similar manner to synthesis example 1 except that <Preparation of octanal solution> in synthesis example 1 was changed to <Preparation of decanal solution> described below and <Synthesis of IMCTA-C8> was changed to <Synthesis of IMCTA-C10> described below. The resulting trehalose analog may be referred to as "IMCTA-C10".

<Preparation of decanal solution>

200 mg of decanal was dissolved in 4 mL of 1-propanol to prepare decanal solution (1.28 mmol).

<Synthesis of IMCTA-C10>

IMCTA-C10 (purified product) was obtained in a similar manner to <Synthesis of IMCTA-C8> in synthesis example 1 except that octanal solution was changed to decanal solution in <Synthesis of IMCTA-C8> in synthesis example 1. The IMCTA-C10 produced was 172.4 mg and the yield was 32.2%.

[Chemical formula 34]

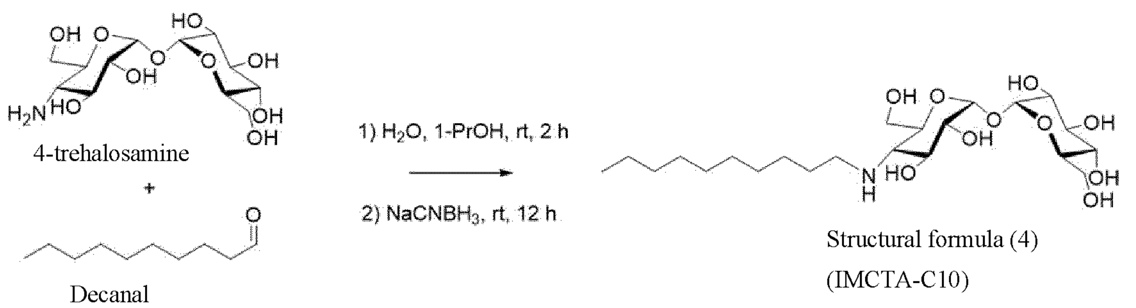


4-trehalosamine
+
Decanal

Structural formula (4)
(IMCTA-C10)

- The physico-chemical properties of the compound (IMCTA-C10) represented by structural formula (4) -

The physico-chemical properties of the IMCTA-C10 obtained were as follows. From these, it was determined that the compound produced in synthesis example 3 was the compound (IMCTA-C10) having the structure represented by the structural formula (4).

(1) Appearance: white powder
(2) Molecular formula: $C_{22}H_{44}NO_{10}$
(3) High resolution mass spectrometry (HRMS: ESI positive ion mode):

Experimental value: m/z 482.2962 $(M+H)^+$
Calculated value: m/z 482.2965 (as $C_{22}H_{44}NO_{10}$)

(4) Specific rotation: $[\alpha]_D^{23}$ = +146.0° (c = 1.010, methanol)
(5) Infrared absorption spectrum:

The measurements by infrared spectroscopy were as shown in Fig. 4A and below.
$\nu_{max}$ (KBr) $cm^{-1}$: 3,372, 2,925, 2,853, 1,645, 1,466, 1,370, 1,148, 1,077, 1,042, 994, 942, 802, 607

(6) Proton nuclear magnetic resonance spectrum:

The results of measurement at 600 MHz in deuterated methanol at 25°C were as shown in Fig. 4B and below. $^1H$ NMR (600 MHz, $CD_3OD$): 5.11 (d, J = 3.8 Hz, 1H), 5.09 (d, J = 3.8 Hz, 1H), 3.74 to 3.85 (m, 6H), 3.64 to 3.69 (m, 2H), 3.48 (dd, J = 9.7, 3.6 Hz, 1H), 3.46 (dd, J = 9.7, 3, 7 Hz, 1H), 3.31 (m, 1H), 2.71 (m, 2H), 2.49 (t, J = 10.0 Hz, 1H), 1.47 (m, 2H), 1.24 to 1.38 (m, 14H), 0.90 (t, J = 7.0 Hz, 3H).

(7) Carbon 13 nuclear magnetic resonance spectrum:
The results of measurement at 150 MHz in deuterated methanol at 25°C were as shown in Fig. 4C and below.

$^{13}C$ NMR (150 MHz, $CD_3OD$): 95.0, 94.9, 74.6, 74.0, 73.8, 73.2, 72.8, 72.7, 72.0, 63.7, 62.6, 62.1, 49.4, 33.1, 31.5, 30.8, 30.7 × 2, 30.5, 28.4, 23.7, 14.4.

(Synthesis example 4: synthesis of IMCTA-C11)

Trehalose analog was synthesized in a similar manner to synthesis example 1 except that <Preparation of octanal solution> in synthesis example 1 was changed to <Preparation of undecanal solution> described below and <Synthesis of IMCTA-C8> was changed to <Synthesis of IMCTA-C11> described below. The resulting trehalose analog may be referred to as "IMCTA-C11".

<Preparation of undecanal solution>

200 mg of undecanal was dissolved in 4 mL of 1-propanol to prepare undecanal solution (1.17 mmol).

<Synthesis of IMCTA-C11>

IMCTA-C11 (purified product) was obtained in a similar manner to <Synthesis of IMCTA-C8> in synthesis example 1 except that octanal solution was changed to undecanal solution in <Synthesis of IMCTA-C8> in synthesis example 1. The IMCTA-C11 produced was 144.0 mg and the yield was 26.2%.

[Chemical formula 35]

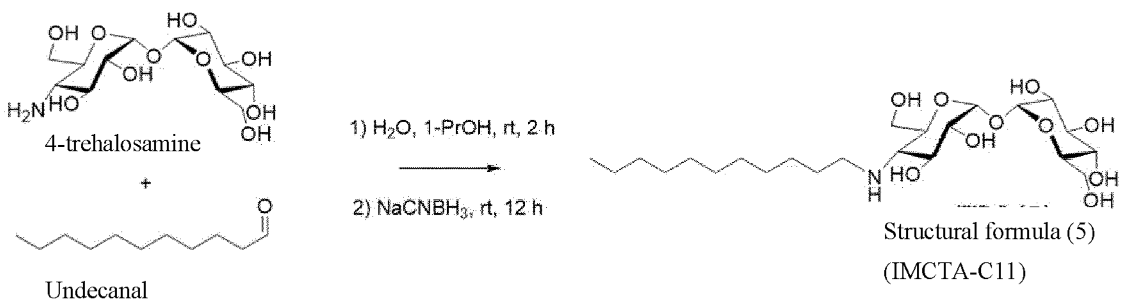


4-trehalosamine

+

Undecanal

Structural formula (5)
(IMCTA-C11)

- The physico-chemical properties of the compound (IMCTA-C11) represented by structural formula (5) -

The physico-chemical properties of the IMCTA-C11 obtained were as follows. From these, it was determined that the compound produced in synthesis example 4 was the compound (IMCTA-C11) having the structure represented by the structural formula (5).

(1) Appearance: white powder
(2) Molecular formula: $C_{23}H_{46}NO_{10}$
(3) High resolution mass spectrometry (HRMS: ESI positive ion mode):

Experimental value: m/z 496.3110 $(M+H)^+$
Calculated value: m/z 496.3122 (as $C_{23}H_{46}NO_{10}$)

(4) Specific rotation: $[\alpha]_D^{23}$ = +141.3° (c = 1.085, methanol)
(5) Infrared absorption spectrum:

The measurements by infrared spectroscopy were as shown in Fig. 5A and below.
$\nu_{max}$ (KBr) $cm^{-1}$: 3,369, 2,924, 2,853, 1,467, 1,370, 1,148, 1,078, 1,048, 993, 939, 840, 802, 607

(6) Proton nuclear magnetic resonance spectrum:

The results of measurement at 600 MHz in deuterated methanol at 25°C were as shown in Fig. 5B and below.
$^1H$ NMR (600 MHz, $CD_3OD$): 5.11 (d, J = 3.7 Hz, 1H), 5.09 (d, J = 3.7 Hz, 1H), 3.74 to 3.85 (m, 6H), 3.64 to 3.69 (m, 2H), 3.48 (dd, J = 9.5, 3.7 Hz, 1H), 3.46 (dd, J = 9.7, 3, 7 Hz, 1H), 3.31 (m, 1H), 2.71 (m, 2H), 2.49 (t, J = 10.0 Hz, 1H), 1.47 (m, 2H), 1.24 to 1.38 (m, 16H), 0.90 (t, J = 7.1 Hz, 3H).

(7) Carbon 13 nuclear magnetic resonance spectrum:
The results of measurement at 150 MHz in deuterated methanol at 25°C were as shown in Fig. 5C and below.

$^{13}C$ NMR (150 MHz, $CD_3OD$): 95.0, 94.9, 74.6, 74.0, 73.8, 73.2, 72.8, 72.7, 72.0, 63.7, 62.6, 62.1, 49.4, 33.1, 31.5, 30.8 × 3, 30.7, 30.5, 28.4, 23.7, 14.4.

(Synthesis example 5: synthesis of IMCTA-C12)

Trehalose analog was synthesized in a similar manner to synthesis example 1 except that <Preparation of octanal solution> in synthesis example 1 was changed to <Preparation of dodecanal solution> described below and <Synthesis of IMCTA-C8> was changed to <Synthesis of IMCTA-C12> described below. The resulting trehalose analog may be referred to as "IMCTA-C12".

<Preparation of dodecanal solution>

200 mg of dodecanal was dissolved in 4 mL of 1-propanol to prepare dodecanal solution (1.09 mmol).

<Synthesis of IMCTA-C12>

IMCTA-C12 (purified product) was obtained in a similar manner to <Synthesis of IMCTA-C8> in synthesis example 1 except that octanal solution was changed to dodecanal solution in <Synthesis of IMCTA-C8> in synthesis example 1. The IMCTA-C12 produced was 96.0 mg and the yield was 17.0%.

[Chemical formula 36]

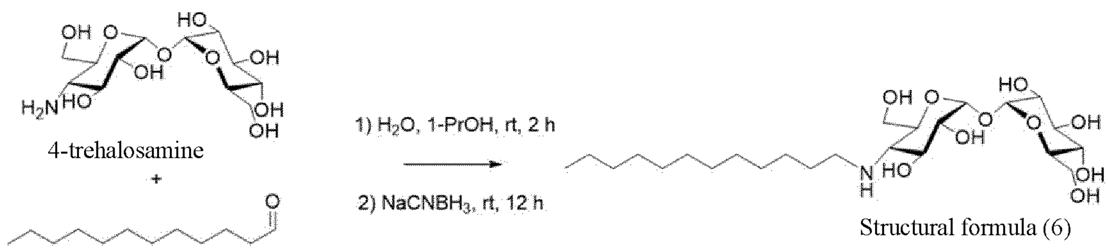


4-trehalosamine
+
Dodecanal

Structural formula (6)
(IMCTA-C12)

- The physico-chemical properties of the compound (IMCTA-C12) represented by structural formula (6) -

The physico-chemical properties of the IMCTA-C12 obtained were as follows. From these, it was determined that the compound produced in synthesis example 5 was the compound (IMCTA-C12) having the structure represented by the structural formula (6).

(1) Appearance: white powder
(2) Molecular formula: $C_{24}H_{48}NO_{10}$
(3) High resolution mass spectrometry (HRMS: ESI positive ion mode):

Experimental value: m/z 510.3278 $(M+H)^+$
Calculated value: m/z 510.3278 (as $C_{24}H_{48}NO_{10}$)

(4) Specific rotation: $[\alpha]_D^{24}$ = +139.7° (c = 1.015, methanol)

(5) Infrared absorption spectrum:

The measurements by infrared spectroscopy were as shown in Fig. 6A and below.
$\nu_{max}$ (KBr) $cm^{-1}$: 3,371, 2,924, 2,853, 1,467, 1,370, 1,149, 1,043, 994, 942, 803, 607

(6) Proton nuclear magnetic resonance spectrum:

The results of measurement at 600 MHz in deuterated methanol at 25°C were as shown in Fig. 6B and below.
$^1H$ NMR (600 MHz, $CD_3OD$): 5.11 (d, J = 3.7 Hz, 1H), 5.09 (d, J = 3.7 Hz, 1H), 3.74 to 3.85 (m, 6H), 3.64 to 3.69 (m, 2H), 3.49 (dd, J = 9.5, 3.7 Hz, 1H), 3.46 (dd, J = 9.8, 3, 7 Hz, 1H), 3.31 (m, 1H), 2.71 (m, 2H), 2.49 (t, J = 10.0 Hz, 1H), 1.47 (m, 2H), 1.22 to 1.41 (m, 18H), 0.90 (t, J = 7.1 Hz, 3H).

(7) Carbon 13 nuclear magnetic resonance spectrum:
The results of measurement at 150 MHz in deuterated methanol at 25°C were as shown in Fig. 6C and below.

$^{13}C$ NMR (150 MHz, $CD_3OD$): 95.0, 94.9, 74.6, 74.0, 73.8, 73.2, 72.8, 72.7, 72.0, 63.7, 62.6, 62.1, 49.4, 33.1, 31.5, 30.8 × 4, 30.7, 30.5, 28.3, 23.7, 14.4.

(Synthesis example 6: synthesis of IMCTA-C13)

Trehalose analog was synthesized in a similar manner to synthesis example 1 except that <Preparation of octanal solution> in synthesis example 1 was changed to <Preparation of tridecanal solution> described below and <Synthesis of IMCTA-C8> was changed to <Synthesis of IMCTA-C13> described below. The resulting trehalose analog may be referred to as "IMCTA-C13".

<Preparation of tridecanal solution>

200 mg of tridecanal was dissolved in 4 mL of 1-propanol to prepare tridecanal solution (1.01 mmol).

<Synthesis of IMCTA-C13>

IMCTA-C13 (purified product) was obtained in a similar manner to <Synthesis of IMCTA-C8> in synthesis example 1 except that octanal solution was changed to tridecanal solution in <Synthesis of IMCTA-C8> in synthesis example 1. The IMCTA-C13 produced was 43.6 mg and the yield was 7.5%.

[Chemical formula 37]

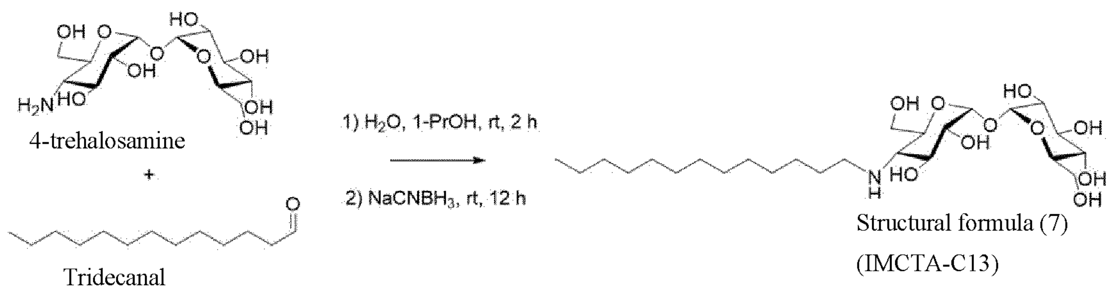


4-trehalosamine

+

Tridecanal

Structural formula (7)
(IMCTA-C13)

- The physico-chemical properties of the compound (IMCTA-C13) represented by structural formula (7) -

The physico-chemical properties of the IMCTA-C13 obtained were as follows. From these, it was determined that the compound produced in synthesis example 6 was the compound (IMCTA-C13) having the structure represented by the structural formula (7).

(1) Appearance: white powder

(2) Molecular formula: $C_{25}H_{50}NO_{10}$

(3) High resolution mass spectrometry (HRMS: ESI positive ion mode):

Experimental value: m/z 524.3428 $(M+H)^+$
Calculated value: m/z 524.3435 (as $C_{25}H_{50}NO_{10}$)

(4) Specific rotation: $[\alpha]_D^{24}$ = +134.6° (c = 1.000, methanol)
(5) Infrared absorption spectrum:

The measurements by infrared spectroscopy were as shown in Fig. 7A and below.
$\nu_{max}$ (KBr) $cm^{-1}$: 3,358, 2,924, 2,853, 1,675, 1,468, 1,204, 1,147, 1,080, 1,043, 994, 837, 801, 721, 605

(6) Proton nuclear magnetic resonance spectrum:

The results of measurement at 600 MHz in deuterated methanol at 25°C were as shown in Fig. 7B and below.
$^1H$ NMR (600 MHz, $CD_3OD$): 5.11 (d, J = 3.7 Hz, 1H), 5.09 (d, J = 3.7 Hz, 1H), 3.74 to 3.85 (m, 6H), 3.64 to 3.69 (m, 2H), 3.48 (dd, J = 9.6, 3.7 Hz, 1H), 3.46 (dd, J = 9.8, 3, 7 Hz, 1H), 3.31 (m, 1H), 2.71 (m, 2H), 2.49 (t, J = 10.0 Hz, 1H), 1.47 (m, 2H), 1.23 to 1.39 (m, 20H), 0.90 (t, J = 7.1 Hz, 3H).

(7) Carbon 13 nuclear magnetic resonance spectrum:
The results of measurement at 150 MHz in deuterated methanol at 25°C were as shown in Fig. 7C and below.

$^{13}C$ NMR (150 MHz, $CD_3OD$): 95.0, 94.9, 74.6, 74.0, 73.8, 73.2, 72.8, 72.7, 72.0, 63.7, 62.6, 62.1, 49.4, 33.1, 31.5, 30.8 × 5, 30.7, 30.5, 28.3, 23.7, 14.4.

(Synthesis example 7: synthesis of IMCTA-C14)

Trehalose analog was synthesized in a similar manner to synthesis example 1 except that <Preparation of octanal solution> in synthesis example 1 was changed to <Preparation of tetradecanal solution> described below and <Synthesis of IMCTA-C8> was changed to <Synthesis of IMCTA-C14> described below. The resulting trehalose analog may be referred to as "IMCTA-C14".

<Preparation of tetradecanal solution>

200 mg of tetradecanal was dissolved in 4 mL of 1-propanol to prepare tetradecanal solution (942 μmol).

<Synthesis of IMCTA-C14>

IMCTA-C14 (purified product) was obtained in a similar manner to <Synthesis of IMCTA-C8> in synthesis example 1 except that octanal solution was changed to tetradecanal solution in <Synthesis of IMCTA-C8> in synthesis example 1. The IMCTA-C14 produced was 370 mg and the yield was 73%.

[Chemical formula 38]

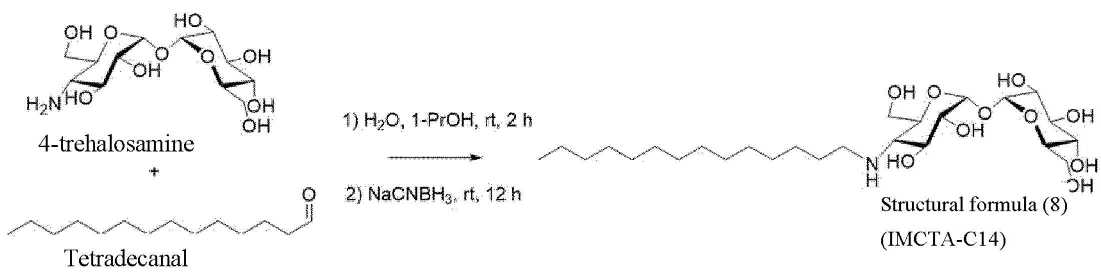


- The physico-chemical properties of the compound (IMCTA-C14) represented by structural formula (8) -

The physico-chemical properties of the IMCTA-C14 obtained were as follows. From these, it was determined that the compound produced in synthesis example 7 was the compound (IMCTA-C14) having the structure represented by the structural formula (8).

(1) Appearance: white powder

(2) Molecular formula: $C_{26}H_{52}NO_{10}$

(3) High resolution mass spectrometry (HRMS: ESI positive ion mode):

Experimental value: m/z 538.3589 $(M+H)^+$
Calculated value: m/z 538.3591 (as $C_{26}H_{52}NO_{10}$)

(4) Specific rotation: $[\alpha]_D^{22}$ = +121.0° (c = 1.045, methanol)

(5) Infrared absorption spectrum:

The measurements by infrared spectroscopy were as shown in Fig. 8A and below.
$\nu_{max}$ (KBr) $cm^{-1}$: 3,372, 2,919, 2,850, 1,468, 1,149, 1,042, 992, 802, 719, 607

s(6) Proton nuclear magnetic resonance spectrum:

The results of measurement at 600 MHz in deuterated methanol at 25°C were as shown in Fig. 8B and below. $^1H$ NMR (600 MHz, $CD_3OD$): 5.11 (d, J = 3.7 Hz, 1H), 5.09 (d, J = 3.7 Hz, 1H), 3.74 to 3.85 (m, 6H), 3.64 to 3.69 (m, 2H), 3.48 (dd, J = 9.5, 3.7 Hz, 1H), 3.46 (dd, J = 9.8, 3, 7 Hz, 1H), 3.31 (m, 1H), 2.71 (m, 2H), 2.49 (t, J = 10.0 Hz, 1H), 1.47 (m, 2H), 1.23 to 1.39 (m, 22H), 0.90 (t, J = 7.1 Hz, 3H).

(7) Carbon 13 nuclear magnetic resonance spectrum:
The results of measurement at 150 MHz in deuterated methanol at 25°C were as shown in Fig. 8C and below.

$^{13}C$ NMR (150 MHz, $CD_3OD$): 95.0, 94.9, 74.6, 74.0, 73.8, 73.2, 72.8, 72.7, 72.0, 63.7, 62.6, 62.1, 49.4, 33.1, 31.5, 30.8 × 6, 30.7, 30.5, 28.4, 23.7, 14.4.

(Synthesis example 8: synthesis of IMCTA-C15)

Trehalose analog was synthesized in a similar manner to synthesis example 1 except that <Preparation of octanal solution> in synthesis example 1 was changed to <Preparation of pentadecanal solution> described below and <Synthesis of IMCTA-C8> was changed to <Synthesis of IMCTA-C15> described below. The resulting trehalose analog may be referred to as "IMCTA-C15".

<Preparation of pentadecanal solution>

200 mg of pentadecanal was dissolved in 4 mL of 1-propanol to prepare pentadecanal solution (883 μmol).

<Synthesis of IMCTA-C15>

IMCTA-C15 (purified product) was obtained in a similar manner to <Synthesis of IMCTA-C8> in synthesis example 1 except that octanal solution was changed to pentadecanal solution in <Synthesis of IMCTA-C8> in synthesis example 1. The IMCTA-C15 produced was 41.6 mg and the yield was 6.8%.

[Chemical formula 39]

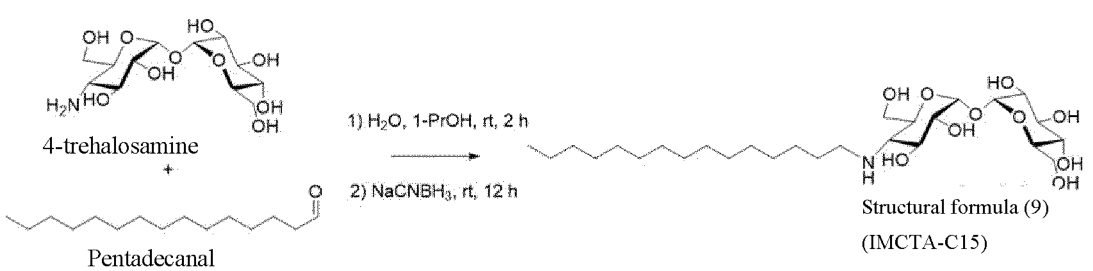


4-trehalosamine
+
Pentadecanal

Structural formula (9)
(IMCTA-C15)

- The physico-chemical properties of the compound (IMCTA-C15) represented by structural formula (9) -

The physico-chemical properties of the IMCTA-C15 obtained were as follows. From these, it was determined

that the compound produced in synthesis example 8 was the compound (IMCTA-C15) having the structure represented by the structural formula (9).

(1) Appearance: white powder
(2) Molecular formula: $C_{27}H_{54}NO_{10}$
(3) High resolution mass spectrometry (HRMS: ESI positive ion mode):

Experimental value: m/z 552.3741 $(M+H)^+$
Calculated value: m/z 552.3748 (as $C_{27}H_{54}NO_{10}$)

(4) Specific rotation: $[\alpha]_D^{24}$ = +124.7° (c = 1.025, methanol)
(5) Infrared absorption spectrum:

The measurements by infrared spectroscopy were as shown in Fig. 9A and below.
$\nu_{max}$ (KBr) $cm^{-1}$: 3,342, 2,918, 2,849, 1,557, 1,469, 1,041, 992, 720

(6) Proton nuclear magnetic resonance spectrum:

The results of measurement at 600 MHz in deuterated DMSO at 25°C were as shown in Fig. 9B and below.
$^1H$ NMR (600 MHz, DMSO-$d_6$): 4.86 (d, J = 3.5 Hz, 1H), 4.84 (d, J = 3.5 Hz, 1H), 4.74 (d, J = 5.2 Hz, 1H), 4.71 (d, J = 4.9 Hz, 1H), 4.69 (d, J = 5.2 Hz, 1H), 4.58 (d, J = 6.5 Hz, 1H), 4.55 (d, J = 6.1, 1H), 4.32 (t, J = 5.9, 1H), 3.57 to 3.65 (m, 3H), 3.50 to 3.56 (m, 3H), 3.43 to 3.49 (m, 2H), 3.23 (m, 2H), 3.12 (ddd, 9.7, 8.8, 5.3 Hz, 1H), 2.62 (m, 1H), 2.53 (m, 1H), 2.25 (t, J = 9.8 Hz, 1H), 1.16 to 1.35 (m, 13H), 0.84 (t, J = 7.0 Hz, 3H).

(7) Carbon 13 nuclear magnetic resonance spectrum:
The results of measurement at 150 MHz in deuterated DMSO at 25°C were as shown in Fig. 9C and below.

$^{13}C$ NMR (150 MHz, $CD_3OD$): 93.1, 92.9, 72.9, 72.4, 72.3, 71.6, 71.5, 71.0, 70.1, 62.1, 60.7 $\times$ 2, 47.5, 31.3, 30.5, 29.1, 29.0 $\times$ 7, 28.7, 26.8, 22.1, 13.9.

(Synthesis example 9: synthesis of IMCTA-fluorescein)

<Preparation of aqueous solution of 4-trehalosamine>

200 mg of the purified product of 4-trehalosamine from the <Purification process> in production example 10 was dissolved in 1 mL of water to prepare aqueous solution of 4-trehalosamine (557 μmol).

<Preparation of 5-carboxyfluorescein N-succinimidyl ester solution>

20 mg of 5-carboxyfluorescein N-succinimidyl ester was dissolved in 200 μL of DMSO to prepare 5-carboxyfluorescein N-succinimidyl ester solution (44.2 μmol).

<Synthesis of IMCTA-fluorescein>

The 1 mL (an excess amount) of aqueous solution of 4-trehalosamine was added to the 200 μL of 5-carboxyfluorescein N-succinimidyl ester solution and the resultant solution was left to stand for 48 hours at room temperature to synthesize trehalose analog. The synthesized trehalose analog may be referred to as "IMCTA-fluorescein".

HPLC purification was repeated twice with a gradient from 0.05% trifluoroacetic acid-containing water 100% to acetone 100% using a reversed-phase column (Hydrosphere C18, length 250 mm, diameter 20 mm, particle size 5 μm, and pore size 12 nm, manufactured by YMC Co., Ltd.) with 0.05% trifluoroacetic acid-containing water - acetone as solvent. The IMCTA-fluorescein produced was 16.5 mg and the yield was 56%.

[Chemical formula 40]

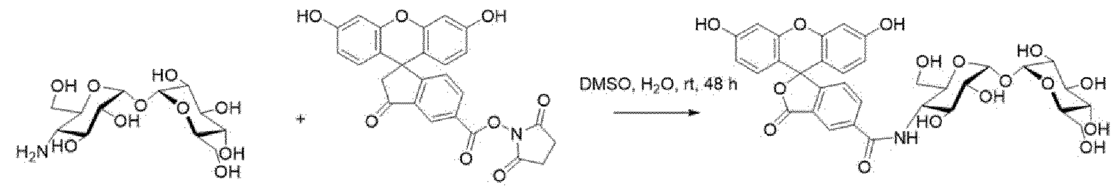


4-trehalosamine

5-carboxyfluorescein N-succinimidyl ester

Structural formula (10) (IMCTA-fluorescein)

- The physico-chemical properties of the compound (IMCTA-fluorescein) represented by structural formula (10) -

The physico-chemical properties of the IMCTA-fluorescein obtained were as follows. From these, it was determined that the compound produced in synthesis example 9 was the compound (IMCTA-fluorescein) having the structure represented by the structural formula (10).

(1) Appearance: powder of turmeric color
(2) Molecular formula: $C_{33}H_{33}NO_{16}Na$
(3) High resolution mass spectrometry (HRMS: ESI positive ion mode):

Experimental value: m/z 722.1698 $(M+H)^+$
Calculated value: m/z 722.1697 (as $C_{33}H_{33}NO_{16}Na$)

(4) Specific rotation: $[\alpha]_D^{23}$ = +89.0° (c = 0.255, methanol)
(5) Infrared absorption spectrum:

The measurements by infrared spectroscopy were as shown in Fig. 10A and below.
$\nu_{max}$ (KBr) $cm^{-1}$: 3,361, 1,593, 1,459, 1,369, 1,308, 1,241, 1,210, 1,173, 1,119, 992, 875, 850, 761, 722, 671

(6) Proton nuclear magnetic resonance spectrum:

The results of measurement at 600 MHz in deuterated methanol at 25°C were as shown in Fig. 10B and below.
$^1H$ NMR (600 MHz, $CD_3OD$): 8.50 (brs, 1H), 8.26 (dd, J = 8.0, 1.5, 1H), 7.33 (d, J = 8.0, 1H), 6.71 (d, J = 1.5, 2H), 6.62 (d, J = 8.0, 2H), 6.56 (dd, J = 8.6, 1.6, 2H), 5.22 (d, J = 3.5, 1H), 5.16 (d, J = 3.7, 1H), 4.11 (t, J = 9.6 Hz, 1H), 4.04 (t, J = 9.6 Hz, 1H), 3.99 (ddd, J = 10.6, 5.3, 2.1, 1H), 3.87 (ddd, J= 10.1, 5.3, 2.2, 1H), 3.83 (t, J = 9.3 Hz, 1H), 3.82 (dd, J = 11.8, 2.2, 1H), 3.70 (dd, J = 11.8, 5.3, 1H), 3.60 to 3.67 (m, 3H), 3.55 (dd, J = 9.7, 3.6, 1H), 3.35 (dd, J = 9.8, 9.1 Hz, 1H).

(7) Carbon 13 nuclear magnetic resonance spectrum:
The results of measurement at 150 MHz in deuterated methanol at 25°C were as shown in Fig. 10C and below.

$^{13}C$ NMR (150 MHz, $CD_3OD$): 170.4, 168.8, 161.7 × 2, 154.3 × 2, 137.7, 135.7, 130.3 × 2, 128.8, 125.9, 125.0, 113.9 × 2, 111.0 × 2, 103.6 × 2, 95.0, 94.9, 74.6, 73.9, 73.7, 73.2 × 2, 72.7, 72.0, 71.9, 63.1, 62.6, 54.1.

(Synthesis example 10: synthesis of IMCTA-biotin)

<Preparation of aqueous solution of 4-trehalosamine>

200 mg of the purified product of 4-trehalosamine from the <Purification process> in production example 10 was dissolved in 1 mL of water to prepare aqueous solution of 4-trehalosamine (557 μmol).

<Preparation of D-biotin N-succinimidyl solution>

20 mg of D-biotin N-succinimidyl was dissolved in 200 μL of DMSO to prepare D-biotin N-succinimidyl solution (58.6 μmol).

<Synthesis of IMCTA-biotin>

The 1 mL (an excess amount) of aqueous solution of 4-trehalosamine was added to the 200 μL of D-biotin N-succinimidyl solution and the resultant solution was left to stand for 48 hours at room temperature to synthesize trehalose analog. The synthesized trehalose analog may be referred to as "IMCTA-biotin".

HPLC purification was repeated twice with a gradient of water 100% or methanol 100% using a reversed-phase column (Hydrosphere C18, length 250 mm, diameter 20 mm, particle size 5 μm, and pore size 12 nm, manufactured by YMC Co., Ltd.) with water-methanol as solvent. The IMCTA-biotin produced was 26 mg and the yield was 78%.

[Chemical formula 41]

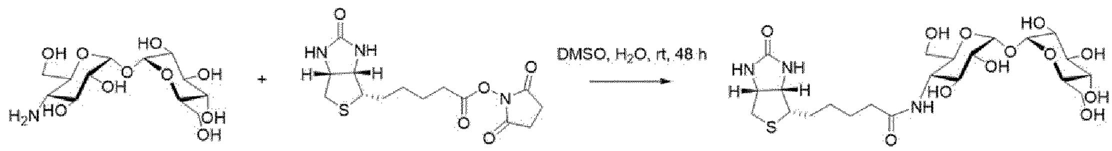


4-trehalosamine D-biotin N-succinimidyl Structural formula (11) (IMCTA-biotin)

- The physico-chemical properties of the compound (IMCTA-biotin) represented by structural formula (11) -

The physico-chemical properties of the IMCTA-biotin obtained were as follows. From these, it was determined that the compound produced in synthesis example 10 was the compound (IMCTA-biotin) having the structure represented by the structural formula (11).

(1) Appearance: white powder
(2) Molecular formula: $C_{22}H_{37}N_3O_{12}SNa$
(3) High resolution mass spectrometry (HRMS: ESI positive ion mode):

Experimental value: m/z 590.1997 $(M+H)^+$
Calculated value: m/z 590.1996 (as $C_{22}H_{37}N_3O_{12}SNa$)

(4) Specific rotation: $[\alpha]_D^{23}$ = +143.5° (c = 1.06, methanol)
(5) Infrared absorption spectrum:

The measurements by infrared spectroscopy were as shown in Fig. 11A and below.
$\nu_{max}$ (KBr) $cm^{-1}$: 3,388, 2,931, 2,348, 1,680, 1,550, 1,464, 1,044, 990, 574

(6) Proton nuclear magnetic resonance spectrum:

The results of measurement at 600 MHz in deuterated methanol at 25°C were as shown in Fig. 11B and below.
$^1H$ NMR (600 MHz, $CD_3OD$): 5.16 (d, J = 3.7 Hz, 1H), 5.11 (d, J = 3.7 Hz, 1H), 4.49 (dd, J = 7.9, 5.0, 1H), 4.31 (dd, J = 7.8, 4.5, 1H), 3.88 (t, J = 9.7 Hz, 1H), 3.71 to 3.85 (m, 5H), 3.68 (dd, J = 11.8, 5.4 Hz, 1H), 3.49 to 3.59 (m, 4H), 3.33 (dd, J = 9.8, 8.8, 1H), 3.21 (m, 1H), 2.93 (dd, J = 12.8, 5.0, 1H), 2.71 (d, J = 12.8 Hz, 1H), 2.27 (t, J = 7.3 Hz, 2H), 1.57 to 1.78 (m, 4H), 1.47 (m, 2H).

(7) Carbon 13 nuclear magnetic resonance spectrum:
The results of measurement at 150 MHz in deuterated methanol at 25°C were as shown in Fig. 11C and below.

$^{13}C$ NMR (150 MHz, $CD_3OD$): 176.9, 166.2, 95.0, 94.9, 74.5, 73.8 × 2, 73.1 × 2, 72.0, 71.8, 63.3, 63.0, 62.6, 61.6, 56.9, 53.2, 41.0, 37.0, 29.8, 29.4, 26.7.

(Synthesis example 11: synthesis of IMCTA-azide)

<Preparation of aqueous solution of 4-trehalosamine>

15 mg of the purified product of 4-trehalosamine from the <Purification process> in production example 10 was dissolved in 150 μL of water to prepare aqueous solution of 4-trehalosamine (41.2 μmol).

<Preparation of 2-azido-1,3-dimethylimidazolinium hexafluorophosphate solution>

100 mg of 2-azido-1,3-dimethylimidazolinium hexafluorophosphate was dissolved in 200 μL of DMSO to prepare 2-azido-1,3-dimethylimidazolinium hexafluorophosphate solution (351 μmol).

<Preparation of N,N-dimethyl-4-aminopyridine solution>

100 mg of N,N-dimethyl-4-aminopyridine (DMAP) was dissolved in 600 μL of 50% by volume aqueous solution of DMSO to prepare N,N-dimethyl-4-aminopyridine solution (819 μmol).

<Synthesis of IMCTA-azide>

The 200 μL of 2-azido-1,3-dimethylimidazolinium hexafluorophosphate solution and the 600 μL of N,N-dimethyl-4-aminopyridine solution were added to and mixed with the 150 μL of aqueous solution of 4-trehalosamine, and the mixture was left to stand at room temperature for 24 hours to synthesize trehalose analog. The synthesized trehalose analog may be referred to as "IMCTA-azide".

After adding 1.2 mL of 8% aqueous solution of sodium hydrogen carbonate ($NaHCO_3$) to solution containing the synthesized IMCTA-azide, HPLC purification was repeated several times with each of a reversed-phase column (Hydrosphere C18, length 250 mm, diameter 20 mm, particle size 5 μm, and pore size 12 nm, manufactured by YMC Co., Ltd.) and a YMC-Pack Polyamine II column (particle size 5 μm and pore size 12 nm, manufactured by YMC Co., Ltd.) with water-methanol as solvent. The IMCTA-azide (4-azide-4-deoxy-trehalose) produced was 9.9 mg and the yield was 61%.

[Chemical formula 42]

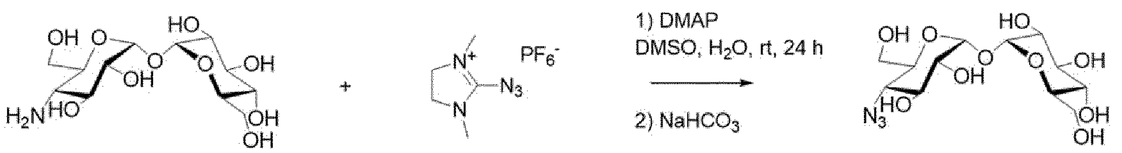


4-trehalosamine

2-azido-1,3-dimethylimidazolinium hexafluorophosphate

Structural formula (12) (IMCTA-azide)

- The physico-chemical properties of the compound (IMCTA-azide) represented by structural formula (12) -

The physico-chemical properties of the IMCTA-azide obtained were as follows. From these, it was determined that the compound produced in synthesis example 11 was the compound (IMCTA-azide) having the structure represented by the structural formula (12).

(1) Appearance: white powder
(2) Molecular formula: $C_{12}H_{21}N_3O_{10}Na$
(3) High resolution mass spectrometry (HRMS: ESI positive ion mode):

Experimental value: m/z 390.1117 $(M+H)^+$
Calculated value: m/z 390.1125 (as $C_{12}H_{21}N_3O_{10}Na$)

(4) Specific rotation: $[\alpha]_D^{21}$ = +184.1° (c = 0.500, methanol)
(5) Infrared absorption spectrum:

The measurements by infrared spectroscopy were as shown in Fig. 12A and below.
$\nu_{max}$ (KBr) $cm^{-1}$: 3,374, 2,934, 2,115, 1,637, 1,264, 1,149, 1,078, 1,045, 992, 935, 842, 801, 567

(6) Proton nuclear magnetic resonance spectrum:

The results of measurement at 600 MHz in deuterated methanol at 25°C were as shown in Fig. 12B and below.
$^1H$ NMR (600 MHz, $CD_3OD$): 5.14 (d, J = 3.8 Hz, 1H), 5.06 (d, J = 3.8 Hz, 1H), 3.92 (t, J = 9.5 Hz, 1H), 3.72 to

3.83 (m, 5H), 3.65 to 3.70 (m, 2H), 3.53 (dd, J = 9.7, 3, 8 Hz, 1H), 3.46 (dd, J = 9.8, 3.8 Hz, 1H), 3.40 (t, J = 10.0 Hz, 1H), 3.33 (t, J = 9.4 Hz, 3H).

(7) Carbon 13 nuclear magnetic resonance spectrum:
The results of measurement at 150 MHz in deuterated methanol at 25°C were as shown in Fig. 12C and below.

$^{13}C$ NMR (150 MHz, $CD_3OD$): 95.2 × 2, 74.5, 73.8, 73.6, 73.3, 73.1, 72.1, 71.9, 63.8, 62.6, 62.3.

(Test example 1: content of 4-trehalosamine in culture fluid)

The content (production) of 4-trehalosamine in the respective culture fluids after 12-day culture resulting from the culture processes of production examples 1 to 10 was determined by two methods: simple evaluation by thin-layer chromatography (TLC) and precise evaluation by liquid chromatograph-mass (LC-MS) analysis.

<TLC analysis>

The respective culture fluids from the culture processes of production examples 1 to 10 were diluted with methanol to 1/10, after which they were centrifuged at 25,300 × g for 5 minutes to obtain their supernatants. 5 μL of each culture supernatant obtained was placed on a 2-mm thick TLC plate (silica gel $60F_{254}$, Merck, Darmstadt, Germany) and dried. Next, the TLC plates were immersed and developed in 60% by volume 1-propanol as developing solvent, and then dried. The dried TLC plates were sprayed with phosphomolybdic acid solution [2.4% disodium molybdate ($Na_2MoO_4$), 1.28% phosphoric acid ($H_3PO_4$), and 5% sulfuric acid ($H_2SO_4$)] and heated on a hot plate to develop color, thus detecting 4-trehalosamine. The results are shown in Fig. 13A. The numbers 1 to 10 on the horizontal axis in Fig. 13A denote the production examples 1 to 10.

<LC-MS analysis>

The respective culture fluids from the culture processes of production examples 1 to 10 were diluted with methanol to 1/10, after which they were centrifuged at 25,300 × g for 5 minutes to obtain their supernatants. The culture supernatants obtained were further diluted with methanol to 1/10 to make test samples, which were used to detect 4-trehalosamine in the respective culture fluids by LC-MS analysis under the analysis conditions below.

Also under those analysis conditions, a calibration curve was created using purified 4-trehalosamine owned by the present inventors. Based on the calibration curve, the concentration (mg/mL) of 4-trehalosamine in the respective culture fluids from the culture processes of production examples 1 to 10 was calculated. The LC-MS analysis was performed with n = 3 (mean ± S.D.). The results are shown in Fig. 13B. The numbers 1 to 10 on the horizontal axis in Fig. 13B denote the production examples 1 to 10.

[LC-MS analysis conditions]

- Equipment: Q-Exactive (manufactured by Thermo Fisher Scientific)
- Column: Hydrophilic Interaction Chromatography column (ACQUITY UPLC, ethylene bridged hybrid (BEH) amide, particle size: 1.7 μm, diameter: 2.1 mm, length: 100 mm, manufactured by Waters, Milford, MA)
- Column temperature: 40°C
- Eluate:

  Liquid A acetonitrile: 5 mM aqueous solution of ammonium acetate (9:1, v/v)
  Liquid B 5 mM aqueous solution of ammonium acetate

- Elution condition: gradient elution of 0 minutes (A:B = 90:10) -> 5.25 minutes (A:B = 40:60) -> 6.25 minutes (A:B = 40:60)
- Flow rate: 0.4 mL/min.
- Retention time: retention time for 4-trehalosamine (3.42 ± 0.2 minutes)
- Ionization: ESI, positive ion mode
- Spray voltage: 4 KV
- Capillary temperature: 350°C
- Capillary voltage: 5 V

- Probe heater temperature: 400°C
- Detection:

Experimental value: m/z 342.1397 $(M+H)^+$
Calculated value: m/z 342.1400 (as $C_{12}H_{24}NO_{10}$)

From the results of Figs. 13A and 13B, it was found that the production of 4-trehalosamine in the culture process was 10 times or higher when the "modified Ko culture medium 8" was used in production example 9 and the "modified Ko culture medium 9" was used in production example 10, compared to when "Ko culture medium" was used in production example 1.

More specifically, as a result of the LC-MS analysis, production of 4.72 g of 4-trehalosamine per liter of "modified Ko culture medium 8" (solid component 96.2 g) was observed for production example 9. Further. For production example 10, production of 5.48 g of 4-trehalosamine per liter of "modified Ko culture medium 9" (solid component 99.1 g) was observed.

(Test example 2: sweetness test)

A sweetness test for 4-trehalosamine was conducted in the following manner.

As test samples, 50 mg/100 μL aqueous solution was prepared with each of the purified product of 4-trehalosamine from the <Purification process> of production example 10, trehalose (manufactured by Hayashibara Co., Ltd.; the same with test examples below), and sucrose.

Twelve professional panelists licked 10 μL of each test sample and scored the sweetness of the test sample, where the sweetness of water alone is "0" and the sweetest is "10". Then, an average of the scores of the professional panelists for each test sample was calculated. The professional panelists were not informed of the material names when they licked the test samples (a blind test). The results are shown in Table 1.

[Table 1]

| | Water | 4-trehalosamine | Trehalose | Sucrose |
|---|---|---|---|---|
| Average | 0 | 7.04 | 5.5 | 9.83 |
| S.D. | 0 | 1.36 | 2.43 | 0.57 |

4-trehalosamine only had a taste of clear sweetness, with no unpleasant flavor or odor.

(Test example 3: properties of IMCTA-C8 to IMCTA-C15)

For the IMCTA-C8, IMCTA-C9, IMCTA-C10, IMCTA-C11, IMCTA-C12, IMCTA-C13, IMCTA-C14, and IMCTA-C15 (IMCTA-Cn) that were obtained in synthesis examples 1 to 8 and for n-dodecyl-β-D-maltoside (DDM) as control, "critical micelle concentration (CMC)", "micelle size", and "Hydrophilic-Lipophilic Balance (HLB) value" were measured or calculated in the following manner. The results are shown in Table 2 below.

<Measurement of critical micelle concentration (CMC)>

CMC was measured with a Detergent Critical Micelle Concentration (CMC) Assay Kit (CMC1000, manufactured by ProFoldin) according to the product protocol.

<Measurement of micelle size>

Micelle sizes were measured by dynamic light scattering with Zetasizer Nano S (manufactured by Malvern Panalytical).

<Measurement of HLB value>

HLB values were calculated by Griffin's method with Marvin Sketch (manufactured by ChemAxon).

[Table 2]

| Compound | CMC (mM) | Micelle size (nm) | HLB value |
|---|---|---|---|
| IMCTA-C8 | 8.02 | 4.3 ± 1.7 | 11.52 |
| IMCTA-C9 | 3.84 | 5.5 ± 1.3 | 11.17 |
| IMCTA-C10 | 2.67 | 5.9 ± 1.8 | 10.85 |
| IMCTA-C11 | 0.96 | 15.9 ± 6.5 | 10.54 |
| IMCTA-C12 | 0.58 | 13.7 ± 6.4 | 10.25 |
| IMCTA-C13 | 0.24 | 17.3 ± 10.9 | 9.98 |
| IMCTA-C14 | 0.11 | 16.5 ± 4.4 | 9.72 |
| IMCTA-C15 | 0.06 | 92.5 ± 49 | 9.47 |
| DDM | 0.23 | 6.1 ± 0.1 | 10.27 |

From the results of Table 2, it was found that IMCTA-Cn has surface activity effect. Also, comparison between IMCTA-C12 and DDM, which have the same fatty chain, showed that IMCTA-C12 had a higher CMC value and a larger micelle size than DDM.

(Test example 4: protein extraction test with IMCTA-C8 to IMCTA-C15)

The protein extraction effect of the IMCTA-Cn obtained in synthesis examples 1 to 8 was evaluated in the following manner.

<Preparation of test samples>

The IMCTA-Cn that were obtained n synthesis examples 1 to 8, and trehalose-C8 (hereinafter, sometimes referred to as "T8") (manufactured by Dojindo Laboratories), trehalose-C12 (hereinafter, sometimes referred to as "T12") (manufactured by Dojindo Laboratories), n-octyl-β-D-glucoside (hereinafter, sometimes referred to as "OG") (manufactured by Dojindo Laboratories), Triton X-100 (hereinafter, sometimes referred to as "TR") (manufactured by MP Biomedicals), Tween 20 (hereinafter, sometimes referred to as "TW") (manufactured by Tokyo Chemical Industry Co., Ltd.), n-dodecyl-β-D-maltoside (DDM) (manufactured by Dojindo Laboratories), 3-[(3-cholamidopropyl) dimethyl ammonio]-1-propanesulfonate (CHAPS; hereinafter, sometimes referred to as "CPS") (manufactured by Dojindo Laboratories), deoxycholic acid sodium (hereinafter, sometimes referred to as "DC") (manufactured by Tokyo Chemical Industry Co., Ltd.), and sodium dodecyl sulfate (SDS) (manufactured by Sigma-Aldrich) were each suspended in tris buffer saline (TBS; manufactured by Takara Bio Inc.), preparing test samples of different concentrations: 0%, 0.005%, 0.01%, 0.02%, 0.05%, 0.1%, 0.2%, 0.5%, and 1%.

<Evaluation of protein extraction efficiency>

- Measurement of protein concentrations -

After human ovarian cancer cells OVK18 (obtained from RIKEN BioResource Research Center (BRC)) were grown in a dish until they were confluent, the culture medium was removed and washed with phosphoric acid buffer saline (PBS; manufactured by Takara Bio Inc.) once, and treated with 0.05% trypsin-EDTA (manufactured by Thermo Fisher Scientific) for 5 minutes. After the trypsin treatment, DMEM culture medium (Dulbecco's modified Eagle's medium "Nissui" 2 (manufactured by Nissui Pharmaceutical Co., Ltd.) containing 10% fetal bovine serum (FBS; manufactured by Sigma-Aldrich), a 1/100 amount of penicillin-streptomycin-L-glutamine solution (× 100) (manufactured by FUJIFILM Wako Pure Chemical Corporation), and 0.12% sodium hydrogen carbonate (manufactured by FUJIFILM Wako Pure Chemical Corporation); for the test examples below, similar compositions were used for "DMEM culture medium") in an amount (V/V) three times the 0.05% trypsin-EDTA was added to stop the reaction of trypsin. Next, cells were stripped off the dish and collected by repeating pipetting seven times. The collected cells were centrifuged for 3 minutes at 400 × g to precipitate cells, which were then washed with tris buffer saline (TBS) once. The precipitation of cells was measured and they were suspended in TBS at 40 mg/mL. This was dispensed in 100 μL portions, to which the test samples of the different concentrations were added, respectively, and rotated for 1 hour at 4°C and at 40 rpm. These mixed solutions were centrifuged for 5 minutes at 700 × g and the protein concentrations of the supernatants were measured with a

BCA protein assay kit (manufactured by Thermo Fisher Scientific) to evaluate the protein extraction efficiency (mean ± S.D., n = 3). The results are shown in Fig. 14A.

- Polyacrylamide electrophoresis (SDS-PAGE) -

Using the cell supernatant obtained in the <Evaluation of protein extraction efficiency> by means of the test sample that was prepared at 0.05% or 0.5% in the <Preparation of test samples>, 2 × SDS-PAGE sample buffer (125 mM tris (tris (hydroxymethyl) aminomethane), 4% SDS, 10% sucrose, 10% 2-mercaptoethanol (2-ME), and 0.004% bromphenol blue (BPB), pH 6.8) in the same amount as the cell supernatant was added, and the mixture was boiled for 5 minutes to make test sample for SDS-PAGE. This test sample for SDS-PAGE was separated by SDS-PAGE using 12.5% acrylamide gel (manufactured by integrate), and stained with Quick-CBB (manufactured by FUJIFILM Wako Pure Chemical Corporation). The results for the 0.05% test sample are shown in Fig. 14B, and the results for the 0.5% test sample are shown in Fig. 14C.

The results of Figs. 14A to 14C revealed that IMCTA-Cn had the ability to extract protein from cells. IMCTA-C11 to IMCTA-C14 in particular exhibited extraction efficiency comparable to surfactants with high extraction efficiency, such as DDM and Triton X-100.

- Determination of membrane protein extraction efficiency -

Using the gel on which SDS-PAGE had been performed in the - Polyacrylamide electrophoresis (SDS-PAGE) -, transfer to Immobilon-P membrane, PVDF (manufactured by Merck) was made and western blotting was performed. For the primary antibodies, antibodies for β-tubulin (manufactured by Cell Signaling Technology) as control soluble protein, and EGFR and $Na^+/K^+$-ATP ase (both manufactured by Cell Signaling Technology) and TRPV6 (manufactured by Proteintech) as membrane proteins were each left to react at 6°C for 12 hours at 1/1,000. For the secondary antibody, HRP-binding anti-rabbit IgG or HRP-binding anti-mouse IgG (both manufactured by Cell Signaling Technology) was used at 1/5,000 in accordance with the primary antibodies, and proteins were detected with a FastGene Western ECL kit (manufactured by NIPPON Genetics Co, Ltd). The results are shown in Fig. 14D.

From the results of Fig. 14D, the membrane protein extraction efficiencies with the different test samples were substantially the same as the patterns for whole protein and soluble protein tubulin by CBB. For IMCTA-C11, IMCTA-C12, IMCTA-C13, and IMCTA-C14 in particular, high efficiency of membrane protein extraction was observed.

- Determination of denaturation effect -

1 μg equivalent of protein that had been extracted from human ovarian cancer cells OVK18 with the 1% test sample mentioned above was mixed with 200 mM of p-nitrophenylphosphate (pNPP) in TBS containing 20 mM magnesium chloride and 0.5 mM dithiothreitol (DTT), and the mixture was left to react for 1 hour at 37°C. After the reaction, absorbance at 420 nm was measured with a microplate reader (Cytation5, manufactured by Bio Tek Instruments) and CIP activity was measured by the amount of p-nitrophenol as enzyme reaction product (n = 3, mean ± S.D.). The results are shown in Fig. 14E.

From the results of Fig. 14E, it was found that SDS, DC, or OG led to lower activity of CIP and had protein denaturation effect, while the other test samples, including the various types of IMCTA-Cn, did not reduce the activity of CIP and had no general protein denaturation effect. Hence, it was suggested that IMCTA-Cn is a mild surfactant that does not denaturalize protein and can be suitably used for extracting membrane protein while maintaining its functionality, for example.

(Test example 5: starch aging inhibiting effect of 4-trehalosamine)

Using the purified product of 4-trehalosamine obtained in the <Purification process> of production example 10, the starch aging inhibiting effect of 4-trehalosamine was determined by two kinds of method: enzyme assay and dynamic viscoelasticity measurement, as described below.

<Enzyme assay 1>

After the individual test samples were mixed into corn starch gel and left to stand at low temperature for 96 hours, the aging level was evaluated via a degradation experiment using β-amylase. The method described below is an evaluation scheme that is an improvement over the BAP method (K. Kainuma, et al, Journal of the Japanese Society of Starch Science 1981, 28, 235.) and only uses β-amylase.

- Preparation of measurement samples -

The 4-trehalosamine (4TA) obtained in production example 10, and trehalose (TRH), sucrose (SCR), and glycerol (hereinafter, sometimes referred to as "GOL") were each dissolved in water to a final concentration of 10% (w/w), thus preparing aqueous solutions. Into each of the aqueous solutions containing the different sugars or water as control, corn starch (manufactured by FUJIFILM Wako Pure Chemical Corporation) was mixed to a final concentration of 15% (w/w) and suspended uniformly, and the suspension was aliquoted in 33.3 μL volumes to four tubes. Next, all of the tubes were treated on a heat block at 100°C for 5 minutes to gel the solutions. One of the four tubes was taken as 0-hour sample, which was used in "- Determination of starch aging inhibiting effect -" described below. The remaining three tubes were left to stand at 6°C for 24, 48, or 96 hours to make 24-hour, 48-hour, and 96-hour samples, respectively.

- Preparation of active enzyme and inactive enzyme -

33 μg/mL (1.37 units/mL) of β-amylase (barley-derived, manufactured by Sigma-Aldrich) was dissolved in 0.8 M acetic acid-sodium hydroxide (NaOH) buffer (pH 6.0), which was used as active enzyme.

A portion of the active enzyme was aliquoted and treated on a heat block at 100°C for 30 minutes to prepare inactive enzyme.

- Determination of starch aging inhibiting effect -

The 0-hour sample was added with 0.8 M acetic acid-sodium hydroxide (NaOH) buffer (pH 6.0) to a total volume of 0.9 mL soon after gelation. The gel in the buffer was loosened by pipetting and suspended as uniformly as possible, and then it was aliquoted in 0.4 mL volumes to two tubes.

100 μL of the active enzyme was added to one of the tubes and 100 μL of the inactive enzyme was added to the other tube and the tubes were sufficiently agitated on a vortex, after which they were left to react at 37°C for 30 minutes. After the reaction, the tubes were treated at 100°C for 5 minutes to deactivate the enzymes and then sufficiently agitated. 10 μL of each was moved to another tube, into which 40 μL of water and 50 μL of Somogyi solution (manufactured by FUJIFILM Wako Pure Chemical Corporation) were added and treated at 100°C for 15 minutes. After they were cooled to room temperature, 80 μL of each was moved to a 96-well plate and 40 μL of Nelson solution (manufactured by FUJIFILM Wako Pure Chemical Corporation) was added. After 5 minutes, absorbance at 660 nm was measured with a plate reader Cytation 5 (manufactured by Bio Tek Instruments), and the absorbance value of the sample with the active enzyme minus the absorbance value of the sample with the inactive enzyme as background was determined.

Quantification (absorbance measurement) of enzyme reaction and sugar were performed for the 24-hour, the 48-hour, or the 96-hour sample in a similar manner to the quantification (absorbance measurement) of enzyme reaction and sugar for the 0-hour sample except that the measurement sample was changed to the 24-hour sample, the 48-hour sample, or the 96-hour sample in the quantification (absorbance measurement) of enzyme reaction and sugar for the 0-hour sample.

With 100% degradation efficiency being the absorbance value of the sample with the active enzyme minus the absorbance value of the sample with the inactive enzyme for the 0-hour sample, the respective degradation efficiencies of 24-hour sample, 48-hour sample, and 96-hour sample were calculated based on the absorbance value of the sample with the active enzyme minus the absorbance value of the sample with the inactive enzyme as background for those samples, and the aging level of starch was evaluated (n = 5, mean ± S.D.). The results are shown in Fig. 15A.

The results of Fig. 15A showed that the corn starch gel containing 4-trehalosamine (4TA) had significantly high rate of degradation by β-amylase at and after 24 hours compared to the other gels; 4-trehalosamine (4TA) had high aging inhibiting effect compared to trehalose (TRH), sucrose (SCR), and glycerol (GCL).

<Enzyme assay 2>

Starch aging inhibiting effect was determined in a similar manner to the <Enzyme assay 1> except that the final concentrations of the aqueous solution of 4-trehalosamine (4TA), the aqueous solution of trehalose (TRH), the aqueous solution of sucrose (SCR), and the aqueous solution of glycerol (GOL) were changed from 10% (w/w) to 2% (w/w), 5% (w/w), or 15% (w/w) in "- Preparation of measurement samples -" in the <Enzyme assay 1>. The results for the 2% (w/w) final concentration of the aqueous solutions of the different sugars are shown in Fig. 15B; the results for the 5% (w/w) final concentration of the aqueous solutions of the different sugars are shown in Fig. 15C; and the results for the 15% (w/w) final concentration of the aqueous solutions of the different sugars are shown in Fig. 15D.

The results of Figs. 15B to 15D showed that the corn starch gel with the final concentration of the aqueous solution of 4-trehalosamine being 5% to 15% maintained β-amylase degradation rate at 96 hours significantly high compared to the other gels; this corn starch gel had even stronger effect of starch aging inhibition.

<Enzyme assay 3>

Starch aging inhibiting effect was determined in a similar manner to the <Enzyme assay 1> except that the "-Preparation of measurement samples -" and "-Determination of starch aging inhibiting effect -" in the <Enzyme assay 1> were changed to the methods below.

- Preparation of measurement samples -

The 4-trehalosamine (4TA) obtained in production example 10, and trehalose (TRH), sucrose (SCR), and glycerol (GOL) were each dissolved in water to a final concentration of 10% (w/w) or 15% (w/w), thus preparing aqueous solutions. Into each of the aqueous solutions containing the different sugars or water as control, potato starch (manufactured by Yoshida Pharmaceutical Company Limited) was mixed to a final concentration of 15% (w/w) and suspended uniformly, and the suspension was aliquoted in 33.3 μL volumes to three tubes. Next, all of the tubes were treated on a heat block at 100°C for 5 minutes to gel the solutions. One of the three tubes was taken as 0-hour sample, which was used in "- Determination of starch aging inhibiting effect - " described below. The remaining two tubes were left to stand at 6°C for 48 or 96 hours to make 48-hour and 96-hour samples, respectively.

- Determination of starch aging inhibiting effect -

Starch aging inhibiting effect was determined in a similar manner to the "-Determination of starch aging inhibiting effect -" of the <Enzyme assay 1> except that measurement of absorbance at 660 nm was changed to measurement of absorbance at 620 nm in the "- Determination of starch aging inhibiting effect -" of the <Enzyme assay 1>, and that quantification (absorbance measurement) of enzyme reaction and sugar was performed for the 0-hour, 48-hour, and 96-hour samples in the quantification (absorbance measurement) of enzyme reaction and sugar. The results for the 10% (w/w) final concentration of the aqueous solutions of the different sugars are shown in Fig. 15E, and the results for the 15% (w/w) final concentration of the different sugars are shown in Fig. 15F.

The results of Figs. 15E and 15F showed that the potato starch gel containing 4-trehalosamine (4TA) had significantly high rate of degradation by β-amylase at and after 48 hours compared to the other gels; 4-trehalosamine (4TA) had high aging inhibiting effect compared to trehalose (TRH), sucrose (SCR), and glycerol (GCL).

<Enzyme assay 4>

Starch aging inhibiting effect was determined in a similar manner to the <Enzyme assay 3> except that potato starch was changed to rice flour (manufactured by OSAWA JAPAN) in the "- Preparation of measurement samples -" of the <Enzyme assay 3>. The results for the 10% (w/w) final concentration of the different sugars are shown in Fig. 15G, and the results for the 15% (w/w) final concentration of the different sugars are shown in Fig. 15H.

The results of Figs. 15G and 15H showed that the rice flour gel containing 4-trehalosamine (4TA) had significantly high rate of degradation by β-amylase at and after 48 hours compared to the other gels; 4-trehalosamine (4TA) had high aging inhibiting effect compared to trehalose (TRH), sucrose (SCR), and glycerol (GCL).

<Enzyme assay 5>

Starch aging inhibiting effect was determined in a similar manner to the <Enzyme assay 3> except that potato starch was changed to wheat flour (organic wheat flour, from Kanazawa Daichi) in the "- Preparation of measurement samples -" of the <Enzyme assay 3>. The results for the 10% (w/w) final concentration of the different sugars are shown in Fig. 15I, and the results for the 15% (w/w) final concentration of the different sugars are shown in Fig. 15J.

The results of Figs. 15I and 15J showed that the wheat flour gel containing 4-trehalosamine (4TA) had significantly high rate of degradation by β-amylase at and after 48 hours compared to the other gels; 4-trehalosamine (4TA) had high aging inhibiting effect compared to trehalose (TRH), sucrose (SCR), and glycerol (GCL).

<Dynamic viscoelasticity measurement>

In 20-mL glass vials, corn starch (manufactured by FUJIFILM Wako Pure Chemical Corporation) was mixed with each of the 10% aqueous solution of 4-trehalosamine (4TA), 10% aqueous solution of trehalose (TRH), 10% aqueous solution of sucrose (SCR), and 10% aqueous solution of glycerol (GOL), which are the test samples, or water as control to a final concentration of 15% (w/w) and uniformly suspended, and the suspensions were autoclaved at 121°C for 20 minutes to gel them. For each of them, 0-hour sample and 96-hour sample were prepared.

After cooling them to room temperature, dynamic viscoelasticity measurement was performed on the 0-hour

sample within 4 hours. The 96-hour sample was left to stand at 4°C for 96 hours and then subjected to dynamic viscoelasticity measurement.

The dynamic viscoelasticity measurement was performed using a MCR300 rheometer (manufactured by Physica, Stuttgart, Germany) under the condition of the gap of a 25-mm parallel plate measurement tool being 1.0 mm. Under the conditions of a fixed frequency of 1.0 Hz, a strain amplitude of 1%, and 25°C, a storage modulus (G') and a loss modulus (G") were measured and a loss tangent (tanδ = G"/G') was calculated. The results are shown in Fig. 15K. In Fig. 15K, the value of tanδ is indicated on the vertical axis (n = 5, mean ± S.D.).

From the results of Fig. 15K, the corn starch gel containing 4-trehalosamine (4TA) exhibited little change or a slight increase in the loss tangent value after 96 hours. In contrast, the corn starch gels containing the other sugars led to reduced loss tangent values as a result of increased elasticity. This demonstrated that 4-trehalosamine (4TA) has high aging inhibiting effect compared to trehalose (TRH), sucrose (SCR), and glycerol (GCL).

(Test example 6-1: protein (CIP) protection effect of 4-trehalosamine)

Test samples 1 to 12 containing bovine alkaline phosphatase (CIP) were prepared as follows and protein-protective activity of various kinds of added sugar under freeze-dried condition was evaluated.

<Preparation of test samples>

Test sample 1. Enzyme solution

After bovine alkaline phosphatase (10 units/μL, manufactured by New England Biolabs) was diluted to 1/40 with TBS (manufactured by Takara Bio Inc.), it was subjected to dialysis for 2 hours at 4°C in TBS of an amount 1,000 times the diluted enzyme solution to remove glycerol. 40 μL of TBS was added to and mixed with 40 μL of the enzyme solution after dialysis and the mixture was left to stand for 20 hours at 4°C, preparing enzyme solution.

Test sample 2. Freeze-thaw product of enzyme

Enzyme solution was prepared in a similar manner to test sample 1 and frozen at -80°C for 20 hours, after which freeze-thaw product of enzyme was prepared.

Test sample 3. Freeze-dried product of enzyme

Enzyme solution was prepared in a similar manner to test sample 1 and frozen at -80°C for 10 minutes. It was then freeze-dried for 20 hours with a freeze dryer (FLEXI-DRY, manufactured by FTS systems INC., Warminster) connected to a vacuum pump (P65D, manufactured by PHIL Sato Vac INC.), preparing freeze-dried product of enzyme.

Test sample 4. Freeze-dried product of enzyme with 1% TRH

After bovine alkaline phosphatase (10 units/μL, manufactured by New England Biolabs) was diluted to 1/40 with TBS (manufactured by Takara Bio Inc.), it was subjected to dialysis for 2 hours at 4°C within TBS of an amount 1,000 times the diluted enzyme solution to remove glycerol. 40 μL of TBS containing 2% trehalose (TRH) was added to and mixed with 40 μL of the enzyme solution after dialysis and the mixture was frozen at -80°C for 10 minutes. It was then freeze-dried for 20 hours with a freeze dryer (FLEXI-DRY, manufactured by FTS systems INC., Warminster) connected to a vacuum pump (P65D, manufactured by PHIL Sato Vac INC.), preparing freeze-dried product of enzyme with 1% TRH.

Test sample 5. Freeze-dried product of enzyme with 3% TRH

Freeze-dried product of enzyme with 3% TRH was prepared in a similar manner to the preparation method of test sample 4 except that the final concentration of trehalose was changed to 3% in the preparation method of test sample 4.

Test sample 6. Freeze-dried product of enzyme with 1% 4TA

Freeze-dried product of enzyme with 1% 4TA was prepared in a similar manner to the preparation method of test sample 4 except that trehalose was changed to 4-trehalosamine (4TA) in the preparation method of test sample 4.

Test sample 7. Freeze-dried product of enzyme with 3% 4TA

Freeze-dried product of enzyme with 3% 4TA was prepared in a similar manner to the preparation method of test sample 5 except that trehalose was changed to 4-trehalosamine (4TA) in the preparation method of test sample 5.

Test sample 8. Freeze-dried product of enzyme with 1% MLT

Freeze-dried product of enzyme with 1% MLT was prepared in a similar manner to the preparation method of test sample 4 except that trehalose was changed to maltose (MLT) in the preparation method of test sample 4.

Test sample 9. Freeze-dried product of enzyme with 3% MLT

Freeze-dried product of enzyme with 3% MLT was prepared in a similar manner to the preparation method of test sample 5 except that trehalose was changed to maltose (MLT) in the preparation method of test sample 5.

Test sample 10. Freeze-dried product of enzyme with 1% SCR

Freeze-dried product of enzyme with 1% SCR was prepared in a similar manner to the preparation method of test sample 4 except that trehalose was changed to sucrose (SCR) in the preparation method of test sample 4.

Test sample 11. Freeze-dried product of enzyme with 3% SCR

Freeze-dried product of enzyme with 3% SCR was prepared in a similar manner to the preparation method of test sample 5 except that trehalose was changed to sucrose (SCR) in the preparation method of test sample 5.

Test sample 12. Freeze-dried product of enzyme with 3% GOL

Freeze-dried product of enzyme with 3% GOL was prepared in a similar manner to the preparation method of test sample 5 except that trehalose was changed to glycerol (GOL) in the preparation method of test sample 5.

<Evaluation of CIP-protective activity>

80 μL of water was added to each of the freeze-dried products of test samples 3 to 12 and the mixture was diluted to 1/100 with TBS. Test samples 1 and 2 were diluted to 1/100 with TBS. 10 μL of each of the diluted test samples 1 to 12 and 20 μL of 0.1 M p-nitrophenylphosphate (pNPP)-TBS solution were added to 70 μL of TBS, and enzyme reaction was left to take place at 37°C for 1 hour. After the reaction, absorbance at 420 nm was measured with a microplate reader (Cytation5, manufactured by Bio Tek Instruments) and CIP activity was measured by the amount of p-nitrophenol as enzyme reaction product (n = 3, mean ± S.D.). The results are shown in Fig. 16A.

From the results of Fig. 16A, enzyme activity significantly dropped with test sample 2 (freeze-thaw product of enzyme) and test sample 12 (freeze-dried product of enzyme with 3% GOL). In contrast, good activity was maintained with the samples that were freeze-dried with 3% 4-trehalosamine, trehalose, maltose, or sucrose added. The protection effect of 4-trehalosamine, in particular, was high compared to the other sugars.

(Test example 6-2: protein (ADH) protection effect of 4-trehalosamine)

Test samples 1 to 12 containing yeast alcohol dehydrogenase (ADH) were prepared as follows and protein-protective activity of various kinds of added sugar under freeze-dried condition was evaluated.

<Preparation of test samples>

Test sample 1. Enzyme solution

Yeast alcohol dehydrogenase (manufactured by Oriental Yeast Co., Ltd.) was dissolved in water at 1,000 units/mL and then diluted to 6 units/mL with TBS (manufactured by Takara Bio Inc.). 50 μL of TBS was added to and mixed with 50 μL of this enzyme solution and the mixture was left to stand at 4°C for 20 hours, preparing enzyme solution.

Test sample 2. Freeze-thaw product of enzyme

Enzyme solution was prepared in a similar manner to test sample 1 and frozen at -80°C for 20 hours, after which freeze-thaw product of enzyme was prepared.

Test sample 3. Freeze-dried product of enzyme

Enzyme solution was prepared in a similar manner to test sample 1 and frozen at -80°C for 10 minutes. It was then freeze-dried for 20 hours with a freeze dryer (FLEXI-DRY, manufactured by FTS systems INC., Warminster) connected to a vacuum pump (P65D, manufactured by PHIL Sato Vac INC.), preparing freeze-dried product of enzyme.

Test sample 4. Freeze-dried product of enzyme with 1% TRH

Yeast alcohol dehydrogenase (manufactured by Oriental Yeast Co., Ltd.) was dissolved in water at 1,000 units/mL and then diluted to 6 units/mL with TBS (manufactured by Takara Bio Inc.). 50 μL of TBS containing 2% trehalose (TRH) was added to and mixed with 50 μL of this enzyme solution and the mixture was frozen at - 80°C for 10 minutes. It was then freeze-dried for 20 hours with a freeze dryer (FLEXI-DRY, manufactured by FTS systems INC., Warminster) connected to a vacuum pump (P65D, manufactured by PHIL Sato Vac INC.), preparing freeze-dried product of enzyme with 1% TRH.

Test sample 5. Freeze-dried product of enzyme with 3% TRH

Freeze-dried product of enzyme with 3% TRH was prepared in a similar manner to the preparation method of test sample 4 except that the final concentration of trehalose was changed to 3% in the preparation method of test sample 4.

Test sample 6. Freeze-dried product of enzyme with 1% 4TA

Freeze-dried product of enzyme with 1% 4TA was prepared in a similar manner to the preparation method of test sample 4 except that trehalose was changed to 4-trehalosamine (4TA) in the preparation method of test sample 4.

Test sample 7. Freeze-dried product of enzyme with 3% 4TA

Freeze-dried product of enzyme with 3% 4TA was prepared in a similar manner to the preparation method of test sample 5 except that trehalose was changed to 4-trehalosamine (4TA) in the preparation method of test sample 5.

Test sample 8. Freeze-dried product of enzyme with 1% MLT

Freeze-dried product of enzyme with 1% MLT was prepared in a similar manner to the preparation method of test sample 4 except that trehalose was changed to maltose (MLT) in the preparation method of test sample 4.

Test sample 9. Freeze-dried product of enzyme with 3% MLT

Freeze-dried product of enzyme with 3% MLT was prepared in a similar manner to the preparation method of test sample 5 except that trehalose was changed to maltose (MLT) in the preparation method of test sample 5.

Test sample 10. Freeze-dried product of enzyme with 1% SCR

Freeze-dried product of enzyme with 1% SCR was prepared in a similar manner to the preparation method of test sample 4 except that trehalose was changed to sucrose (SCR) in the preparation method of test sample 4.

Test sample 11. Freeze-dried product of enzyme with 3% SCR

Freeze-dried product of enzyme with 3% SCR was prepared in a similar manner to the preparation method of test sample 5 except that trehalose was changed to sucrose (SCR) in the preparation method of test sample 5.

Test sample 12. Freeze-dried product of enzyme with 3% GOL

Freeze-dried product of enzyme with 3% GOL was prepared in a similar manner to the preparation method of test sample 5 except that trehalose was changed to glycerol (GOL) in the preparation method of test sample 5.

<Evaluation of ADH-protective activity>

The enzyme solution from test sample 1, the freeze-thaw product of enzyme from test sample 2, and the freeze-dried products from test samples 3 to 12 were each diluted to 0.012 units/mL with TBS. 0.5 mM β-$NAD^+$ (manufactured by Oriental Yeast Co., Ltd.) and 0.62 M ethanol were added, and enzyme reaction was left to take place at room temperature for 1 hour. After the reaction, absorbance at 340 nm was measured with a microplate reader (Cytation5, manufactured by Bio Tek Instruments) (n = 3, mean ± S.D.). The value of this absorbance at 340 nm was divided by the value of absorbance at 340 nm for the enzyme solution of test sample 1 (control) to determine ADH activity. The results are shown in Fig. 16B.

From the results of Fig. 16B, as with test example 6-1, enzyme activity significantly dropped with test sample 2 (freeze-thaw product of enzyme) and test sample 12 (freeze-dried product of enzyme with 3% GOL). In contrast, good activity was maintained with the samples that were freeze-dried with 1% or 3% 4-trehalosamine, trehalose, maltose, or sucrose added. The protection effect of 4-trehalosamine, in particular, was high compared to the other sugars.

(Test example 7-1: protective effect of 4-trehalosamine on microorganism (baker's yeast))

Using test samples 1 to 8 containing baker's yeast prepared in the following manner, microorganism-protective activity with various kinds of sugar in a freeze-dried condition was evaluated.

<Preparation of test samples>

Test sample 1. Control (NF: Non-freezing)

Frozen stock of baker's yeast (Saccharomyces cerevisiae F-7, owned by the Microbial Chemistry Research Foundation) was inoculated in 10 mL of YPD culture fluid (1% yeast extract, 2% polypeptone, 2% glucose) and cultured at 27°C for 2 days while shaking it at 220 rpm. After culture, it was centrifuged at 1,000 × g for 5 minutes, washed once with 10 mL of water, and then suspended in 10 mL of water, thus obtaining yeast suspension. 50 μL of water was added to and mixed with 50 μL of this yeast suspension and the mixture was left to stand at 4°C for 12 hours, preparing control (NF).

Test sample 2. Freeze-thaw product (FT: Freeze-Thaw) of yeast

Yeast suspension was prepared in a similar manner to test sample 1 and frozen at -80°C for 12 hours, after which freeze-thaw product (FT) of the yeast was prepared.

Test sample 3. Freeze-dried product (FD: Freeze-Dry) of yeast

Yeast suspension was prepared in a similar manner to test sample 1 and frozen at -80°C for 1 hour. It was then freeze-dried for 12 hours with a freeze dryer (FLEXI-DRY, manufactured by FTS systems INC., Warminster) connected to a vacuum pump (P65D, manufactured by PHIL Sato Vac INC.), preparing freeze-dried product (FD) of yeast.

Test sample 4. Freeze-dried product of yeast with TRH

After yeast suspension was prepared in a similar manner to test sample 1, 50 μL of 6% aqueous solution of trehalose was added to and mixed with 50 μL of the yeast suspension, and the mixture was frozen at -80°C for 1 hour. It was then freeze-dried for 12 hours with a freeze dryer (FLEXI-DRY, manufactured by FTS systems INC., Warminster) connected to a vacuum pump (P65D, manufactured by PHIL Sato Vac INC.), preparing freeze-dried product (TRH) of yeast with TRH.

Test sample 5. Freeze-dried product of yeast with 4TA

Freeze-dried product (4TA) of yeast with 4TA was obtained in a similar manner to the preparation method of test sample 4 except that trehalose was changed to 4-trehalosamine (4TA) in the preparation method of test sample 4.

Test sample 6. Freeze-dried product of yeast with MLT

Freeze-dried product (MLT) of yeast with MLT was obtained in a similar manner to the preparation method of test sample 4 except that trehalose was changed to maltose (MLT) in the preparation method of test sample 4.

Test sample 7. Freeze-dried product of yeast with SCR

Freeze-dried product (SCR) of yeast with SCR was obtained in a similar manner to the preparation method of test sample 4 except that trehalose was changed to sucrose (SCR) in the preparation method of test sample 4.

Test sample 8. Freeze-dried product of yeast with GOL

Freeze-dried product (GOL) of yeast with GOL was obtained in a similar manner to the preparation method of test sample 4 except that trehalose was changed to glycerol (GOL) in the preparation method of test sample 4.

<Evaluation of baker's yeast protective activity>

For test samples 1 and 2, serial suspensions were made by diluting them to 1/10 with saline. 100 μL of water was added to each of the freeze-dried products of test samples 3 to 8 and diluted to 1/10 with saline to make their serial suspensions. 60 μL of water was added to 30 μL of each diluted solution.

In a 6-well microplate, 2% Agar-YPD was prepared at 3 mL/well, and 90 μL of each diluted solution was inoculated on the 2% Agar-YPD and cultured at 30°C for two days. The results are shown in Fig. 17A.

Wells in which the number of colonies was measurable were selected and the numbers (relative numbers) of live bacterial cells per well on test samples 1, 2, and 4 to 8 when the number of live bacterial cells per well on test sample 3 (FD) was "1" were calculated in consideration of the dilution concentrations of those test samples. The results of the same are shown in Table 3 below.

[Table 3]

| Test samples | | Numbers of live bacterial cells (relative numbers) |
|---|---|---|
| Test sample 1 | NF | > 100 |
| Test sample 2 | FT | 1 |
| Test sample 3 | FD | 1 |
| Test sample 4 | TRH | 100 |
| Test sample 5 | 4TA | 100 |
| Test sample 6 | MLT | 10 |
| Test sample 7 | SCR | 10 |
| Test sample 8 | GOL | 0 |

From the results of Fig. 17A and Table 3, when comparing test sample 1 (NF) and test sample 3 (FD), a significant decrease in the viability of the bacterial cells of baker's yeast (Saccharomyces cerevisiae) was observed as a consequence of freeze drying. In contrast, it was found that the viability was improved by 10 times or more when adding 6% (final concentration 3%) of sugars other than glycerol. With the addition of 4-trehalosamine, in particular, protective effect on microorganisms was stronger than when maltose or sucrose was added.

(Test example 7-2: protective effect of 4-trehalosamine on microorganism (Escherichia coli))

Using test samples 1 to 8 containing Escherichia coli prepared in the following manner, microorganism-protective activity with various kinds of sugar in a freeze-dried condition was evaluated.

<Preparation of test samples>

Test sample 1. Control (NF: Non-freezing)

Frozen stock of Escherichia coli (Escherichia coli K-12, owned by the Microbial Chemistry Research Foundation) was inoculated in 10 mL of Luria-Bertani (LB) culture medium (manufactured by Difco) and cultured at 37°C for 24 hours while shaking it at 220 rpm. After culture, it was centrifuged at 3,000 × g for 5 minutes, washed once with 10 mL of water, and then suspended in 10 mL of water, thus obtaining Escherichia coli suspension. 50 μL of water was added to and mixed with 50 μL of this Escherichia coli suspension and the mixture was left to stand at 4°C for 12 hours, preparing control (NF).

Test sample 2. Freeze-thaw product (FT) of Escherichia coli

Escherichia coli suspension was prepared in a similar manner to test sample 1 and frozen at -80°C for 12 hours, after which freeze-thaw product (FT) of Escherichia coli was prepared.

Test sample 3. Freeze-dried product (FD) of Escherichia coli

Escherichia coli suspension was prepared in a similar manner to test sample 1 and frozen at -80°C for 1 hour. It was then freeze-dried for 12 hours with a freeze dryer (FLEXI-DRY, manufactured by FTS systems INC., Warminster) connected to a vacuum pump (P65D, manufactured by PHIL Sato Vac INC.), preparing freeze-dried product (FD) of Escherichia coli.

Test sample 4. Freeze-dried product of Escherichia coli with TRH

After Escherichia coli suspension was prepared in a similar manner to test sample 1, 50 μL of 6% aqueous solution of trehalose was added to and mixed with 50 μL of the Escherichia coli suspension, and the mixture was frozen at -80°C for 1 hour. It was then freeze-dried for 12 hours with a freeze dryer (FLEXI-DRY, manufactured by FTS systems INC., Warminster) connected to a vacuum pump (P65D, manufactured by PHIL Sato Vac INC.), preparing freeze-dried product (TRH) of Escherichia coli with TRH.

Test sample 5. Freeze-dried product of Escherichia coli with 4TA

Freeze-dried product (4TA) of Escherichia coli with 4TA was obtained in a similar manner to the preparation method of test sample 4 except that trehalose was changed to 4-trehalosamine (4TA) in the preparation method of test sample 4.

Test sample 6. Freeze-dried product of Escherichia coli with MLT

Freeze-dried product (MLT) of Escherichia coli with MLT was obtained in a similar manner to the preparation method of test sample 4 except that trehalose was changed to maltose (MLT) in the preparation method of test sample 4.

Test sample 7. Freeze-dried product of Escherichia coli with SCR

Freeze-dried product (SCR) of Escherichia coli with SCR was obtained in a similar manner to the preparation method of test sample 4 except that trehalose was changed to sucrose (SCR) in the preparation method of test sample 4.

Test sample 8. Freeze-dried product of Escherichia coli with GOL

Freeze-dried product (GOL) of Escherichia coli with GOL was obtained in a similar manner to the preparation method of test sample 4 except that trehalose was changed to glycerol (GOL) in the preparation method of test sample 4.

<Evaluation of Escherichia coli protective activity>

For test samples 1 and 2, serial suspensions were made by diluting them to 1/10 with water. 100 μL of water was added to each of the freeze-dried products of test samples 3 to 8 and diluted to 1/10 with water to make their serial suspensions. 80 μL of water was added to 10 μL of each diluted solution.

In a 6-well microplate, 2% Agar-LB was prepared at 3 mL/well, and 90 μL of each diluted solution was inoculated

on the 2% Agar-LB and cultured at 37°C for 24 hours. The results are shown in Fig. 17B.

Wells in which the number of colonies was measurable were selected and the numbers (relative numbers) of live bacterial cells per well on test samples 1, 2, and 4 to 8 when the number of live bacterial cells per well on test sample 3 (FD) was " 1" were calculated in consideration of the dilution concentrations of those test samples. The results of the same are shown in Table 4 below.

[Table 4]

| Test samples | | Numbers of live bacterial cells (relative numbers) |
|---|---|---|
| Test sample 1 | NF | 100 |
| Test sample 2 | FT | 100 |
| Test sample 3 | FD | 1 |
| Test sample 4 | TRH | 10 |
| Test sample 5 | 4TA | 100 |
| Test sample 6 | MLT | 10 |
| Test sample 7 | SCR | 100 |
| Test sample 8 | GOL | 0 |

From the results of Fig. 17B and Table 4, when comparing test sample 1 (NF) and test sample 3 (FD), a significant decrease in the viability of the bacterial cells of Escherichia coli was observed as a consequence of freeze drying. In contrast, it was found that the viability was improved by 10 times or more when adding 6% (final concentration 3%) of sugars other than glycerol. With the addition of 4-trehalosamine, in particular, protective effect on microorganisms was stronger than when the other sugars were added; 4-trehalosamine improved the viability of bacteria and fungi to a similar level to test sample 1 (NF), which was not freeze-dried.

(Test example 7-3: protective effect of 4-trehalosamine on microorganism (Bacillus subtilis))

Using test samples 1 to 5 containing Bacillus subtilis prepared in the following manner, microorganism-protective activity with trehalose or 4-trehalosamine in a freeze-dried condition was evaluated.

<Preparation of test samples>

Test sample 1. Control (NF: Non-freezing)

Frozen stock of Bacillus subtilis (Bacillus subtilis 168 (obtained from ATCC)) was inoculated in 10 mL of Luria-Bertani (LB) culture medium (manufactured by Difco) and cultured at 37°C for 24 hours while shaking it at 220 rpm. After culture, it was centrifuged at 3,000 × g for 5 minutes, washed once with 10 mL of water, and then suspended in 10 mL of water, thus obtaining Bacillus subtilis suspension. 50 μL of water was added to and mixed with 50 μL of this Bacillus subtilis suspension, and the mixture was left to stand at 4°C for 20 hours, preparing control (NF).

Test sample 2. Freeze-thaw product (FT) of Bacillus subtilis

Bacillus subtilis suspension was prepared in a similar manner to test sample 1 and frozen at -80°C for 20 hours, after which freeze-thaw product (FT) of Bacillus subtilis was obtained.

Test sample 3. Freeze-dried product (FD) of Bacillus subtilis

Bacillus subtilis suspension was prepared in a similar manner to test sample 1 and frozen at -80°C for 10 minutes. It was then freeze-dried for 20 hours with a freeze dryer (FLEXI-DRY, manufactured by FTS systems INC., Warminster) connected to a vacuum pump (P65D, manufactured by PHIL Sato Vac INC.), preparing freeze-dried product (FD) of Bacillus subtilis.

Test sample 4. Freeze-dried product of Bacillus subtilis with TRH

After Bacillus subtilis suspension was prepared in a similar manner to test sample 1, 50 μL of 6% aqueous solution of trehalose was added to and mixed with 50 μL of the Bacillus subtilis suspension, and the mixture was frozen at -80°C for 10 minutes. It was then freeze-dried for 20 hours with a freeze dryer (FLEXI-DRY, manufactured by FTS systems INC., Warminster) connected to a vacuum pump (P65D, manufactured by PHIL Sato Vac INC.), preparing freeze-dried product (TRH) of Bacillus subtilis with TRH.

Test sample 5. Freeze-dried product of Bacillus subtilis with 4TA

Freeze-dried product (4TA) of Bacillus subtilis with 4TA was obtained in a similar manner to the preparation method of test sample 4 except that trehalose was changed to 4-trehalosamine (4TA) in the preparation method of test sample 4.

<Evaluation of Bacillus subtilis protective activity>

For test samples 1 and 2, serial suspensions were made by diluting them to 1/10 with water. 100 μL of water was added to each of the freeze-dried products of test samples 3 to 5 and diluted to 1/10 with water to make their serial suspensions. 80 μL of water was added to 10 μL of each diluted solution.

In a 6-well microplate, 2% Agar-LB was prepared at 3 mL/well, and 90 μL of each diluted solution was inoculated on the 2% Agar-LB and cultured at 37°C for 24 hours. The results are shown in Fig. 17C.

Wells in which the number of colonies was measurable were selected and the numbers (relative numbers) of live bacterial cells per well on test samples 1, 2, 4, and 5 when the number of live bacterial cells per well on test sample 3 (FD) was " 1" were calculated in consideration of the dilution concentrations of those test samples. The results of the same are shown in Table 5 below.

[Table 5]

| Test samples | | Numbers of live bacterial cells (relative numbers) |
|---|---|---|
| Test sample 1 | NF | 1000 |
| Test sample 2 | FT | 1000 |
| Test sample 3 | FD | 1 |
| Test sample 4 | TRH | 10 |
| Test sample 5 | 4TA | 100 |

From the results of Fig. 17C and Table 5, when comparing test sample 1 (NF) and test sample 3 (FD), a significant decrease in the viability of the bacterial cells of Bacillus subtilis was observed as a consequence of freeze drying. In contrast, it was found that the viability was improved by 10 times or more when adding 6% (final concentration 3%) of trehalose or 4-trehalosamine. With the addition of 4-trehalosamine, in particular, protective effect on microorganisms was stronger than when trehalose was added.

(Test example 7-4: protective effect of 4-trehalosamine on microorganism (mycobacterium))

Using test samples 1 to 5 containing mycobacterium prepared in the following manner, microorganism-protective activity with trehalose or 4-trehalosamine in a freeze-dried condition was evaluated.

<Preparation of test samples>

Test sample 1. Control (NF: Non-freezing)

Frozen stock of mycobacterium (155 strains of Mycobacterium smegmatis mc$^2$ (American Type Culture Collection (ATCC) 700084)) was inoculated in 10 mL of Middlebrook 7H9 culture medium (manufactured by Becton, Dickinson and Company) and cultured at 37°C for 72 hours while shaking it at 220 rpm. After culture, it was centrifuged at 3,000 × g for 5 minutes, washed once with 10 mL of water, and then suspended in 10 mL of water, thus obtaining mycobacterium suspension. 50 μL of water was added to and mixed with 50 μL of this mycobacterium suspension, and the mixture was

left to stand at 4°C for 20 hours, preparing control (NF).

Test sample 2. Freeze-thaw product (FT) of mycobacterium

Mycobacterium suspension was prepared in a similar manner to test sample 1 and frozen at -80°C for 20 hours, after which freeze-thaw product (FT) of mycobacterium was obtained.

Test sample 3. Freeze-dried product (FD) of mycobacterium

Mycobacterium suspension was prepared in a similar manner to test sample 1 and frozen at -80°C for 10 minutes. It was then freeze-dried for 20 hours with a freeze dryer (FLEXI-DRY, manufactured by FTS systems INC., Warminster) connected to a vacuum pump (P65D, manufactured by PHIL Sato Vac INC.), preparing freeze-dried product (FD) of mycobacterium.

Test sample 4. Freeze-dried product of mycobacterium with TRH

After mycobacterium suspension was prepared in a similar manner to test sample 1, 50 μL of 6% aqueous solution of trehalose was added to and mixed with 50 μL of the mycobacterium suspension, and the mixture was frozen at -80°C for 10 minutes. It was then freeze-dried for 20 hours with a freeze dryer (FLEXI-DRY, manufactured by FTS systems INC., Warminster) connected to a vacuum pump (P65D, manufactured by PHIL Sato Vac INC.), preparing freeze-dried product (TRH) of mycobacterium with TRH.

Test sample 5. Freeze-dried product of mycobacterium with 4TA

Freeze-dried product (4TA) of mycobacterium with 4TA was obtained in a similar manner to the preparation method of test sample 4 except that trehalose was changed to 4-trehalosamine (4TA) in the preparation method of test sample 4.

<Evaluation of mycobacterium-protective activity>

For test samples 1 and 2, serial suspensions were made by diluting them to 1/10 with water. 100 μL of water was added to each of the freeze-dried products of test samples 3 to 5 and diluted to 1/10 with water to make their serial suspensions. 80 μL of water was added to 10 μL of each diluted solution.

In a 6-well microplate, 2% Agar-Middlebrook 7H9 culture medium was prepared at 3 mL/well, and 90 μL of each diluted solution was inoculated on the 2% Agar-Middlebrook 7H9 culture medium and cultured at 37°C for 24 hours. The results are shown in Fig. 17D.

Wells in which the number of colonies was measurable were selected and the numbers (relative numbers) of live bacterial cells per well on test samples 1, 2, 4, and 5 when the number of live bacterial cells per well on test sample 3 (FD) was "1" were calculated in consideration of the dilution concentrations of those test samples. The results of the same are shown in Table 6 below.

[Table 6]

| Test samples | | Numbers of live bacterial cells (relative numbers) |
|---|---|---|
| Test sample 1 | NF | 100 |
| Test sample 2 | FT | 100 |
| Test sample 3 | FD | 1 |
| Test sample 4 | TRH | 10 |
| Test sample 5 | 4TA | 100 |

From the results of Fig. 17D and Table 5, when comparing test sample 1 (NF) and test sample 3 (FD), a significant decrease in the viability of the bacterial cells of mycobacterium (Mycobacterium smegmatis) was observed as a consequence of freeze drying. In contrast, it was found that the viability was improved by 10 times or more when adding 6% (final concentration 3%) of trehalose or 4-trehalosamine. With the addition of 4-trehalosamine, in particular, protective effect on microorganisms was stronger than when trehalose was added.

(Test example 8: pH buffer effect of 4-trehalosamine)

Using the purified product of 4-trehalosamine obtained in the <Purification process> of production example 10, the functionality of 4-trehalosamine as buffer reagent was determined in the following manner.
A change in pH while 50 μL to 100 μL of 1 M hydrochloric acid (HCl) or 1 M sodium hydroxide (NaOH) was added to 50 mL of aqueous solution of 10 mM 4-trehalosamine (production example 10), 50 mL of aqueous solution of 10 mM trehalose, 50 mL of 10 mM tris, 50 mL of aqueous solution of 10 mM MES (2-(N-morpholino) ethanesulfonic acid), or 50 mL of aqueous solution of 10 mM HEPES (4-(2-hydroxyethyl)-1-piperazinylethane sulfonic acid) was measured with a pH meter (LAQUA F-51, manufactured by HORIBA, Ltd.). The results are shown in Fig. 18.
From the results of Fig. 18, strong pH buffer effect comparable that of tris was observed for 4-trehalosamine (4TA). A pH range with strong buffering was around pH 5.5 to pH 8.0, which is lower than that of tris by about 1.0, and pKa was estimated to be 6.99.

(Test example 9-1: determination 1 of degradation of 4-trehalosamine by trehalose degradation enzyme)

Trehalose (TRH) or the 4-trehalosamine (4TA) from the <Purification process> of production example 10 was added to and mixed with 135 mM sodium citrate buffer solution (pH 5.7) so that the final concentration was 0 mM, 0.01 mM, 0.1 mM, 1 mM, or 10 mM. Into them, trehalase derived from pig kidney (0.24 units/mL, manufactured by Sigma-Aldrich) was added and left to react at 37°C for 20 minutes. Into the solution after reaction, a triple amount of Glucose assay reagent (manufactured by Sigma-Aldrich) was added and left to react at room temperature for 15 minutes. After that, absorbance at 340 nm was measured with a plate reader Cytation 5 (manufactured by Bio Tek Instruments) and the amount of glucose released as degradation product was estimated. The results are shown in Figs. 19A and 19B. Fig. 19B is a zoomed-in view of Fig. 19A.
From the results of Figs. 19A and 19B, while trehalose was degraded in this reaction and release of glucose was observed, no release of glucose was observed for 4-trehalosamine and it was determined that 4-trehalosamine was not degraded by pig trehalase.

(Test example 9-2: determination 2 of degradation of 4-trehalosamine by trehalose degradation enzyme)

<Making 10 strains of KP4-TREH>

In order to perform a degradation experiment with human trehalase, high expression strains of the same were made.
First, from human small intestine total RNA (manufactured by Takara Bio Inc.), cDNA was synthesized with PrimeScript II Reverse Transcriptase (manufactured by Takara Bio Inc.).
From this cDNA, PCR was performed using primer TREH1F represented by sequence number: 2 (TTAGCGGCCGCGCCACCATGCCAGGGAGGAC) and primer TREH1R represented by sequence number: 3
(GGGAATTCTCACCATGGCAGGAGGCTGA), and KOD-Plus DNA polymerase (manufactured by TOYOBO Co., Ltd.) to amplify the DNA of human trehalase gene TREH. This amplified DNA was degraded with restriction enzymes EcoRI and NotI and ligated to pSF-CMV-FMDV-Neo/G418 (manufactured by Oxford Genetics) that had been also degraded with those enzymes via a Quick Ligation Kit (manufactured by New England BioLabs), and it was introduced into Escherichia coli DH5α competent cells (manufactured by BioDynamics Laboratory Inc.). Escherichia coli after introduction of plasmid was cultured on 2% Agar-LB culture medium containing 100 μg/mL of kanamycin and Escherichia coli clones with the target plasmid maintained were selected. A number of (about ten) clones were separately cultured on LB culture medium containing 100 μg/mL of kanamycin and the plasmid was purified with a Wizard plasmid purification system (manufactured by Promega). For each plasmid clone, the DNA sequence of the TREH portion was determined via an ABI Prism 3130 Genetic Analyzer (manufactured by ABI), and plasmid clone pSF-TREH 3-3 into which the target sequence had been properly inserted was obtained.
This plasmid was introduced into various kinds of cultured human cells using Viafect (manufactured by Promega), and the expression level of transient trehalase protein was analyzed by western blotting with 1/2,000-diluted anti-trehalase antibody (manufactured by Santa Cruz). Human pancreatic cancer cells KP4 for which high expression of trehalase was observed in this analysis was chosen for use as cells for expression in the subsequent experiments. Human pancreatic cancer cells KP4 into which pSF-TREH 3-3 plasmid had been introduced as described above was diluted to one cell or less per well, and applied to a 96-well microplate. They were cultured on DMEM culture medium containing 400 μg/mL of G418 (manufactured by Thermo Fisher Scientific), and clones with stable trehalase expression were selected and amplified. Ten strains of KP4-TREH which were the clones with the highest trehalase expression were chosen for use in the subsequent experiments.

<Determination of trehalase expression in cultured cells>

Expression patterns of trehalase in human pancreatic cancer cells KP4 (obtained from RIKEN BRC) and in 10 strains of KP4-TREH, which are cell strains with stable and high expression of human trehalase, were determined by western blotting. For detection of trehalase (the primary antibody), anti-trehalase antibody (manufactured by Santa Cruz) was used at 1/2,000 dilution. Also, anti-tubulin antibody (manufactured by Cell Signaling Technology) as control was used at 1/5,000 dilution. The results are shown in Fig. 19C.

When compared with the amount of protein and the number of units for the pig-kidney derived trehalase used in test example 9-1, the amount of trehalase within the total extracted protein of the 10 strains of KP4-TREH was estimated to be 0.32 units/mg to 0.45 units/mg.

<Determination of degradation by human trehalase>

Degradation of 4-trehalosamine and trehalose by cell extract liquids of human pancreatic cancer cells KP4 and 10 strains of KP4-TREH, which are cells with high human trehalase expression, was examined.

Using DMEM culture medium, each of the human pancreatic cancer cells KP4 and the 10 strains of KP4-TREH was amplified in a $CO_2$ incubator at 37°C until they were confluent. After that, the culture medium was removed and washed with PBS (manufactured by Takara Bio Inc.) three times, and cells were collected with a cell scraper in the PBS. After centrifuging the collected cell suspension at 400 × g for 3 minutes and removing supernatant, 135 mM citric acid buffer solution (pH 5.7) containing 0.5% Triton (registered trademark) in an amount 15 times the precipitated cells was added and homogenized. The cells were centrifuged at 21,300 × g for 10 minutes and the respective supernatants were collected. Aqueous solution of 4-trehalosamine or aqueous solution of trehalose was added at 20 μg/mL to each supernatant and they were left to stand at 37°C. At 0 hours, 2 hours, 8 hours, or 24 hours from the start of reaction, the reaction solutions were collected, was diluted with methanol to 1/10, and centrifuged at 21,300 × g for 10 minutes. The respective supernatants were collected and LC-MS analysis was performed under similar conditions to test example 1 to determine the persistence rates of 4-trehalosamine and trehalose in the reaction solution (4TA C) of 4-trehalosamine and the cell extract liquid of human pancreatic cancer cells KP4, the reaction solution (4TA T) of 4-trehalosamine and the cell extract liquid of 10 strains of KP4-TREH, the reaction solution (TRH C) of trehalose and the cell extract liquid of human pancreatic cancer cells KP4, and the reaction solution (TRH T) of trehalose and the cell extract liquid of 10 strains of KP4-TREH. The results are shown in Fig. 19D.

From the results of Fig. 19D, neither trehalose nor 4-trehalosamine was degraded by the cell extract liquid of human pancreatic cancer cells KP4. On the other hand, trehalose was degraded by the cell extract liquid of 10 strains of KP4-TREH, which are cells with high human trehalase expression, and almost disappeared after 8 hours. By contrast, for 4-trehalosamine, almost no degradation or decrease by the cell extract liquid of 10 strains of KP4-TREH was observed.

(Test example 9-3: determination of dynamics upon oral administration of 4-trehalosamine to mice)

4-trehalosamine or trehalose dissolved in saline at 0.5 mg/10 μL per g of body weight was orally administered in a single dose to ICR mice (9-week to 10-week old, female, n = 5) that had been fasted overnight. The five mice on which the same treatment had been performed were placed in the same cage.

<Determination of cumulative excretion of 4-trehalosamine in urine and feces>

Urine and feces were regularly collected together. The collected feces were suspended in water at 200 mg/mL and centrifuged at 21,300 × g for 5 minutes. The centrifuged supernatant of feces suspension and the collected urine were each diluted with methanol to 1/1,000, on which LC-MS analysis was performed under similar conditions to test example 1 and cumulative excretion of 4-trehalosamine (4TA) or trehalose (TRH) over time was calculated. The results for urine are shown in Fig. 19E and the results for feces are shown in Fig. 19F.

<Determination of blood level of 4-trehalosamine>

Blood was regularly collected from tail vein in an amount of around 10 μL to 20 μL, and 200 μL of methanol was added to it after measurement. After agitation on a vortex, it was centrifuged at 21,000 × g for 5 minutes. The supernatant was diluted with methanol to 1/5 and LC-MS analysis was performed on it under similar conditions to test example 1. The results for 4-trehalosamine are shown in Fig. 19G and the results for trehalose are shown in Fig. 19H for each individual (n = 5).

From the results of Fig. 19G, it was found that a portion of orally administered 4-trehalosamine is absorbed and circulates in blood for a certain amount of time. It was also found that most of the absorbed 4-trehalosamine is

excreted through feces and urine in the original form, without being degraded or modified.

<Determination of change in blood glucose level>

In a 96-well plate, the centrifuged supernatant sample of dilution of blood with methanol prepared in <Determination of blood level of 4-trehalosamine> (a sample similar to the sample used in the LC-MS analysis) was placed at 5 μL/well and dried by suction with a vacuum pump. After that, it was dissolved in 25 μL of water, 75 μL of Glucose assay reagent (manufactured by Sigma-Aldrich) was added, and the solution was left to stand at 37°C for 1 hour. Absorbance at 340 nm was then measured with a plate reader Cytation 5 (manufactured by Bio Tek Instruments) and the amount of glucose estimated. The results for 4-trehalosamine are shown in Fig. 19I and the results for trehalose are shown in Fig. 19J (n = 5).

From the results of Fig. 19J, elevation in the blood glucose level was observed 30 minutes to 1 hour after administration for the group with oral administration of trehalose, whereas no elevation in the blood glucose level was observed for the group with administration of 4-trehalosamine.

(Test example 10-1: autophagy inducing effect of 4-trehalosamine and trehalose analog on cultured cells)

Expression and phosphorylation patterns of autophagy-related proteins when human ovarian cancer cells or human malignant melanoma cells were treated with 4-trehalosamine and trehalose analog for 24 hours were determined by western blotting.

Human ovarian cancer cells OVK18 (obtained from RIKEN BRC) and human malignant melanoma cells Mewo (JCRB0066) were each inoculated on a 12-well microplate at $8 \times 10^4$ cells/mL/well and cultured in DMEM culture medium at 37°C for 72 hours, after which they were treated with test samples.

The test samples used were the 4-trehalosamine (4TA) obtained in the <Purification process> of production example 10, trehalose (TRH), the IMCTA-Cn compounds obtained in synthesis examples 1 to 8, trehalose ester (TRH-Cn) (manufactured by Dojindo Laboratories), n-dodecyl-β-D-maltoside (DDM), maltose (MLT), sucrose (SCR), and rapamycin (RM). Untreated cells without addition of compounds were used as control.

In treatment with the test samples, in order to avoid contact of a compound with locally high concentration with cells, a half of the culture fluid in each well was taken, and a required amount of test sample was dissolved in it. After dissolving it sufficiently, it was returned to the original well and treated at 37°C for 24 hours.

Subsequently, culture medium containing the test samples was removed, washed once with PBS, and dissolved with 2 × SDS sample buffer not containing 2-mercaptoethanol (2-ME) or bromphenol blue (BPB). After measuring the protein concentration in the dissolved sample with a BCA protein assay kit (manufactured by Thermo Fisher Scientific), 2-ME and BPB were added and the sample was boiled for 5 minutes, which was used as a sample for western blotting.

The western blotting was performed in a similar manner to test example 4 except that anti-LC3 antibody, which is a marker for autophagy, anti-p62, anti-cathepsin D antibody, anti-Phospho-Akt (Ser473) antibody, anti-Akt antibody, anti-Phospho-p70 S6K (T389) antibody, and anti-p70 S6K antibody (all manufactured by Cell Signaling Technology) were used at 1/2,000 dilution as the primary antibodies in the method described in test example 4.

The results for human ovarian cancer cells OVK18 are shown in Figs. 20A to 20C, and the results for human malignant melanoma cells Mewo are shown in Figs. 20D to 20F. In Figs. 20A, 20B, 20D, and 20E, "PCD" denotes precursors of cathepsin D and "CD-HC" denotes cathepsin D heavy chain.

From the results of Figs. 20A to 20F, although there are variations associated with treatment concentration, cells treated with 4-trehalosamine and trehalose analog both exhibited elevated expression of LC3-II, which is an autophagy marker. For IMCTA-C13, IMCTA-C14, and IMCTA-C15, particularly strong expression was observed; the same or higher amount of expression was observed at a concentration several thousand times lower than trehalose. For trehalose ester (TRH-Cn), elevation in the expression of LC3-II at low concentration was also observed, but the activity was not as strong as IMCTA-Cn. In addition, expression of LC3-II was also observed with sucrose (SCR) at high concentration.

In the treatments with IMCTA-C13, IMCTA-C14, and IMCTA-C15, no accumulation of PCD was observed, unlike the treatment with trehalose; however, they showed similar results to trehalose in that there were accumulation trend of p62 and decreasing trend of pAkt, and that p70 S6K was not dephosphorylated. From this, the action mechanism of autophagy induction by IMCTA-Cn was considered to be basically common with that of trehalose.

(Test example 10-2: determination of nature of autophagy marker expression elevation by 4-trehalosamine or trehalose analog when used in combination of autophagy inhibitor)

Elevated expression of LC3-II is a phenomenon that can be seen when autophagy is actively taking place and also in a situation like when autophagy is inhibited in progress and autophagosome is accumulated. Thus, in order to

ascertain whether the elevated expression of LC3-II as a result of treatment with 4-trehalosamine or trehalose analog in test example 10-1 was caused by autophagy induction or by inhibition, we conducted an experiment with combined use of 4-trehalosamine or trehalose analog and bafilomycin (BM) or chloroquine (CQ), which are autophagy inhibitors. If the amount of expression of LC3-II increases in the presence of an inhibitor, it is determined that 4-trehalosamine or trehalose analog acts to induce autophagy, and if the amount of expression of LC3-II does not change, it is determined that 4-trehalosamine or trehalose analog inhibits autophagy.

As with the test example 10-1, human ovarian cancer cells OVK18 (obtained from RIKEN BRC) and human malignant melanoma cells Mewo (JCRB0066) were each inoculated on a 12-well microplate at $8 \times 10^4$ cells/mL/well, and cultured in DMEM culture medium at 37°C for 72 hours. After that, a half of the culture fluid in each well was taken and each compound was dissolved so that the final concentration is 50 mM 4-trehalosamine, 50 μM IMCTA-C14, or 100 mM trehalose. After dissolving it sufficiently, the cells were returned to the original well and treated at 37°C for 14 hours. Untreated cells without addition of the compounds were used as control.

After that, each autophagy inhibitor was added so that the final concentration is 200 nM bafilomycin (BM) or 100 μM chloroquine (CQ), and the cells were cultured at 37°C for 4 hours. A group with no inhibitor added was cultured in a similar way. After culture, they were turned into samples for western blotting in a similar manner to test example 10-1.

The western blotting was performed in a similar manner to test example 4 except that anti-LC3 antibody (manufactured by Cell Signaling Technology) was used at 1/2,000 dilution as the primary antibody in the method described in test example 4. The results for human ovarian cancer cells OVK18 are shown in Fig. 21A, and the results for human malignant melanoma cells Mewo are shown in Fig. 21B.

Also, the results (graphs) of image analysis on the results of western blotting via Image J (manufactured by National Institutes of Health) are shown in Figs. 21A and 21B (n = 10, mean ± S.D.). The vertical axis of the graph indicates the value of the amount of expression (expression amount ratio) of LC3-II for the groups with the different compounds added when the amount of expression of LC3-II in the control (untreated cells) is 1. The bars of the graph are indicated by dotted oblique lines when there is a significant difference ($p < 0.05$) compared to control as a result of Dunnett's test, by gray color when there is a significant difference ($p < 0.05$) compared to the case of no inhibitor addition, and by black color when there is a significant difference ($p < 0.05$) compared to both of no compound addition (the control) and no inhibitor addition.

From the results of Figs. 21A and 21B, 4-trehalosamine, trehalose, and IMCTA-C14 all led to increased expression of LC3-II in combination with bafilomycin (BM) or chloroquine (CQ) and hence were considered to exhibit autophagy inducing effect on cultured cells.

(Test example 10-3: determination of elimination effect on aggregated protein in cells by 4-trehalosamine or trehalose analog)

The effect of 4-trehalosamine or trehalose analog on Q74, which is partial peptide of huntingtin (Htt), the causal protein of Huntington's disease, and SynA53T, which is a mutation of α-synuclein, the causal protein of Parkinson's disease, (both aggregated proteins) was determined in the following manner.

Neuroblastoma cells SH-SY5Y (obtained from European Collection of Authenticated Cell Cultures (ECACC)) and human neuroblastoma cells NH-12 (obtained from RIKEN BRC) were each inoculated at $14 \times 10^4$ cells/mL and cultured in DMEM culture medium at 37°C for 48 hours.

After the culture of SH-SY5Y cells and NH-12 cells, pEGFP-Q74 vector or EGFP-alphasynuclein-A53T (both obtained from addgene), which are expression vectors of aggregated protein, were transfected with transfection reagent (Viafect, manufactured by Promega). After 24 hours from the transfection, 100 mM 4-trehalosamine, 70 μM IMCTA-C14, or 100 mM trehalose was added and the cells were further treated for 14 hours. Untreated cells without addition of the compounds were used as control. After washing the cells three times with PBS, hyposmotic fluid (solution of one tablet of cOmplete, Mini, EDTA-free Protease Inhibitor Cocktail (CM, manufactured by Merck) in 50 mL of pure water) was added and the mixture was shaken on ice for 10 minutes. It was then centrifuged at 21,300 × g for 10 minutes to be separated into soluble protein (Sol) and aggregated protein (Agg) of supernatant. Thereafter, they were turned into samples for western blotting in a similar manner to test example 10-1.

The western blotting was performed in a similar manner to test example 4 except that anti-GFP antibody (manufactured by Proteintech) was used at 1/5,000 dilution and anti-LC3 antibody (manufactured by Cell Signaling Technology) was used at 1/2,000 dilution as the primary antibodies in the method described in test example 4. The results for neuroblastoma cells SH-SY5Y are shown in Figs. 22A and 22B, and the results for human neuroblastoma cells NH-12 are shown in Figs. 22C and 22D.

Also, the results (graphs) of image analysis on the results of western blotting via Image J (manufactured by National Institutes of Health) are shown in Figs. 22A to 22D (n = 5, mean ± S.D.). The vertical axis of the graph indicates the value of the amount of expression (expression amount ratio) of each protein for the groups with the different compounds added when the amount of expression of each protein in the control (untreated cells) is 1. The bars of the graph

are indicated with "*" when there is a significant difference ($p < 0.01$) compared to control as a result of t-test.

From the results of Figs. 22A to 22D, it was determined that aggregate of partial peptide Q74 as the causal protein of Huntington's disease and aggregate of SynA53T as the causal protein of Parkinson's disease which had been expressed in the SH-SY5Y cells and NH-12 cells used as nerve cell models were significantly decreased by 4-trehalosamine, IMCTA-C14, and trehalose. For IMCTA-C14, in particular, activity comparable to or higher than that with trehalose was observed at or below a concentration 1/1,000 of trehalose.

(Test example 10-4: determination of elevated expression and nuclear localization of autophagy-related transcription factor TFEB due to 4-trehalosamine or trehalose analog)

Elevated expression and nuclear localization of autophagy-related transcription factor EB (TFEB) when human ovarian cancer cells or human malignant melanoma cells were treated with 4-trehalosamine or trehalose analog were determined by western blotting and immunostaining.

Fractionation of nuclear and cytoplasm was performed in the following manner using buffer that was a modification of the buffer according to the report of Dignam et al. (J. D. Dignam et al., Nucleic Acids Res, 1983, 11 (5), p. 1475-1489).

<Determination by western blotting>

Human ovarian cancer cells OVK18 (obtained from RIKEN BRC) and human malignant melanoma cells Mewo (JCRB0066) were each inoculated on a 6-well plate at $2 \times 10^5$ cells/2 mL/well, and cultured in DMEM culture medium at 37°C for 48 hours. After that, as with test example 10-1, a half of the culture fluid in each well was taken, and each compound was dissolved so that the final concentration is 50 mM 4-trehalosamine, 50 μM IMCTA-C14, 100 μM IMCTA-C14, or 100 mM trehalose. After dissolving it sufficiently, the cells were returned to the original well and treated at 37°C for 14 hours. Untreated cells without addition of the compounds were used as control.

The cells after treatment with the compounds were washed with PBS once, and then treated with lysis buffer (10 mM HEPES (pH 7.9), 10 mM potassium chloride, 1.5 mM magnesium chloride, 0.4% Igepal CA-630 (manufactured by Sigma-Aldrich), 1 mM DTT, and 1 tablet/50 mL of CM (manufactured by Merck)) on ice for 30 minutes. Next, cells were scraped off with a cell scraper and collected, after which they were gently shaken at 6°C for 10 minutes. After they were centrifuged at $21,300 \times g$ for 5 minutes, the supernatant was collected as cytoplasm fraction.

The precipitation was washed with lysis buffer once and then dissolved with nuclear buffer (20 mM HEPES (pH 7.9), 0.4 M sodium chloride, 1.5 mM magnesium chloride, 5% glycerol, 1 mM DTT, and 1 tablet/50 mL of CM). It was centrifuged at $21,300 \times g$ for 5 minutes, after which the supernatant was collected as nuclear fraction.

The protein concentration of each fraction was measured with protein quantification reagent (XI,-Bradford, integrate) and the amount of expression of TFEB in each fraction was examined by western blotting. The western blotting was performed in a similar manner to test example 4 except that anti-TFEB antibody (manufactured by Cell Signaling Technology) was used at 1/2,000 dilution, anti-GAPDH antibody (manufactured by MEDICAL & BIOLOGICAL LABORATORIES CO., LTD.) was used at 1/40,000 dilution in order to indicate the accuracies of cytoplasm (Cyt) fraction and nuclear (Nuc) fraction, and anti-PARP antibody (manufactured by Cell Signaling Technology) was used at 1/10,000 dilution as the primary antibodies in the method described in test example 4. The results for human ovarian cancer cells OVK18 are shown in Fig. 23A, and the results for human malignant melanoma cells Mewo are shown in Fig. 23B.

<Determination by immunostaining>

Human ovarian cancer cells OVK18 and human malignant melanoma cells Mewo were each inoculated on a 48-well microplate at $2.4 \times 10^4$ cells/0.6 mL/well, and cultured in DMEM culture medium at 37°C for 48 hours. After that, as with test example 10-1, a half of the culture fluid in each well was taken, and each compound was dissolved so that the final concentration is 50 mM 4-trehalosamine, 100 μM IMCTA-C14, or 100 mM trehalose. After dissolving it sufficiently, the cells were returned to the original well and treated at 37°C for 14 hours. Untreated cells without addition of the compounds were used as control.

All of the subsequent reactions were performed at room temperature while gently shaking the samples. The culture fluid was removed from the treated cells, and after adding 4% paraformaldehyde-PBS (manufactured by FUJIFILM Wako Pure Chemical Corporation) at 300 μL/well, the cells were fixed for 15 minutes. After washing the cells with PBS three times, 0.3% Triton X-100-PBS containing 5% BSA was added at 300 μL/well and blocked for 1 hour. After removing the blocking fluid, anti-TFEB antibody (manufactured by Santa Cruz Biotechnology) diluted at 1/33 with 0.3% Triton X-100-PBS containing 1% BSA was added at 50 μL/well and reaction was left to proceed for 4 hours. After washing the cells with PBS three times, Alexa Fluor 488 binding anti-mouse IgG antibody (manufactured by Cell Signaling Technology) diluted at 1/500 with 0.3% Triton X-100-PBS containing 1% BSA was added at 50 μL/well and reaction was left to proceed

for 2 hours. After removing the antibody fluid, 0.3% Triton X-100-PBS was added to the cells at 1 mL/well to wash them for 5 minutes three times, and microscopic observation was performed with PBS added at 300 μL/well. For detection of Alexa Fluor 488, a B filter (excitation filter (Ex) 420 nm to 490 nm, absorption filter (Em) 520 nm) was used. After taking phase contrast and fluorescent images, DAPI (4',6-diamidino-2-phenylindole)-PBS solution of 50 ng/mL was added to the cells at 50 μL/well, and after treatment for 2 hours, the cells were washed with PBS three times. The stained nuclei were observed through a UV filter (Ex. 330 nm to 350 nm, Em. 420 nm).

The results for human ovarian cancer cells OVK18 are shown in Fig. 23C, and the results for human malignant melanoma cells Mewo are shown in Fig. 23D. In Figs. 23C and 23D, "PC" indicates phase contrast observation images and "DAPI" indicates staining patterns of nuclei.

From the results of Figs. 23A to 23D, it was determined that treatment of cells with 4-trehalosamine and trehalose analog led to elevated expression and nuclear localization of TFEB, which is known to be expressed at elevated level and transported into the nucleus during autophagy induction. For IMCTA-C14, in particular, activity comparable to or higher than that with trehalose was observed at or below a concentration 1/1,000 of trehalose.

(Test example 11-1: staining of mycobacterium with IMCTA-fluorescein, IMCTA-biotin, or IMCTA-azide)

Using the IMCTA-fluorescein from synthesis example 9, the IMCTA-biotin from synthesis example 10, and the IMCTA-azide from synthesis example 11, a staining experiment of mycobacterium (Mycobacterium smegmatis) was performed.

155 strains of Mycobacterium smegmatis $mc^2$ (ATCC 700084) that had been stored at 4°C were inoculated at 0.5 μL/mL on Middlebrook 7H9 culture medium (manufactured by Becton, Dickinson and Company) containing addition of 0.5% glycerol, 0.05% Tween 80, and 10% BD BBL Middlebrook OADC Enrichment (manufactured by Becton, Dickinson and Company), and were cultured at 37°C for 24 hours. After adding aqueous solution of trehalose or aqueous solution of sucrose used for blocking to this culture fluid to a final concentration of 1% or 3%, the compounds were added such that the final concentration was 0.1% DMSO, 10 μg/mL 5-carboxyfluorescein (5-FC), 10 μg/mL IMCTA-fluorescein (synthesis example 9), 30 μg/mL IMCTA-biotin (synthesis example 10), or 10 μg/mL IMCTA-azide (synthesis example 11), respectively. After they were cultured at 37°C for 8 hours, the culture fluids were centrifuged at 21,300 × g for 1 minute and the precipitations were washed with 0.05% Tween 80-PBS three times. The bacterial cells were suspended in 0.05% Tween 80-PBS, preparing bacterial cell suspension. Cells that were cultured in the same manner without addition of aqueous solution of trehalose or aqueous solution of sucrose were used as "No block".

The bacterial cell suspension prepared with IMCTA-fluorescein was observed for phase contrast and fluorescent images with a fluorescence microscope (ECLIPSE-TE2000-U, manufactured by NIKON CORPORATION). For fluorescence observation, a B filter (Ex. 420 nm to 490 nm, Em. 520 nm) was used. The results are shown in Fig. 24A.

After taking phase contrast and fluorescent images, fluorescence intensity per bacterial cell volume was analyzed with Image J (manufactured by National Institutes of Health), and the intensity of fluorescence per bacterial cell volume was turned into numerical values and graphs (n = 3, mean ± S.D.). The results are shown in Fig. 25A.

The bacterial cell suspension prepared with IMCTA-biotin was cultured at 37°C for 1 hour with the addition of 30 μg/mL DyLight 488 Streptavidin (manufactured by Vector Laboratories) and washed with 0.05% Tween 80-PBS three times. After that, in 0.05% Tween 80-PBS, fluorescence microscope observation and image analysis were performed under similar conditions to the observation of the bacterial cell suspension prepared with IMCTA-fluorescein. The results of fluorescence microscope observation are shown in Fig. 24B, and the results of image analysis are shown in Fig. 25B.

The bacterial cell suspension prepared with IMCTA-azide was added with 5-FAM-Alkyne (final concentration 10 μg/mL, manufactured by Jena Bioscience), $CuSO_4 \cdot 5H_2O$ (100 μg/mL), and ascorbic acid (100 μg/mL), which were mixed in 0.05% Tween 80-PBS beforehand, and was treated at 37°C for 1 hour. The bacterial cells were washed with 2% aqueous solution of SDS three times, and then suspended in 2% aqueous solution of SDS, and fluorescence microscope observation and image analysis were performed under similar conditions to the observation of the bacterial cell suspension prepared with IMCTA-fluorescein. The results of fluorescence microscope observation are shown in Fig. 24C, and the results of image analysis are shown in Fig. 25C.

Although it was assumed that by treating raw material with fluorescent substance, Mycobacterium smegmatis bacterial cells would not emit fluorescence and the substance would not be ingested into the bacterial cells, from the results of Figs. 24A to 24C, all of IMCTA-fluorescein, IMCTA-biotin, and IMCTA-azide were ingested into Mycobacterium smegmatis bacterial cells and emitted fluorescence.

For ingestion of IMCTA-fluorescein, no significant inhibition was seen even in the presence of an excess amount of sucrose, whereas inhibition was observed in the presence of 1% trehalose. This suggested that IMCTA-fluorescein was ingested competitively with trehalose.

However, some differences were observed in the respective staining patterns of IMCTA-fluorescein, IMCTA-biotin, and IMCTA-azide. When the same amount of labeller was used, the strongest fluorescence was observed for the treatment with IMCTA-azide. Meanwhile, although the cause is unknown, suppression of ingestion was observed not

only with trehalose but with sucrose.

For IMCTA-biotin, a patch-like staining pattern was recognized. In the case of treatment with IMCTA-biotin, some suppression of ingestion by sucrose was also observed.

(Test example 11-2: TLC analysis of lipid components of IMCTA-fluorescein stained cells)

TLC analysis was performed on the lipid components of bacterial cell suspension of Mycobacterium smegmatis bacterial cells stained with IMCTA-fluorescein in test example 11-1 or bacterial cell suspension of Mycobacterium smegmatis bacterial cells treated with DMSO.

The TLC analysis was performed based on the method of Kai et al. (M. Kai et al., FEBS Lett 2007, 581, p. 3345-3350).

The bacterial cell suspension of Mycobacterium smegmatis bacterial cells stained with IMCTA-fluorescein in test example 11-1 or the bacterial cell suspension of Mycobacterium smegmatis bacterial cells treated with DMSO was centrifuged at 21,300 $\times$ g for 1 minute. After that, a 20-times amount of chloroform/methanol (2:1, v/v) was added to the precipitation and the solution was strongly agitated with a tube mixer (TWIN3-28N, manufactured by AGC Techno Glass Co., Ltd) for 5 minutes. After the solution was centrifuged at 21,300 $\times$ g for 1 minute, supernatant was collected. Similar extraction treatment was performed on the precipitation twice more, and the supernatants were combined and dried with a Savant SpeedVac (manufactured by Thermo Fisher Scientific) to remove the solvent. The dried product was suspended in chloroform/methanol in a 0.75% amount of the amount of the initial culture in test example 11-1 and vigorously agitated for 5 minutes. It was then centrifuged at 21,300 $\times$ g for 1 minute and the supernatant was gained as TLC sample.

In the TLC analysis, a Silica gel 60 TLC plate (manufactured by Merck) was used and the solvent was developed with chloroform/methanol/acetone/acetonitrile (90:10:6:1, v/v). The lipid as a whole was detected with phosphomolybdic acid solution similar to that in test example 1 and fluorescence was detected with a UV transilluminator (NTFM-20, manufactured by Analytik Jena US LLC).

The phosphomolybdic acid staining patterns for the entire component are shown in Fig. 26A and the fluorescence detection patterns are shown in Fig. 26B. In Figs. 26A and 26B, "1" indicates the result for bacterial cells treated with DMSO, "2" indicates the result for bacterial cells treated with IMCTA-fluorescein, and "3" indicates the result of spotting 1.3 $\mu$g of IMCTA-fluorescein itself. In Fig. 26B, the arrows indicate components for which fluorescence is seen only in the IMCTA-fluorescein treated sample.

The results of Figs. 26A and 26B suggest that two fluorescent lipids were detected in bacterial cells which had ingested IMCTA-fluorescein and IMCTA-fluorescein was ingested in the trehalose portions of trehalose monomycolate and trehalose dimycolate instead of trehalose.

(Test example 12-1: determination of degradation of 4-trehalosamine by microorganisms)

Degradation of 4-trehalosamine by the 12 kinds of microorganisms below was determined.

<Microorganisms>

1. EC: Escherichia coli K-12 (owned by the Microbial Chemistry Research Foundation)
2. SM: Serratia marcescens B-0524 (owned by the Microbial Chemistry Research Foundation)
3. EF: Enterococcus faecalis JCM5803 (obtained from RIKEN BRC)
4. AN: Aspergillus niger F16 (owned by the Microbial Chemistry Research Foundation)
5. SE: Salmonella enteritidis 1891 (owned by the Microbial Chemistry Research Foundation)
6. MS: Mycobacterium smegmatis ATCC-607 (obtained from ATCC)
7. BS: Bacillus subtilis 168 (obtained from ATCC)
8. PA: Pseudomonas aeruginosa A3 (owned by the Microbial Chemistry Research Foundation)
9. ML: Micrococcus luteus IFO3333 (obtained from IFO)
10. BF: Bacteroides fragilis JCM11019 (obtained from RIKEN BRC)
11. SC: Saccharomyces cerevisiae F-7 (owned by the Microbial Chemistry Research Foundation)
12. CA: Candida albicans 3147 (obtained from IFO)

- Culture methods -

EC, SM, PA, and ML were pre-cultured on Nutrient culture medium (1% polypeptone (manufactured by Nihon

Pharmaceutical Co., Ltd.), 1% fish extract for bacteria (manufactured by Kyokuto Pharmaceutical Industrial Co., Ltd.), 0.2% sodium chloride).

MS was pre-cultured on Nutrient culture medium with 2% glycerol.

EF and SE were pre-cultured on Bacto Heart infusion culture medium (HI, manufactured by Becton, Dickinson and Company (BD)).

BF was pre-cultured on Gifu anaerobic culture medium (GAM culture medium, manufactured by Nissui Pharmaceutical Co., Ltd.).

SC, CA, and AN were pre-cultured on RPMI-1640 culture medium (manufactured by Nissui Pharmaceutical Co., Ltd.).

BS was pre-cultured on LB culture medium (manufactured by Difco).

After placing 196 μL of culture fluid to be used with each of the microorganisms 1 to 12 in a 96-well plate, 2 μL of each of the pre-cultured microorganism suspensions was added. 2 μL of the mixture of 5 mg/L aqueous solution of 4-trehalosamine and 5 mg/L aqueous solution of trehalose (both at a final concentration of 50 μg/mL) was added, and the bacteria were cultured at 37°C and the fungi were cultured at 30°C under a humid condition for 5 days.

The humid condition refers to a condition where the 96-well plate is wrapped in a paper towel saturated with water and placed in a zip bag, with the zipper of the bag kept open over 2 to 3 cm as a vent.

After the culture, the culture fluid was diluted to 1/10 with methanol, vigorously agitated on a vortex, and then centrifuged at 21,300 × g for 5 minutes. The resultant supernatant was subjected to LC-MS analysis under similar conditions to test example 1 and the persistence rate (%) of 4-trehalosamine or trehalose was calculated. The results are shown in Fig. 27A.

From the results of Fig. 27A, 4-trehalosamine was almost not detected with 8 out of the 12 kinds of microorganism.

(Test example 12-2: determination of assimilation of 4-trehalosamine by microorganisms)

Assimilation of 4-trehalosamine by the 12 kinds of microorganisms described in test example 12-1 was examined.

The 12 kinds of microorganisms described in test example 12-1 were cultured until they were saturated in the respective culture fluids used, after which a 1/100 amount of each was added to the corresponding one of the culture fluids free of carbon source below.

- Culture fluids free of carbon source -

For EC, SM, AN, SE, ML, and BF, RPMI-1640 (containing no glucose) (manufactured by FUJIFILM Wako Pure Chemical Corporation) was used.

For MS, BS, and CA, modified 805 culture medium (0.1% yeast extract (manufactured by BD), 0.07% dipotassium hydrogen phosphate, 0.01% potassium dihydrogen phosphate, 0.1% magnesium sulfate heptahydrate), which is a modification of NBRC805-1 culture medium (National Institute of Technology and Evaluation), was used.

For PA, modified 805-4 culture medium (0.025% triptone (manufactured by BD), 0.07% dipotassium hydrogen phosphate, 0.01% potassium dihydrogen phosphate, 0.1% magnesium sulfate heptahydrate) was used.

For EF and SC, an LB culture medium not containing yeast extract (1% triptone (manufactured by BD), 0.05% sodium chloride) was used.

Next, 4-trehalosamine, trehalose, or glucose was added to PA at a final concentration of 0.1% and to the microorganisms other than PA at a final concentration of 0.4%, and the bacteria were cultured at 37°C and the fungi were cultured at 30°C for 5 days under a humid condition (a similar condition to test example 12-1). For the culture fluids after 5 days, absorbance at 620 nm was measured with a plate reader Cytation 5 (manufactured by Bio Tek Instruments) to examine the proliferation of the microorganisms.

The results are shown in Fig. 27B. In Fig. 27B, "1" indicates control without addition of 4-trehalosamine, trehalose, or glucose, "2" indicates those cultured with addition of glucose, "3" indicates those cultured with addition of trehalose, and "4" indicates those cultured with addition of 4-trehalosamine.

In test example 12-2, experiments were conducted under a condition that increases proliferation rate when glucose was added to the microorganisms. From the results of Fig. 27B, activated proliferation was observed with 6 kinds of bacteria in the case of adding trehalose; it was considered that trehalose was degraded and assimilated by these bacteria. In contrast, in the case of adding 4-trehalosamine, activated proliferation was not observed with any of the 12 kinds of microorganisms. From the results of Figs. 27A and 27B, presumably, 4-trehalosamine became undetectable as 4-trehalosamine due to modification of part of its structure rather than being degraded by the microorganisms such that glucose is released, and it was confirmed that 4-trehalosamine would not be a carbon or nutrient source.

(Test example 13: binding model of 4-trehalosamine to human trehalase)

A homology model of human trehalase (O43280) was built using Promod3 3.1.1 at the SWISS-MODEL web server (see A. Waterhouse et al., Nucleic Acids Res, 2018, 46, W296., N. Guex et al., Electrophoresis, 2009, 30 Suppl 1, S162., and S. Bienert et al., Nucleic Acids Res, 2017, 45, D313.). Of 148 kinds of templates, we selected one indicative of 39% sequence homology (PDB 5z66) (see A. Adhav et al., FEBS J, 2019, 286, 1700.) and used it as base. The quality of the model was assessed by Qmean and QMEANDisCo scoring at the QMEAN-Server (https: //swissmodel.expasy.org/qmean) (see G. Studer et al., Bioinformatics, 2020, 36, 1765., M. Bertoni et al., Sci Rep, 2017, 7, 10480., C. Camacho et al., BMC Bioinformatics, 2009, 10, 421., M. Steinegger et al., BMC Bioinformatics, 2019, 20, 473., and M. Mirdita et al., Nucleic Acids Res, 2017, 45, D170.). QMEAN score was -3.15 and QMEANDisCo Global score was 0.70 $\pm$ 0.05.

Subsequently, a binding model of trehalase and trehalose (ZINC4095531) was made with AutoDock Vina on Chimera (see O. Trott et al., J Comput Chem, 2010, 31, 455. and E. F. Pettersen et al., J Comput Chem, 2004, 25, 1605.). Attribution of charge was made using AMBER ff14SB for standard amino acid residues and Gasteiger for the others. A docking pose of 4-trehalosamine was also built in a similar manner.

For creation of binding model views, PyMOL (The PyMOL Molecular Graphics System, Version 2.1, Schrodinger, LLC. 2010.) was used.

Fig. 28A shows a binding model of trehalose to human trehalase, and Fig. 28B shows a binding model of 4-trehalosamine to human trehalase.

The binding model of trehalose to human trehalase in Fig. 28A was an optimal model, for which AutoDock score indicated -9.4 kcal/mol. The catalytic site of trehalase is small, such that little extra space is left once trehalose binds to it. It is considered that degradation is prevented because molecules larger than trehalose cannot enter it.

For 4-trehalosamine, which is of a similar size to trehalose, several binding models were created. For every one of those models, it was inferred that binding might be hindered because there were amino acid residues agonistic to the amino group of 4-trehalosamine at the catalytic site.

The present international application claims the priority based on Japanese Patent Application No. 2021-144962 filed on September 6, 2021 and Japanese Patent Application No. 2021-144962 is incorporated herein in its entirety by reference.

## Accession number

**[0564]** NITE BP-03495 (National Institute of Technology and Evaluation (NITE) Patent Microorganisms Depositary (NPMD))

## Claims

1. A protective agent, a surfactant composition, a composition for blood glucose level control, an autophagy inducer, a mycobacterium stain, or a protein extractant which contains compounds, or pharmaceutically acceptable salts thereof, or solvates thereof represented by general formula (1) below:

[Chemical formula 1]

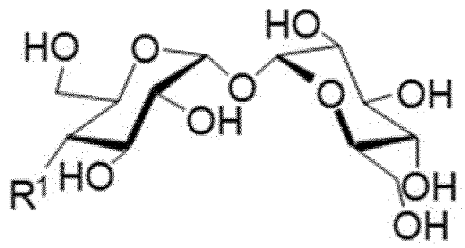

General formula (1)

where, in the general formula (1) above, $R^1$ is $-NH_2$, a substituent represented by structural formula (A) below, a substituent represented by structural formula (B) below, or $-NH(CH_2)_mCH_3$, and the m denotes an integer from 7 to 14,

[Chemical formula 2]

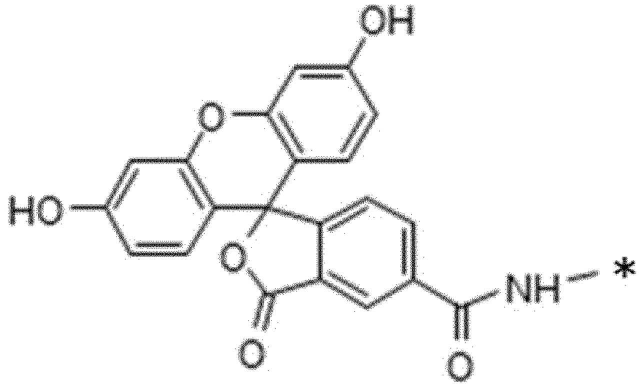

Structural formula (A)

[Chemical formula 3]

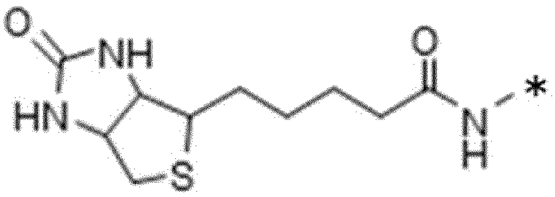

Structural formula (B)

where, in the structural formula (A) above or the structural formula (B) below, "*" denotes a dangling bond.

**2.** Compounds, or pharmaceutically acceptable salts thereof, or solvates thereof represented by general formula (2) below:

[Chemical formula 4]

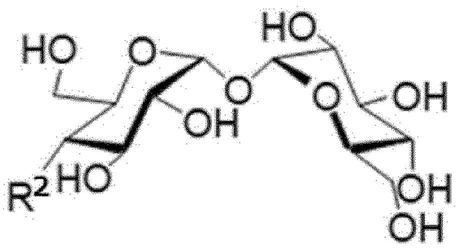

General formula (2)

where, in the general formula (2) above, $R^2$ is a substituent represented by structural formula (A) below, a substituent represented by structural formula (B) below, or $-NH(CH_2)mCH_3$, and the m denotes an integer from 7 to 14,

[Chemical formula 5]

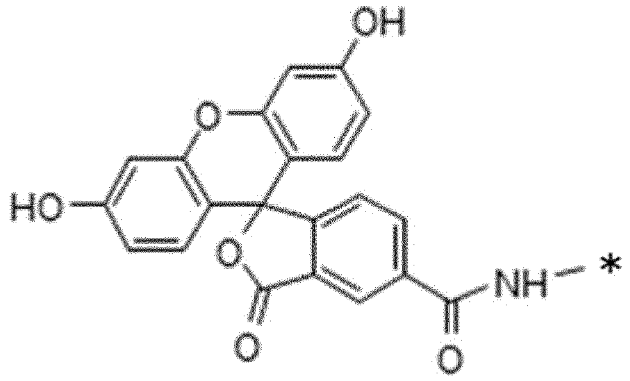

Structural formula (A)

[Chemical formula 6]

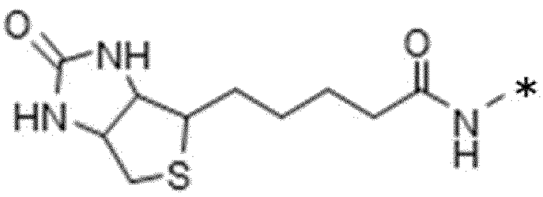

Structural formula (B)

where, in the structural formula (A) above or the structural formula (B) below, "*" denotes a dangling bond.

**3.** A compound-containing composition containing the compounds, or pharmaceutically acceptable salts thereof, or solvates thereof according to Claim 2.

**4.** A method for producing 4-trehalosamine comprising:
a culture process for culturing Streptomyces sp. MK186-mF5 (accession number: NITE BP-03495).

**5.** The method for producing 4-trehalosamine according to Claim 4, wherein a culture medium contains at least any one of 3% by mass to 9% by mass of soluble starch, 0.001% by mass to 0.02% by mass of zinc chloride, and 0.1% by mass to 1% by mass of potassium chloride in the culture process.

**6.** A method for producing compound represented by general formula (3) below, the method comprising:

a process for reacting an amino group at position 4 of 4-trehalosamine with $CH_3(CH_2)_pCOH$ (where the p denotes an integer from 6 to 13), 2-azido-1,3-dimethylimidazolinium hexafluorophosphate, 5-carboxyfluorescein N-succinimidyl ester, or D-biotin N-succinimidyl, by using 4-trehalosamine as a starting material:

[Chemical formula 7]

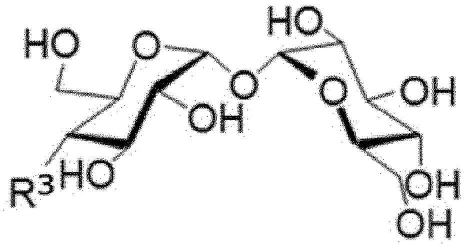

General formula (3)

where, in the general formula (3) above, $R^3$ is $-N(CH_2)_mCH_3$, $-N_3$, a substituent represented by structural formula (A) below, or a substituent represented by structural formula (B) below, and the m denotes an integer from 7 to 14,

[Chemical formula 8]

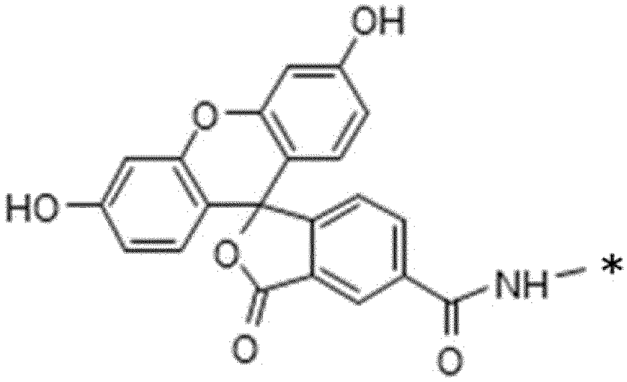

Structural formula (A)

[Chemical formula 9]

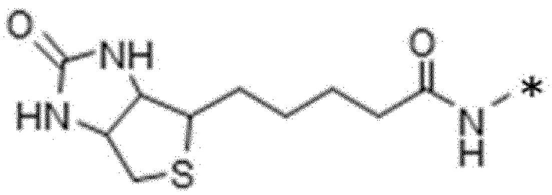

Structural formula (B)

where, in the structural formula (A) above or the structural formula (B) below, "*" denotes a dangling bond.

**7.** A microorganism which is Streptomyces sp. MK186-mF5 (accession number: NITE BP-03495), and is capable of producing 4-trehalosamine.

FIG. 1A

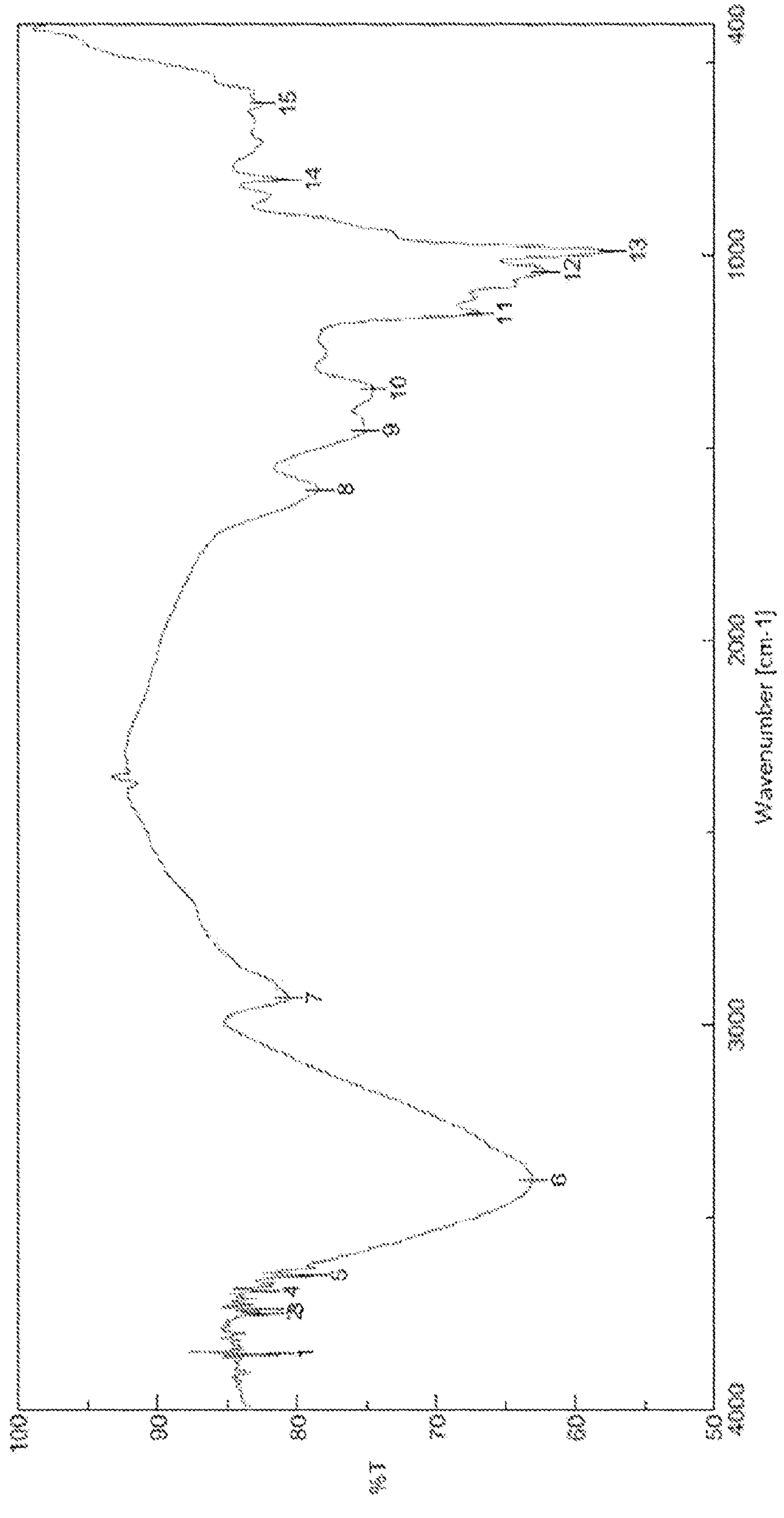
%T
Wavenumber [cm-1]
100
90
80
70
60
50
4000
3000
2000
1000
400
1
2
3
4
5
6
7
8
9
10
11
12
13
14
15

FIG. 1B

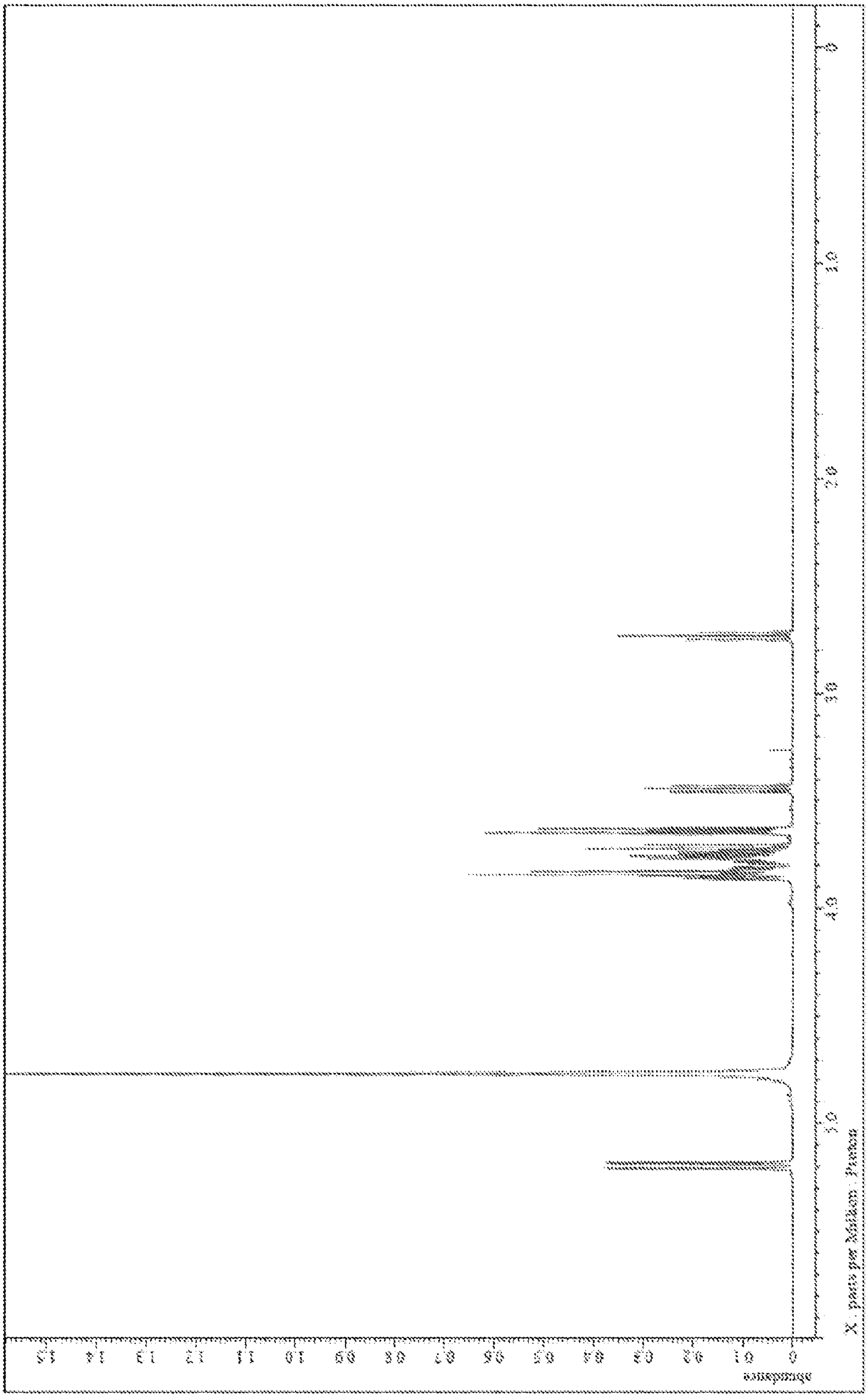

FIG. 1C

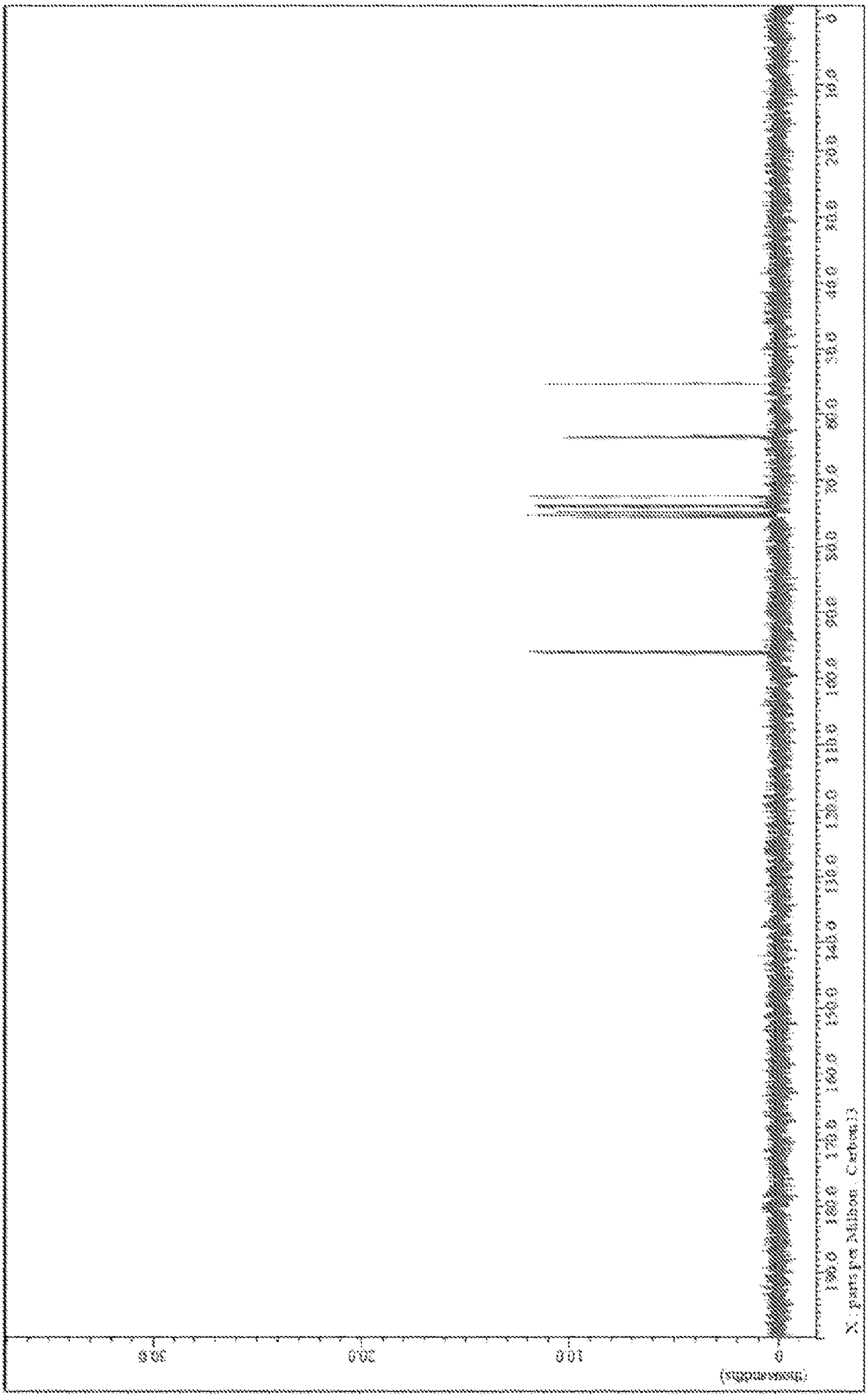

FIG. 2A

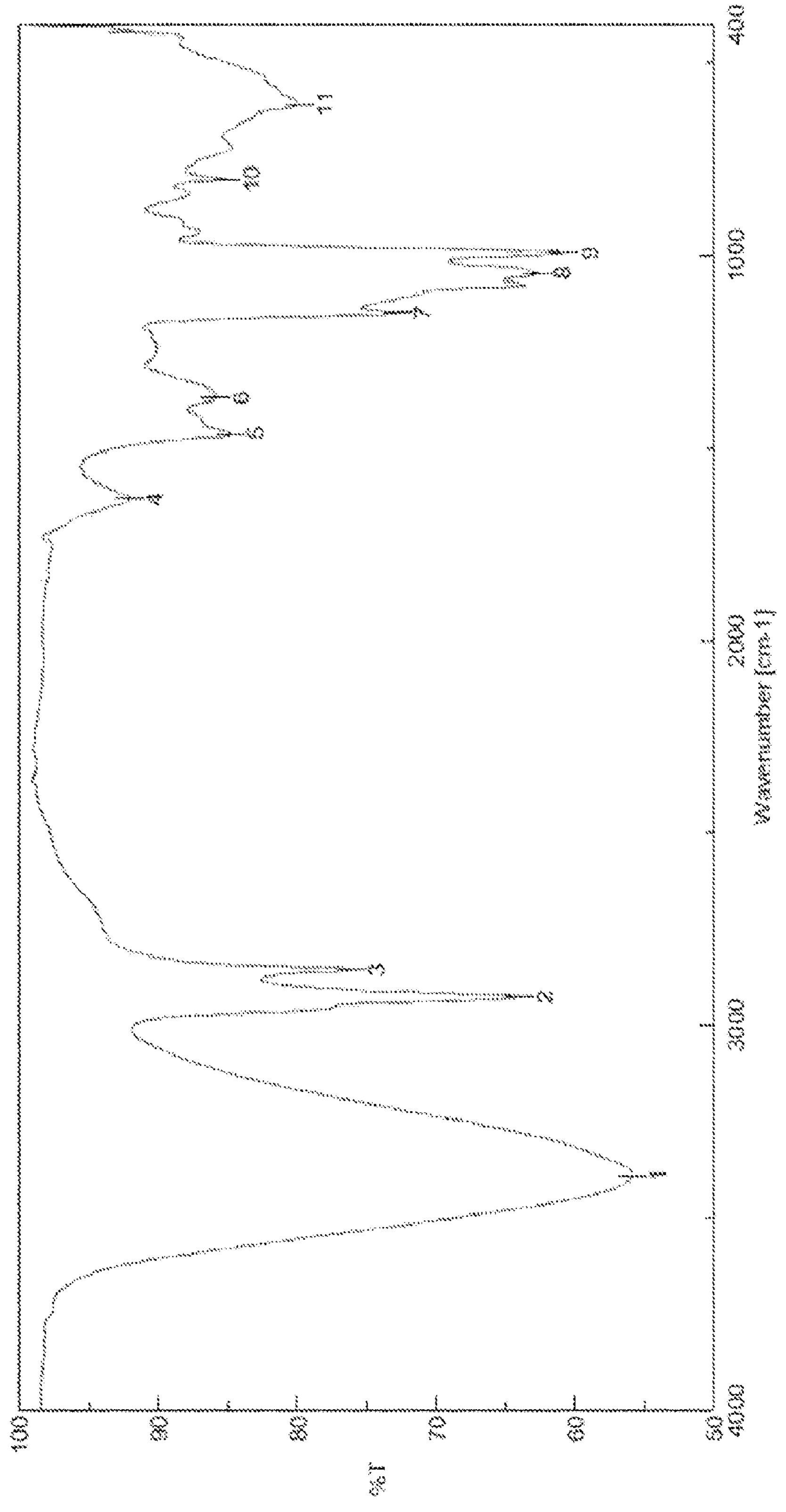
%T
100
90
80
70
60
50
4000
3000
2000
1000
400
Wavenumber [cm-1]
1
2
3
4
5
6
7
8
9
10
11

FIG. 2B

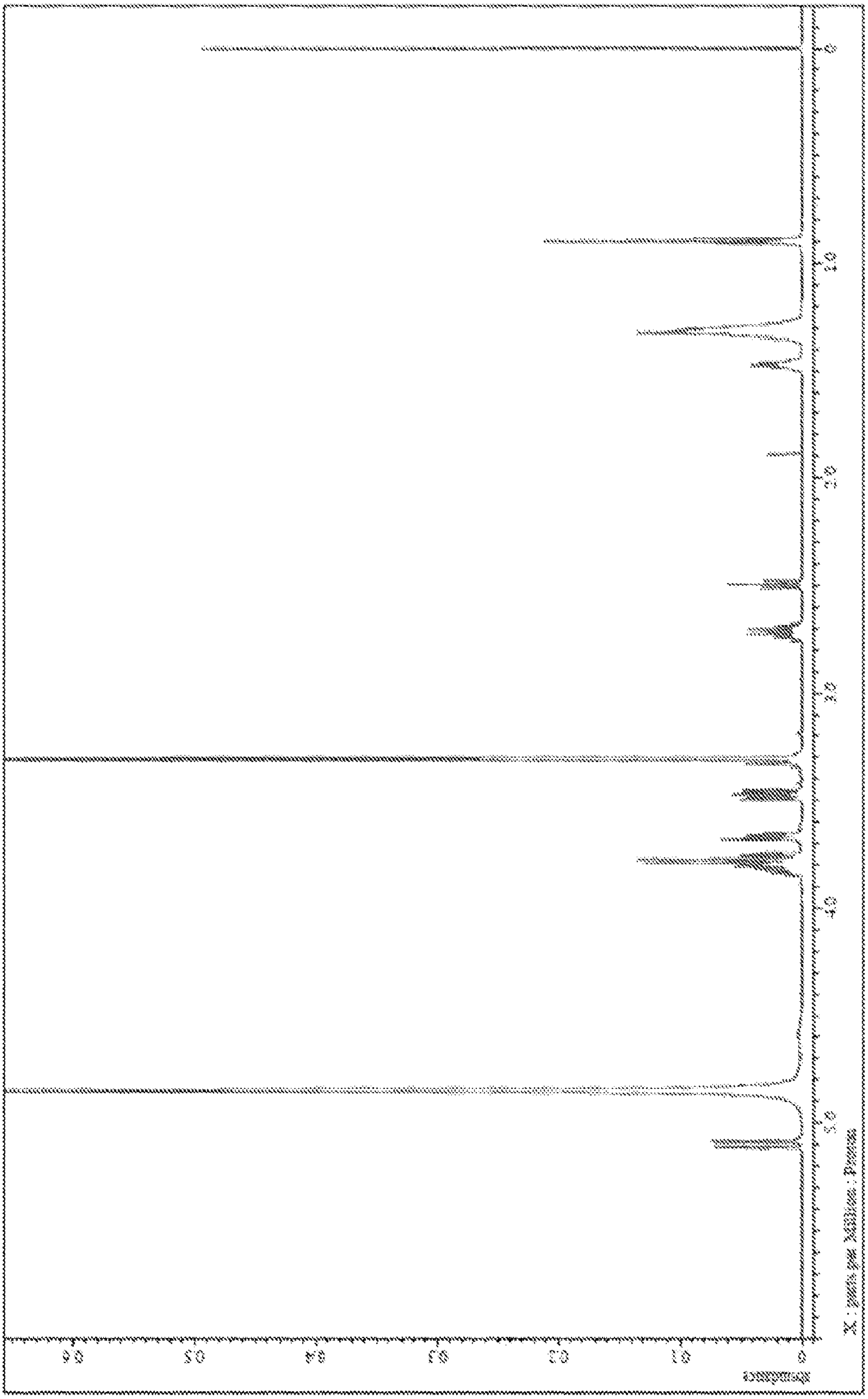

FIG. 2C

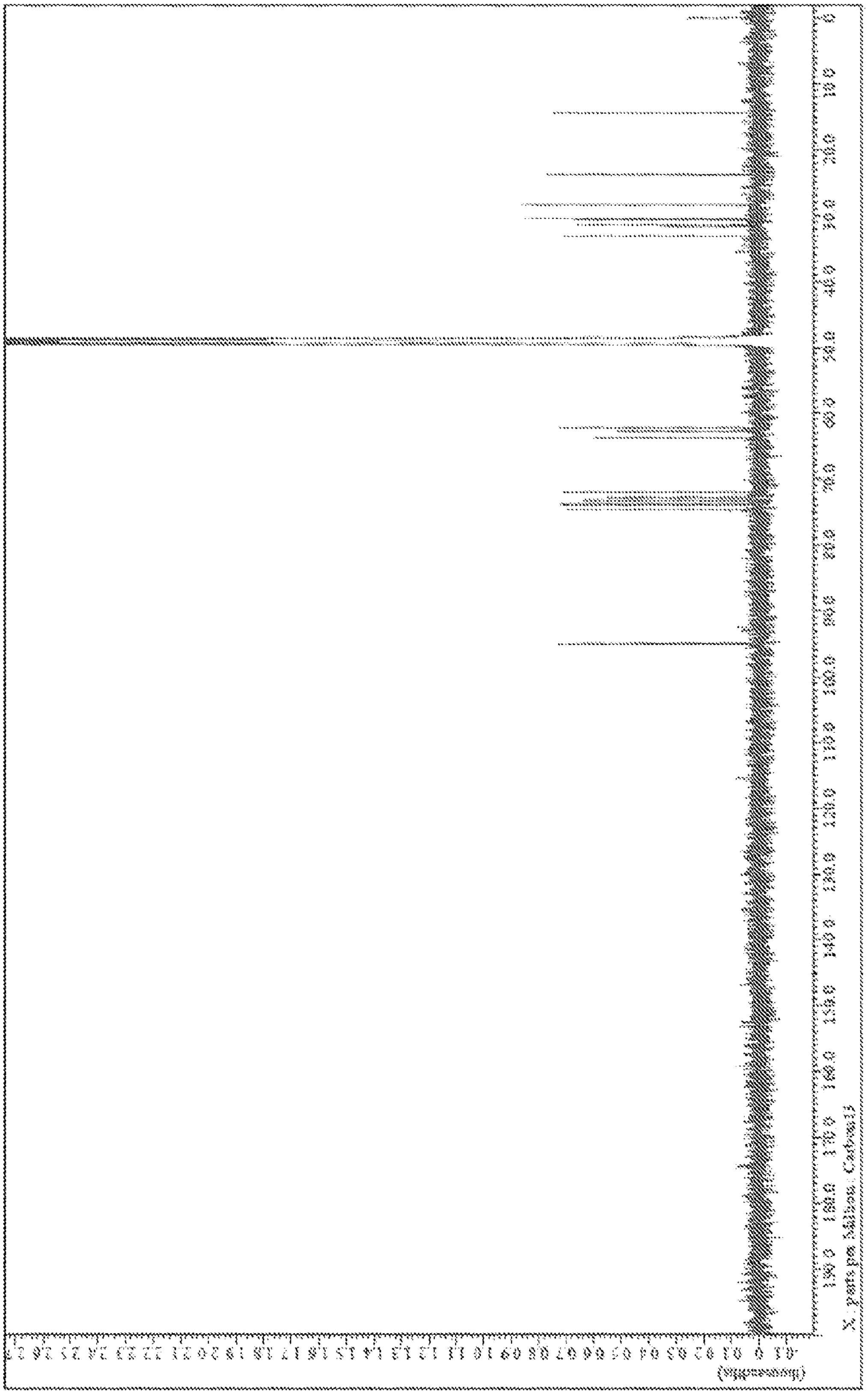

FIG. 3A

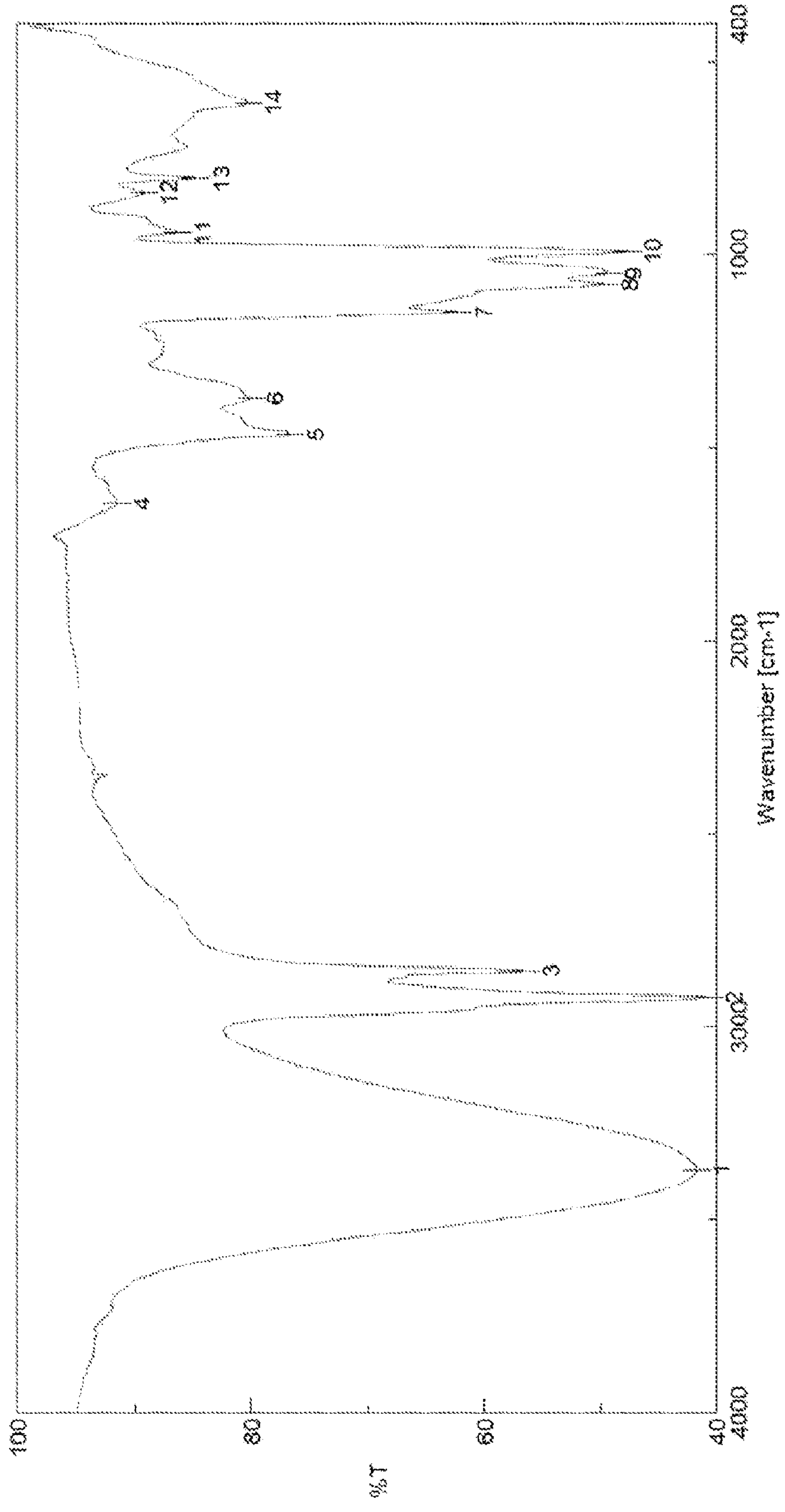
%T
100
80
60
40
4000
3000
2000
1000
400
Wavenumber [cm-1]
1
2
3
4
5
6
7
8
9
10
11
12
13
14

FIG. 3B

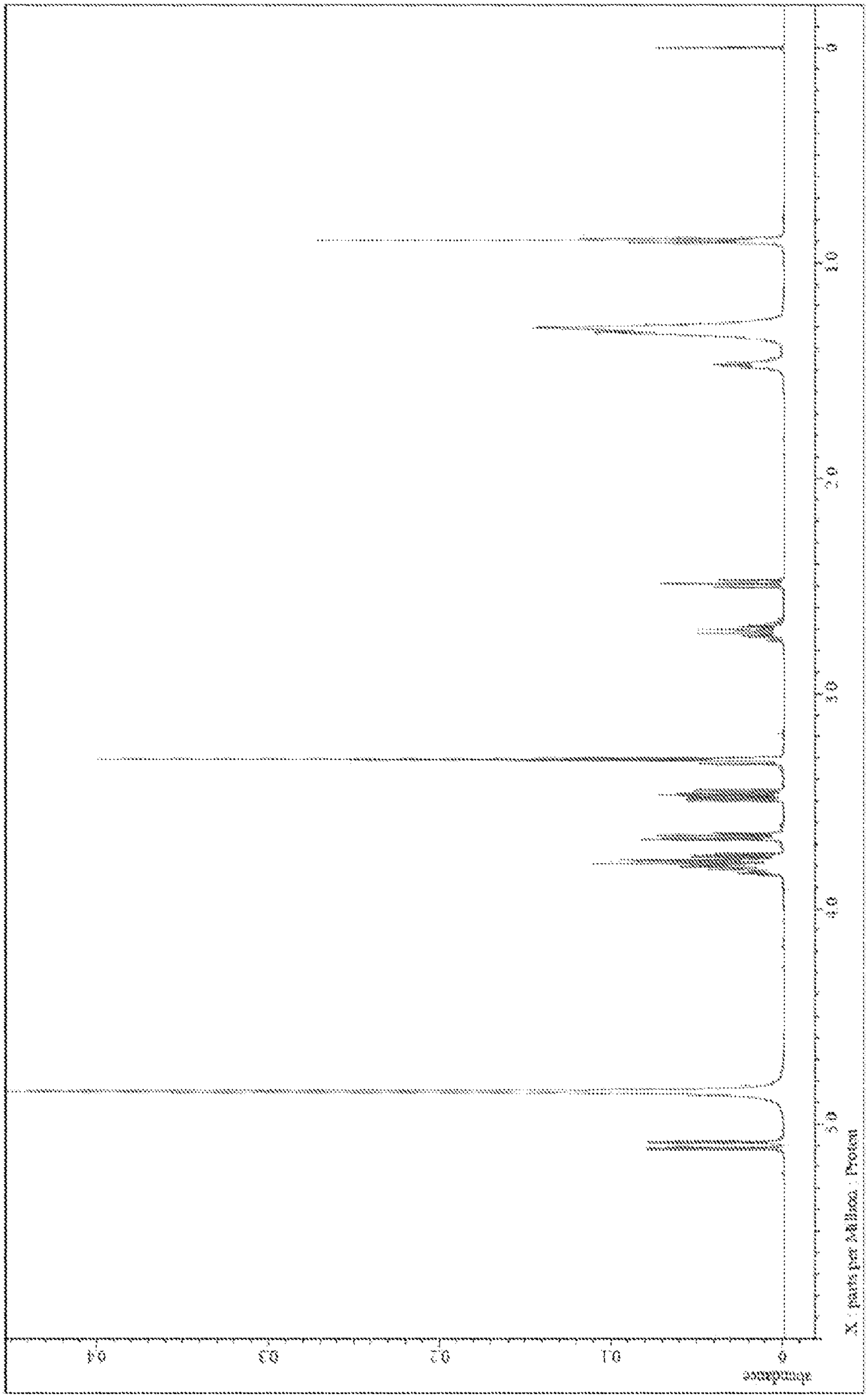

FIG. 3C

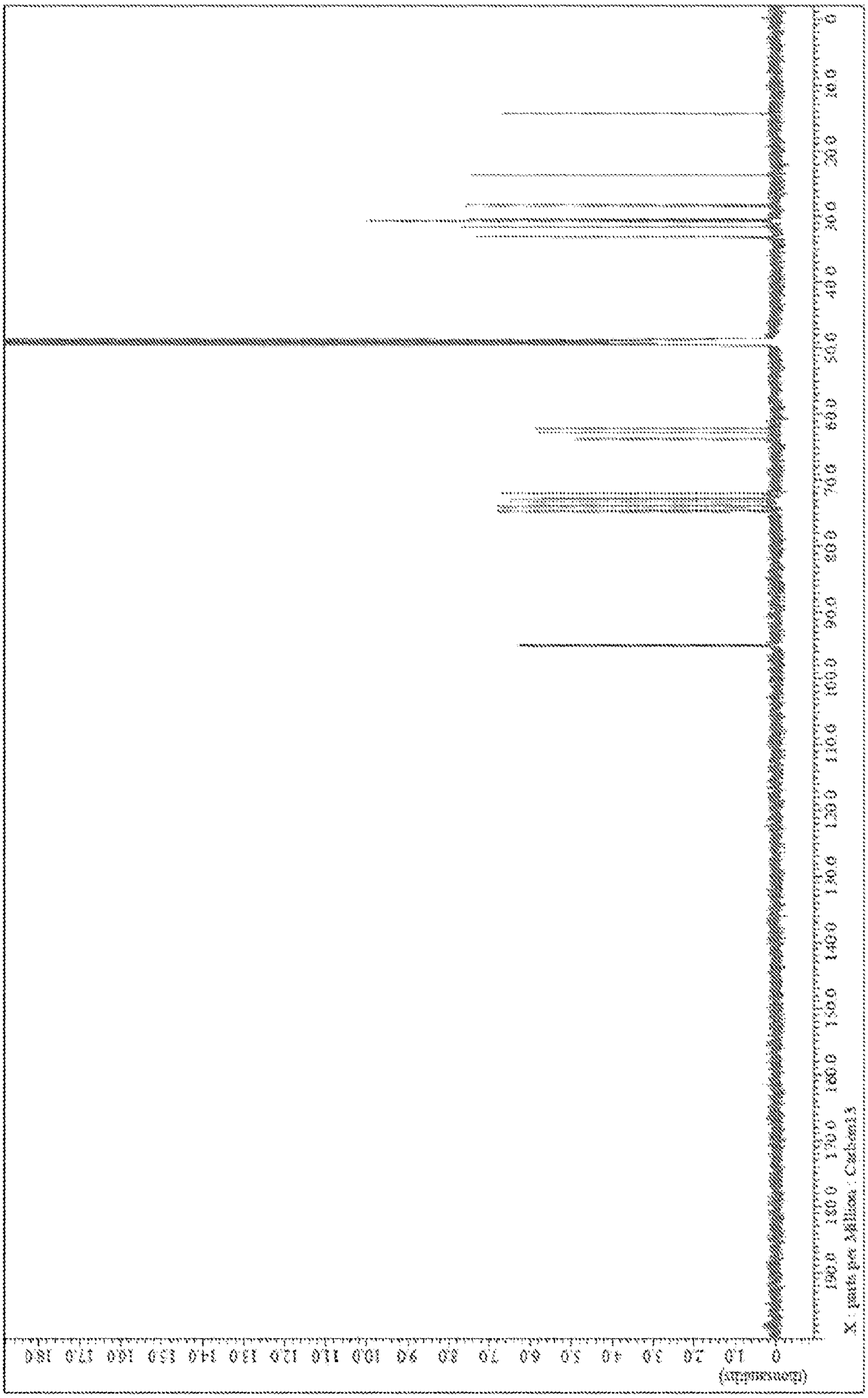

FIG. 4A

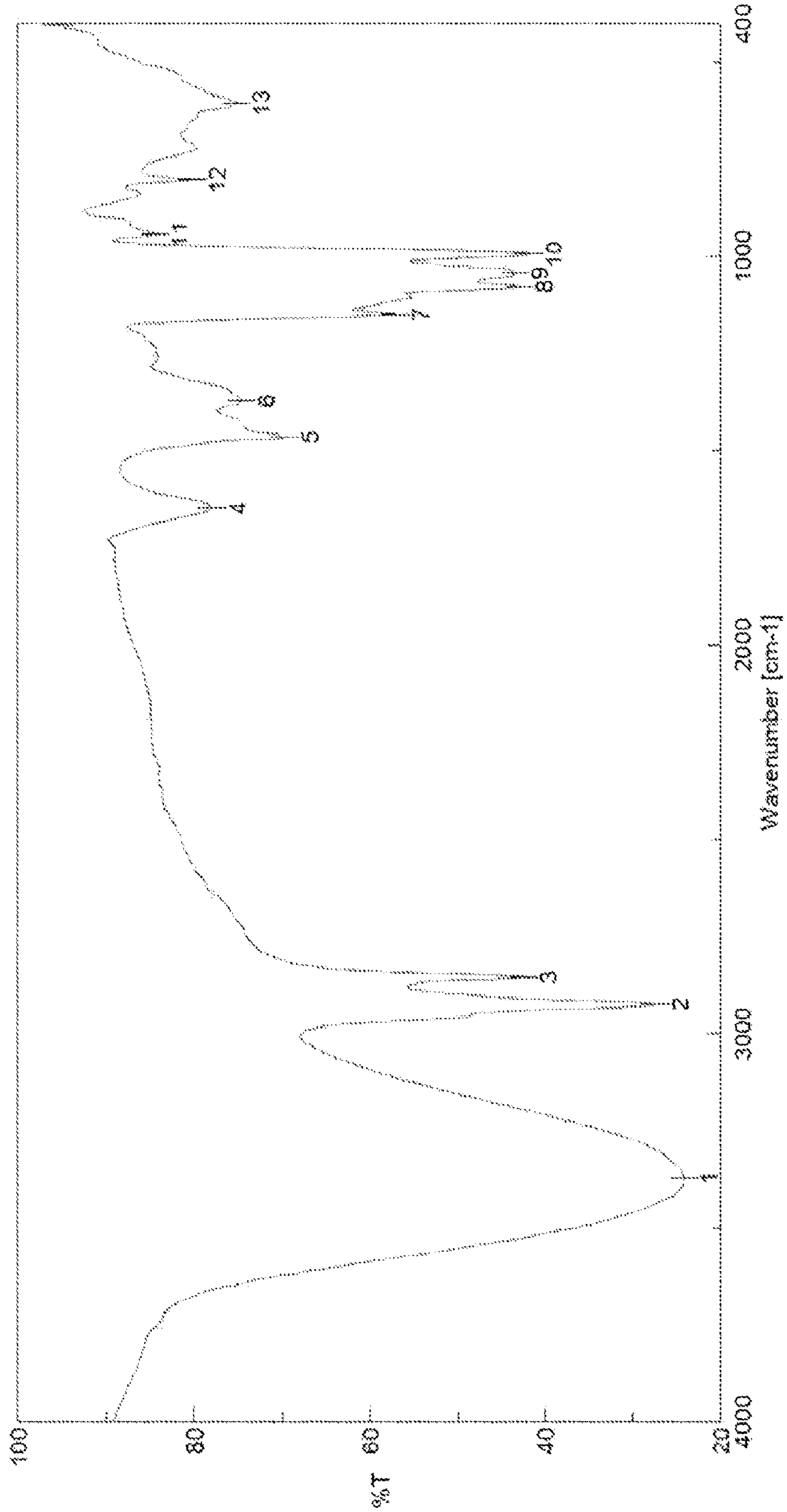
%T
100
80
60
40
20
4000
3000
2000
1000
400
Wavenumber [cm-1]
1
2
3
4
5
6
7
8
9
10
11
12
13

FIG. 4B

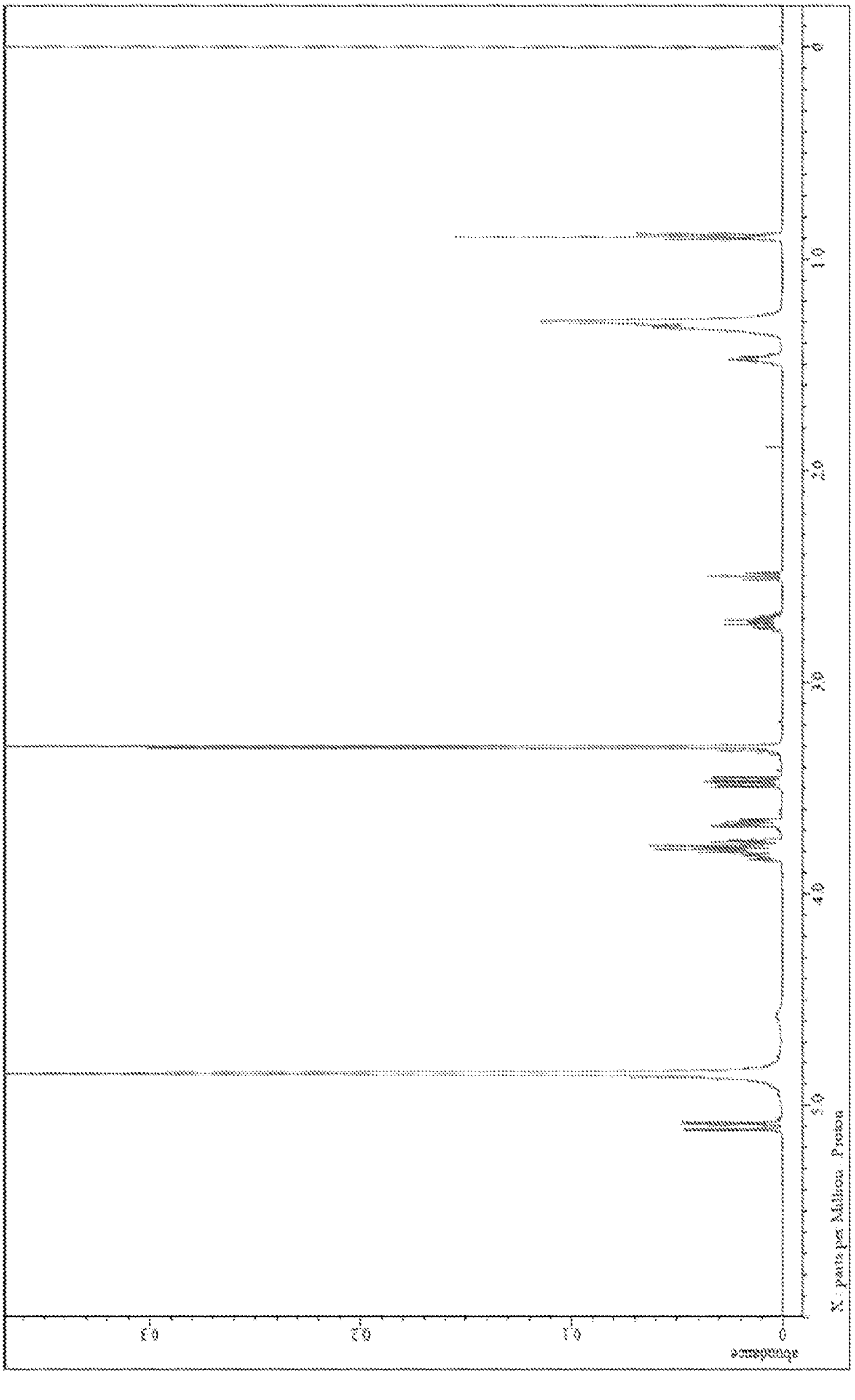

FIG. 4C

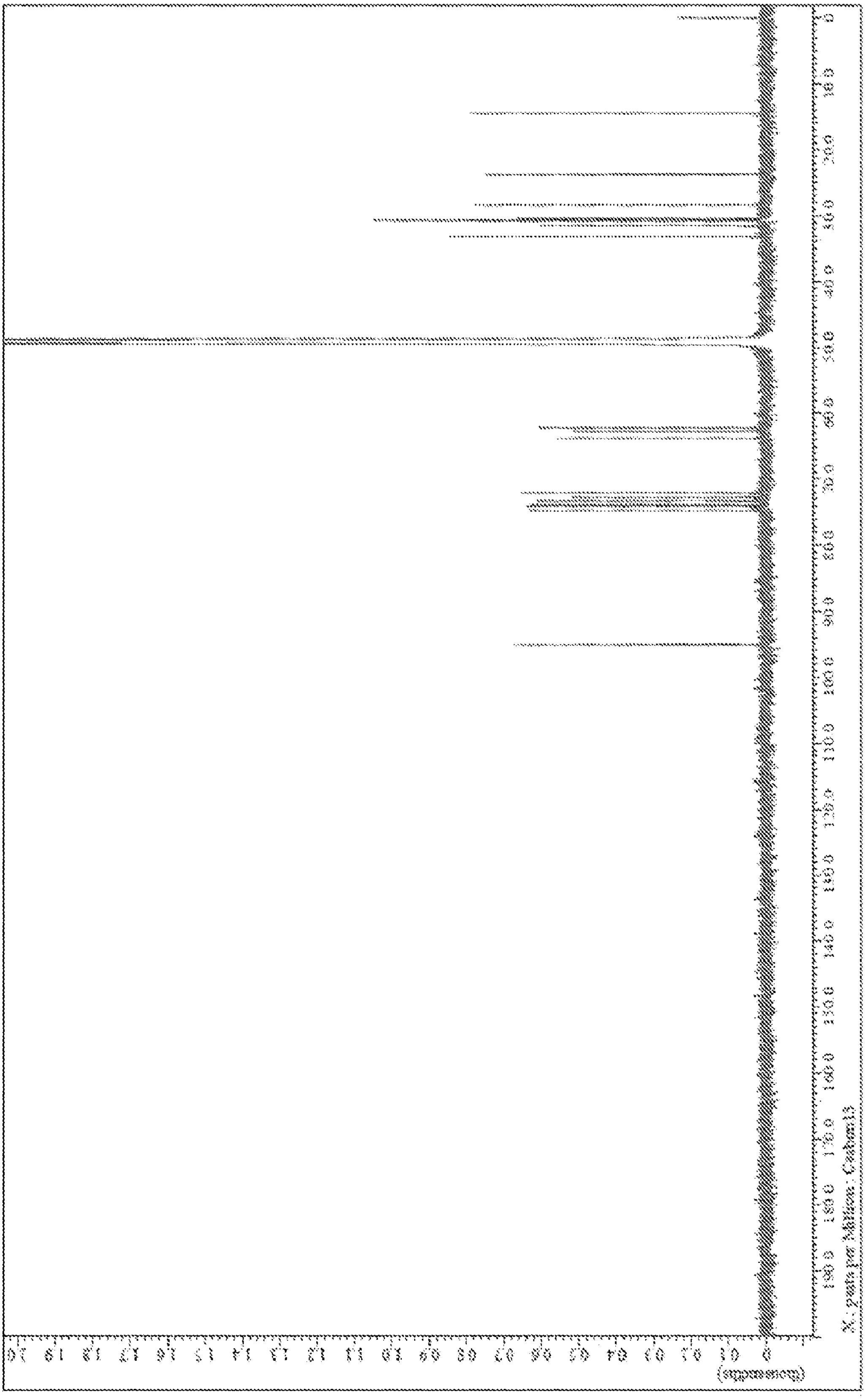

FIG. 5A

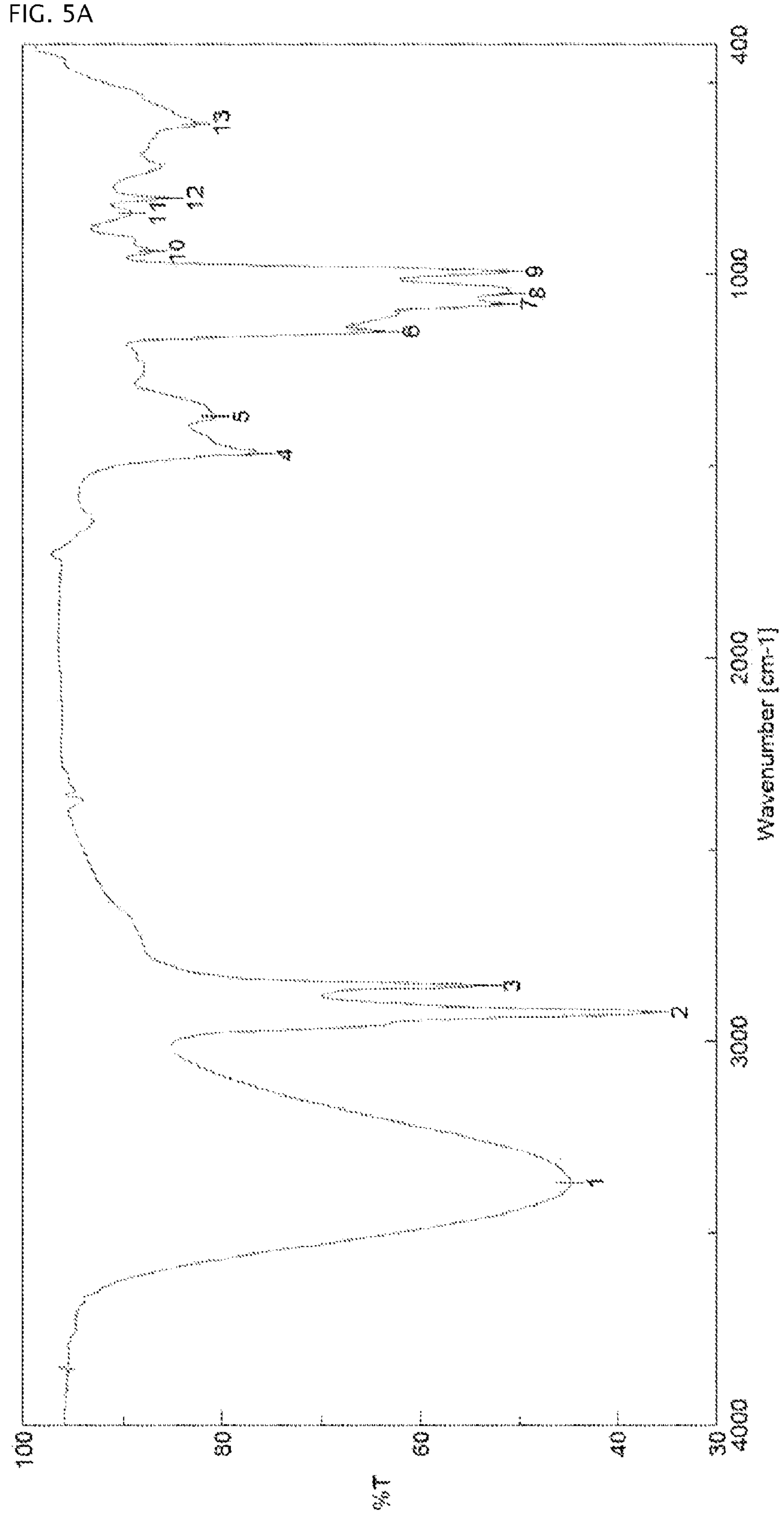
%T
Wavenumber [cm-1]
100
80
60
40
30
4000
3000
2000
1000
400
1
2
3
4
5
6
7
8
9
10
11
12
13

FIG. 5B

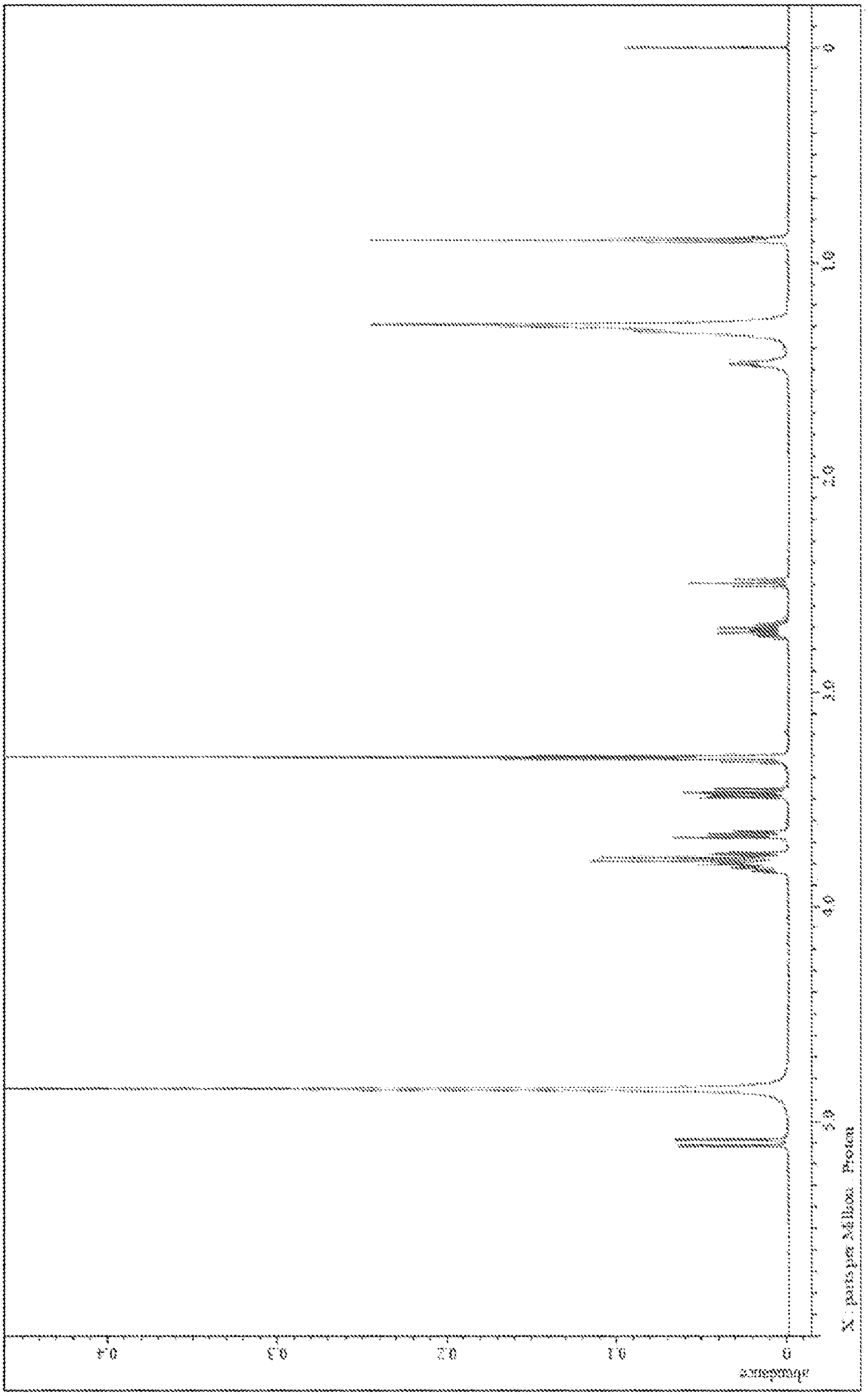

FIG. 5C

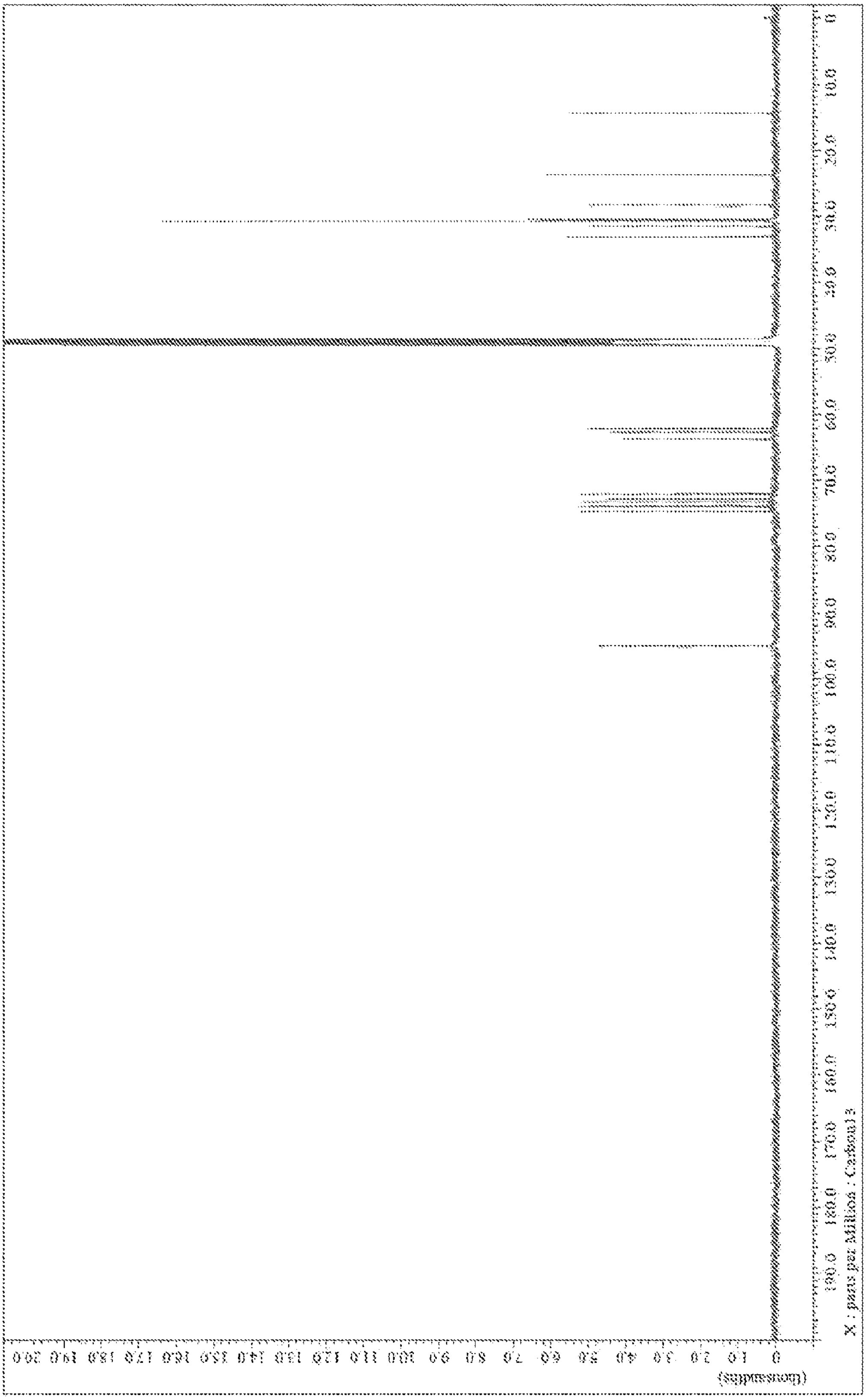

FIG. 6A

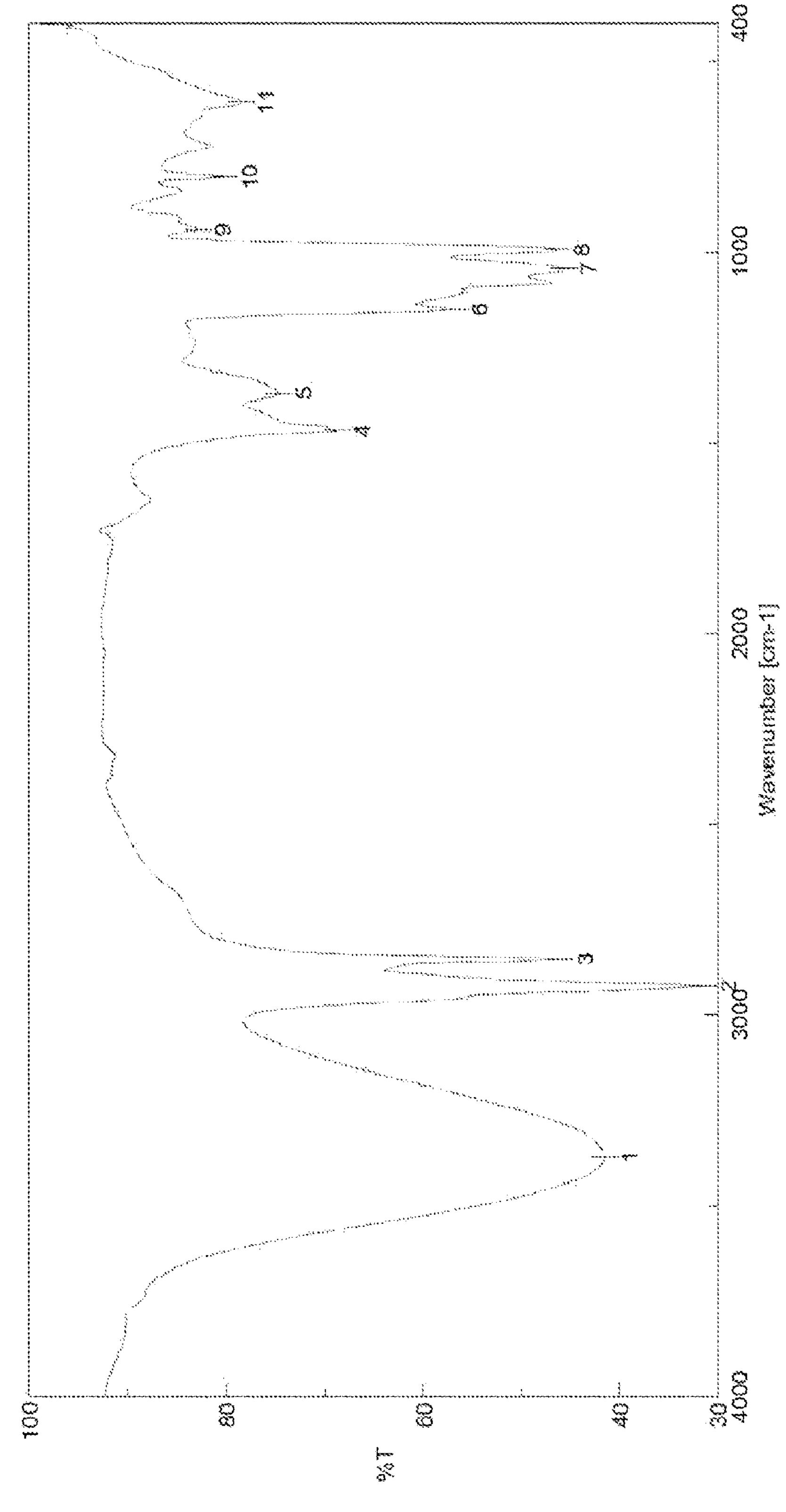
%T
100
80
60
40
30
4000
3000
2000
1000
400
Wavenumber [cm-1]
1
2
3
4
5
6
7
8
9
10
11

FIG. 6B

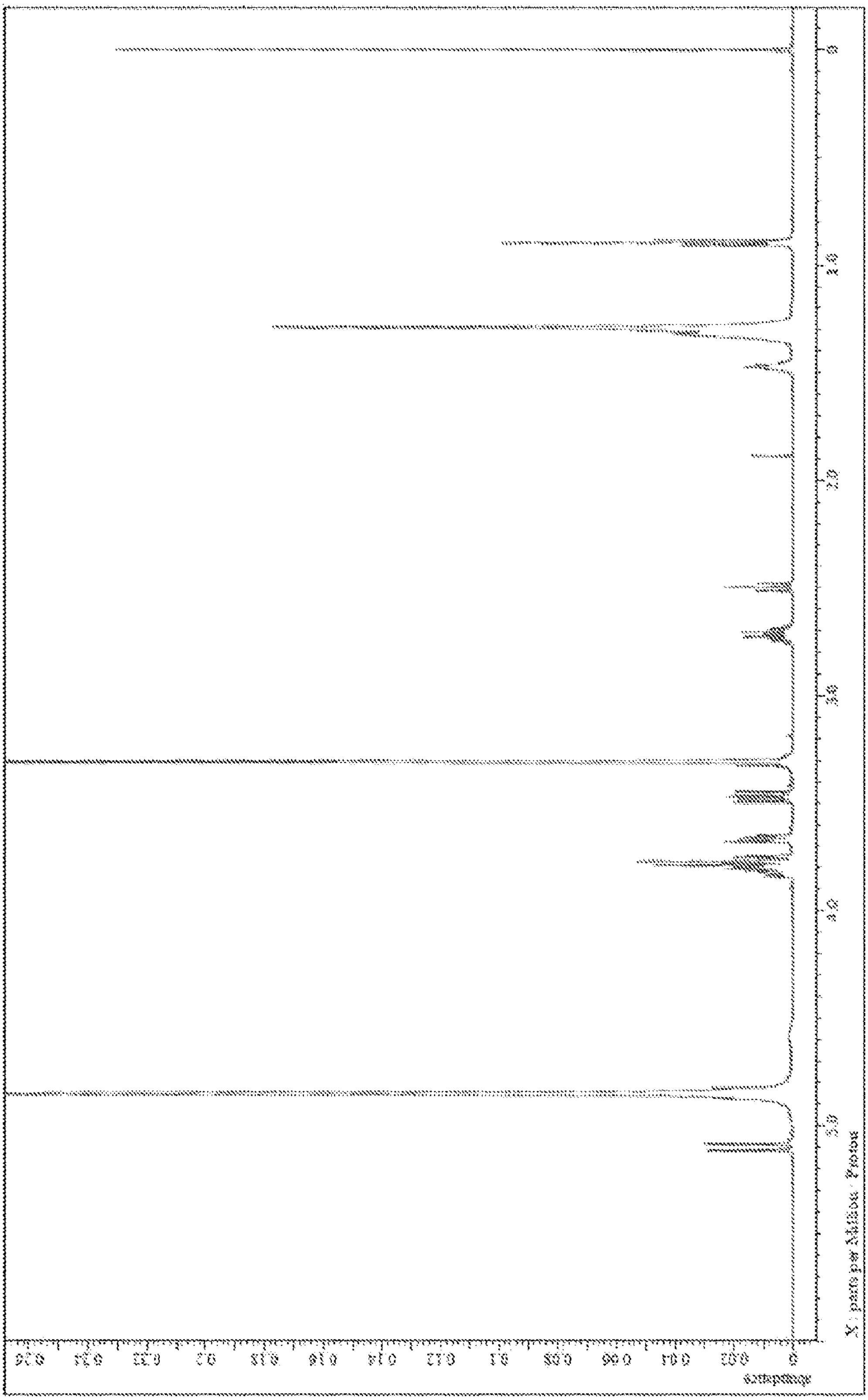

FIG. 6C

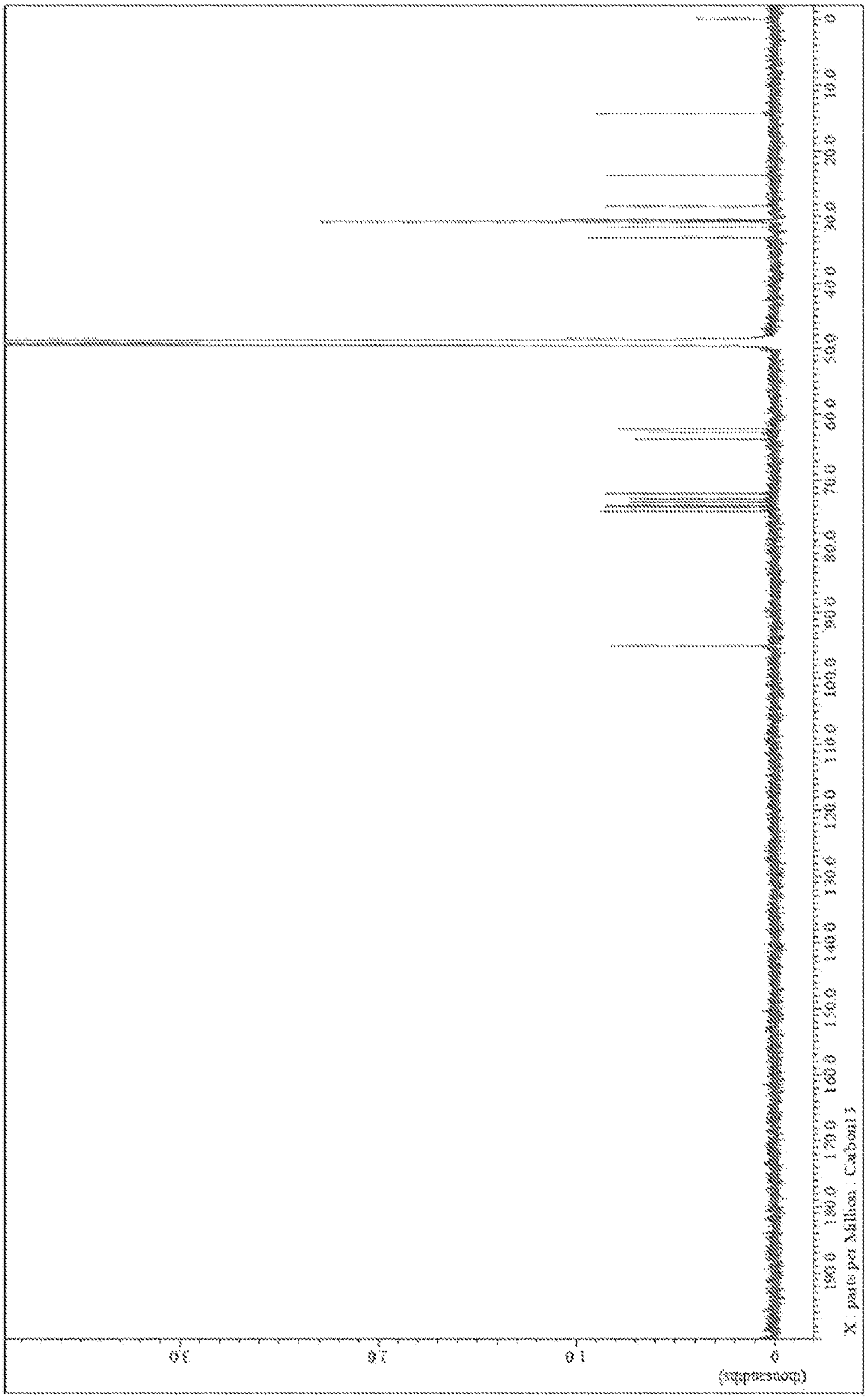

FIG. 7A

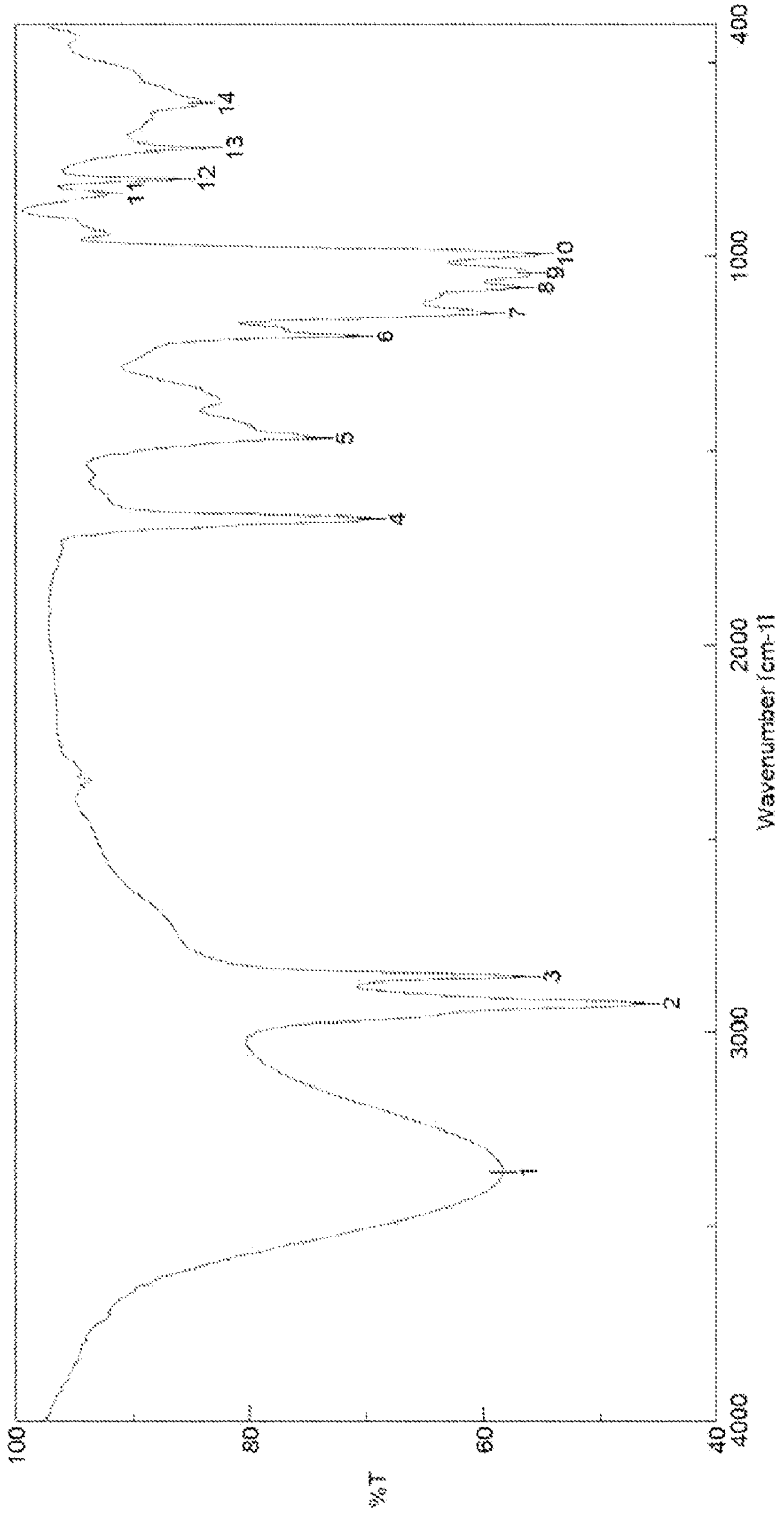

100
80
60
40
%T
4000
3000
2000
1000
400
Wavenumber [cm-1]
1
2
3
4
5
6
7
8
9
10
11
12
13
14

FIG. 7B

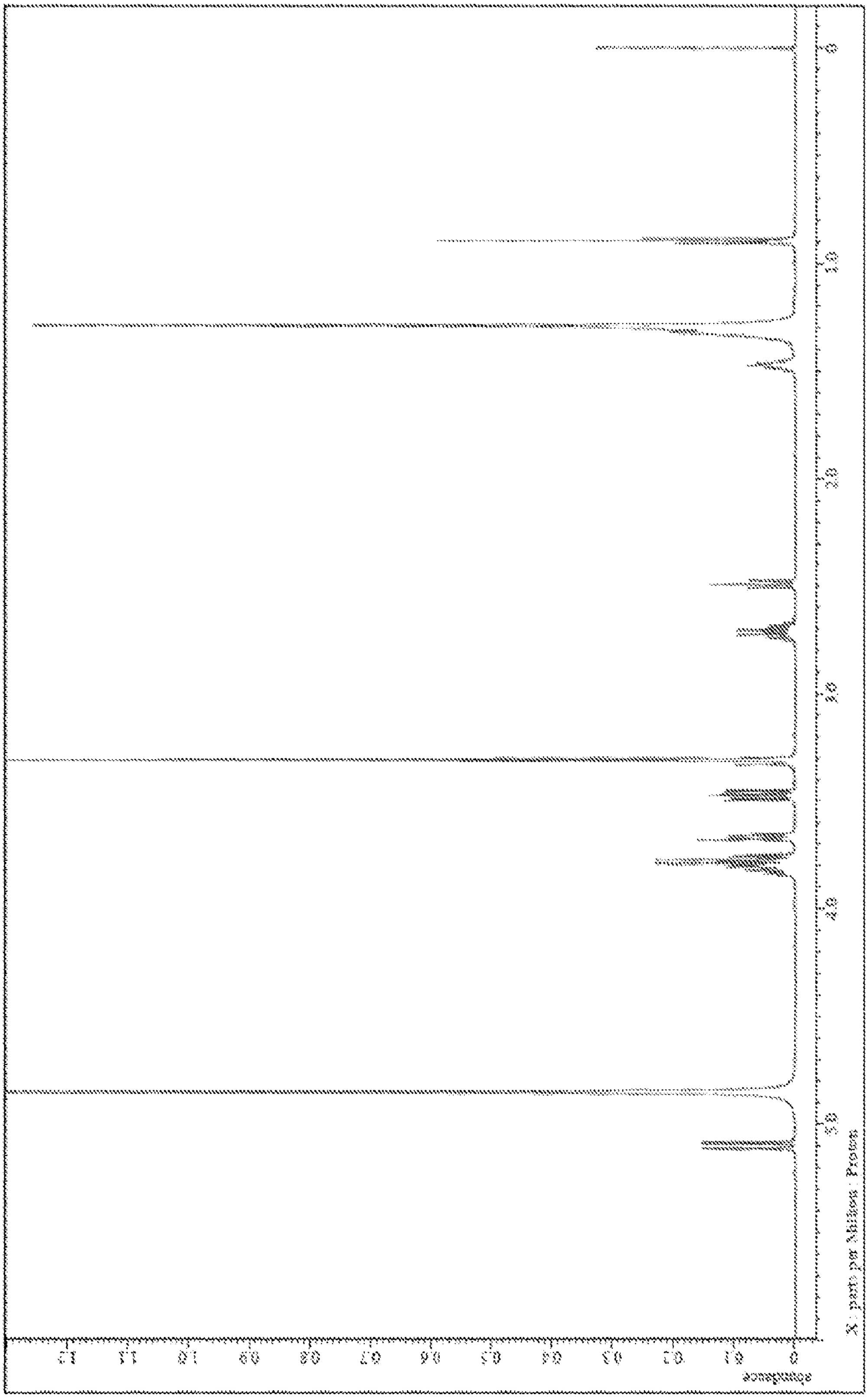

FIG. 7C

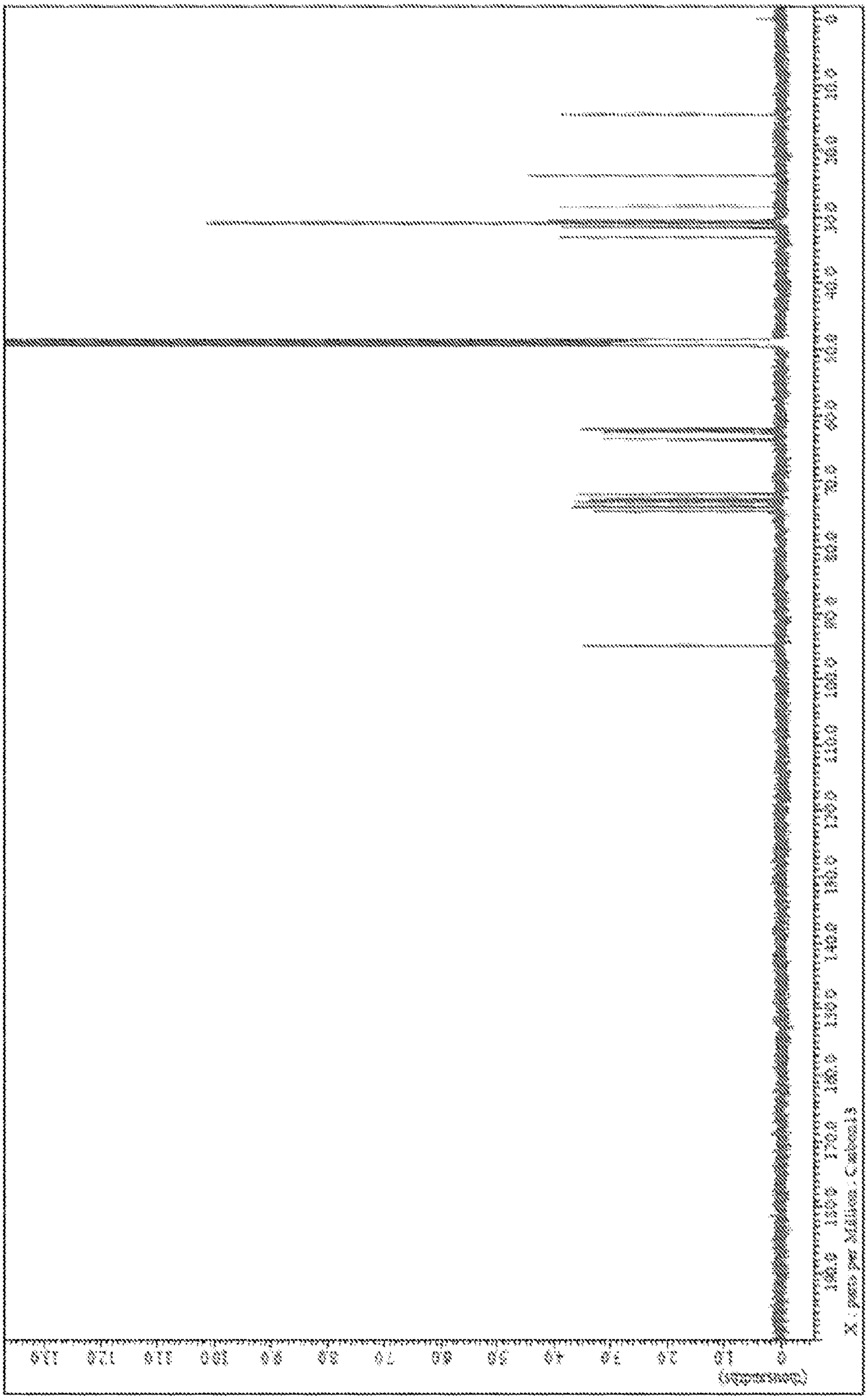

FIG. 8A

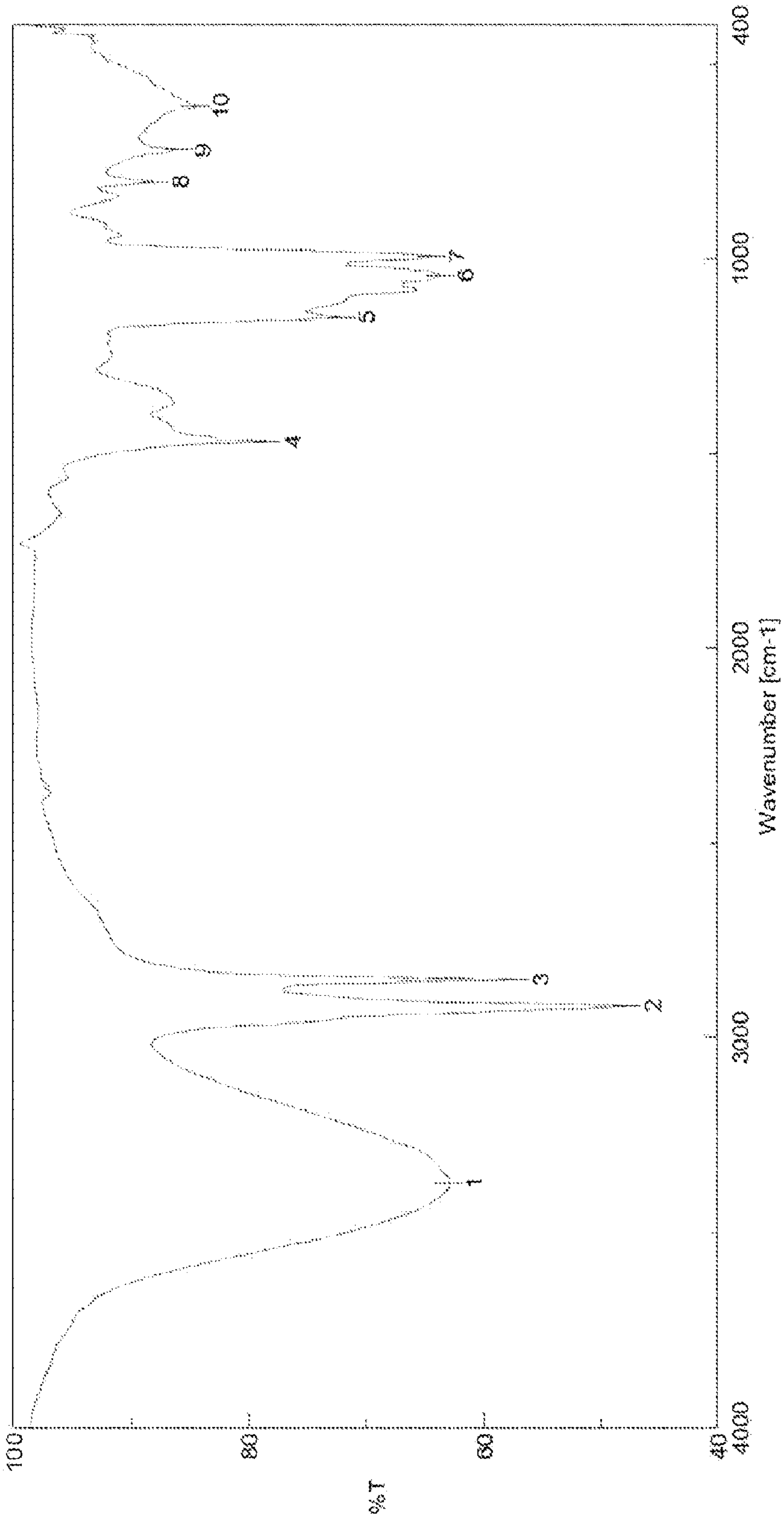
%T
100
80
60
40
4000
3000
2000
1000
400
Wavenumber [cm-1]
1
2
3
4
5
6
7
8
9
10

FIG. 8B

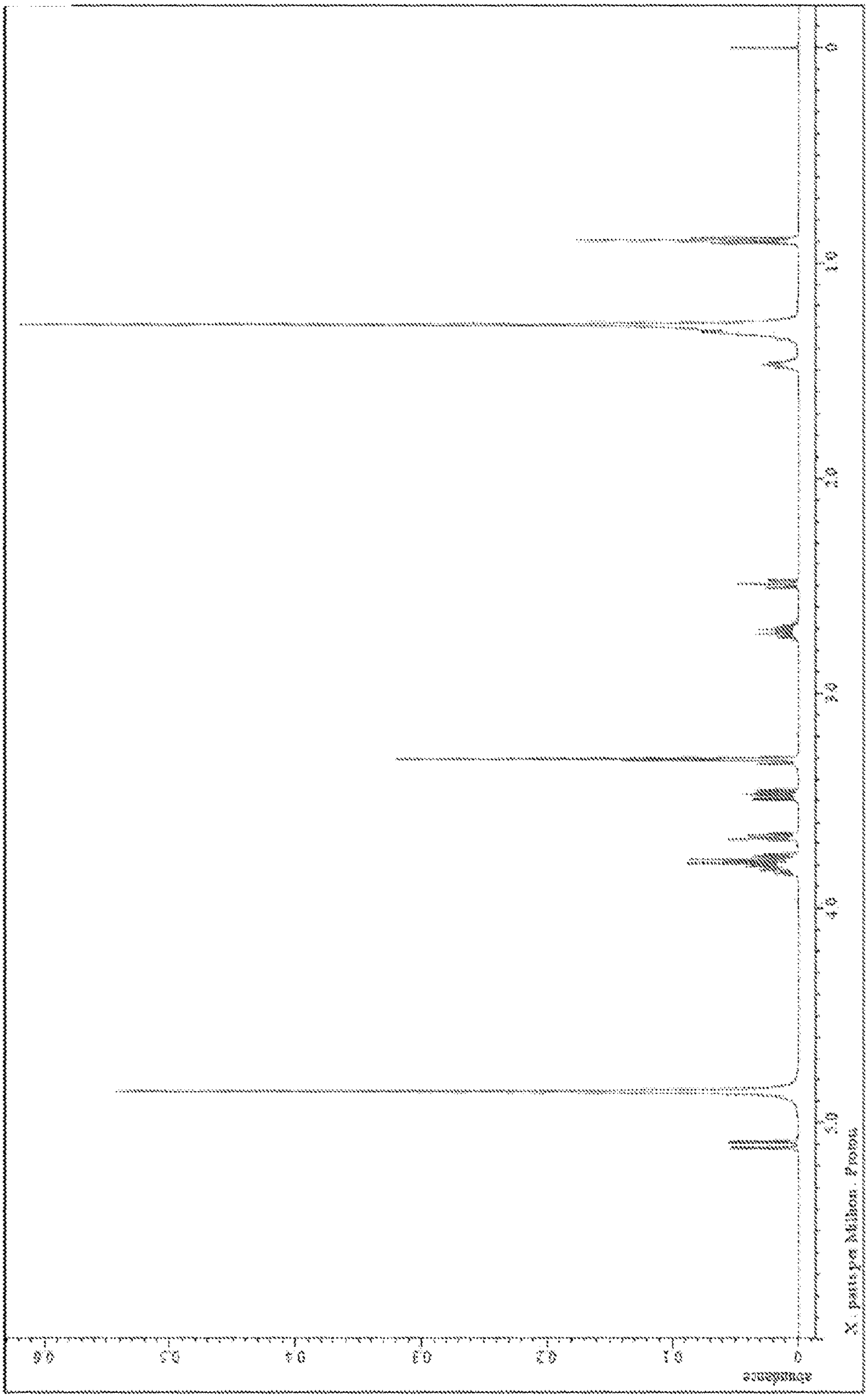

FIG. 8C

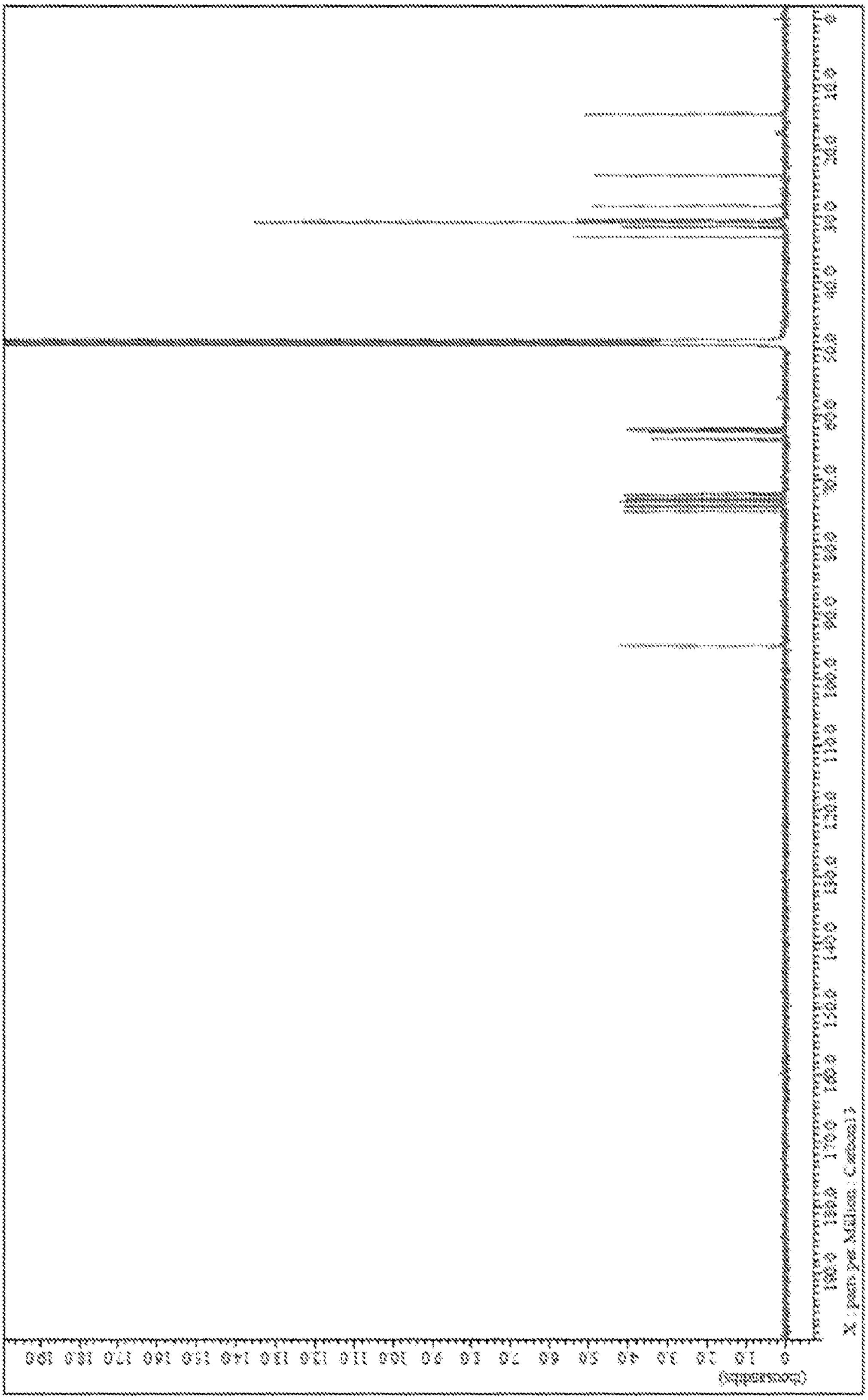

FIG. 9A

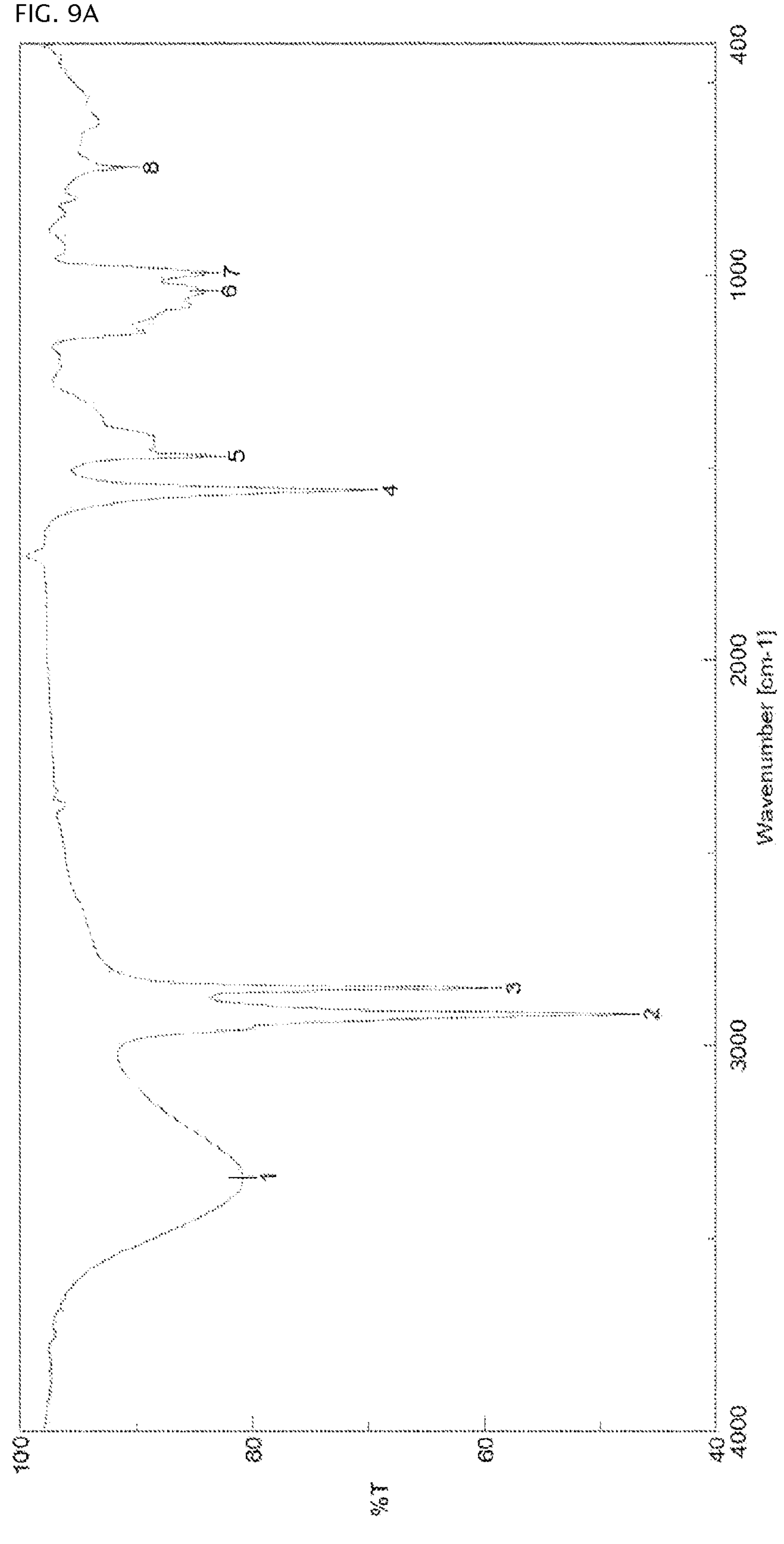
%T
100
80
60
40
4000
3000
2000
1000
400
Wavenumber [cm-1]
1
2
3
4
5
6 7
8

FIG. 9B

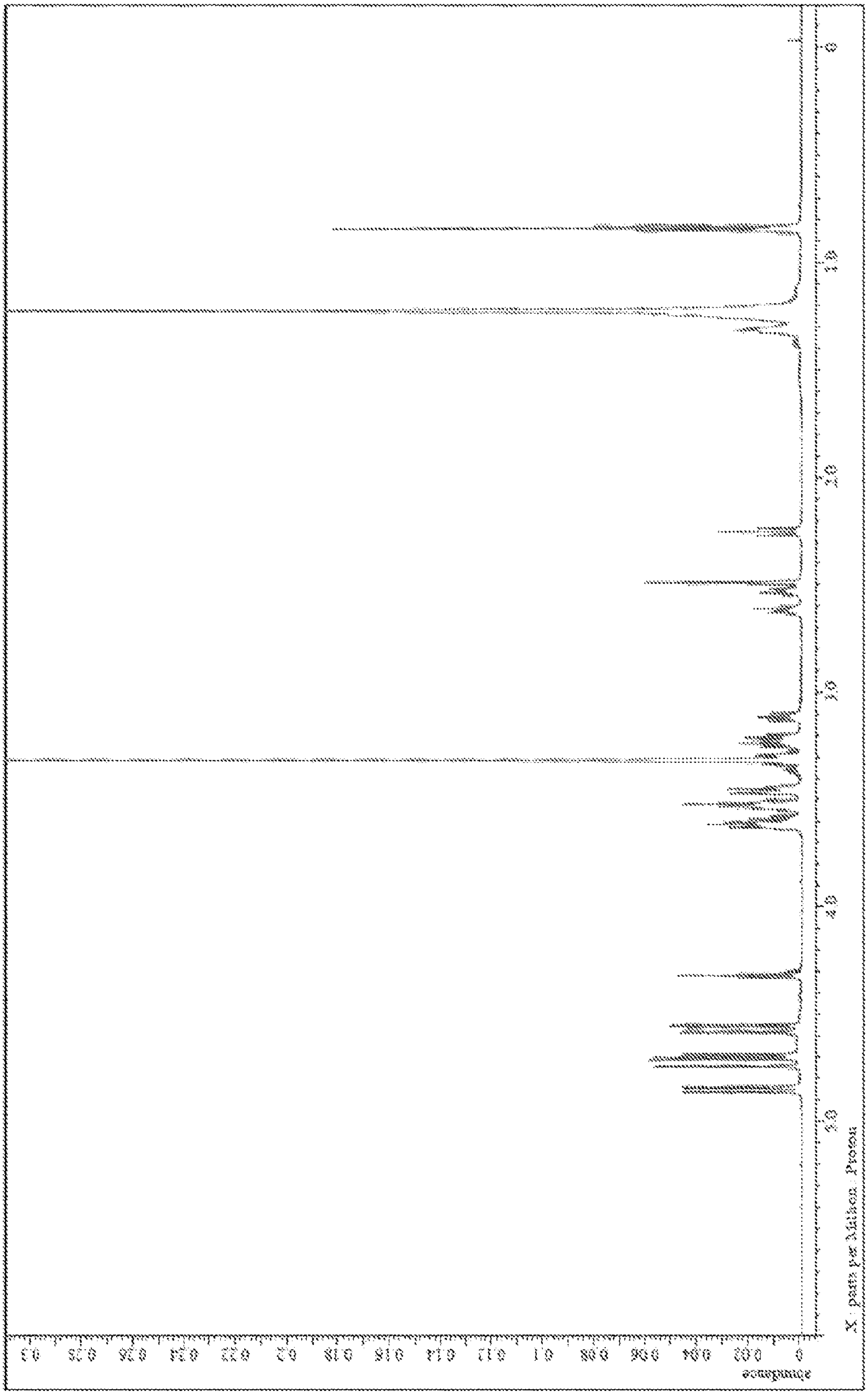

FIG. 9C

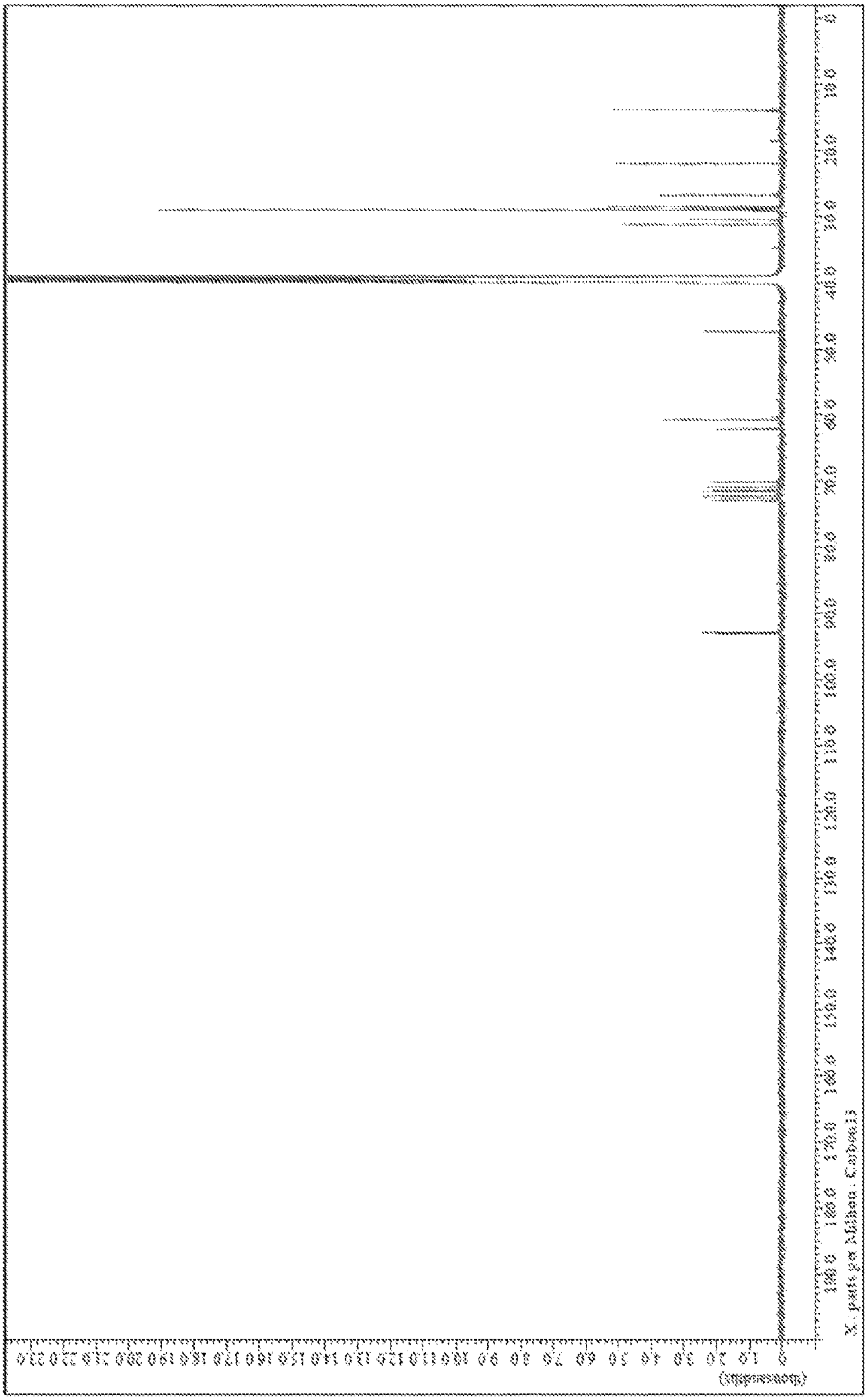

FIG. 10A

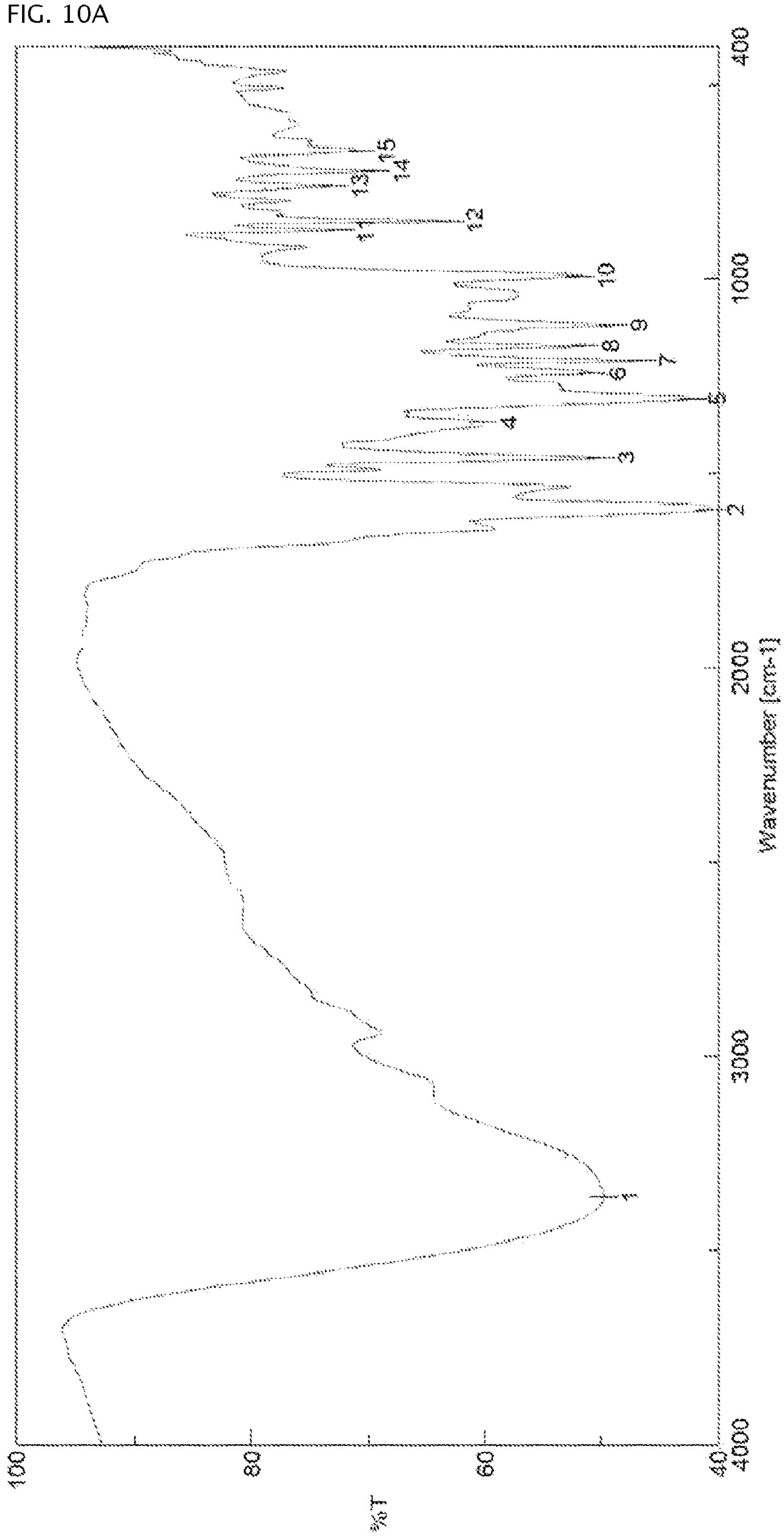
%T
100
80
60
40
4000
3000
2000
1000
400
Wavenumber [cm-1]
1
2
3
4
5
6
7
8
9
10
11
12
13
14
15

FIG. 10B

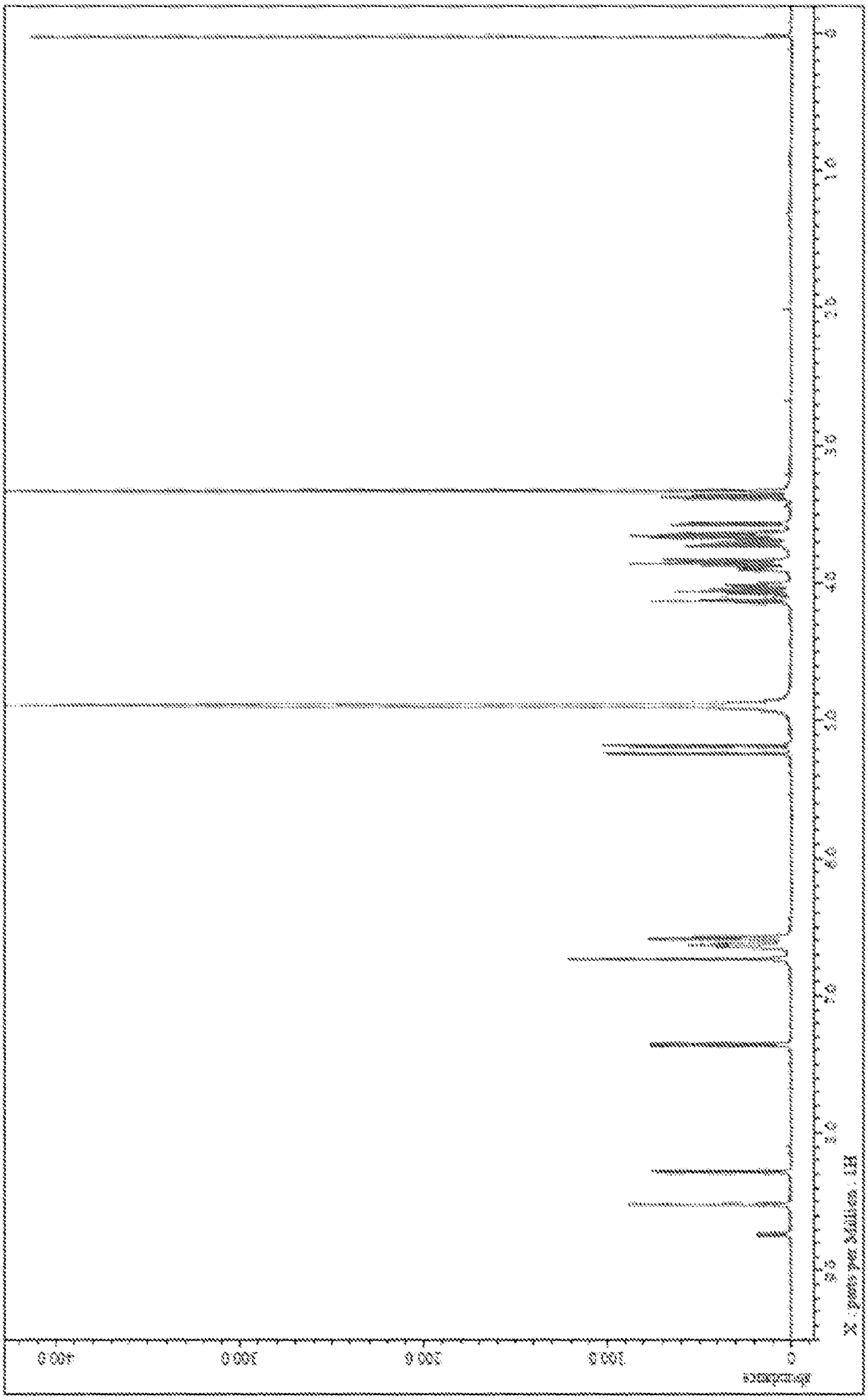

FIG. 10C

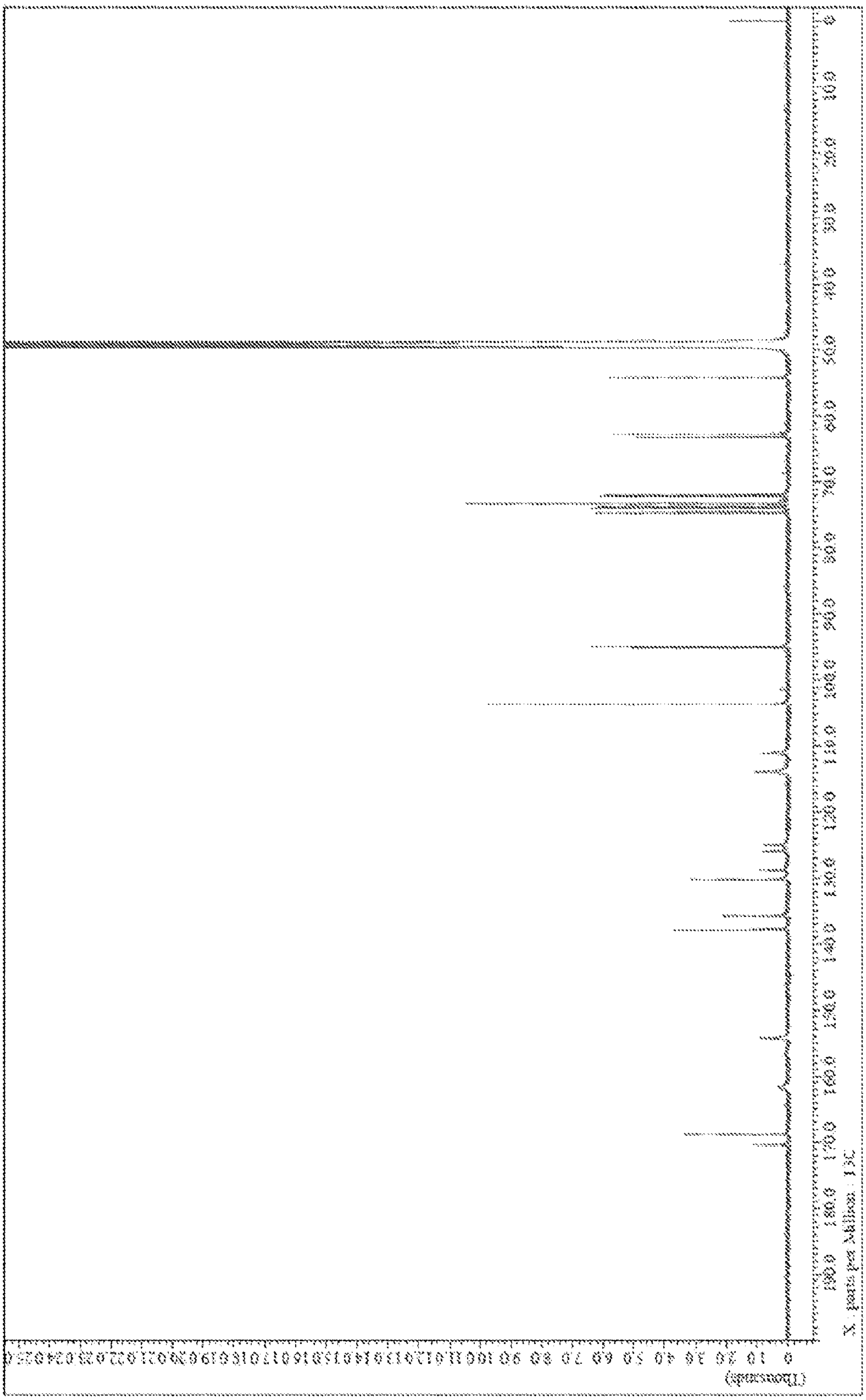

FIG. 11A

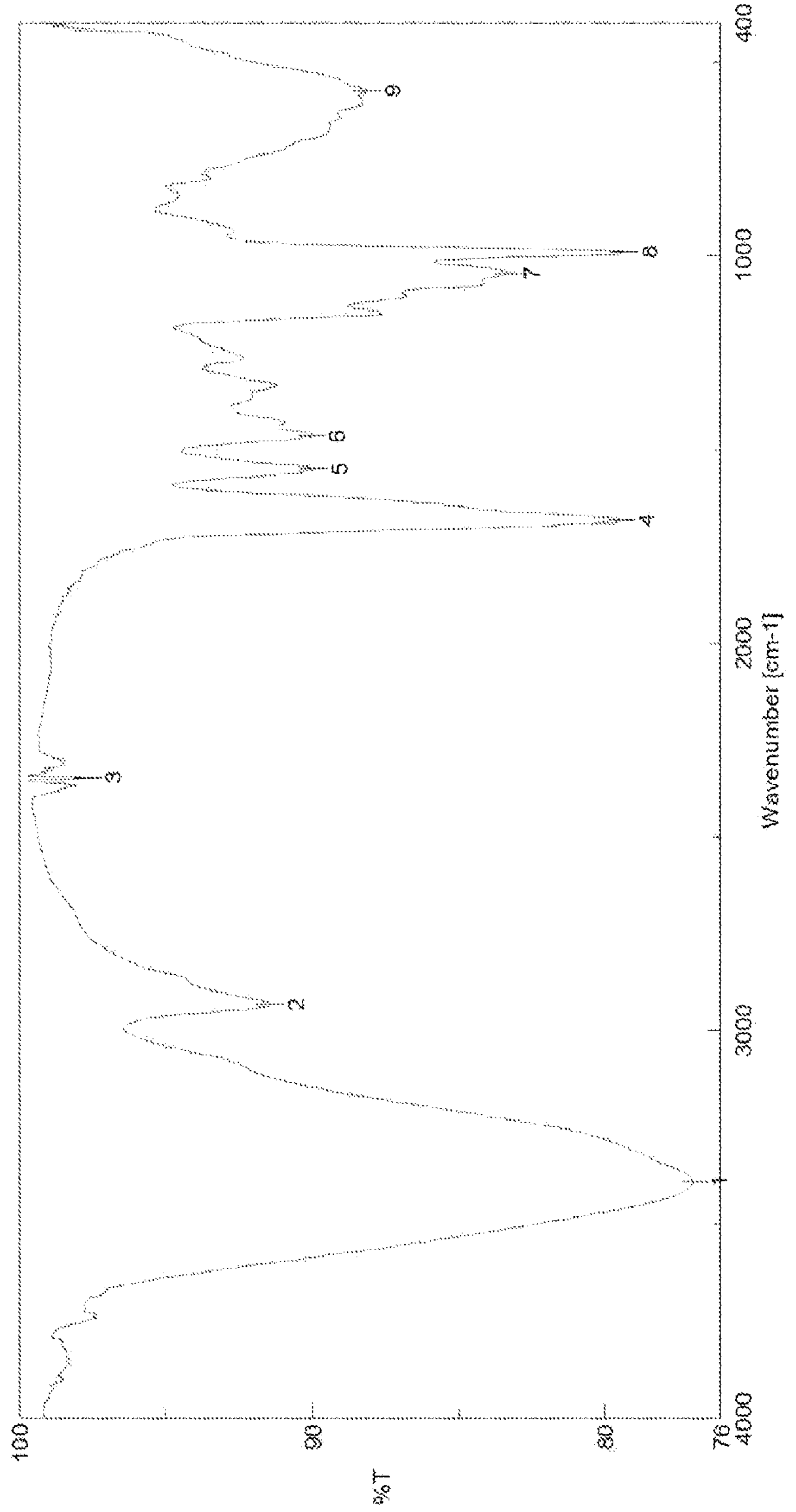
100
90
80
76
%T
4000
3000
2000
1000
400
Wavenumber [cm-1]
1
2
3
4
5
6
7
8
9

FIG. 11B

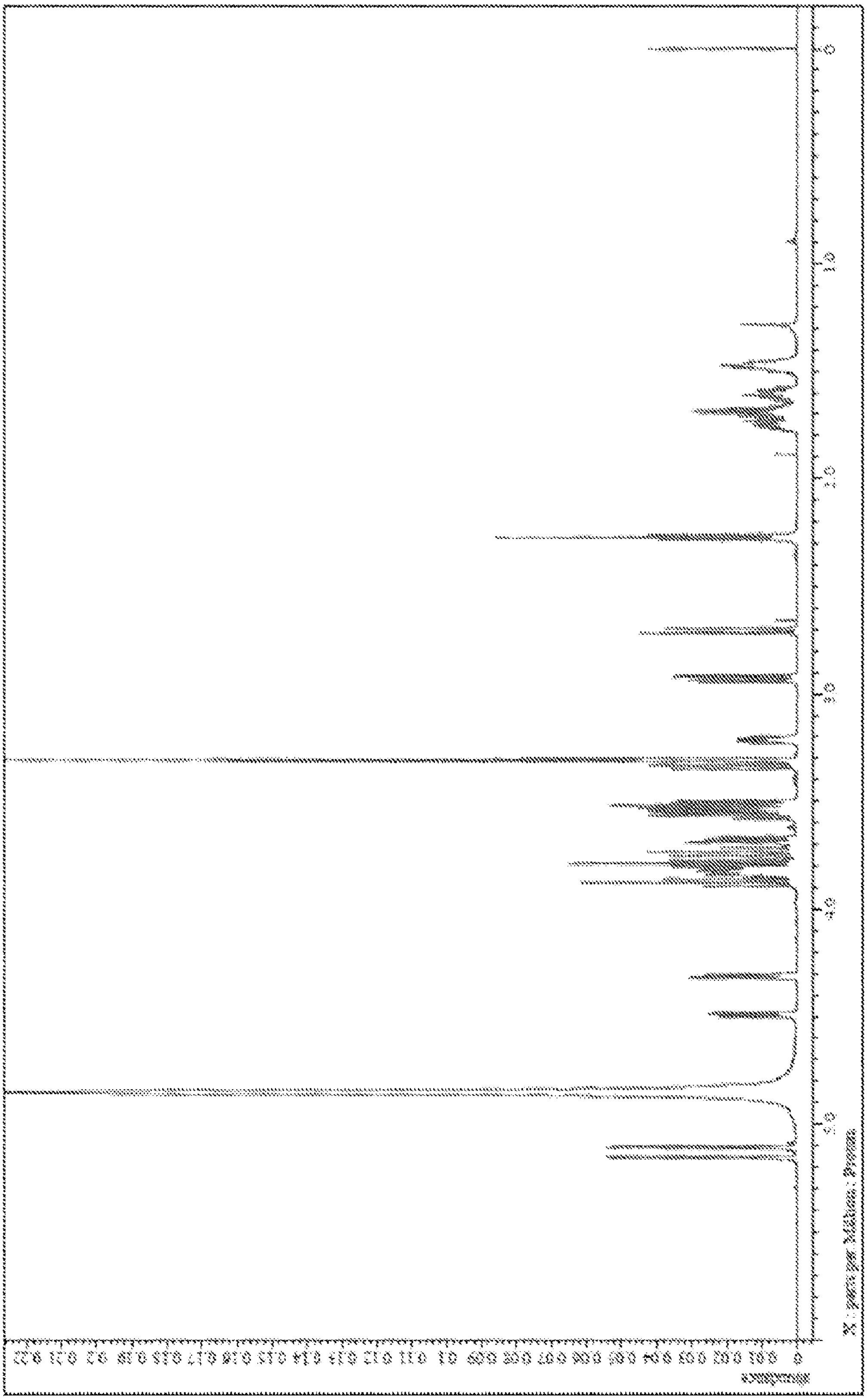

FIG. 11C

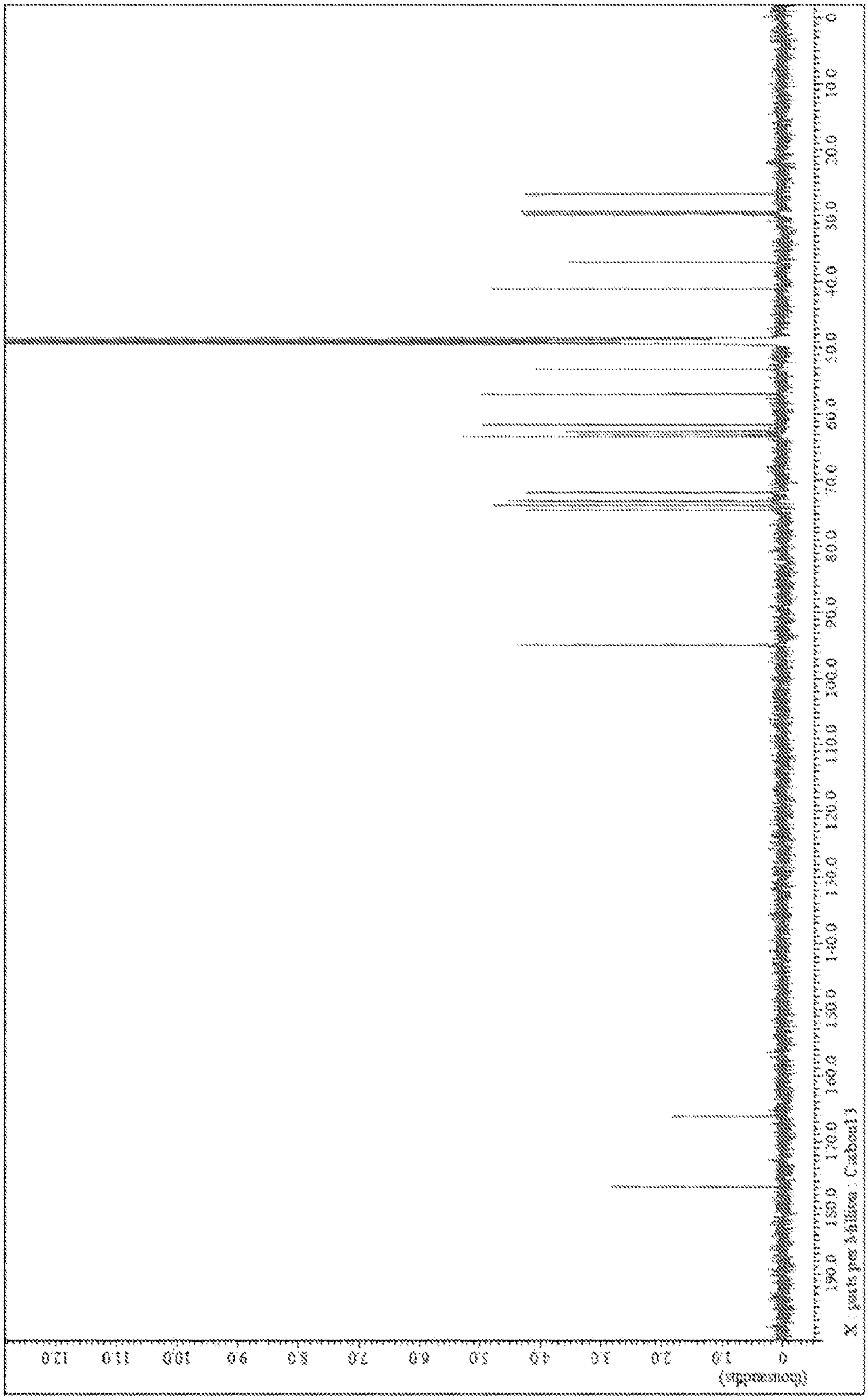

FIG. 12A

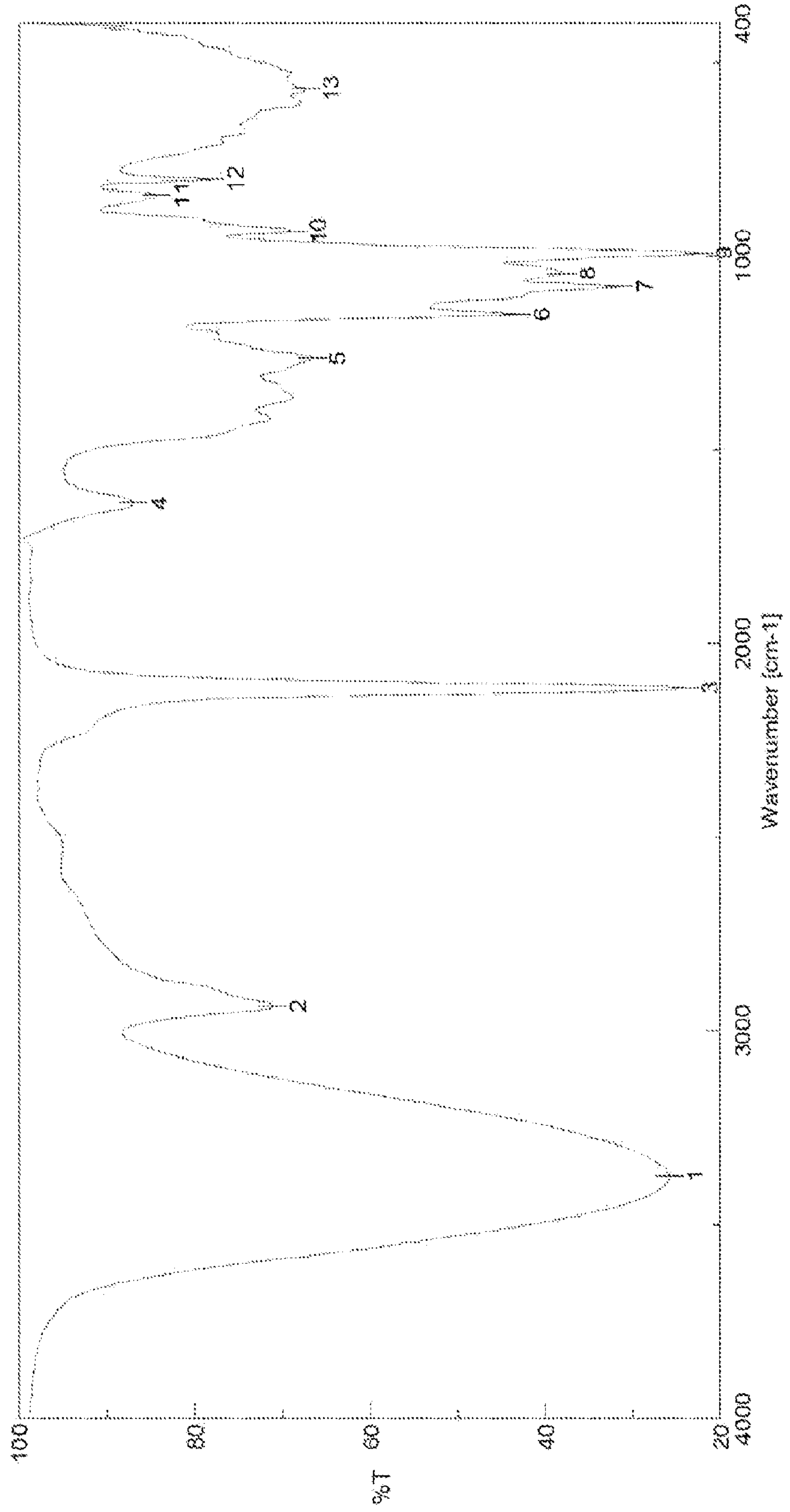
%T
100
80
60
40
20
4000
3000
2000
1000
400
Wavenumber [cm-1]
1
2
3
4
5
6
7
8
9
10
11
12
13

FIG. 12B

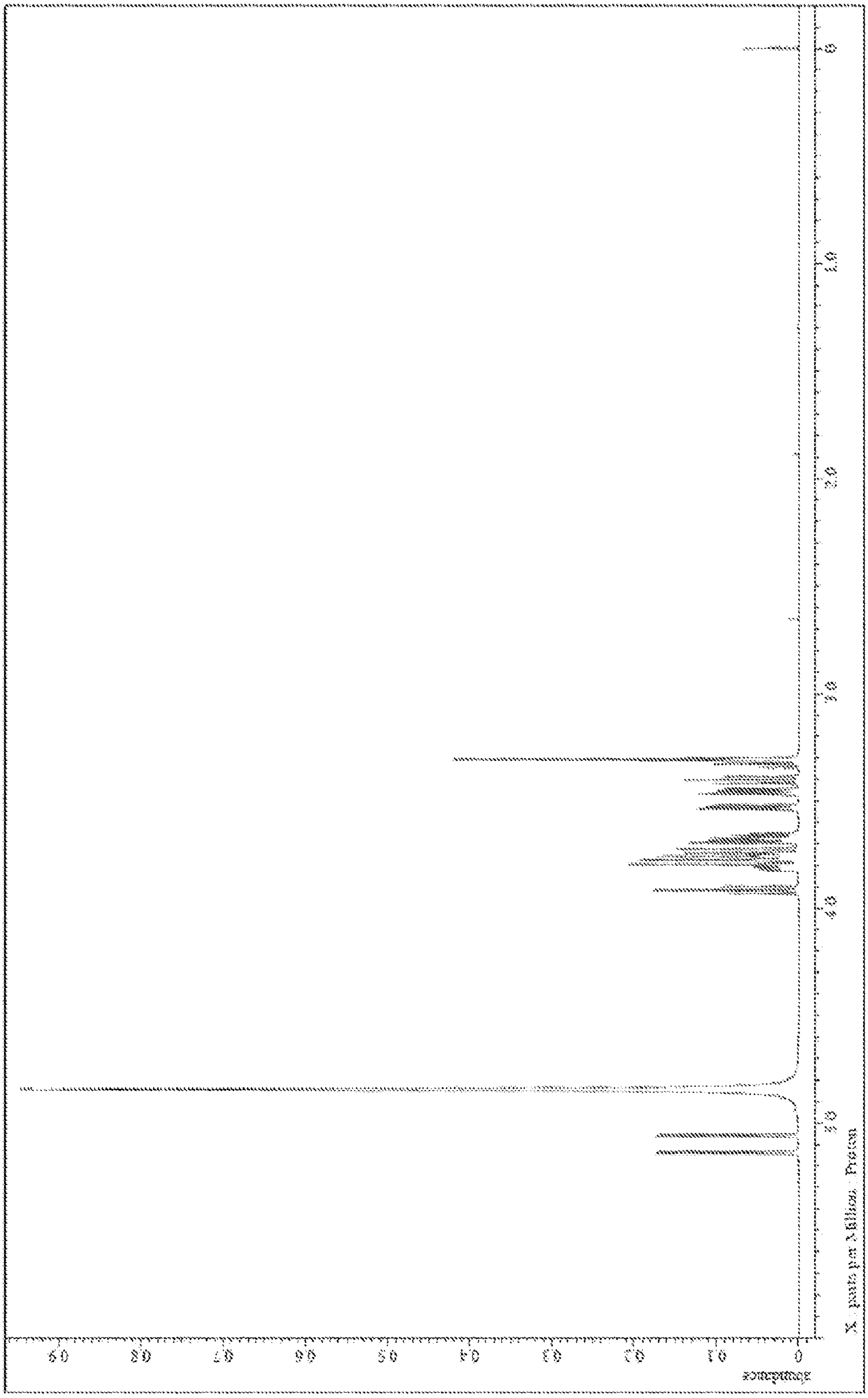

FIG. 12C

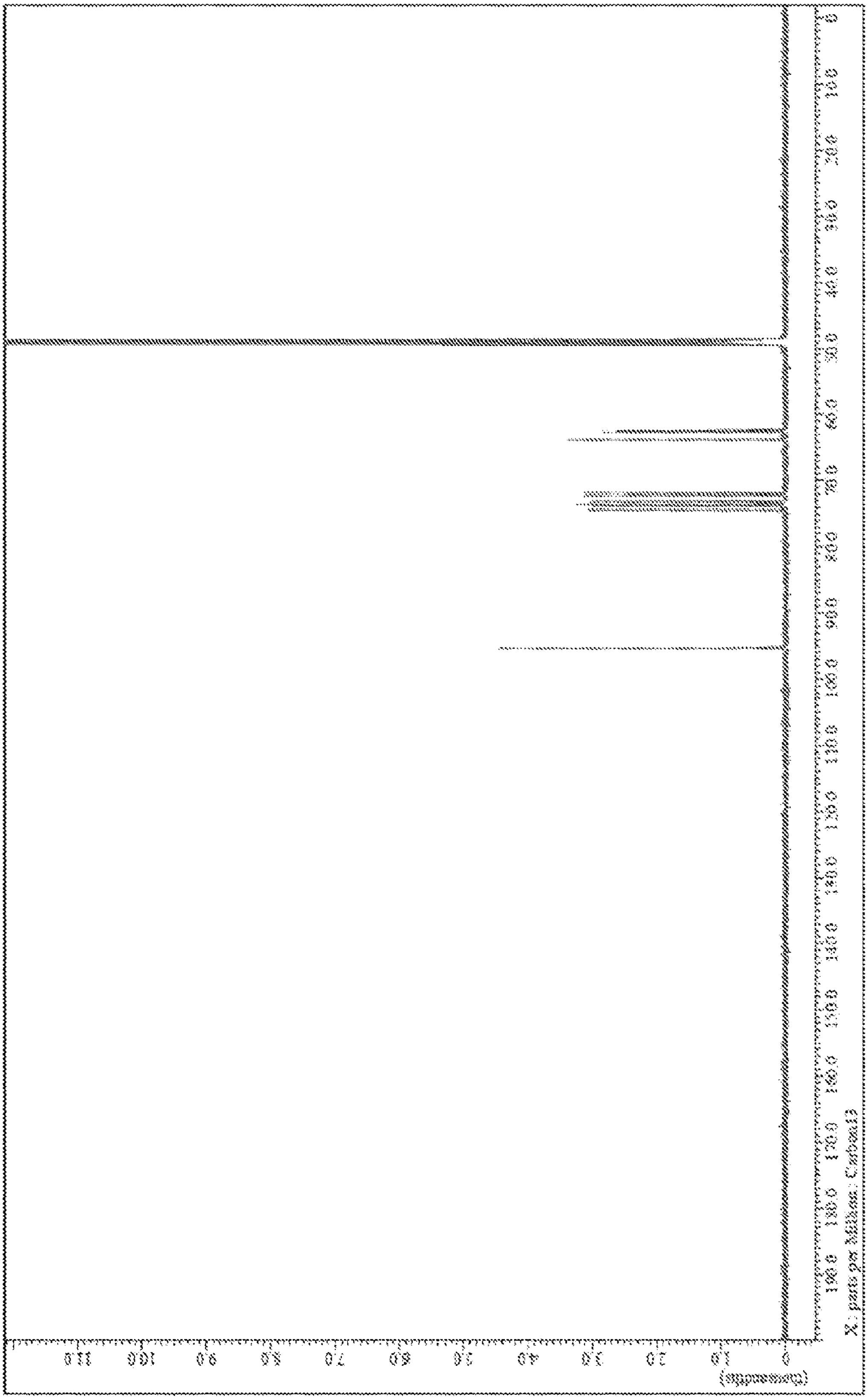

FIG. 13A

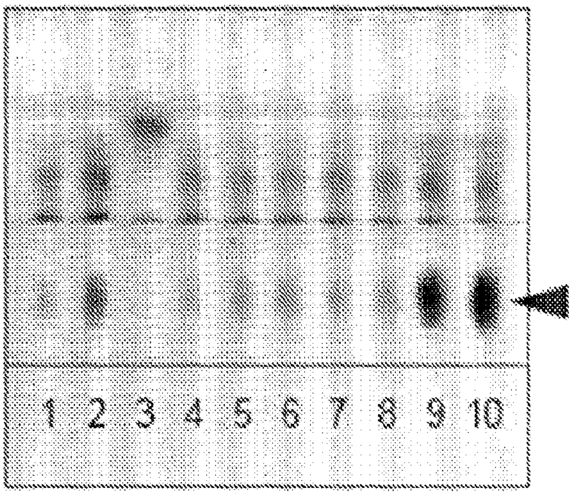


FIG. 13B

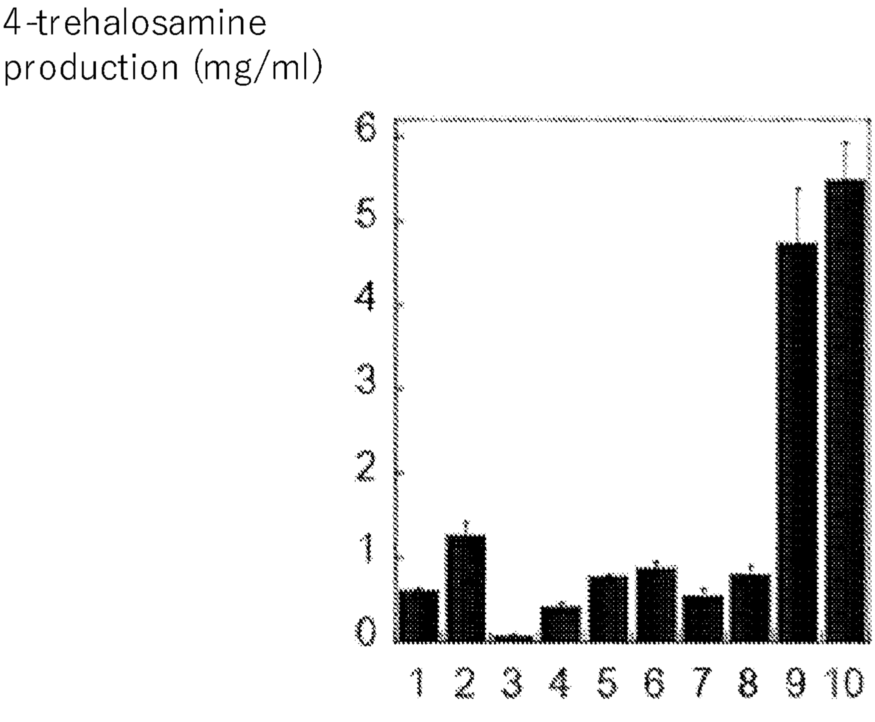

FIG. 14A

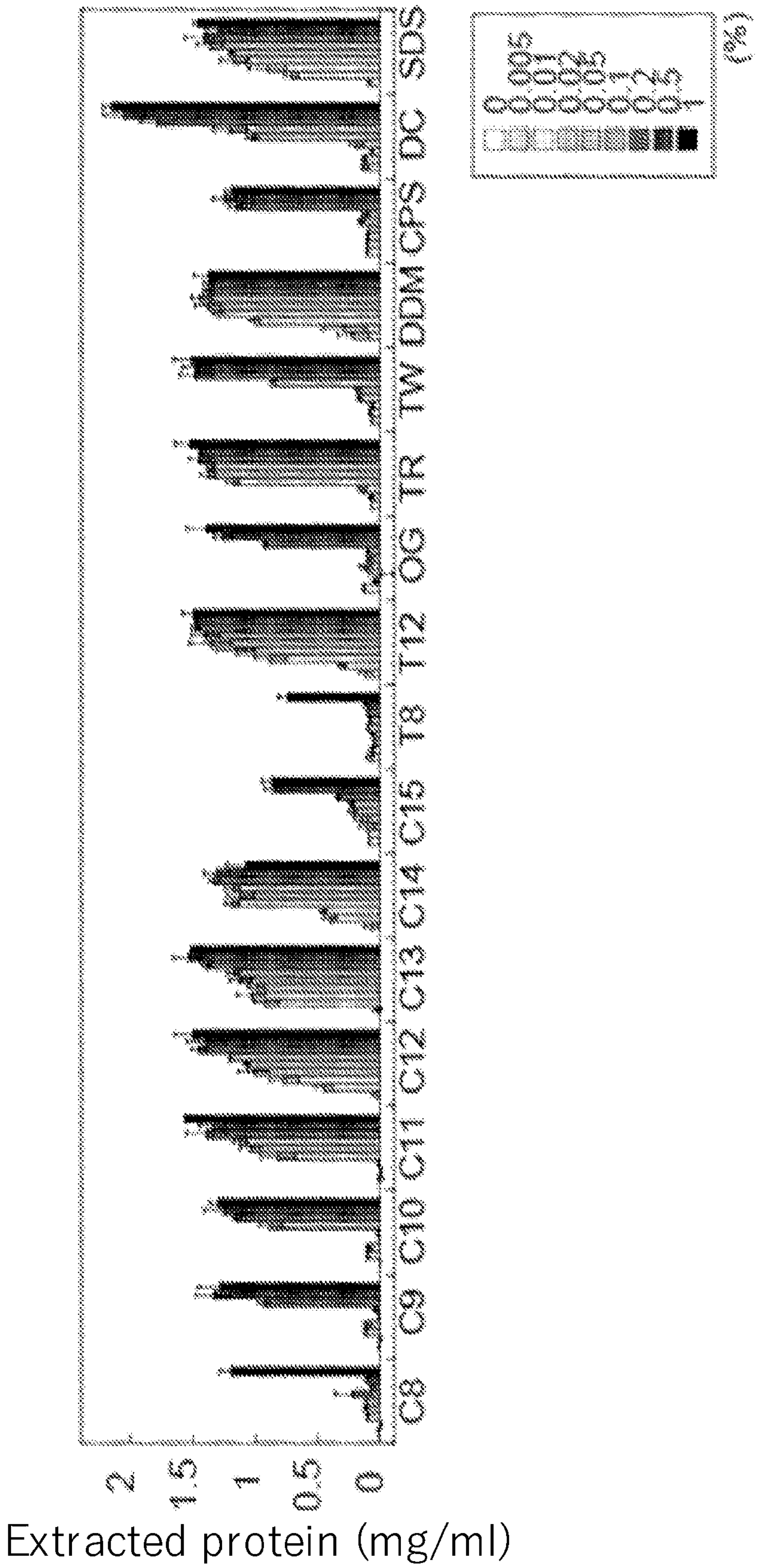
C8
C9
C10
C11
C12
C13
C14
C15
T8
T12
OG
TR
TW
DDM
CPS
DC
SDS
(%)
0
0.5
1
1.5
2
Extracted protein (mg/ml)

FIG. 14B

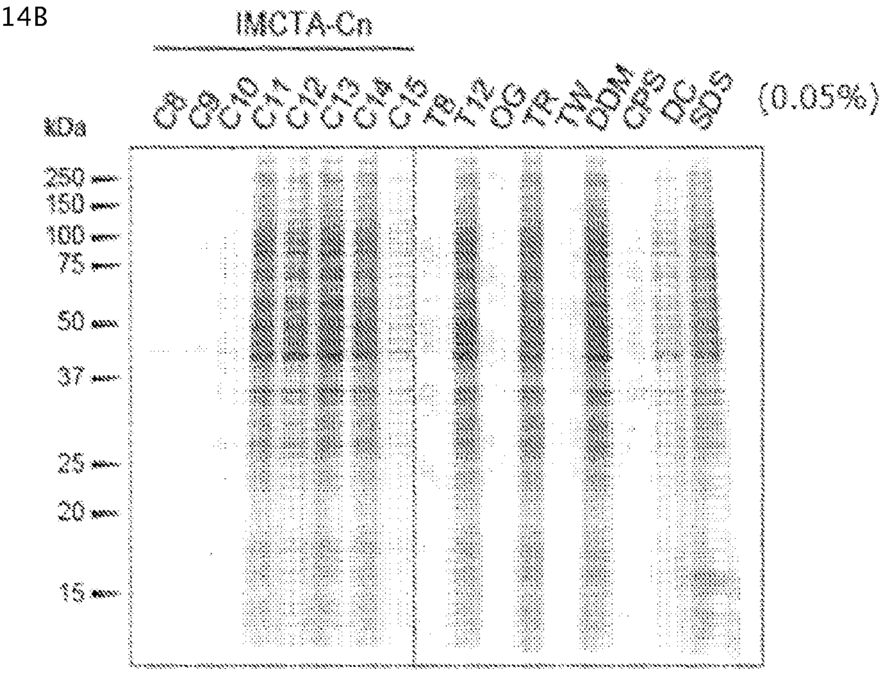


FIG. 14C

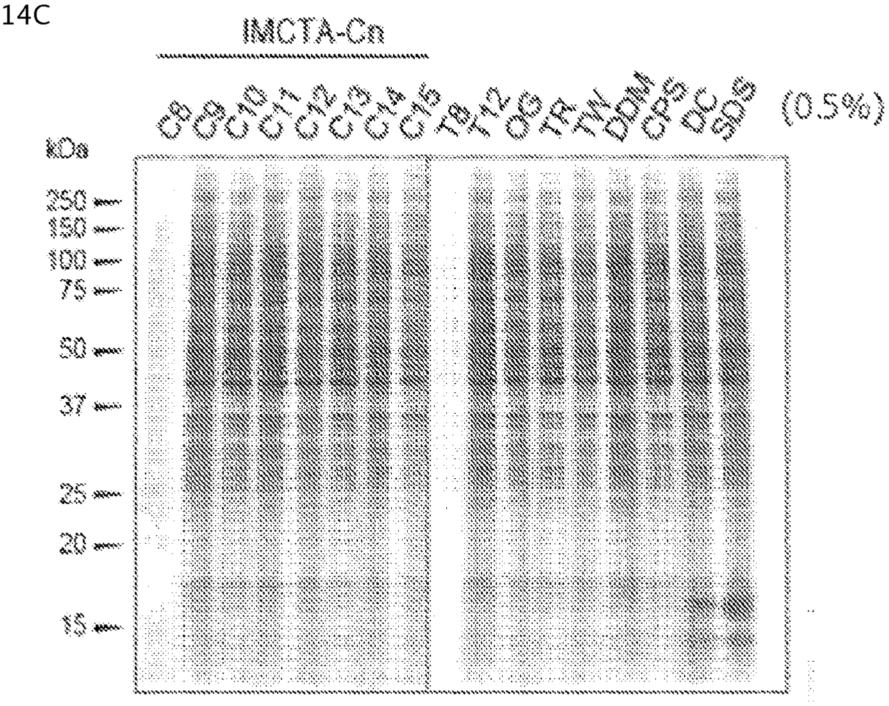

FIG. 14D

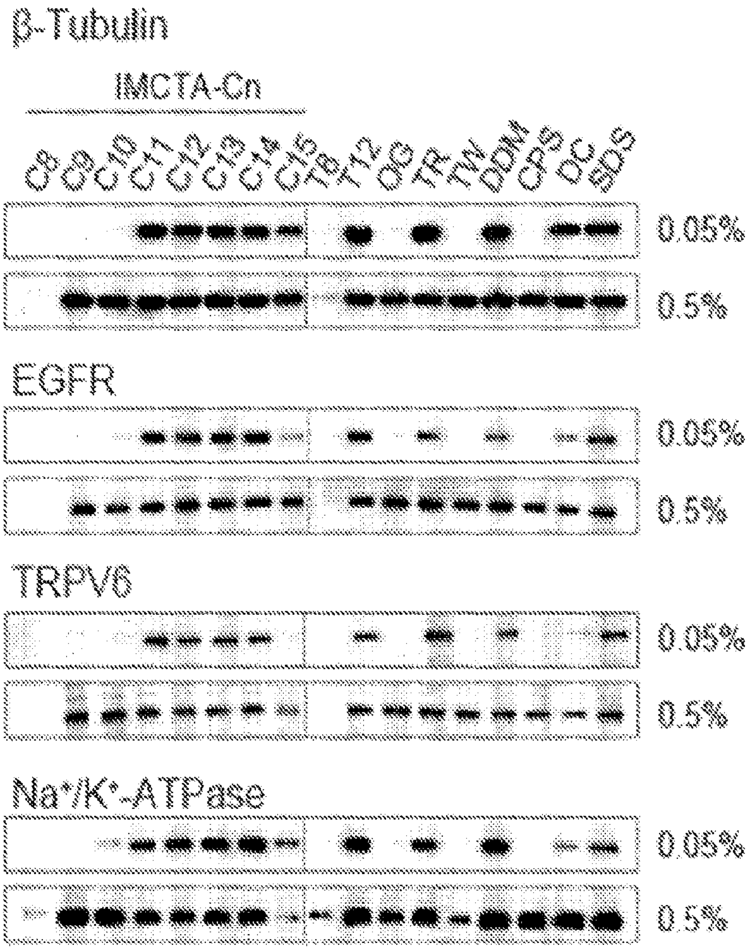


FIG. 14E

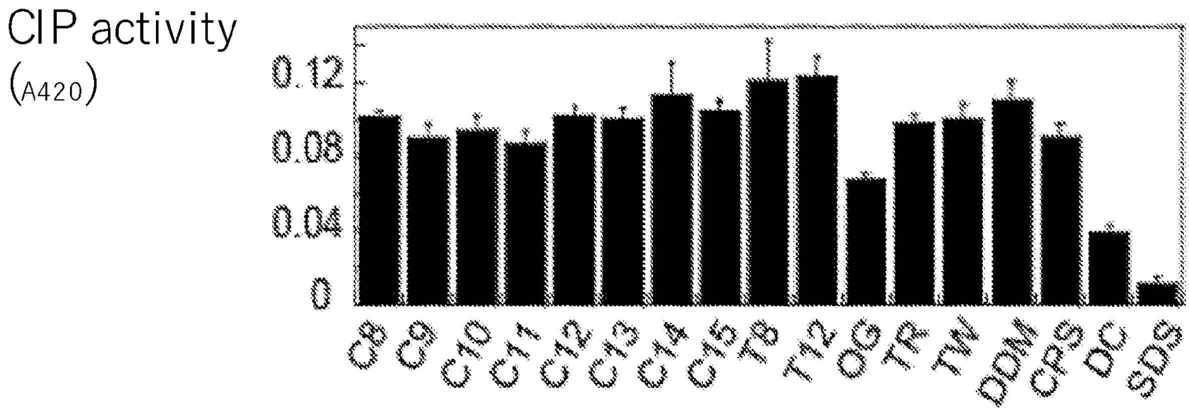

FIG. 15A

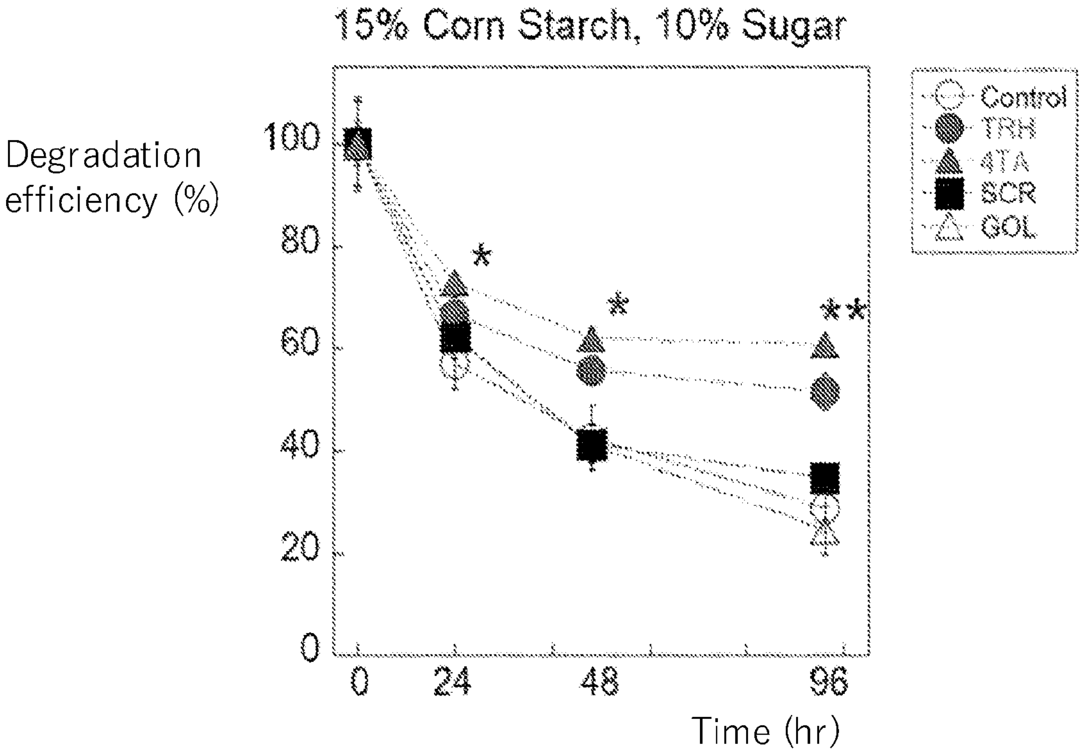


FIG. 15B

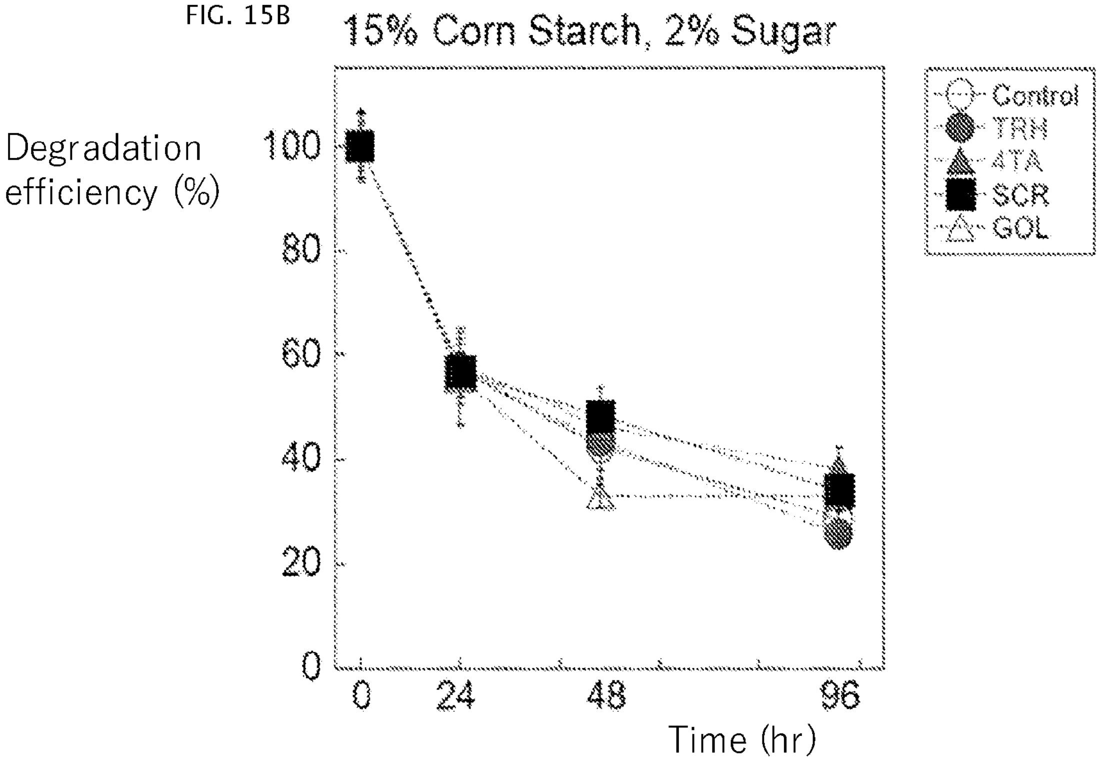

FIG. 15C

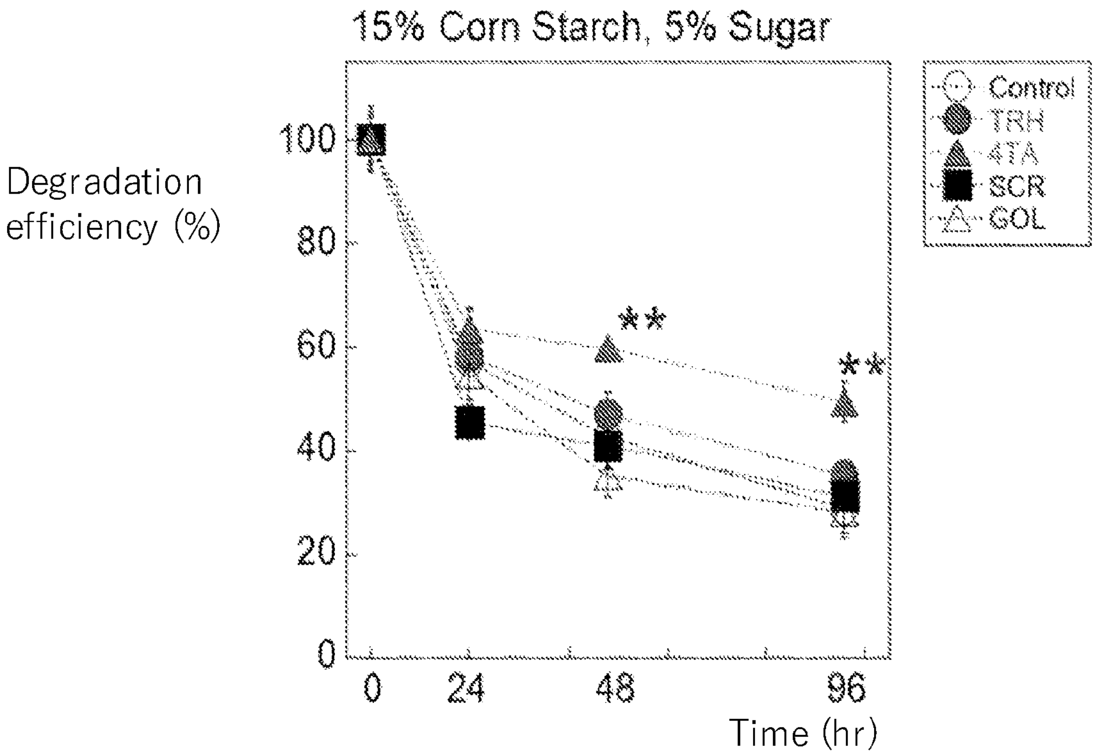


FIG. 15D

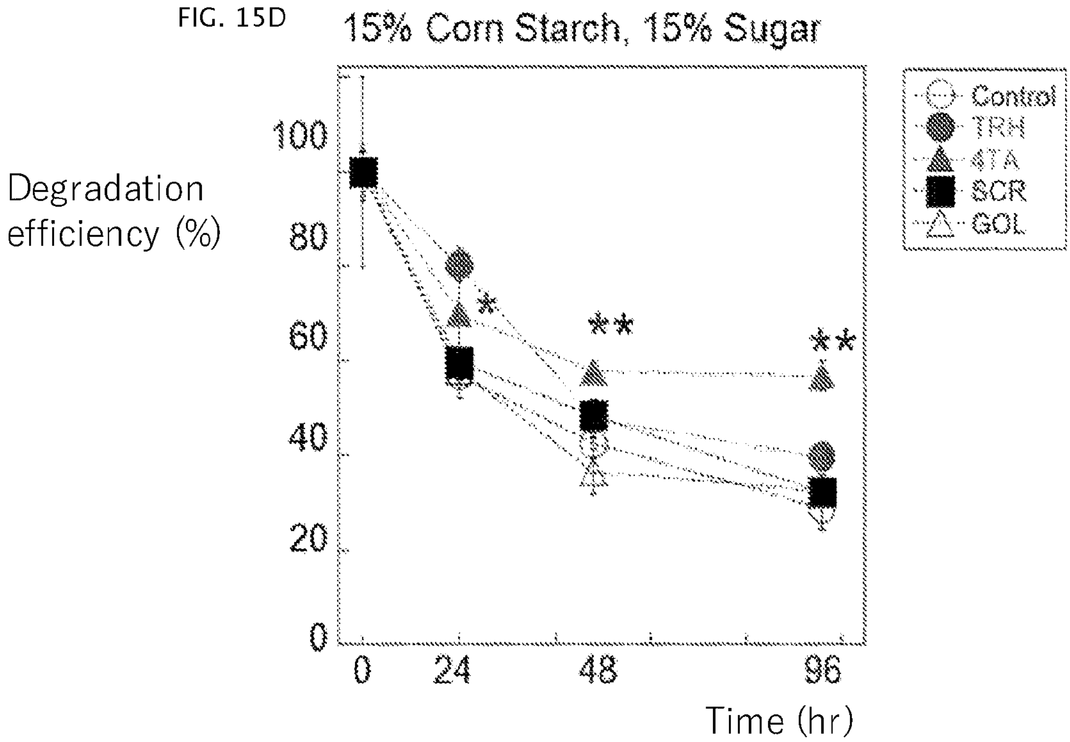

FIG. 15E

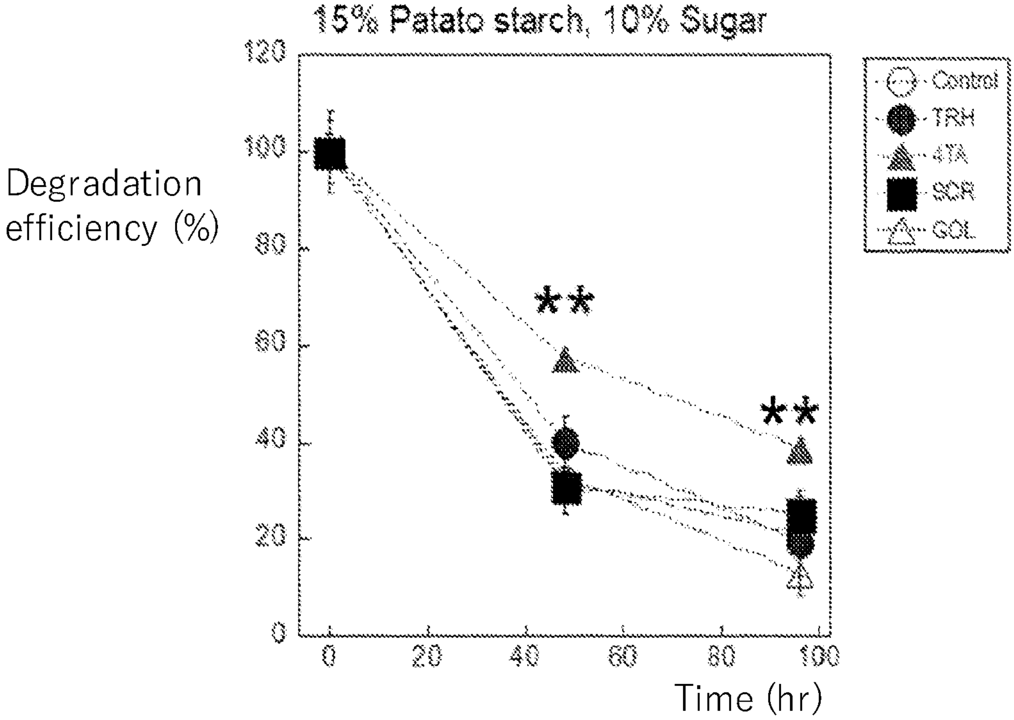


FIG. 15F

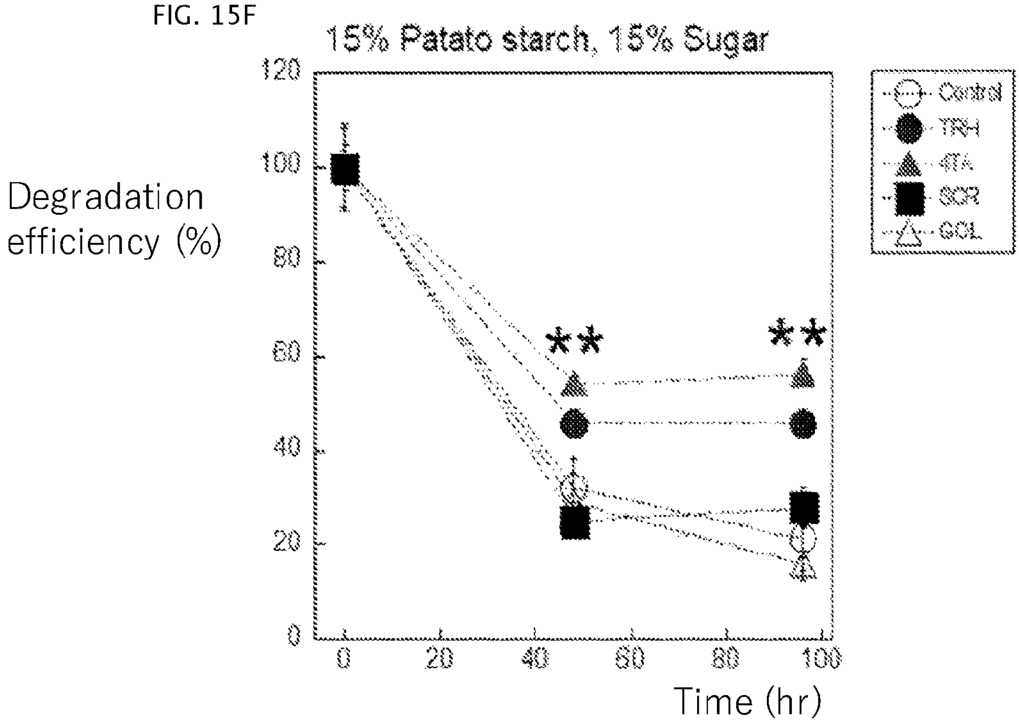

FIG. 15G

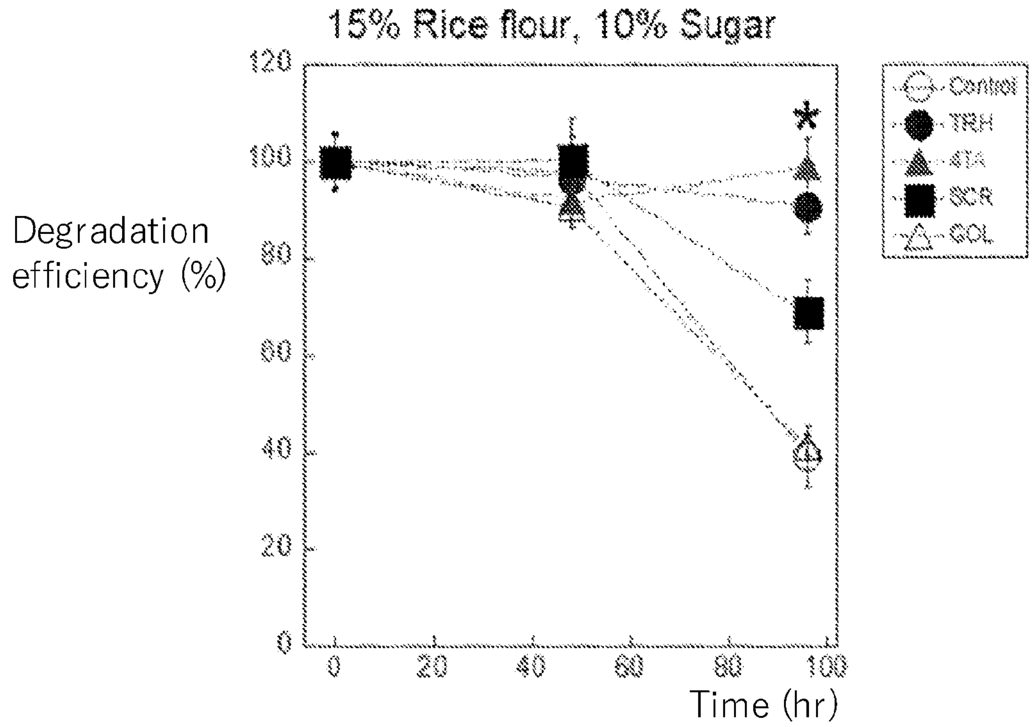


FIG. 15H

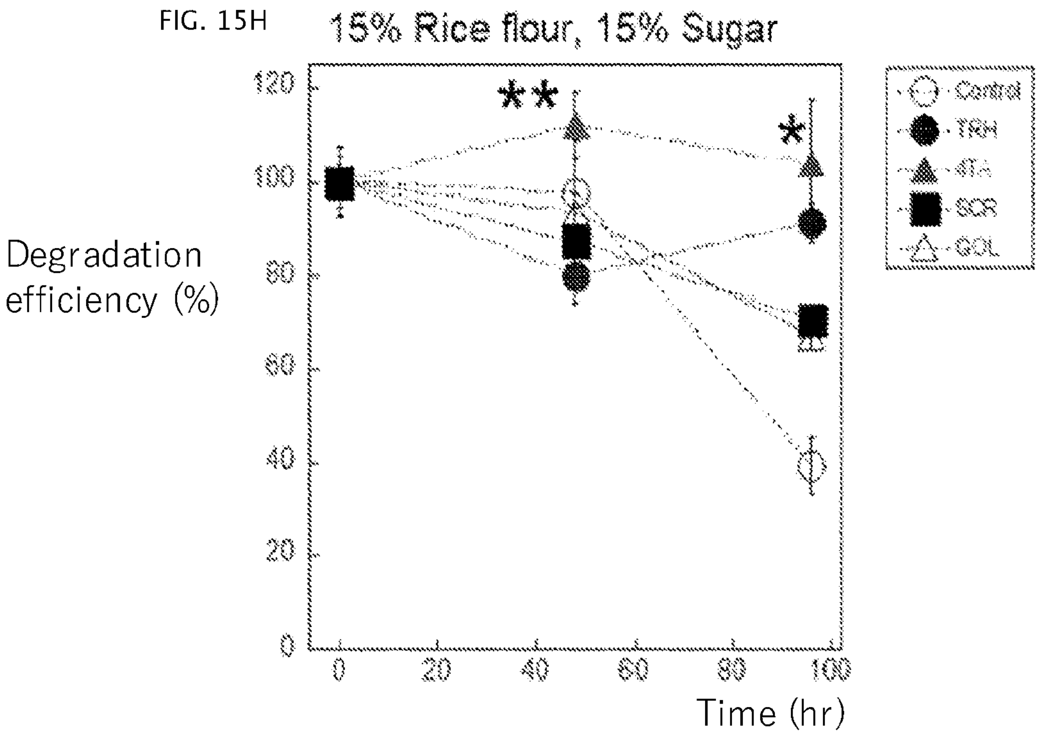

FIG. 15I

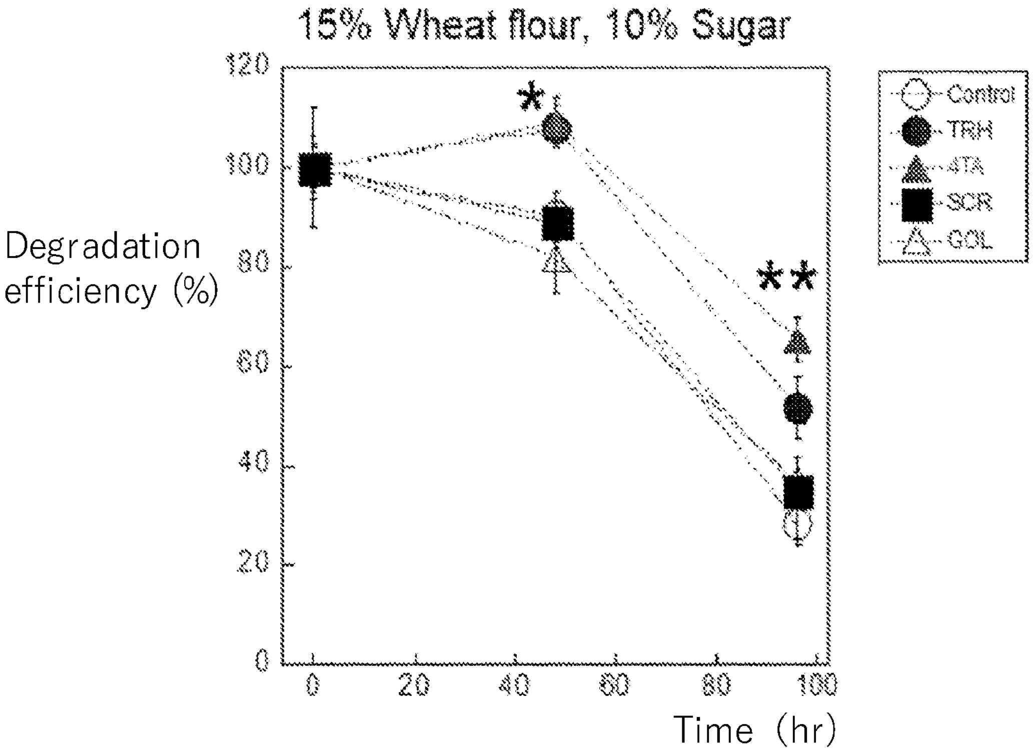


FIG. 15J

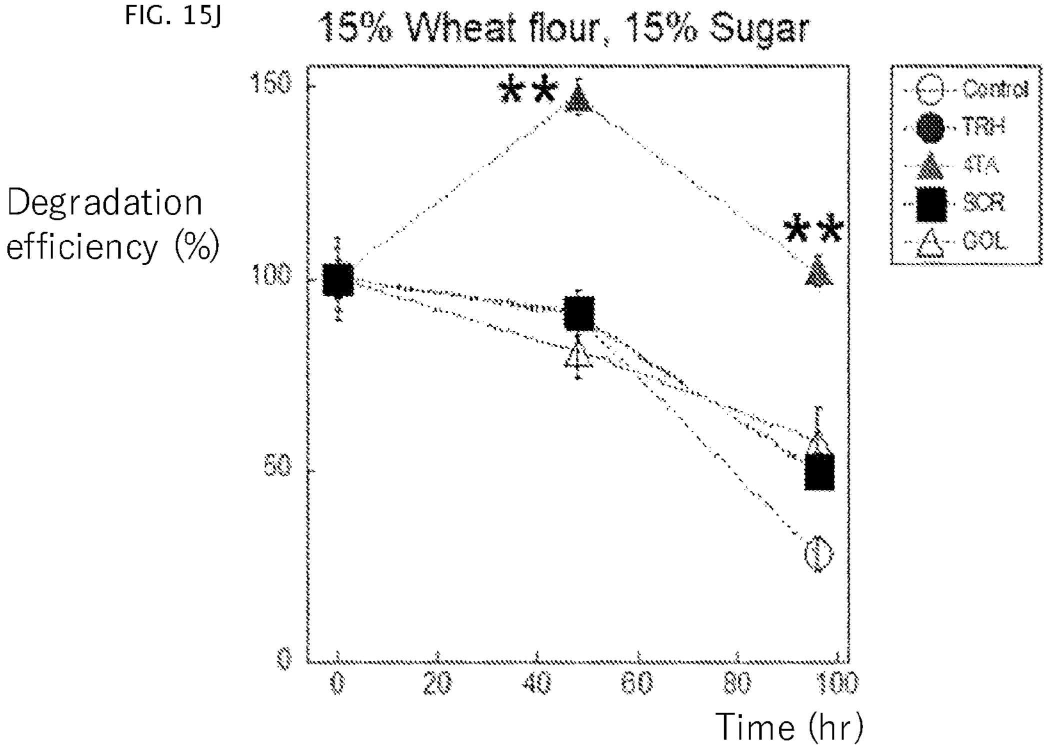

FIG. 15K

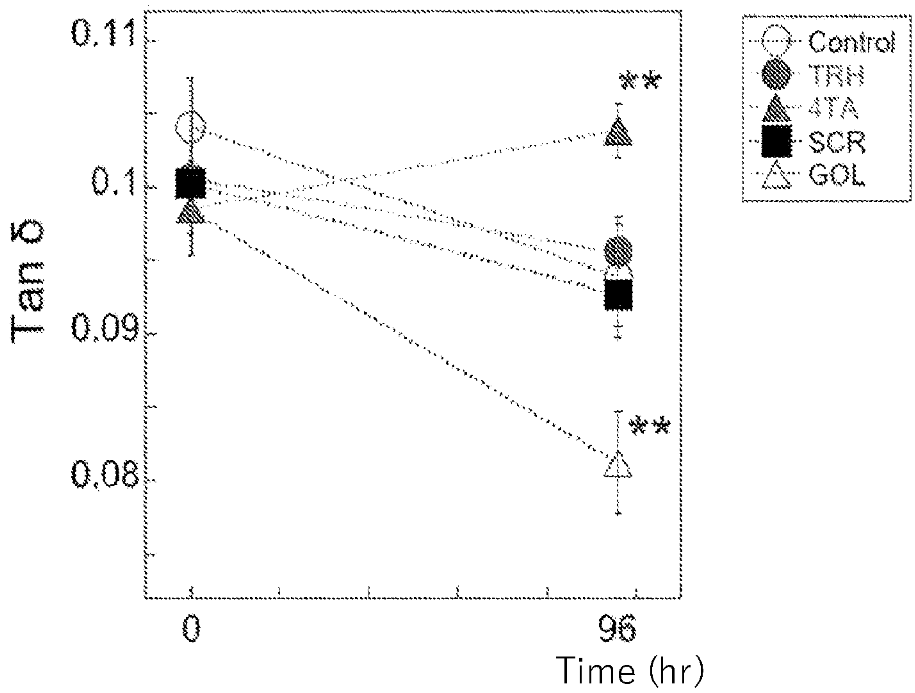


FIG. 16A

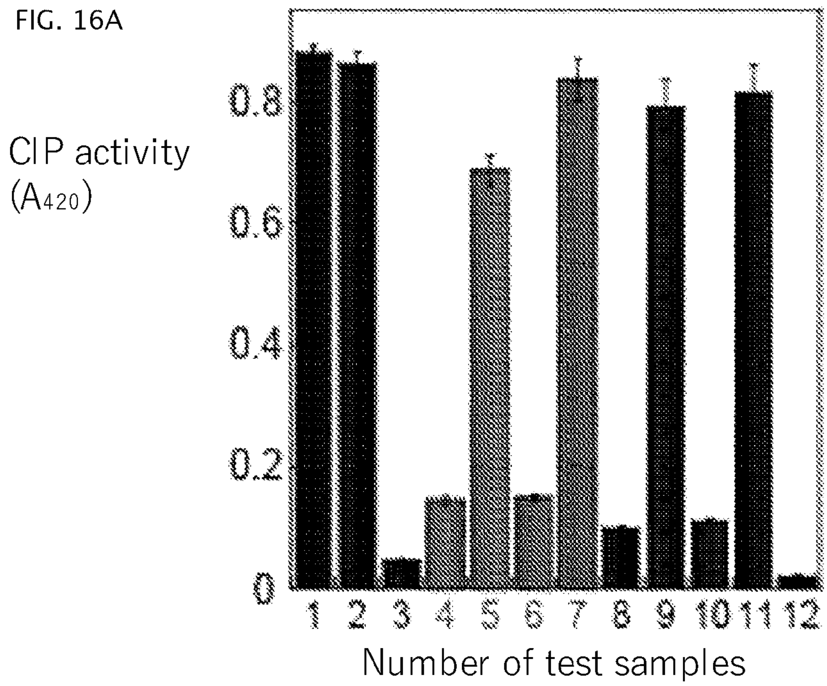

FIG. 16B

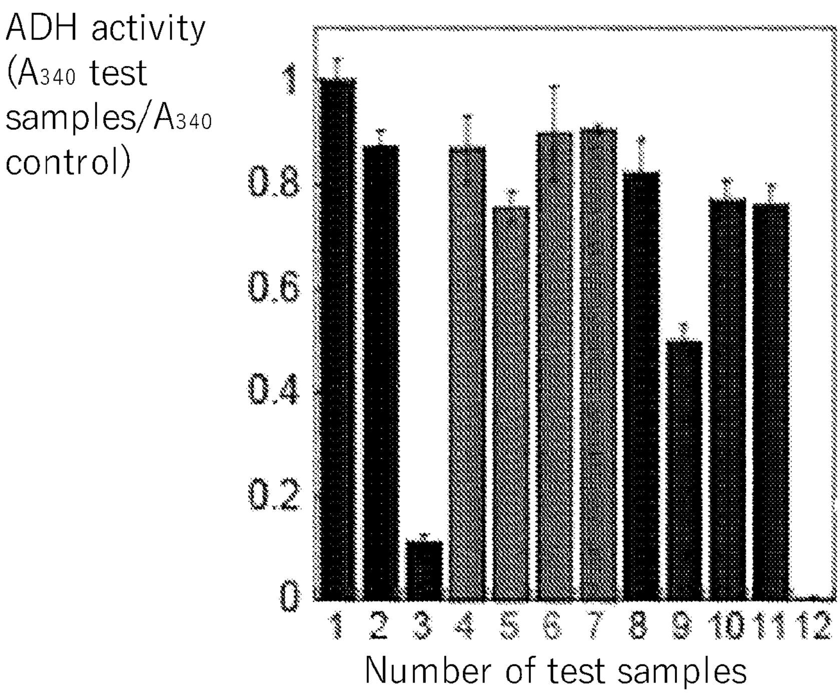
ADH activity
(A340 test
samples/A340
control)
1
0.8
0.6
0.4
0.2
0
1 2 3 4 5 6 7 8 9 10 11 12
Number of test samples

FIG. 17A

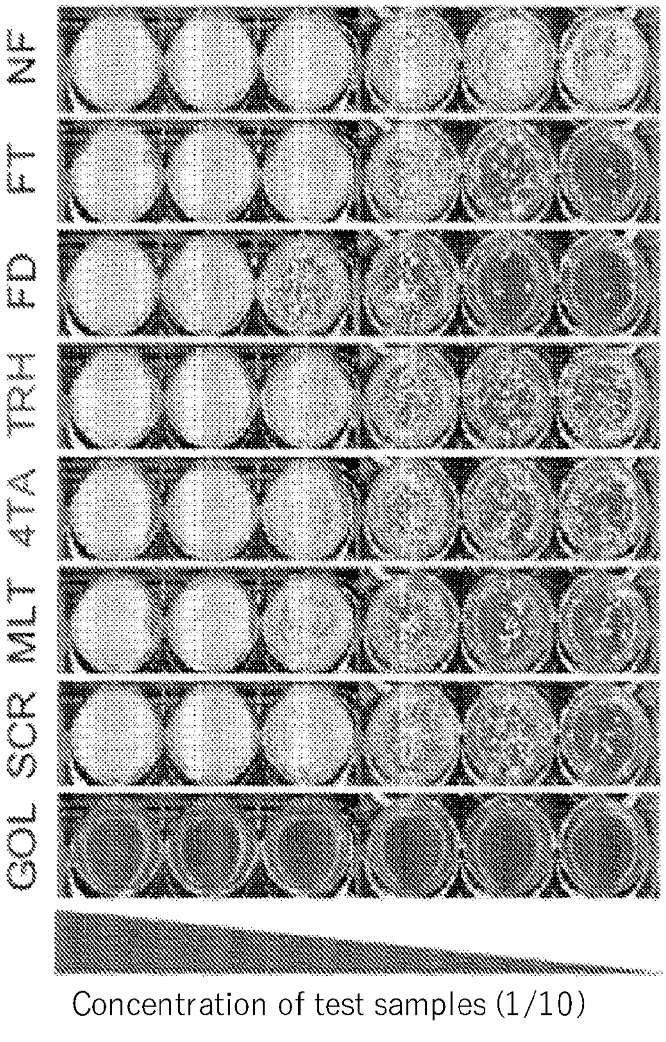
NF
FT
FD
TRH
4TA
MLT
SCR
GOL
Concentration of test samples (1/10)

FIG. 17B

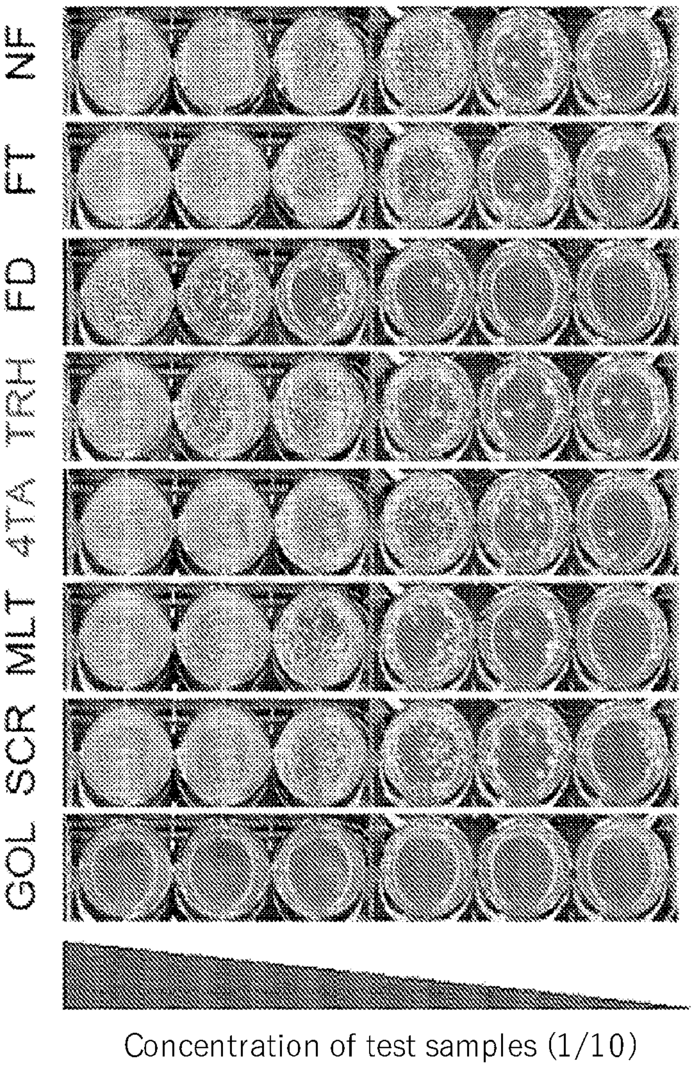
NF
FT
FD
TRH
4TA
MLT
SCR
GOL
Concentration of test samples (1/10)

FIG. 17C

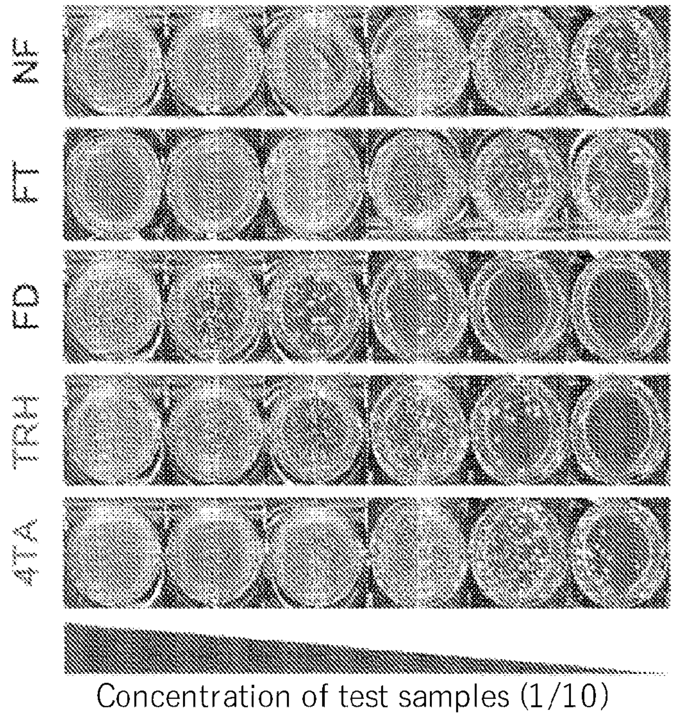


FIG. 17D

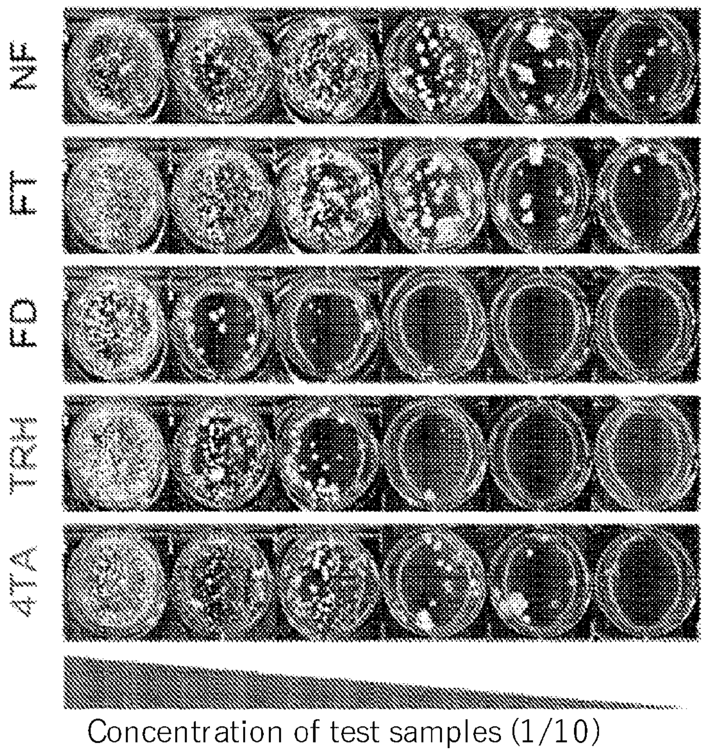

FIG. 18

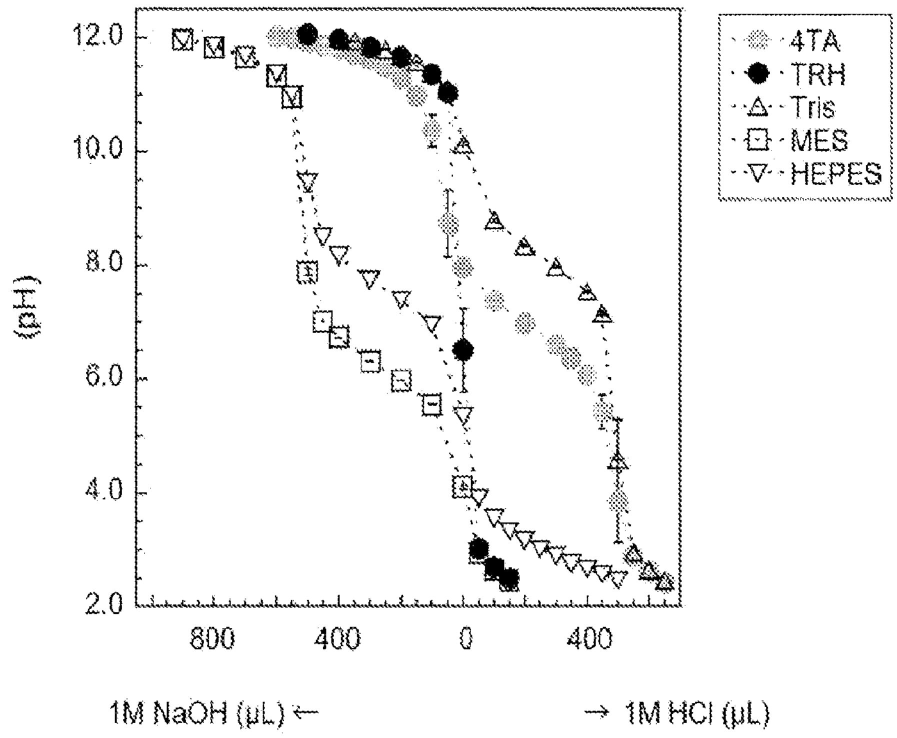


FIG. 19A

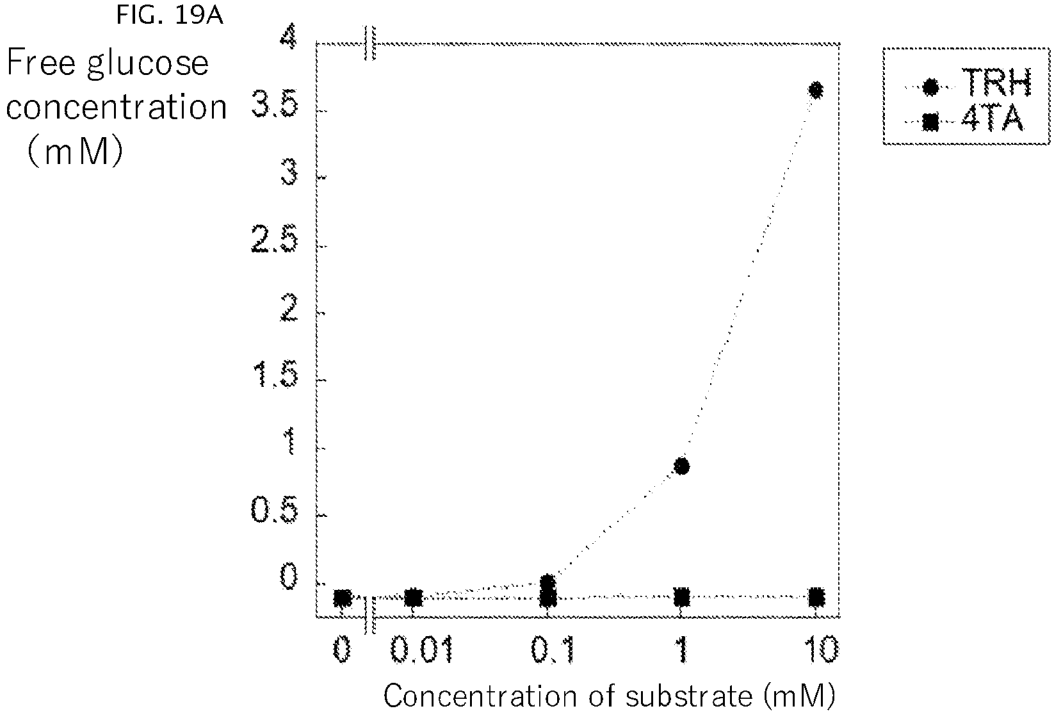

FIG. 19B

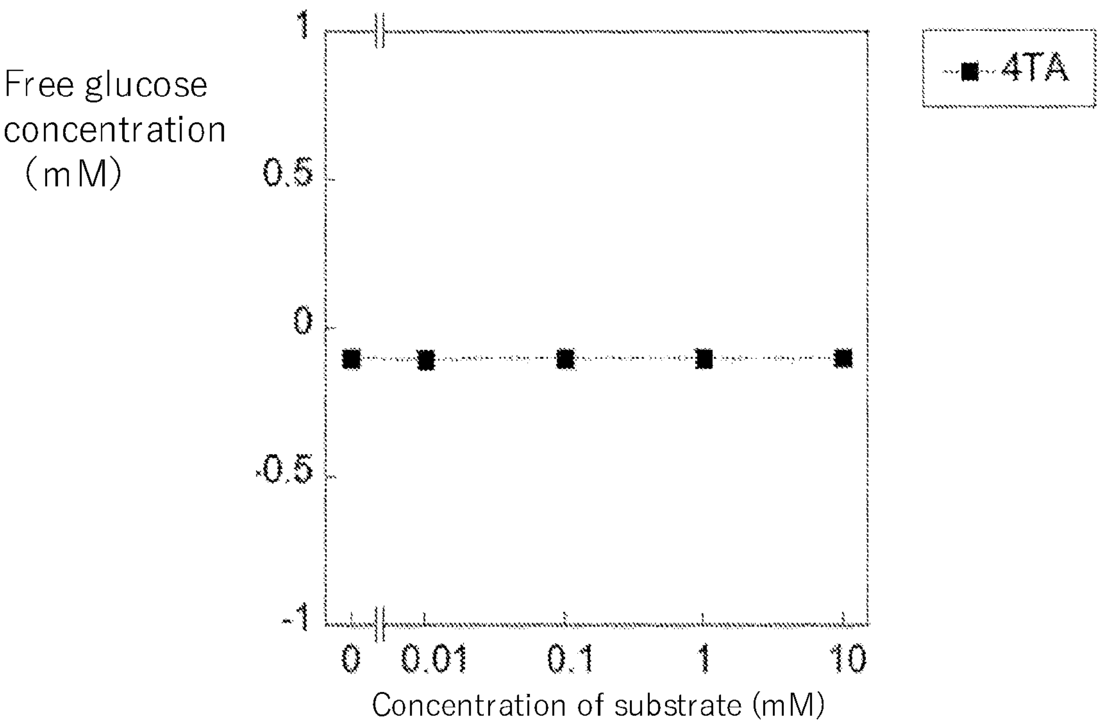


FIG. 19C

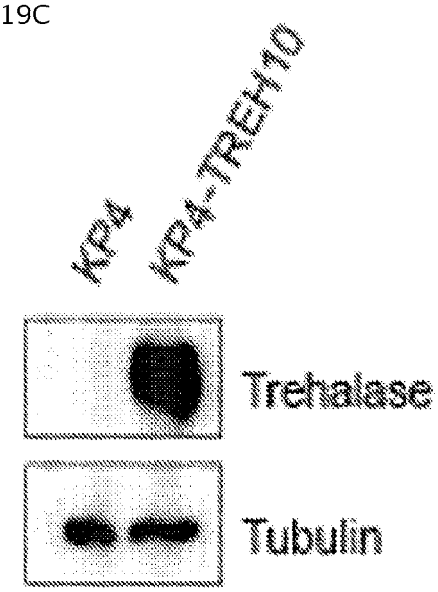

FIG. 19D

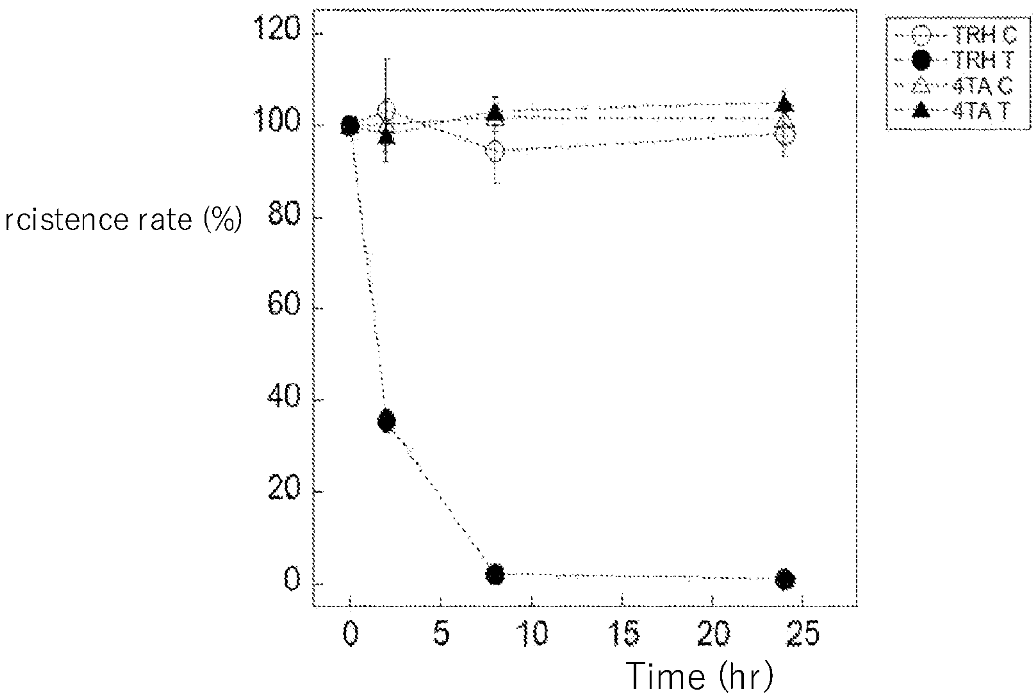


FIG. 19E

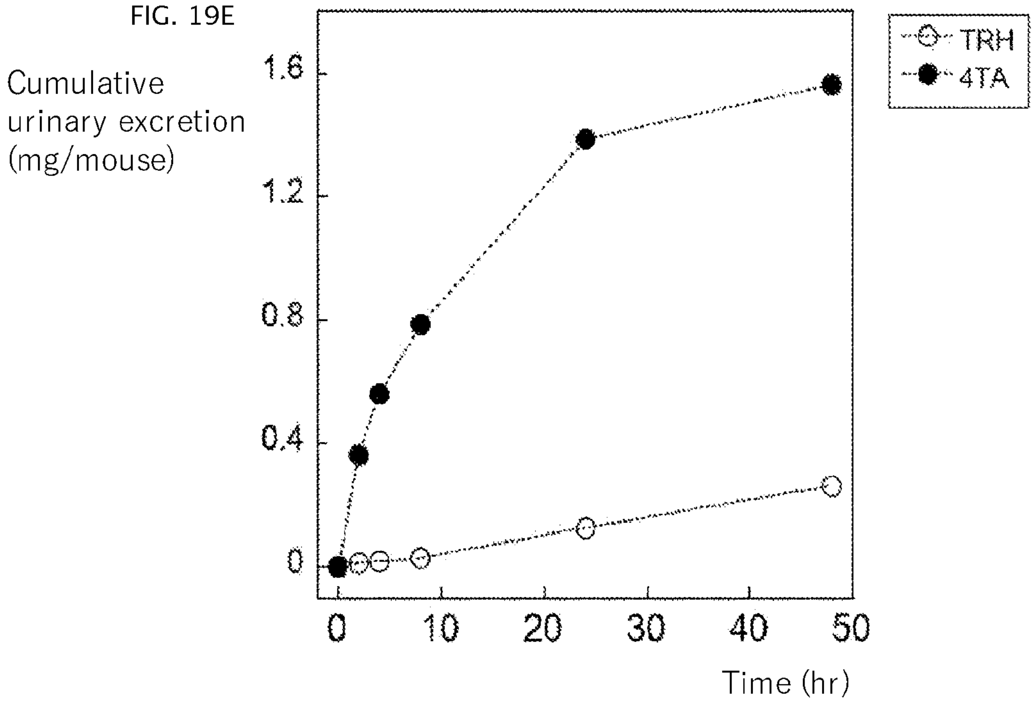

FIG. 19F

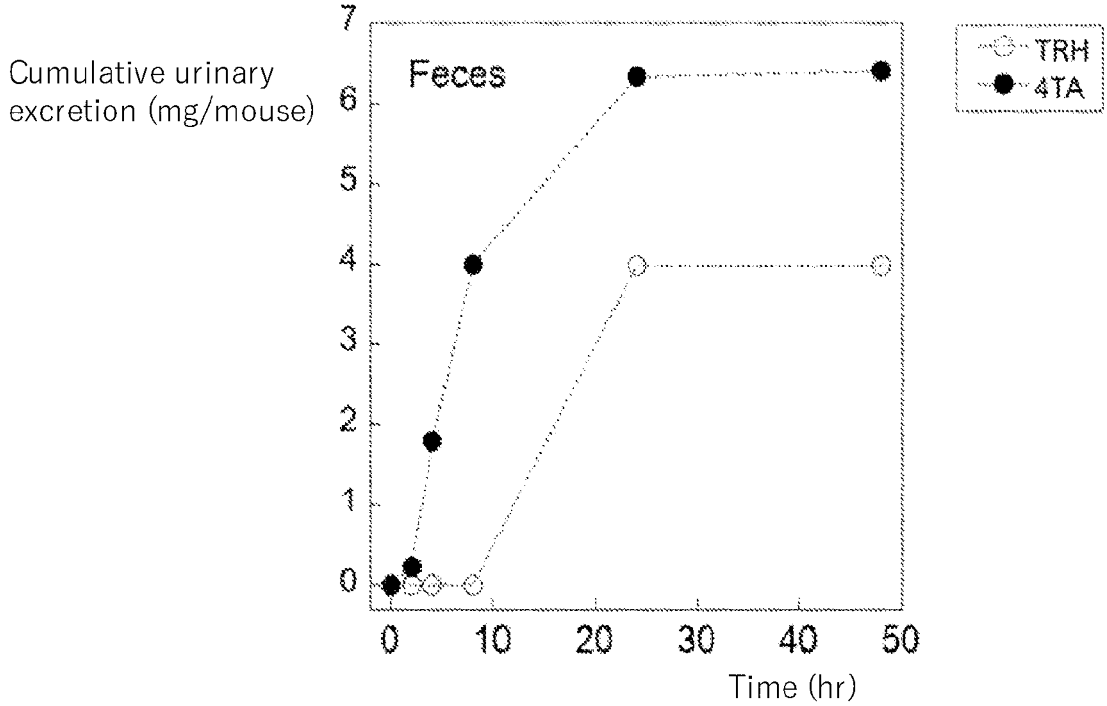


FIG. 19G

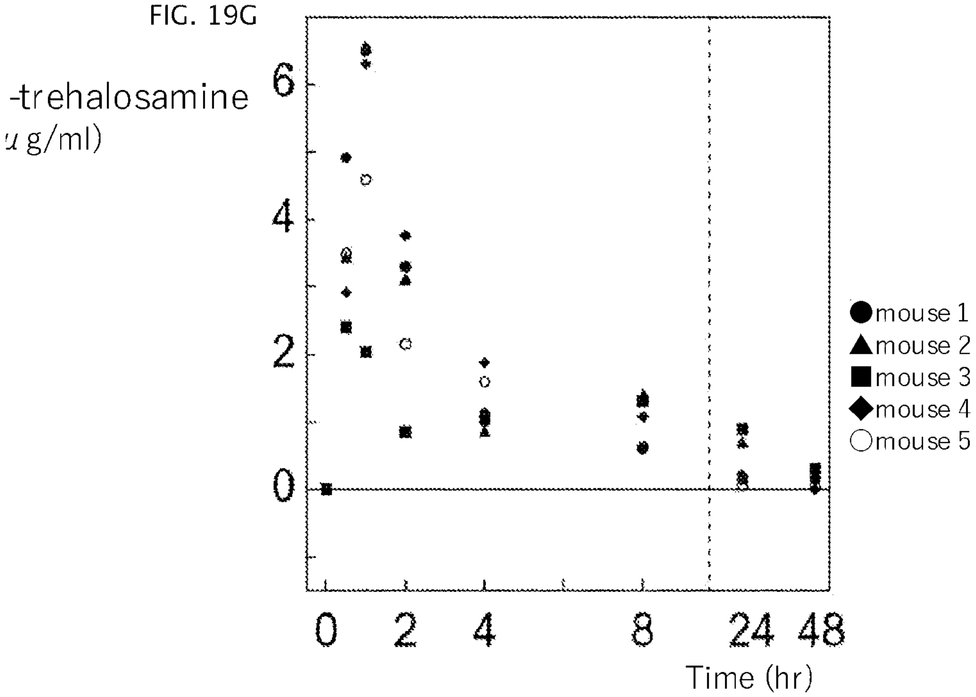

FIG. 19H

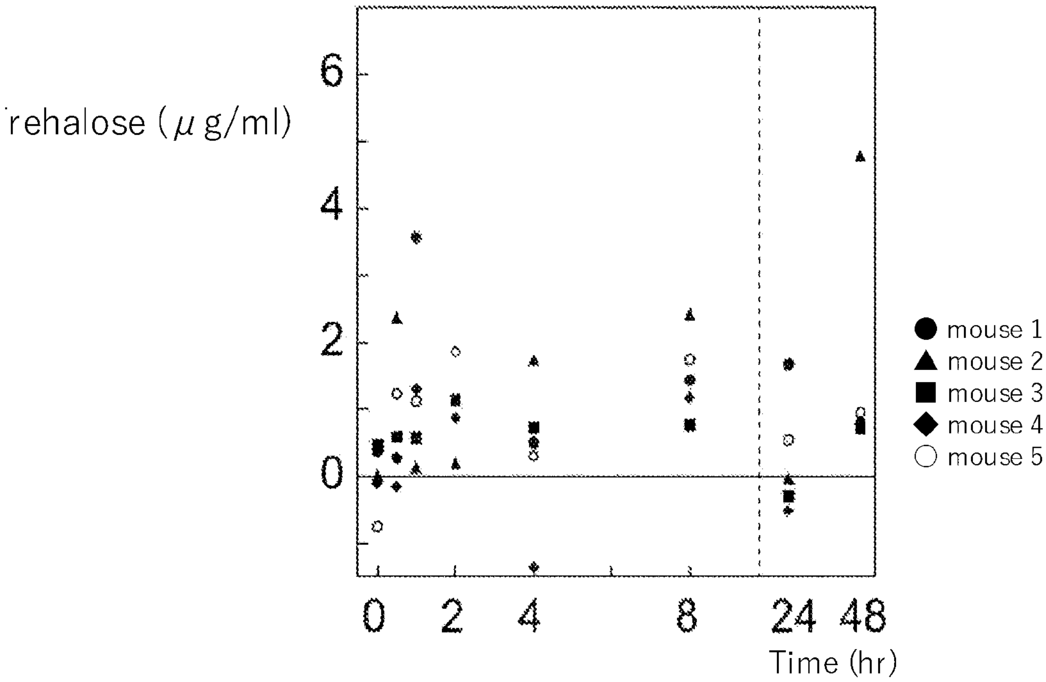


FIG. 19I

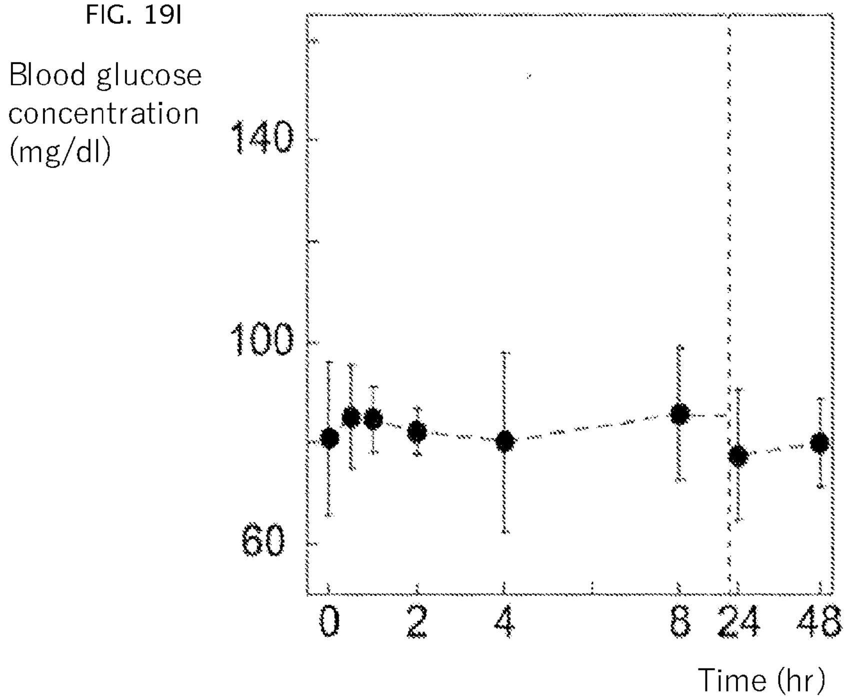

FIG. 19J

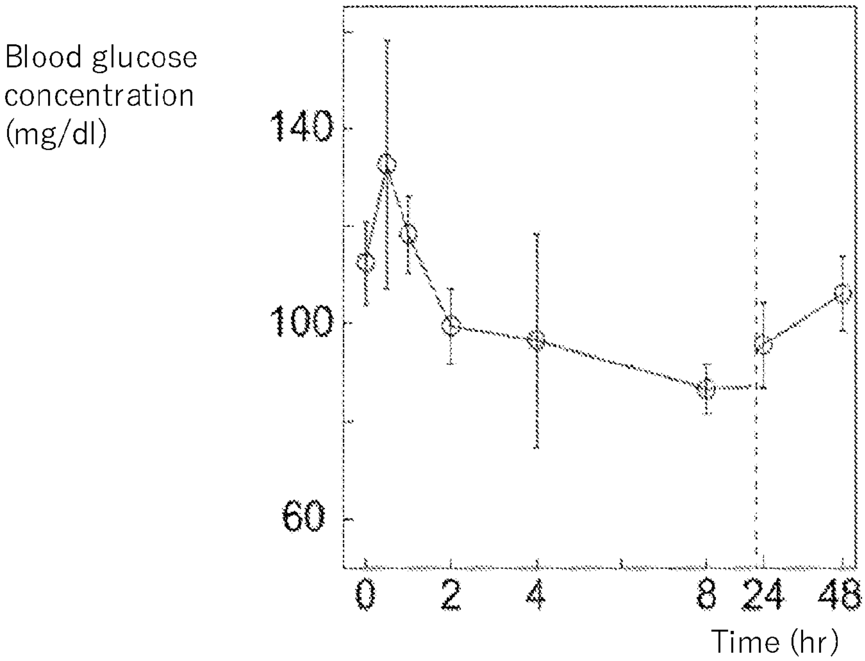


FIG. 20A

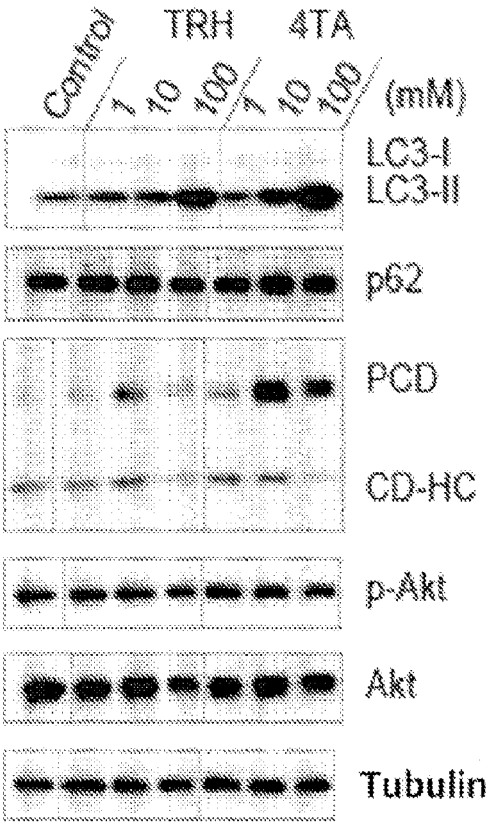

FIG. 20B

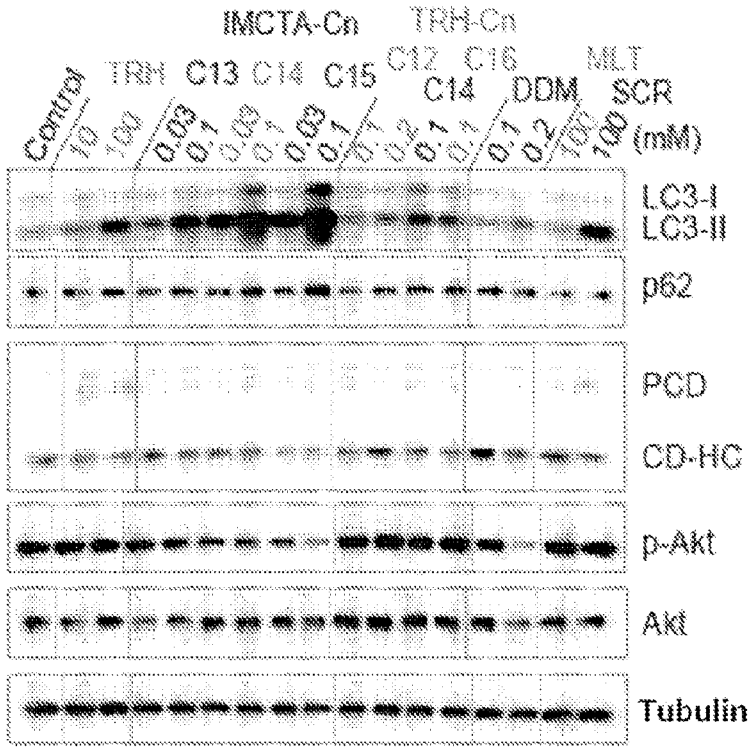


FIG. 20C

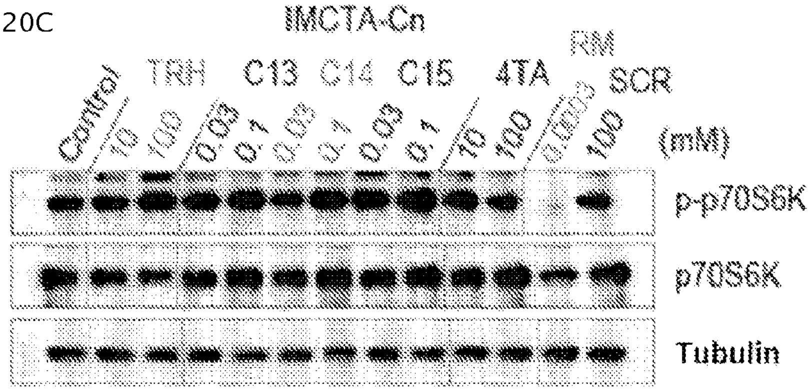

FIG. 20D

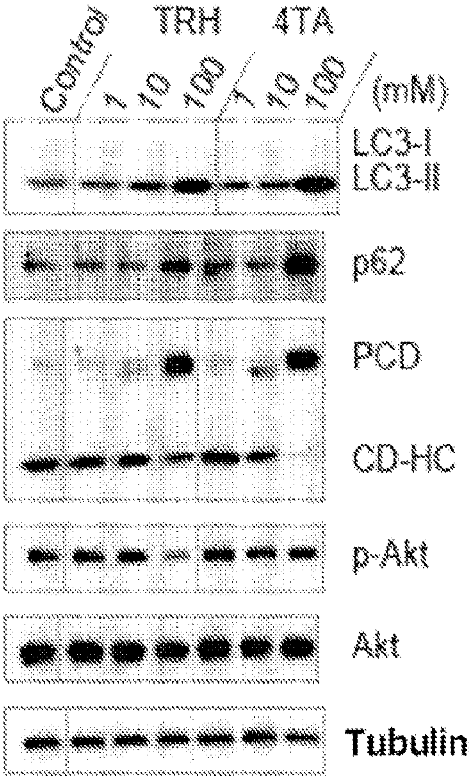


FIG. 20E

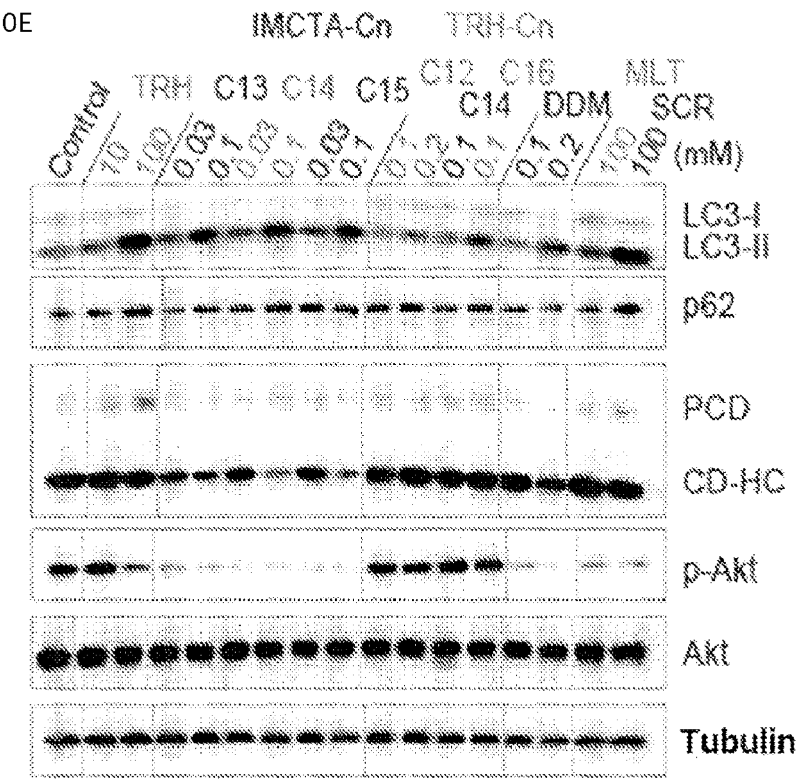

FIG. 20F

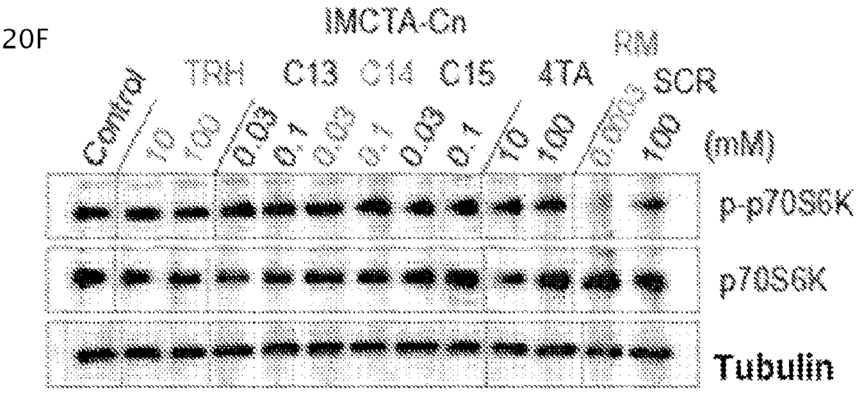


FIG. 21A

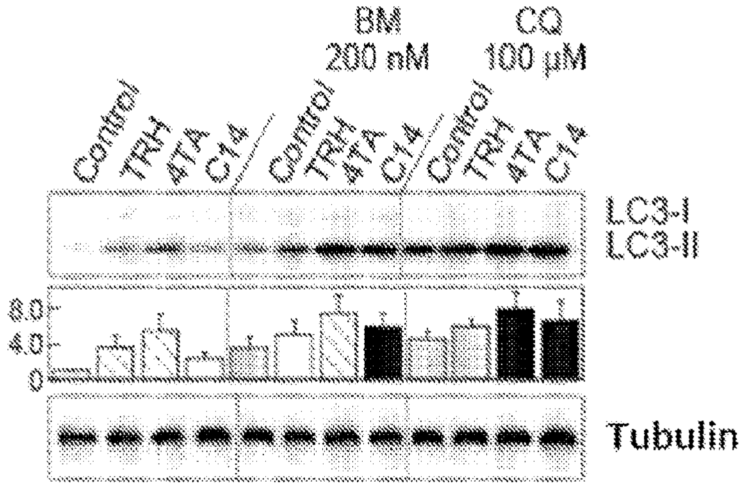


FIG. 21B

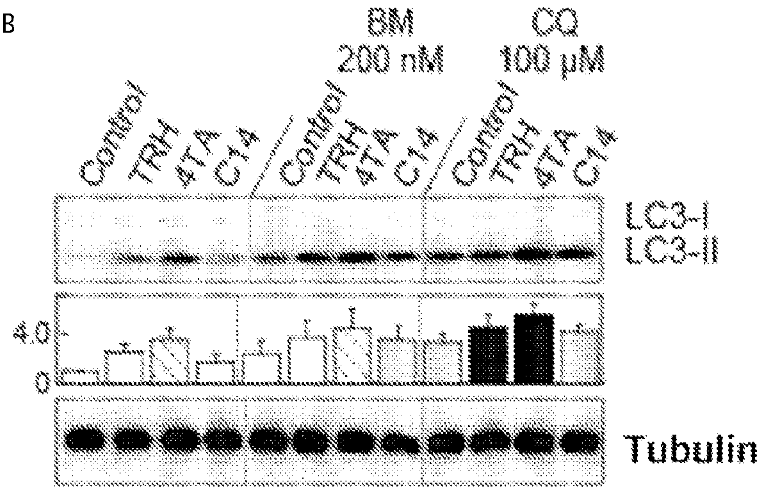

FIG. 22A

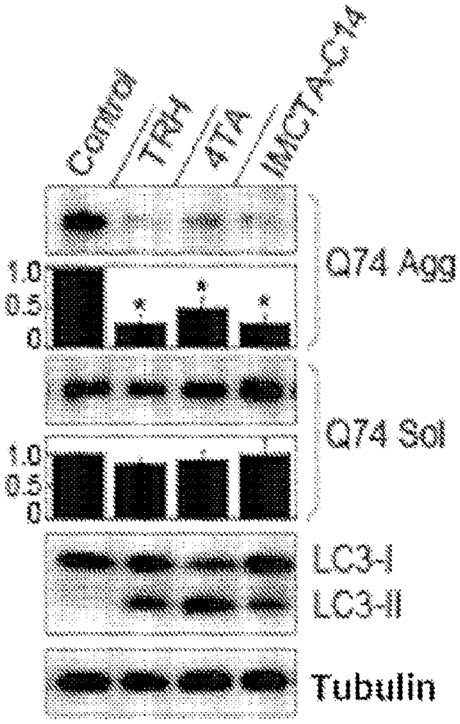


FIG. 22B

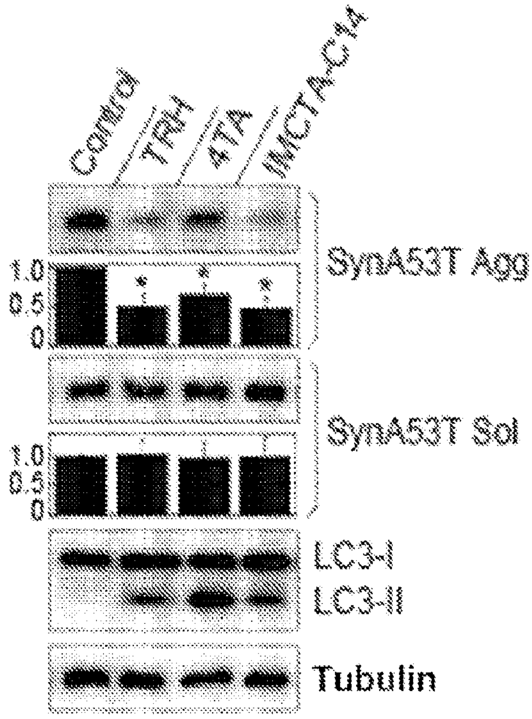

FIG. 22C

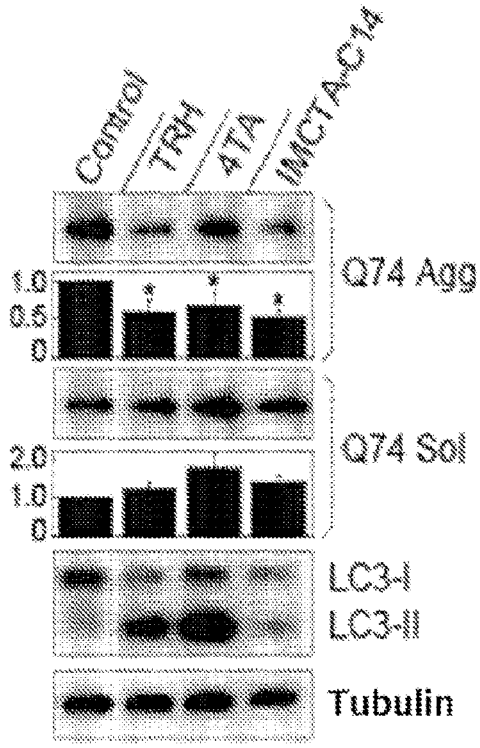


FIG. 22D

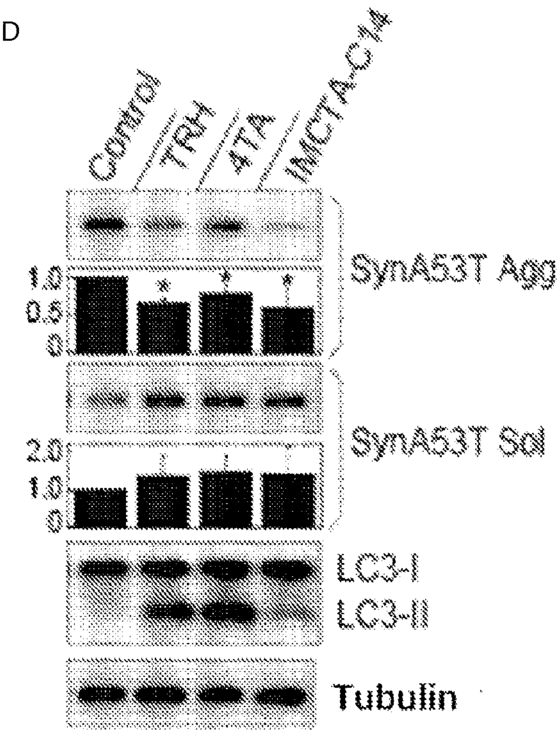

FIG. 23A

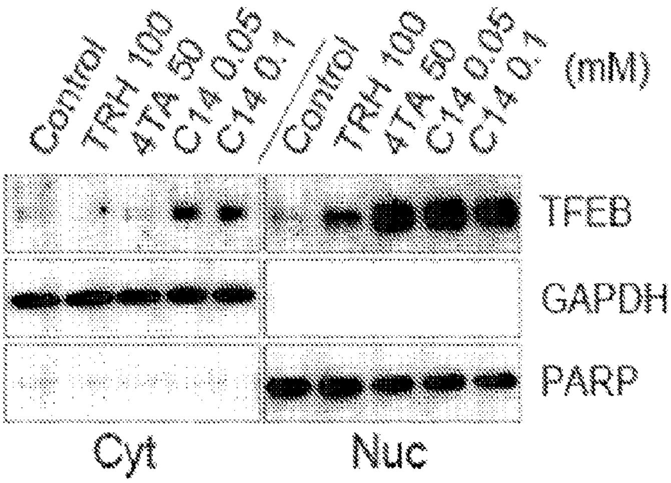


FIG. 23B

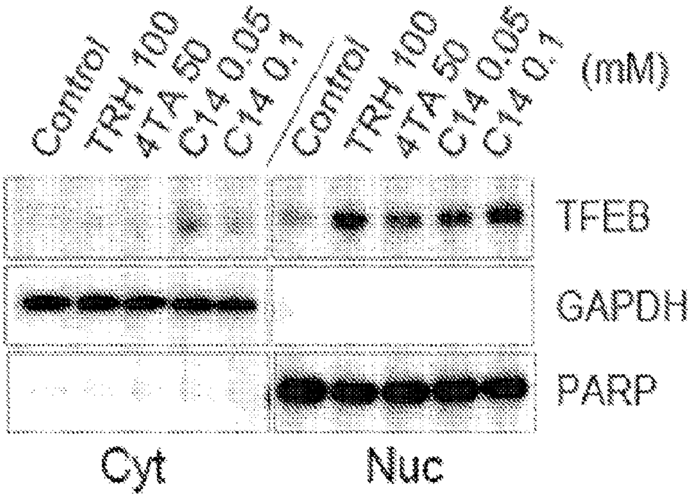

FIG. 23C

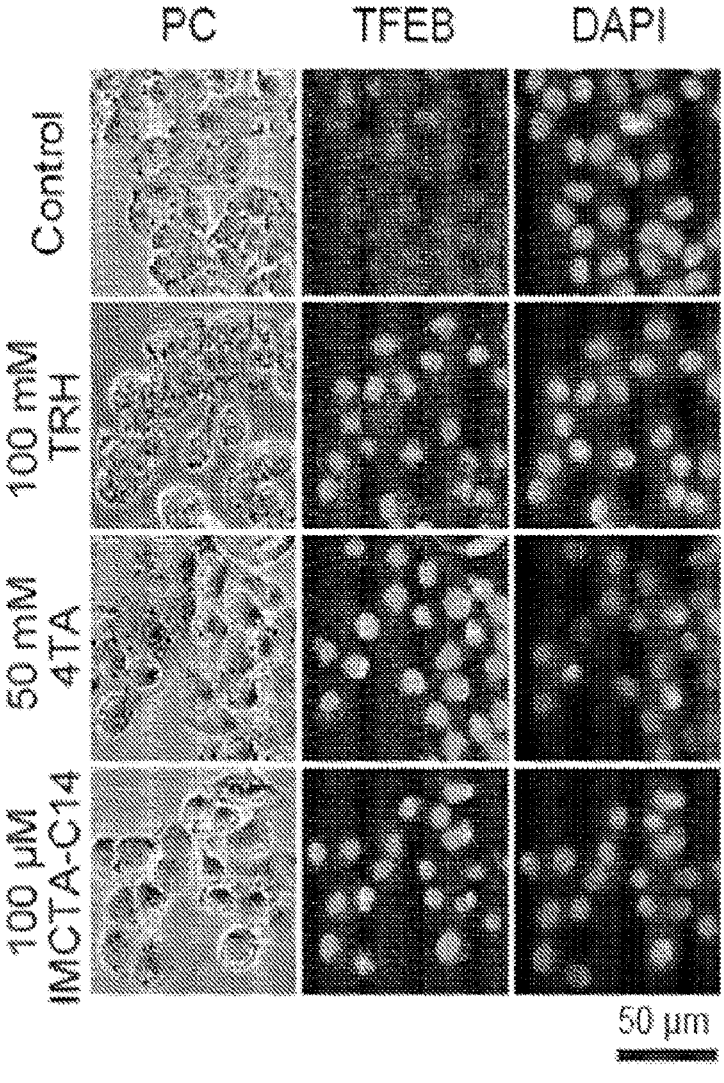

FIG. 23D

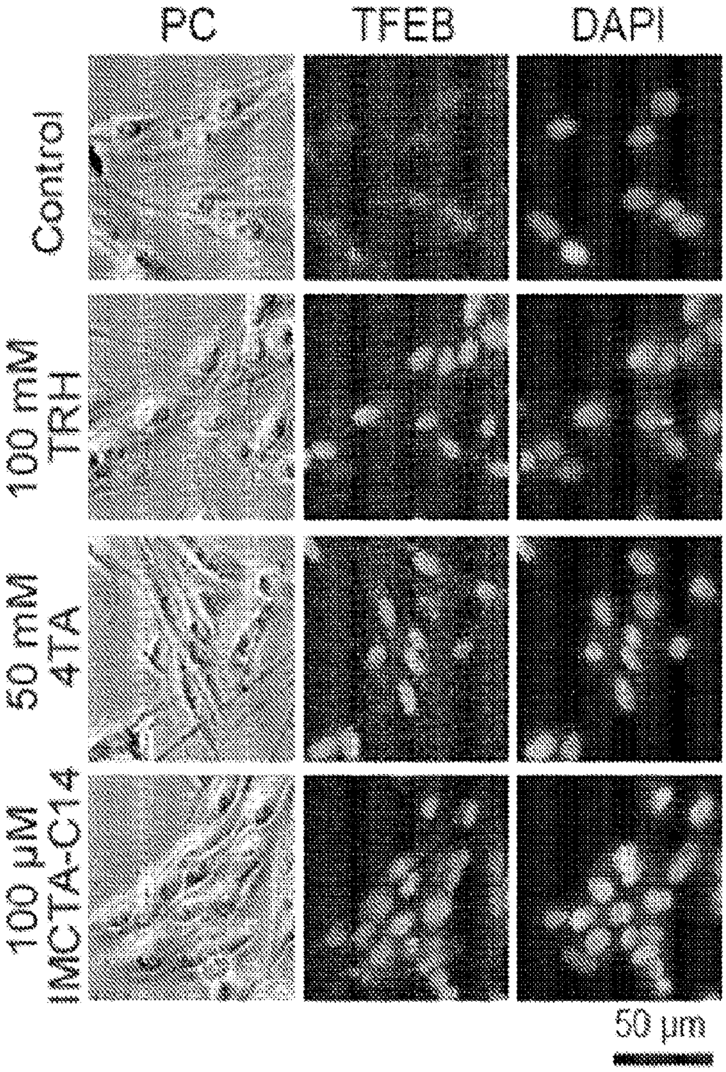

FIG. 24A

IMCTA-Fluorescein

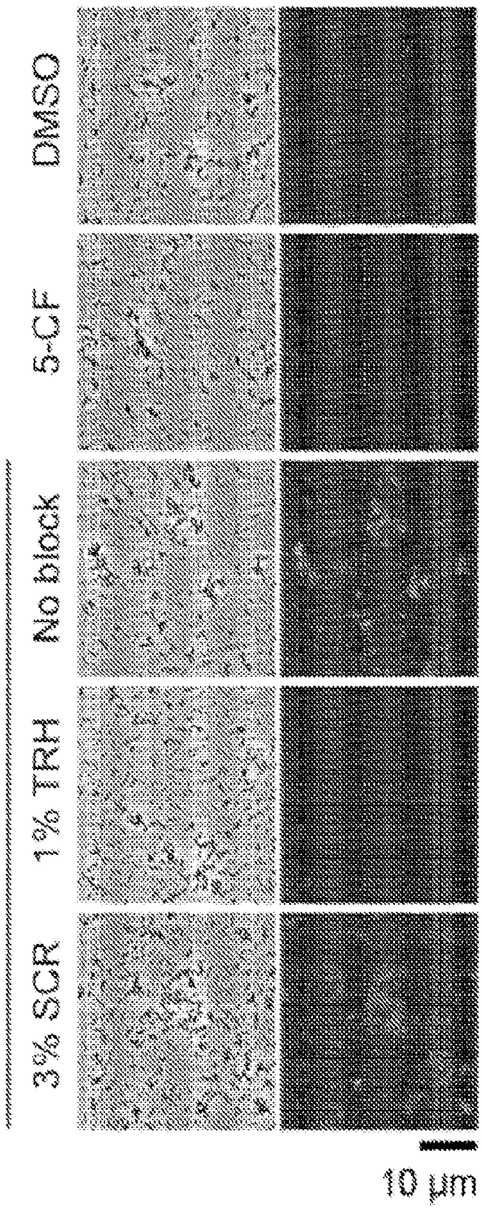

FIG. 24B

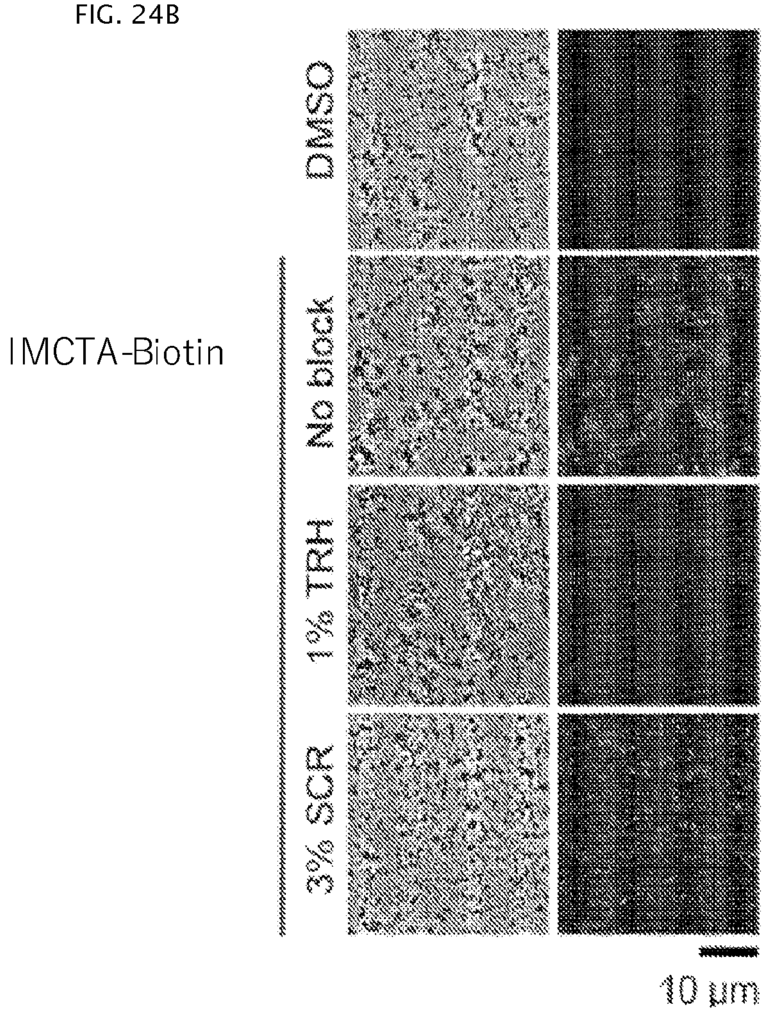

FIG. 24C

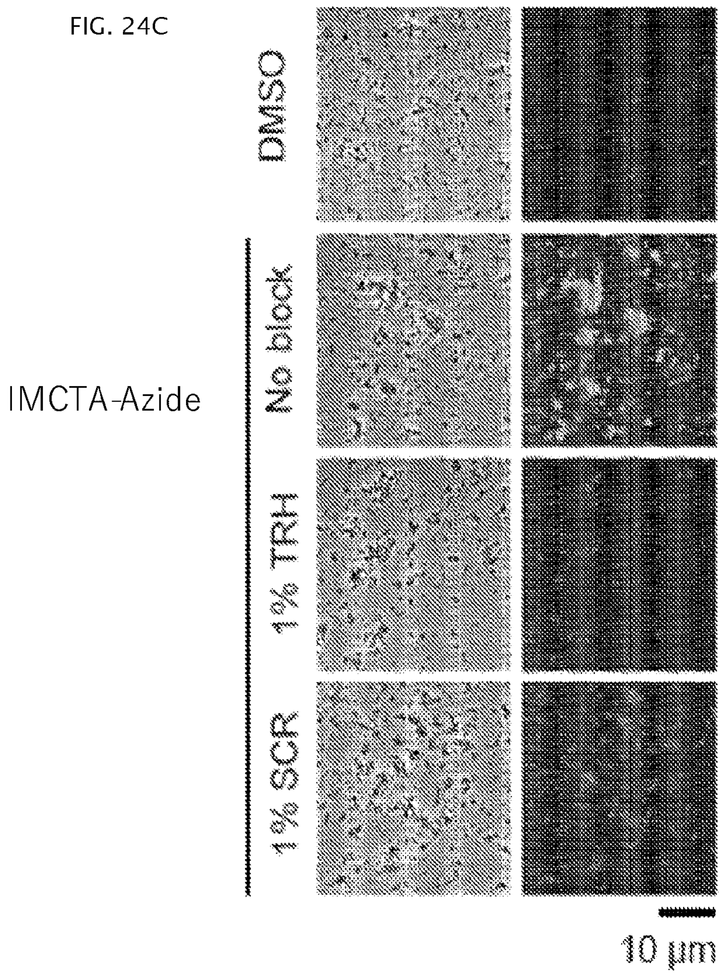


FIG. 25A

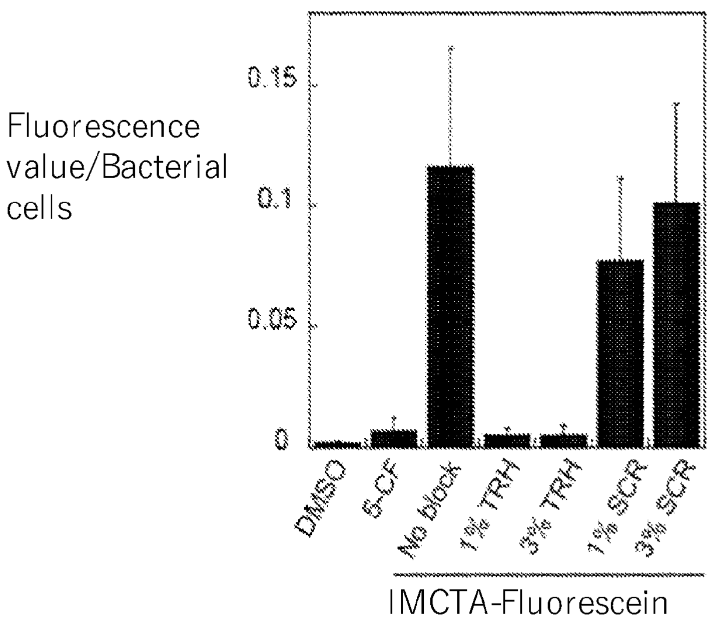

FIG. 25B

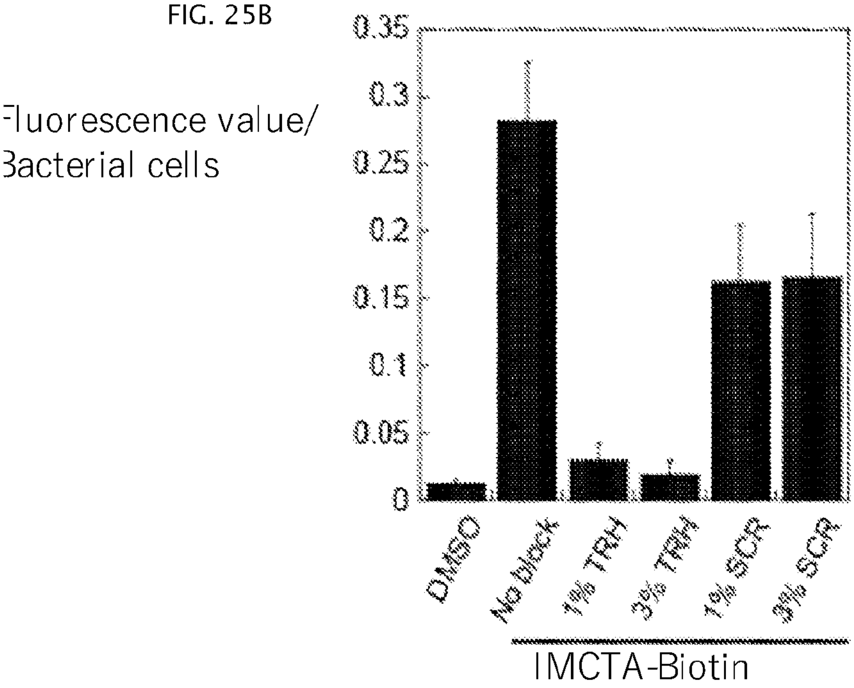


FIG. 25C

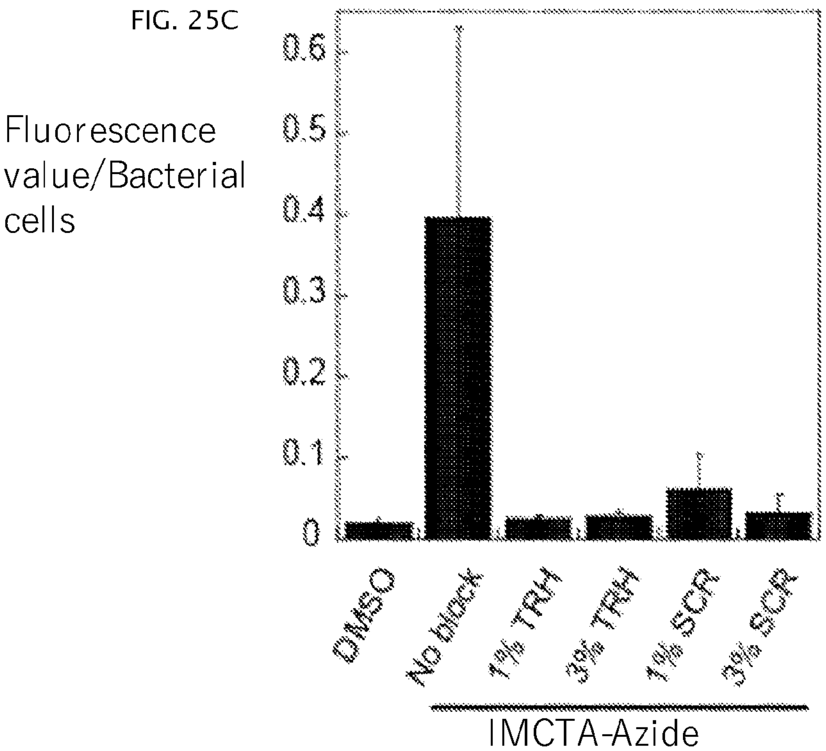

FIG. 26A

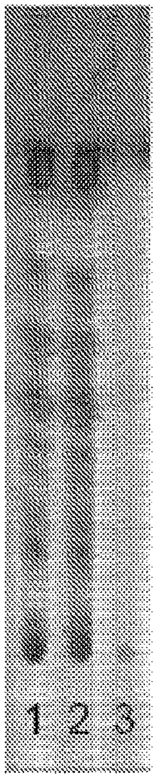


FIG. 26B

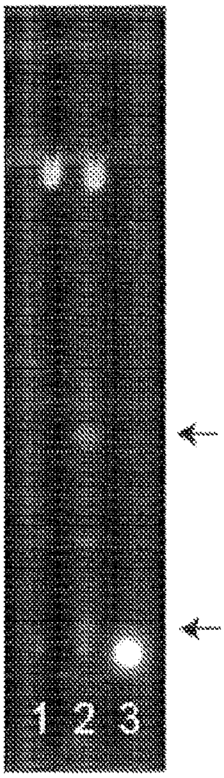

FIG. 27A

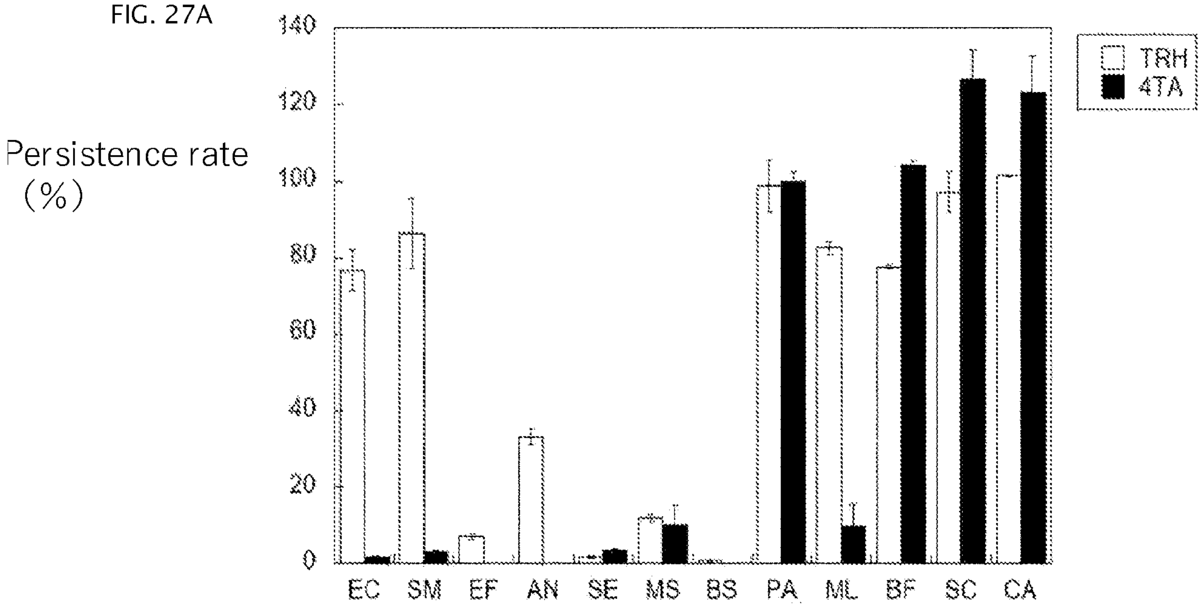


FIG. 27B

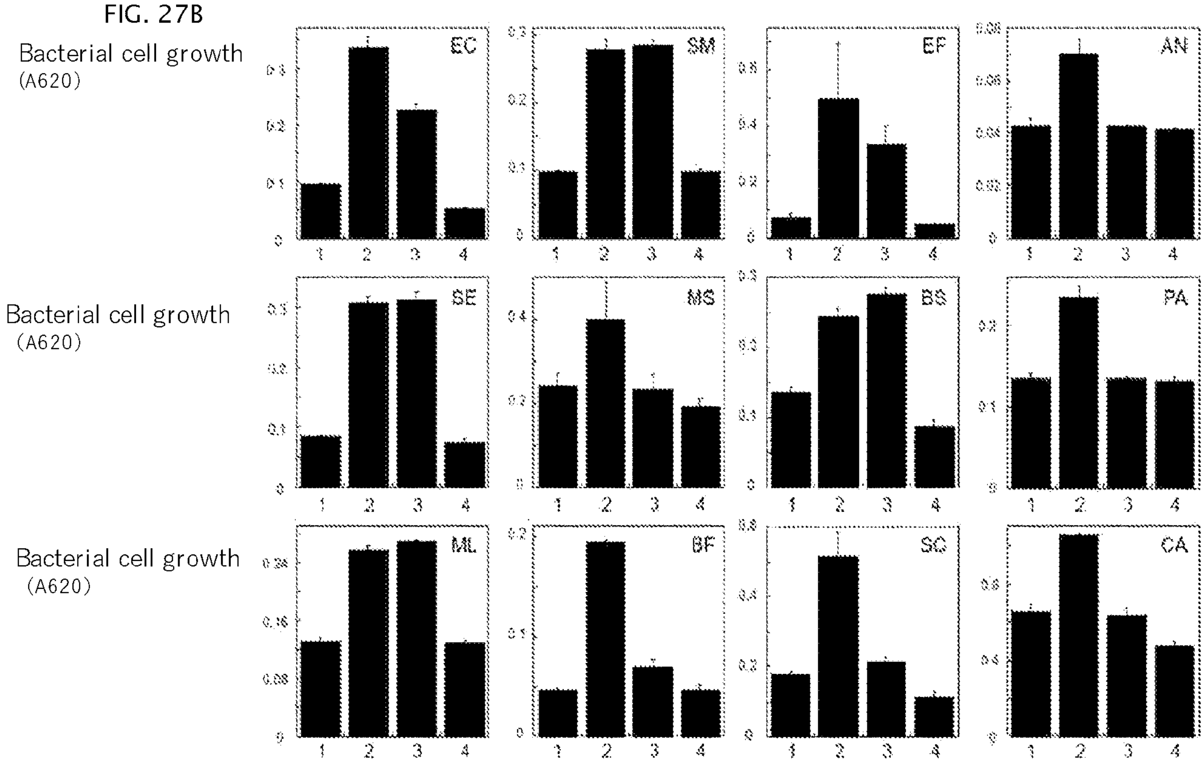

FIG. 28A

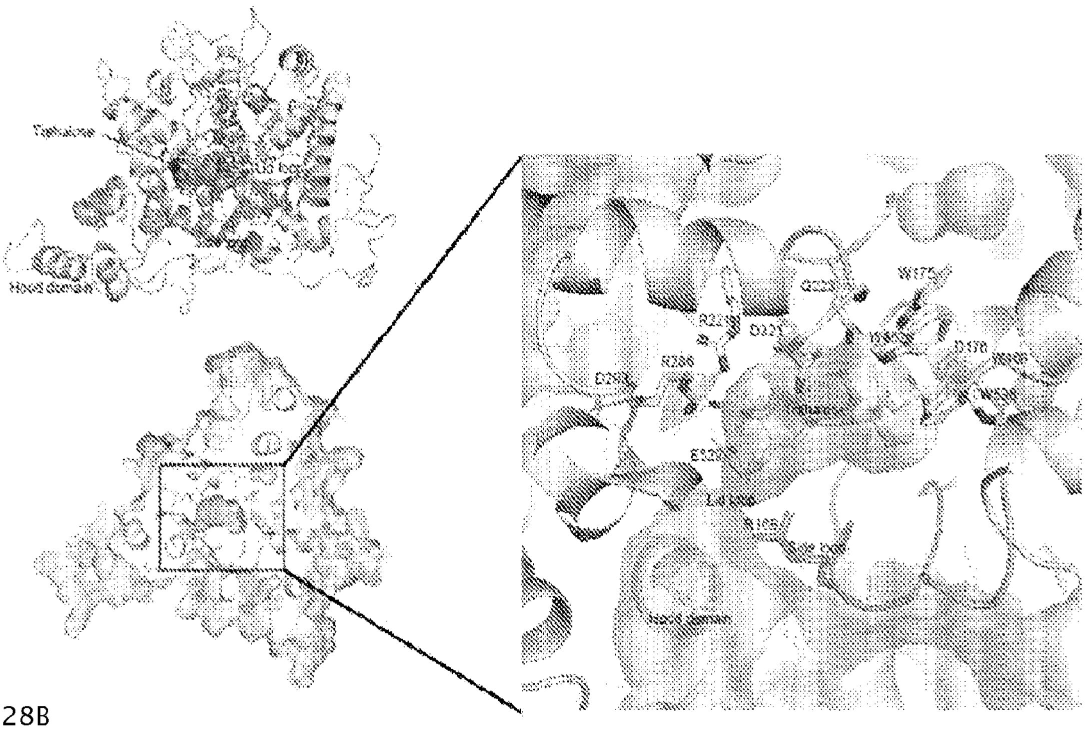


FIG. 28B

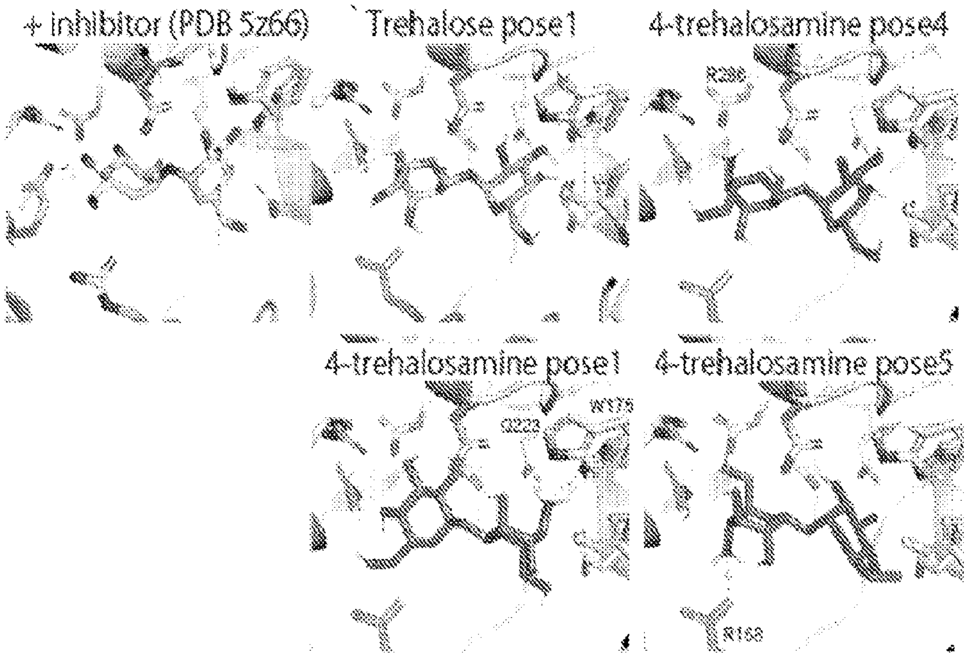

## INTERNATIONAL SEARCH REPORT

International application No. **PCT/JP2022/031876**

### A. CLASSIFICATION OF SUBJECT MATTER

***C12P 19/12***(2006.01)i; ***A61K 31/7008***(2006.01)i; ***A61K 31/7048***(2006.01)i; ***A61K 31/7056***(2006.01)i; ***A61P 3/10***(2006.01)i; ***A61P 43/00***(2006.01)i; ***C07H 5/06***(2006.01)i; ***C07H 15/12***(2006.01)i; ***C07H 15/26***(2006.01)i; ***C12N 1/20***(2006.01)i; ***C12P 19/44***(2006.01)i

FI: C12P19/12; C12P19/44; C12N1/20 E; A61K31/7008; A61K31/7048; A61K31/7056; C07H15/12; C07H15/26; A61P3/10; A61P43/00 105; C07H5/06 CSP

According to International Patent Classification (IPC) or to both national classification and IPC

### B. FIELDS SEARCHED

Minimum documentation searched (classification system followed by classification symbols)

C12P19/12; A61K31/7008; A61K31/7048; A61K31/7056; A61P3/10; A61P43/00; C07H5/06; C07H15/12; C07H15/26; C12N1/20; C12P19/44

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

### C. DOCUMENTS CONSIDERED TO BE RELEVANT

| Category* | Citation of document, with indication, where appropriate, of the relevant passages | Relevant to claim No. |
|---|---|---|
| X | WO 2021/060440 A1 (NOF CORP) 01 April 2021 (2021-04-01)<br>claims, paragraph [0164] | 1 |
| X | RODRIGUEZ-RIVERA, Frances P. et al. Visualization of mycobacterial membrane dynamics in live cells. Journal of the American Chemical Society. 2017, vol. 139, pp. 3488-3495, doi:10.1021/jacs.6b12541<br>RESULTS AND DISCUSSION, fig. 2 | 2-3, 6 |
| X | NAGANAWA, Hiroshi et al. 4-AMINO-4-DEOXY-α,α-TREHALOSE, A NEW METABOLITE OF A STREPTOMYCES. The Journal of Antibiotics. 1974, vol. 27, no. 2, pp. 145-146<br>p. 145, left column | 4-5, 7 |
| A | WO 2016/052656 A1 (MICROBIAL CHEM RES FOUND) 07 April 2016 (2016-04-07)<br>entire text | 1 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **02 November 2022** | **15 November 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**

International application No.
**PCT/JP2022/031876**

**C. DOCUMENTS CONSIDERED TO BE RELEVANT**

| Category* | Citation of document, with indication, where appropriate, of the relevant passages | Relevant to claim No. |
|---|---|---|
| A | JP 9-70528 A (AJINOMOTO CO INC) 18 March 1997 (1997-03-18) paragraph [0003] | 1 |
| P, X | WADA, Shunichi et al. Rediscovery of 4-Trehalosamine as a Biologically Stable, Mass-Producible, and Chemically Modifiable Trehalose Analog. Advanced Biology. 17 March 2022, vol. 6, 2101309, doi:10.1002/adbi.202101309 whole document | 1–7 |

Form PCT/ISA/210 (second sheet) (January 2015)

**INTERNATIONAL SEARCH REPORT**

International application No.
**PCT/JP2022/031876**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   - a. ☑ forming part of the international application as filed:
     - ☑ in the form of an Annex C/ST.25 text file.
     - ☐ on paper or in the form of an image file.
   - b. ☐ furnished together with the international application under PCT Rule 13*ter*.1(a) for the purposes of international search only in the form of an Annex C/ST.25 text file.
   - c. ☐ furnished subsequent to the international filing date for the purposes of international search only:
     - ☐ in the form of an Annex C/ST.25 text file (Rule 13*ter*.1(a)).
     - ☐ on paper or in the form of an image file (Rule 13*ter*.1(b) and Administrative Instructions, Section 713).
2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.
3. Additional comments:

   The “form of an Annex C/ST.25 text file” is replaced with the “form of ST.26.”

Form PCT/ISA/210 (continuation of first sheet) (January 2015)

**INTERNATIONAL SEARCH REPORT**
**Information on patent family members**

International application No.
**PCT/JP2022/031876**

| Patent document cited in search report | Publication date (day/month/year) | Patent family member(s) | Publication date (day/month/year) |
|---|---|---|---|
| WO 2021/060440 A1 | 01 April 2021 | EP 4035686 A1<br>claims, paragraph [0125]<br>CA 3156033 A | |
| WO 2016/052656 A1 | 07 April 2016 | (Family: none) | |
| JP 9-70528 A | 18 March 1997 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP H5276882 A
- JP 2001139599 A
- JP 2002101869 A
- US 5731419 A
- JP S538792 B
- JP S61265085 A
- JP 2021144962 A


### Non-patent literature cited in the description


- *THE JOURNAL OF ANTIBIOTICS,* 1974, vol. XXVII (2), 145
- *Protein Science,* January 2011, vol. 20 (1), 42-50
- **ANA S. CARVALHO et al.** Relevant factors for the preparation of freeze-dried lactic acid bacteria. *International Dairy Journal,* 2004, vol. 14 (10), 835-847
- **K. KAINUMA et al.** *Journal of the Japanese Society of Starch Science,* 1981, vol. 28, 235
- **J. D. DIGNAM et al.** *Nucleic Acids Res,* 1983, vol. 11 (5), 1475-1489
- **M. KAI et al.** *FEBS Lett,* 2007, vol. 581, 3345-3350
- **A. WATERHOUSE et al.** *Nucleic Acids Res,* 2018, vol. 46, W296
- **N. GUEX et al.** *Electrophoresis,* 2009, vol. 30 (1), S162
- **S. BIENERT et al.** *Nucleic Acids Res,* 2017, vol. 45, D313
- **A. ADHAV et al.** *FEBS J,* 2019, vol. 286, 1700
- **G. STUDER et al.** *Bioinformatics,* 2020, vol. 36, 1765
- **M. BERTONI et al.** *Sci Rep,* 2017, vol. 7, 10480
- **C. CAMACHO et al.** *BMC Bioinformatics,* 2009, vol. 10, 421
- **M. STEINEGGER et al.** *BMC Bioinformatics,* 2019, vol. 20, 473
- **M. MIRDITA et al.** *Nucleic Acids Res,* 2017, vol. 45, D170
- **O. TROTT et al.** *J Comput Chem,* 2010, vol. 31, 455
- **E. F. PETTERSEN et al.** *J Comput Chem,* 2004, vol. 25, 1605